(19) Europäisches Patentamt
European Patent Office
Office européen des brevets

(11) EP 4 389 750 A1

(12) **EUROPEAN PATENT APPLICATION**
published in accordance with Art. 153(4) EPC

(43) Date of publication:
**26.06.2024 Bulletin 2024/26**

(21) Application number: **22858533.7**

(22) Date of filing: **19.08.2022**

(51) International Patent Classification (IPC):
***C07D 487/04*** *(2006.01)* ***A61K 31/53*** *(2006.01)*
***A61P 31/16*** *(2006.01)*

(52) Cooperative Patent Classification (CPC):
**A61K 31/53; A61P 31/16; C07D 487/04**

(86) International application number:
**PCT/JP2022/031308**

(87) International publication number:
**WO 2023/022216 (23.02.2023 Gazette 2023/08)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **20.08.2021 JP 2021134801**

(71) Applicant: **Shionogi & Co., Ltd.**
**Osaka-shi, Osaka 541-0045 (JP)**

(72) Inventors:
- **KATO, Issei**
**Osaka-shi, Osaka 541-0045 (JP)**
- **KANEDA, Masato**
**Osaka-shi, Osaka 541-0045 (JP)**
- **MIYAGAWA, Masayoshi**
**Osaka-shi, Osaka 541-0045 (JP)**
- **YAMAMOTO, Ryota**
**Osaka-shi, Osaka 541-0045 (JP)**

(74) Representative: **Vossius & Partner**
**Patentanwälte Rechtsanwälte mbB**
**Siebertstrasse 3**
**81675 München (DE)**

(54) **NUCLEOSIDE DERIVATIVES AND PRODRUGS THEREOF HAVING VIRAL GROWTH INHIBITORY ACTION**

(57) The present invention provides a compound represented by the following formula (I):

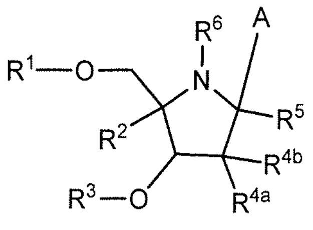

(I)

wherein A is a base or the like, $R^1$ is hydrogen or the like, $R^2$ is hydrogen or the like, $R^3$ is hydrogen or the like, $R^{4a}$ is hydrogen or the like, $R^{4b}$ is hydrogen or the like, $R^5$ is hydrogen or the like, and $R^6$ is hydrogen or the like.

EP 4 389 750 A1

## Description

[TECHNICAL FIELD]

The present invention relates to azasugar- or sugar-containing nucleoside derivatives, prodrugs thereof, pharmaceutically acceptable salts thereof, and pharmaceutical compositions containing the same, which are suitable for use in inhibiting viral RNA polymerase activity or viral growth, and treating viral infection.

[BACKGROUND ART]

Viruses are responsible for many infectious diseases in animals, including mammals and especially humans. Unlike infections with bacteria, relatively few agents are effective for the prevention and treatment of viral infections. The biology of viral diseases, including viral genome transcription, translation and replication, is now well understood. RNA-dependent RNA polymerase is an enzyme essential for viral genome replication that all RNA viruses encode. This enzyme catalyzes synthesis of an RNA strand complementary to a given RNA template. Since virus replication depends on RNA polymerase, this enzyme is a promising target in the development of new anti-viral compounds.

Although not limited to the viruses described below, for example, RNA-dependent RNA polymerase (hereinafter, it is referred to as RdRp), which is an enzyme derived from an influenza virus, is an enzyme essential for replication of a viral genome, and is an amino acid sequence that is highly conserved among types and subtypes of influenza virus. Therefore, it is considered that the RNA-dependent RNA polymerase is suitable as a target of an anti-influenza drug that exhibits effects on a wide range of types and subtypes. RdRp of an influenza virus is a protein complex having an active center in a PB1 region. RdRp synthesizes positive-strand complementary RNA, which is an intermediate product for transcription into mRNA and replication of progeny vRNA, using negative (-) single-stranded genomic RNA (vRNA) as a template. That is, it is considered that a substance that inhibits RdRp inhibits growth of virus as a result of inhibiting synthesis of viral protein by inhibiting replication of viral genome.

As a compound that inhibits RNA-dependent RNA polymerase, Favipiravir (Non-Patent Documents 1 and 2) is clinically approved, and there are safety problems such as teratogenic risk, and the use thereof (new or re-emerging influenza virus infection (limited to those to which other anti-influenza viral drugs are ineffective or insufficiently effective) is limited. In addition, N-hydroxycytidine (Non Patent Documents 3 and 4), compounds described in recent years in Patent Documents, and the like, have been reported, but have not yet been clinically used as an anti-influenza drug.

In addition, RNA-dependent RNA polymerase (hereinafter, it is referred to as RdRp), which is an enzyme derived from SARS-CoV-2, is an enzyme essential for replication of a viral genome, and is an amino acid sequence that is highly conserved among viruses. Therefore, it is considered that the RNA-dependent RNA polymerase is suitable as a target of therapeutic agents against conventional and various new mutant viruses. RdRp is a complex enzyme composed of three types of nsp proteins (nsp7, nsp8, nsp12) encoded by the virus. RdRp synthesizes (-) strand RNA from a (+) strand viral genome, and further replicates (+) strand genomic RNA using the (-) strand as a template. That is, it is considered that a substance that inhibits RdRp inhibits growth of virus as a result of inhibiting synthesis of viral protein by inhibiting replication of viral genome.

As a compound that inhibits RNA-dependent RNA polymerase derived from SARS-CoV-2, Remdesivir (Non-Patent Documents 5 and 6) is clinically approved. However, it is an intravenously administered agent, and the use of the same is limited (to adult and adolescent COVID-19 patients who require supplemental oxygen and have pneumonia (aged 12 years or older and weighing 40 kg or more)). Other examples such as Favipiravir (Non-Patent Documents 7 and 8), N-hydroxycytidine (Non-Patent Documents 9 to 11), AT-527 (Non-Patent Document 12), and compounds described in patent documents in recent years have been reported, but have not yet been clinically used as anti-SARS-CoV-2 drugs.

In addition to these, Patent Documents 1 to 34 describe azasugar- or sugar-containing nucleoside derivatives.

[PRIOR ART REFERENCES]

[Patent Document]

[Patent Document 1] WO 1999/019338A Pamphlet
[Patent Document 2] Specification of US Patent No. 6066722
[Patent Document 3] WO 2002/018371A Pamphlet
[Patent Document 4] WO 2003/080620A Pamphlet
[Patent Document 5] WO 2006/002231A Pamphlet

[Patent Document 6] WO 2007/069924A Pamphlet
[Patent Document 7] WO 2012/074912A Pamphlet
[Patent Document 8] WO 2013/158746A Pamphlet
[Patent Document 9] WO 2014/078778A Pamphlet
[Patent Document 10] WO 2014/186465A Pamphlet
[Patent Document 11] WO 2018/199048A Pamphlet
[Patent Document 12] WO 2018/230479A Pamphlet
[Patent Document 13] WO 2019/140365A Pamphlet
[Patent Document 14] WO 2004/096286A
[Patent Document 15] WO 2016/069825A
[Patent Document 16] WO 2016/069826A
[Patent Document 17] WO 2016/069827A
[Patent Document 18] Chinese Patent Application Publication CN 112010916A
[Patent Document 191 Chinese Patent Application Publication CN 112062800A
[Patent Document 20] WO 2008/141079A Pamphlet
[Patent Document 21] WO 2008/089105A Pamphlet
[Patent Document 22] WO 2021/040356A Pamphlet
[Patent Document 23] WO 2014/035140A Pamphlet
[Patent Document 24] WO 2010/002877 Pamphlet
[Patent Document 25] WO 2012/037038 Pamphlet
[Patent Document 26] WO 2016/069975 Pamphlet
[Patent Document 27] Chinese Patent Application Publication CN 112778310A
[Patent Document 28] WO 2015/069939 Pamphlet
[Patent Document 29] WO 2015/148746 Pamphlet
[Patent Document 30] WO 2014/093924 Pamphlet
[Patent Document 31] WO 2003/093290 Pamphlet
[Patent Document 32] WO 2005/123087 Pamphlet
[Patent Document 33] WO 2006/050161A Pamphlet
[Patent Document 34] Specification of EP 71227

[Non-patent Documents]

[Non-patent Document 1] American Society for Microbiology Antimicrobial Agents and Chemotherapy Volume 46, Issue 4, April 2002, 977-981
[Non-patent Document 2] Antiviral Chemistry & Chemotherapy 14, 235-241
[Non-patent Document 3] American Society for Microbiology Antimicrobial Agents and Chemotherapy Volume 62, Issue 8, August 2018
[Non-patent Document 4] Toots et al., Sci. Transl. Med. 11, eaax5866 (2019) 23 October 2019
[Non-patent Document 5] n engl j med, Volume 383, No. 19, November 5, 2020, Pages 1813
[Non-patent Document 6] n engl j med, Volume 384, NO. 9, March 4, 2021, Pages 795
[Non-patent Document 7] Q. Cai et al. Engineering 6 (2020) 1192-1198
[Non-patent Document 8] NATURE COMMUNICATIONS (2021) 12, 1735, 1-13
[Non-patent Document 9] Sheahan et al., Sci. Transl. Med. 12, eabb5883 (2020), 1-15
[Non-patent Document 10] Nature Microbiology, VOL 6, January 2021, 11-18
[Non-patent Document 11] NATURE COMMUNICATIONS (2021), 12, 2295, 1-8
[Non-patent Document 12] Antimicrobial Agents and Chemotherapy, April 2021 Volume 65 Issue 4 e02479-20

[SUMMARY OF THE INVENTION]

[PROBLEMS TO BE SOLVED BY THE INVENTION]

An object of the present invention is to provide a compound suitable for use in inhibiting viral RNA polymerase activity or viral growth, and treating viral infection. Preferably, the present invention provides a compound having viral growth inhibitory action against influenza virus and/or coronavirus.
Another object of the present invention is to make a compound to be administered (for example, orally administered) to a living body a prodrug, thereby to provide a compound that is efficiently absorbed into the body after administration and exhibits a high pharmacological effect.

In addition, another object of the present invention is to provide a compound having a lower frequency of appearance of a slow-sensitivity strain, compared to existing drugs.

[MEANS FOR SOLVING THE PROBLEM]

The present invention relates to the following.

[1] A compound represented by Formula (I):

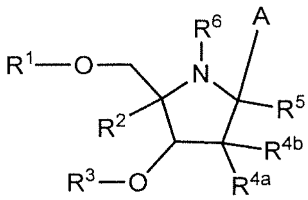

(I)

wherein

A is a group represented by any one of the following formulae:

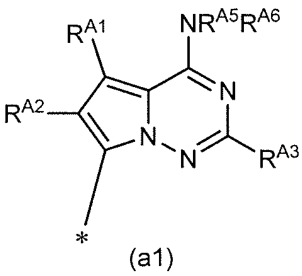

(a1)

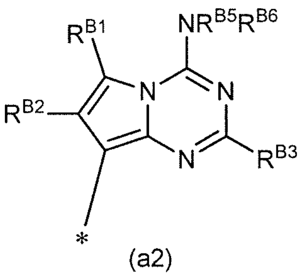

(a2)

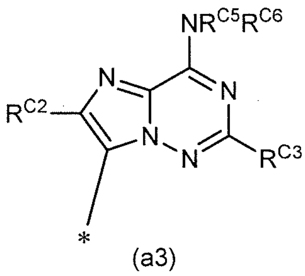

(a3)

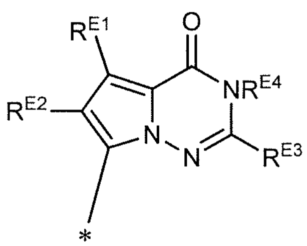

(b1)

wherein

$R^{A1}$, $R^{B1}$, and $R^{E1}$ are each independently hydrogen, halogen, hydroxy, - $B(OH)_2$, amino optionally substituted with substituent group $\gamma$, an aromatic heterocyclyl optionally substituted with substituent group $\alpha$, a non-aromatic heterocyclyl optionally substituted with substituent group $\alpha$, alkyl optionally substituted with substituent group $\beta$, or a group represented by any of the following formulae: $-C(=O)-N(R^{a1})(R^{a2})$, $-C(=NR^{a3})-NH_2$, and -C(=NOH)-H,
wherein $R^{a1}$ is hydrogen, $R^{a2}$ is hydrogen or alkyl optionally substituted with substituent group $\beta$, and $R^{a3}$ is hydrogen or hydroxy,
wherein

the substituent group $\alpha$: halogen, hydroxy, alkyl, and hydroxyalkyl,
the substituent group $\beta$: cyano and hydroxy, and
the substituent group $\gamma$: alkyl,

$R^{A2}$, $R^{B2}$, $R^{C2}$, and $R^{E2}$ are each independently hydrogen;
$R^{A3}$, $R^{B3}$, $R^{C3}$, and $R^{E3}$ are each independently hydrogen, halogen, amino, or alkyl,
$R^{E4}$ is hydrogen,
$R^{A5}$, $R^{B5}$, and $R^{C5}$ are each independently hydrogen, alkyl, or a group forming a prodrug,
$R^{A6}$, $R^{B6}$, and $R^{C6}$ are each independently hydrogen, hydroxy, alkylcarbonyl, or a group forming a prodrug,

$R^1$ is hydrogen, a group forming a prodrug, or a group selected from the group consisting of the following:

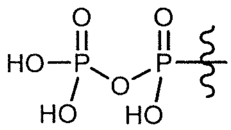 , 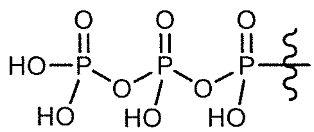

$R^2$ is hydrogen, halogen, C1-C3 alkyl, C2-C3 alkenyl, C2-C3 alkynyl, halo C1-C3 alkyl, halo C2-C3 alkenyl, or halo C2-C3 alkynyl,
$R^3$ is hydrogen or a group forming a prodrug,
$R^{4a}$ is hydrogen, hydroxy, or halogen,
$R^{4b}$ is hydrogen, halogen, C1-C3 alkyl, C1-C3 haloalkyl, C2-C3 alkenyl, or C2-C3 alkynyl,
$R^5$ is hydrogen, C1-C3 alkyl, C2-C3 alkenyl, or C2-C3 alkynyl, and
$R^6$ is hydrogen, C1-C3 alkyl, or a group forming a prodrug,
provided that the following compound is excluded:

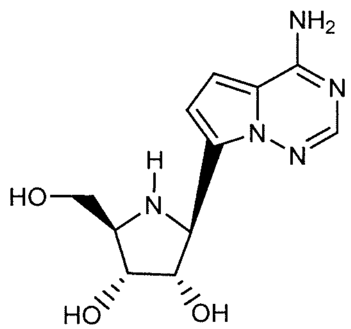

or a pharmaceutically acceptable salt thereof.

[2] The compound according to [1], represented by Formula:

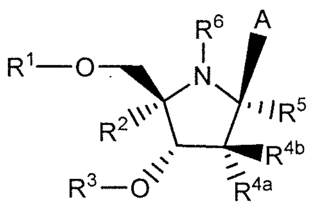 (I-1)

wherein

A is a group represented by either one of the following formulae:

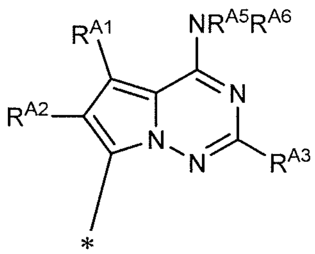
(a1)

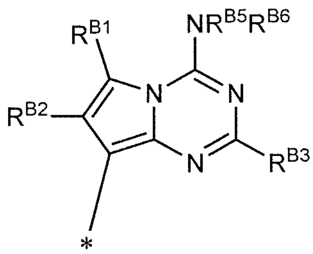
(a2)

wherein

$R^{A1}$ and $R^{B1}$ are each independently hydrogen, halogen, hydroxy, - $B(OH)_2$, amino, an aromatic heterocyclyl optionally substituted with substituent group $\alpha$, a non-aromatic heterocyclyl optionally substituted with substituent group α, alkyl optionally substituted with substituent group β, or a group represented by any of the following formulae: $-C(=O)-N(R^{a1})(R^{a2})$, $-C(=NR^{a3})-NH_2$, and -C(=NOH)-H,
wherein $R^{a1}$ is hydrogen, $R^{a2}$ is hydrogen or alkyl optionally substituted with substituent group β, and $R^{a3}$ is hydrogen or hydroxy,
wherein

the substituent group α: halogen, hydroxy, alkyl, and hydroxyalkyl, and
the substituent group β: cyano and hydroxy,

$R^{A2}$ and $R^{B2}$ are hydrogen,
$R^{A3}$ and $R^{B3}$ are each independently hydrogen, halogen, or alkyl,
$R^{A5}$ and $R^{B5}$ are each independently hydrogen, or a group forming a prodrug, and
$R^{A6}$ and $R^{B6}$ are each independently hydrogen, hydroxy, alkylcarbonyl, or a group forming a prodrug,

$R^1$ is hydrogen, a group forming a prodrug, or a group selected from the group consisting of the following:

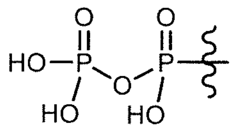 , 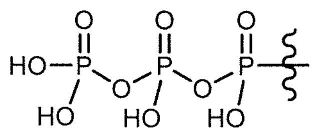

$R^2$ is hydrogen,
$R^3$ is hydrogen or a group forming a prodrug,
$R^{4a}$ is hydroxy,
$R^{4b}$ is hydrogen,
$R^5$ is hydrogen, and
$R^6$ is hydrogen, C1-C3 alkyl, or a group forming a prodrug,

or a pharmaceutically acceptable salt thereof.
[3] The compound according to [2],
wherein

$R^{A6}$ and $R^{B6}$ are each independently hydrogen, hydroxy, or alkylcarbonyl,
$R^3$ is hydrogen, and
$R^6$ is hydrogen, or C1-C3 alkyl,

or a pharmaceutically acceptable salt thereof.
[4] The compound according to any one of [1] to [3],

wherein A is a group represented by either one of the following formulae:

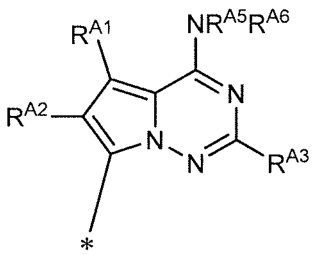
(a1)

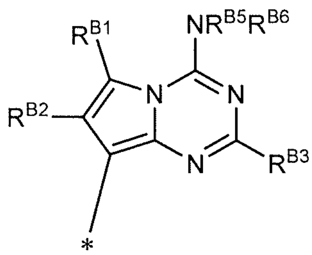
(a2)

wherein
$R^{A1}$ and $R^{B1}$ are each independently hydroxy, amino, an aromatic heterocyclyl optionally substituted with substituent group α, or a group represented by any of the following formulae: $-C(=O)-N(R^{a1})(R^{a2})$, and $-C(=NR^{a3})-NH_2$,

wherein $R^{a1}$ is hydrogen, $R^{a2}$ is hydrogen or cyanomethyl, and $R^{a3}$ is hydrogen or hydroxy, wherein the substituent group $\alpha$: fluorine and C1-C3 alkyl, and

$R^{A3}$ and $R^{B3}$ are each independently hydrogen or fluorine,

or a pharmaceutically acceptable salt thereof.

[5] The compound according to any one of [1] to [4], wherein $R^{A1}$ and $R^{B1}$ are hydroxy, a 5-membered aromatic heterocyclyl, or a group represented by either one of the following formulae: -C(=O)-$NH_2$, and -C(=NOH)-$NH_2$, or a pharmaceutically acceptable salt thereof.

[6] The compound according to any one of [1] to [3],

wherein A is a group represented by either one of the following formulae:

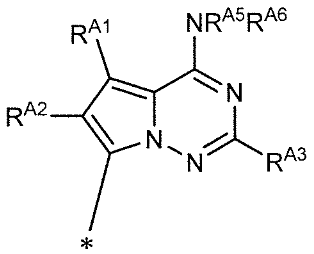
(a1)

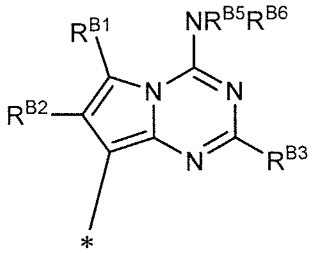
(a2)

wherein
$R^{A1}$ and $R^{B1}$ are each independently hydrogen, fluorine, hydroxy, -$B(OH)_2$, amino, an aromatic heterocyclyl optionally substituted with substituent group $\alpha$, cyanomethyl, C1-C3 alkyl substituted with hydroxy, or a group represented by the following formula: -C(=NOH)-H,
wherein the substituent group $\alpha$: C1-C3 alkyl, and
$R^{A3}$ and $R^{B3}$ are each independently hydrogen, fluorine, or methyl,

or a pharmaceutically acceptable salt thereof.

[7] The compound according to any one of [1], [2], [3], and [6], wherein $R^{A1}$ and $R^{B1}$ are fluorine, a 5- or 6-membered aromatic heterocyclyl, cyanomethyl, or a group represented by the following formula: -C(=NOH)-H, or a pharmaceutically acceptable salt thereof.

[8] A compound selected from the group consisting of those represented by the following formulae:

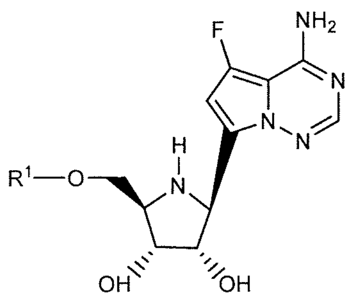

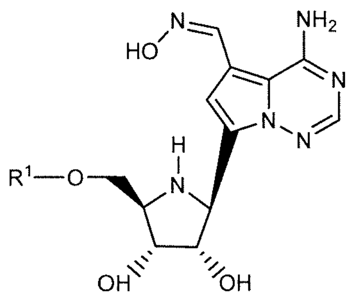

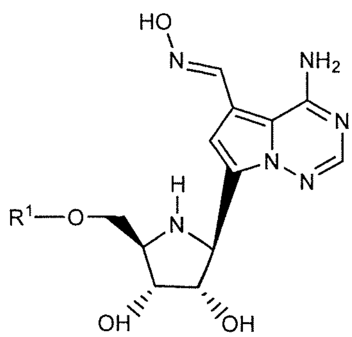

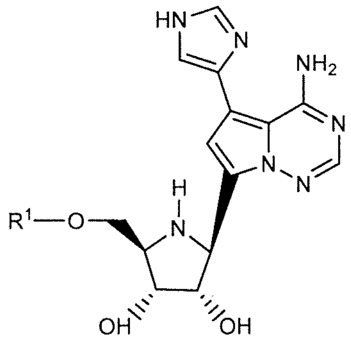

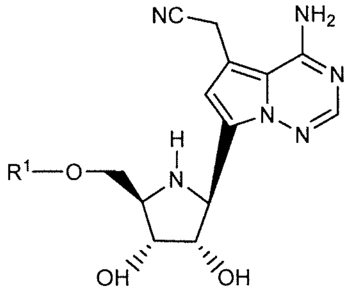

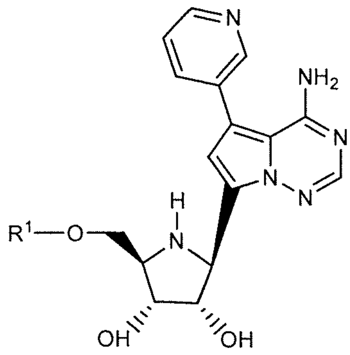

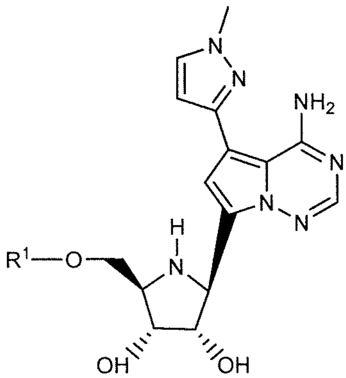

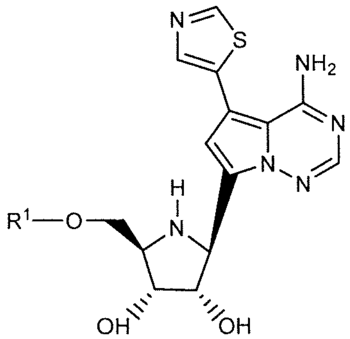

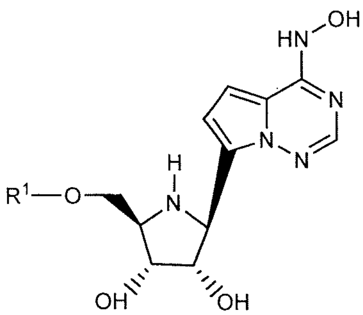

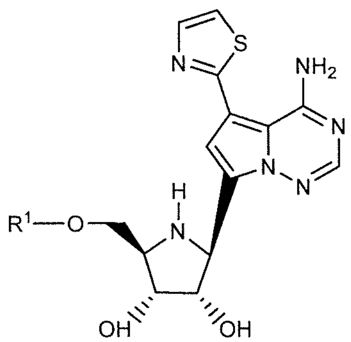

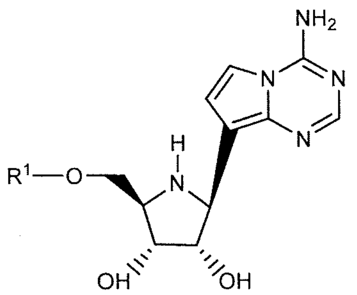

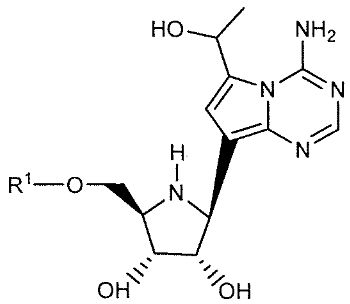

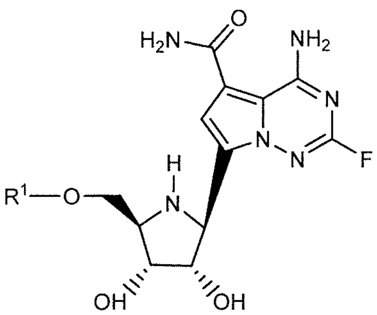

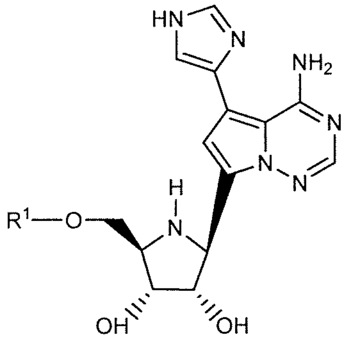

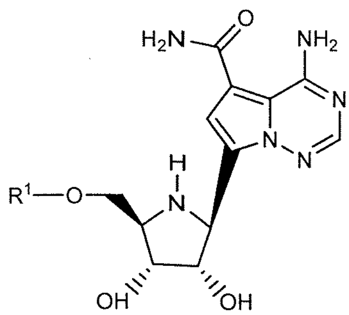

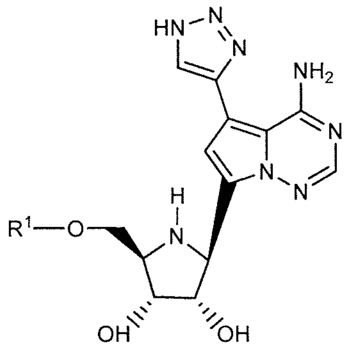
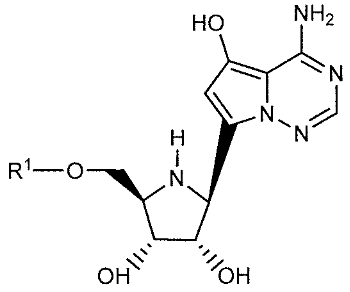
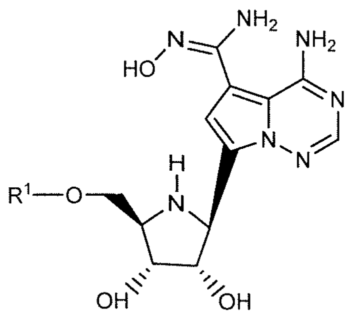
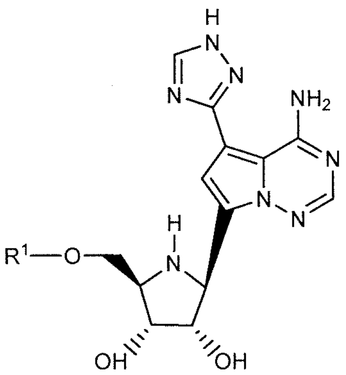
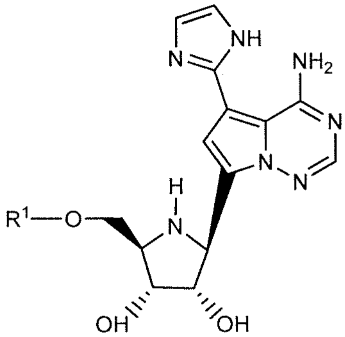
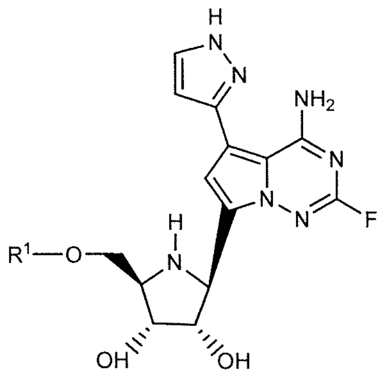
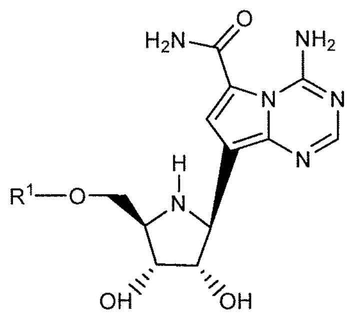
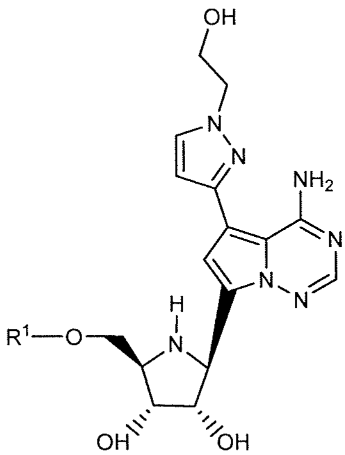
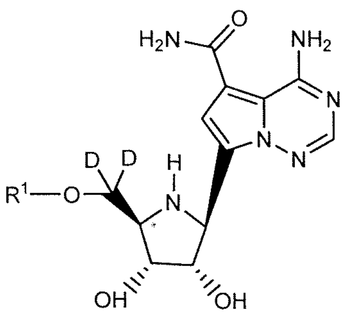

wherein $R^1$ is hydrogen, a group forming a prodrug, or a group selected from the group consisting of the following:

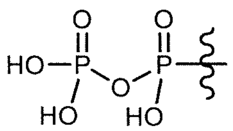
,
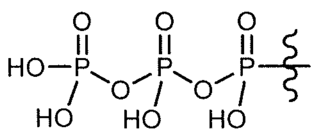

or a pharmaceutically acceptable salt thereof.

[9] A compound represented by Formula (II):

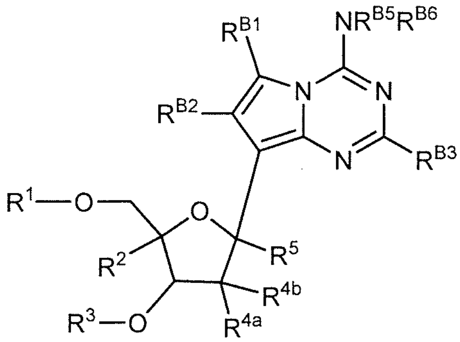

(II)

wherein

$R^{B1}$ is hydrogen, halogen, hydroxy, $-B(OH)_2$, amino optionally substituted with substituent group $\gamma$, an aromatic heterocyclyl optionally substituted with substituent group $\alpha$, alkyl optionally substituted with substituent group $\beta$, or a group represented by any one of the following formulae: $-C(=O)-N(R^{a1})(R^{a2})$, $-C(=NR^{a3})-NH_2$, and -C(=NOH)-H,
wherein
$R^{a1}$ is hydrogen, $R^{a2}$ is hydrogen or alkyl optionally substituted with substituent group $\beta$, and $R^{a3}$ is hydrogen or hydroxy,
wherein

the substituent group $\alpha$: halogen and alkyl,
the substituent group $\beta$: cyano and hydroxy, and
the substituent group $\gamma$: alkyl,

$R^{B2}$ is hydrogen,
$R^{B3}$ is hydrogen, halogen, amino, or alkyl,
$R^{B5}$ is hydrogen, alkyl, or a group forming a prodrug,
$R^{B6}$ is hydrogen, hydroxy, alkylcarbonyl, or a group forming a prodrug,
$R^1$ is hydrogen, a group forming a prodrug, or a group selected from the group consisting of the following:

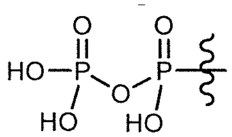
,
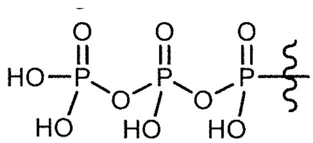

$R^2$ is hydrogen, halogen, C1-C3 alkyl, C2-C3 alkenyl, C2-C3 alkynyl, halo C1-C3 alkyl, halo C2-C3 alkenyl, or halo C2-C3 alkynyl,
$R^3$ is hydrogen or a group forming a prodrug,
$R^{4a}$ is hydrogen, hydroxy, or halogen,
$R^{4b}$ is hydrogen, halogen, C1-C3 alkyl, C1-C3 haloalkyl, C2-C3 alkenyl, or C2-C3 alkynyl, and
$R^5$ is hydrogen, or C1-C3 alkyl,

or a pharmaceutically acceptable salt thereof.
[10] A compound represented by Formula (II-a2-1):

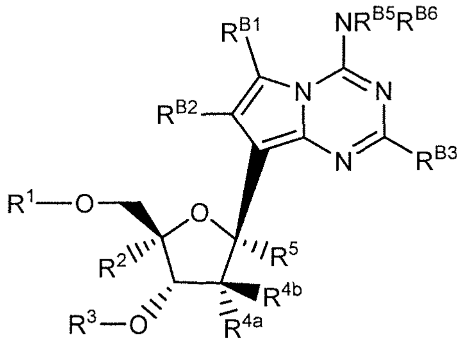

(II-a2-1)

wherein

$R^{B1}$ is hydrogen, halogen, hydroxy, $-B(OH)_2$, amino, an aromatic heterocyclyl optionally substituted with substituent group $\alpha$, alkyl optionally substituted with substituent group $\beta$, or a group represented by any one of the following formulae: - $C(=O)-N(R^{a1})(R^{a2})$, $-C(=NR^{a3})-NH_2$, and -C(=NOH)-H,
wherein
$R^{a1}$ is hydrogen, $R^{a2}$ is hydrogen or alkyl optionally substituted with substituent group $\beta$, and $R^{a3}$ is hydrogen or hydroxy,
wherein

the substituent group $\alpha$: halogen and alkyl, and
the substituent group $\beta$: cyano and hydroxy,

$R^{B2}$ is hydrogen,
$R^{B3}$ is hydrogen, halogen, or alkyl,
$R^{B5}$ is hydrogen or a group forming a prodrug,
$R^{B6}$ is hydrogen, hydroxy, alkylcarbonyl, or a group forming a prodrug,
$R^1$ is hydrogen, a group forming a prodrug, or a group selected from the group consisting of the following:

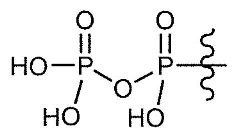 , 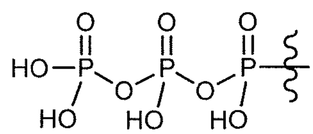

$R^2$ is hydrogen or halogen,
$R^3$ is hydrogen or a group forming a prodrug,
$R^{4a}$ is hydroxy or halogen,
$R^{4b}$ is hydrogen or halogen, and
$R^5$ is hydrogen or cyano,

or a pharmaceutically acceptable salt thereof.
[11] A compound selected from the group consisting of those represented by the following formulae:

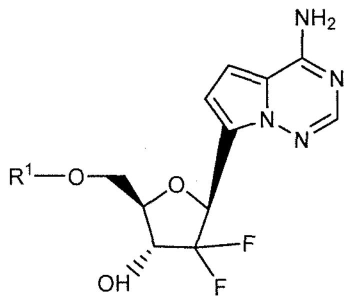

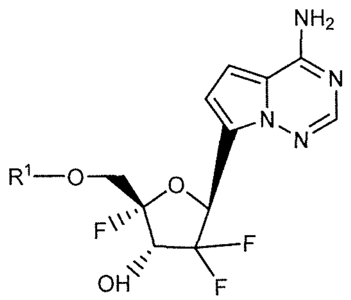

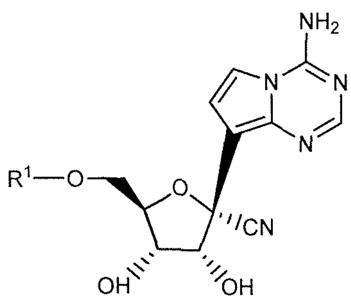

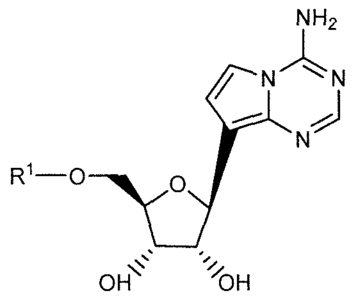
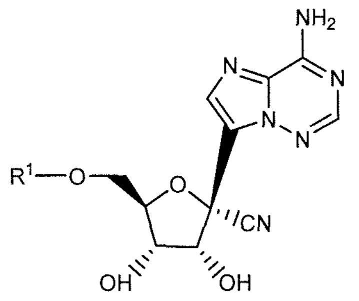
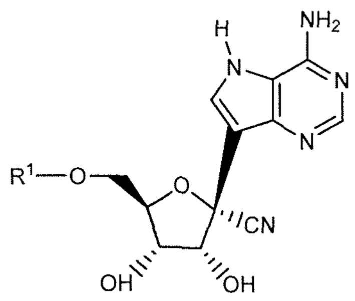

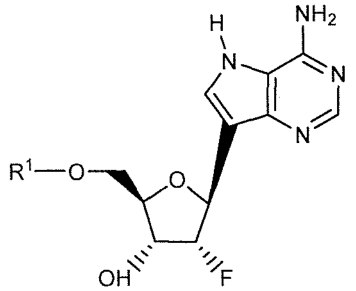

wherein $R^1$ is hydrogen, a group forming a prodrug, or a group selected from the group consisting of the following:

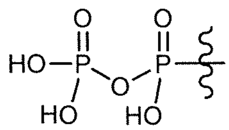
,
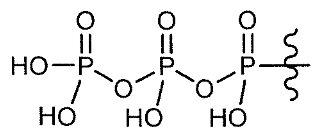

or a pharmaceutically acceptable salt thereof.

[12] The compound according to any one of [1] to [11],

wherein the groups forming the prodrugs of $R^1$ and $R^3$ are groups selected from those represented by the following formulae:

a) $-C(=O)-P^{R0}$;
b) $-C(=O)-L-P^{R1}$;
c) $-C(=O)-L-O-P^{R0}$;
d) $-C(=O)-L-S-P^{R0}$;
e) $-C(=O)-L-N(-P^{R2})_2$;
f) $-C(=O)-C(P^{R3})_2-NH_2$;
g) $-C(=O)-O-P^{R0}$;
h) $-C(P^{R4})_2-O-C(=O)-P^{R5}$;
i) $-C(P^{R4})_2-O-C(=O)-L-P^{R6}$;
j) $-C(P^{R4})_2-O-C(=O)-O-P^{R5}$;
k) $-C(P^{R4})_2-O-C(=O)-O-L-P^{R6}$;
l) $-P(=O)(-OP^{R7})_2$;
m) $-P(=O)(-OP^{R7})-N(-P^{R8})_2$; and
n) $-P(=O)(-N(-P^{R8})_2)_2$,

the groups forming the prodrugs of $R^{A5}$, $R^{B5}$, $R^{C5}$, $R^{A6}$, $R^{B6}$, $R^{C6}$, and $R^6$ are groups selected from those represented by the following formulae:

a) $-C(=O)-P^{R0}$;
g) $-C(=O)-O-P^{R0}$;
o) $-C(=O)-O-L-P^{R1}$;
p) $-C(=O)-L-O-C(=O)-P^{R0}$; and

q) $-C(P^{R4})_2-P^{R1}$, or

the groups forming prodrugs of $R^1$ and $R^3$ may be taken together to form a group represented by the following formula: $-P(=O)(-OP^{R7})$, or alternatively,
the groups forming prodrugs of $R^1$ and $R^6$ may be taken together to form a group represented by the following formula: $-P(=O)(-OP^{R7})$, or $-P(=O)(-N(-P^{R8})_2)$,
wherein L's are each independently a linear or branched alkylene,
$P^{R0}$s are each independently alkyl optionally substituted with substituent group A, a carbocyclyl optionally substituted with substituent group B, or a heterocyclyl optionally substituted with substituent group B,
$P^{R1}$s are each independently a carbocyclyl optionally substituted with substituent group B, or a heterocyclyl optionally substituted with substituent group B,
$P^{R2}$s are each independently alkyl optionally substituted with substituent group A, a carbocyclyl optionally substituted with substituent group B, or a heterocyclyl optionally substituted with substituent group B, or
two $P^{R2}$s may be taken together with an adjacent atom to form a heterocycle optionally substituted with substituent group E,
$P^{R3}$s are each independently hydrogen or alkyl optionally substituted with substituent group C,
$P^{R4}$s are each independently hydrogen or alkyl,
$P^{R5}$s are each independently alkyl optionally substituted with substituent group A, a carbocyclyl optionally substituted with substituent group B, or a heterocyclyl optionally substituted with substituent group B,
$P^{R6}$s are each independently a carbocyclyl optionally substituted with substituent group B, or a heterocyclyl optionally substituted with substituent group B,
$P^{R7}$s are each independently hydrogen, alkyl optionally substituted with substituent group D, phenyl optionally substituted with substituent group E, or naphthyl optionally substituted with substituent group E, or
two $P^{R7}$s may be taken together with adjacent atoms to form a heterocycle optionally substituted with substituent group E, and
$P^{R8}$s are each independently hydrogen or alkyl optionally substituted with substituent group F,
wherein the substituent group A: halogen,
the substituent group B: hydroxy, alkyl, and oxo,
the substituent group C: a carbocyclyl optionally substituted with substituent group G, a heterocyclyl optionally substituted with substituent group G, and alkylthio,
the substituent group D: a carbocyclyl optionally substituted with substituent group G, a heterocyclyl optionally substituted with substituent group G, a carbocyclylalkyloxy optionally substituted with substituent group G, alkyloxy, alkyloxycarbonyl, alkylcarbonyloxy, alkylthio, and alkylcarbonylthio,
the substituent group E: halogen, alkyl, and alkyloxy,
the substituent group F: a carbocyclyl, a heterocyclyl, halogen, and alkyloxycarbonyl, and
the substituent group G: halogen and cyano, or
a pharmaceutically acceptable salt thereof.

[13] The compound according to any one of [1] to [11],

wherein $R^3$, $R^6$, $R^{A5}$, $R^{B5}$, $R^{C5}$, $R^{A6}$, $R^{B6}$, and $R^{C6}$ are groups other than the group forming a prodrug, and $R^1$ is hydrogen or a group selected from those represented by the following formulae:

a) $-C(=O)-P^{R0}$;
b) $-C(=O)-L-P^{R1}$;
f) $-C(=O)-C(P^{R3})_2-NH_2$;
g) $-C(=O)-O-P^{R0}$;
i) $-C(P^{R4})_2-O-C(=O)-L-P^{R6}$;
l) $-P(=O)(-OP^{R7})_2$; and
m) $-P(=O)(-OP^{R7})-N(-P^{R8})_2$,

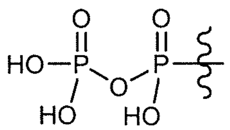 , 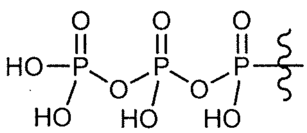

wherein

L's are each independently a linear or branched alkylene,
$P^{R0}$s are each independently alkyl, a carbocyclyl optionally substituted with substituent group B, or a heterocyclyl optionally substituted with substituent group B,
$P^{R1}$s are each independently a carbocyclyl optionally substituted with substituent group B, or a heterocyclyl optionally substituted with substituent group B,
$P^{R4}$s are each independently hydrogen or alkyl,
$P^{R6}$s are each independently a carbocyclyl optionally substituted with substituent group B, or a heterocyclyl optionally substituted with substituent group B,
$P^{R7}$s are each independently hydrogen, alkyl optionally substituted with substituent group D, or phenyl optionally substituted with substituent group E, and
$P^{R8}$s are each independently hydrogen or alkyl optionally substituted with substituent group F,
wherein
the substituent group B: hydroxy, alkyl, and oxo,
the substituent group D: a carbocyclylalkyloxy optionally substituted with substituent group G, and alkyloxy,
the substituent group E: halogen, alkyl, and alkyloxy,
the substituent group F: alkyloxycarbonyl, and
the substituent group G: halogen and cyano,

or a pharmaceutically acceptable salt thereof.
[14] The compound according to [13],
wherein

$R^3$, $R^6$, $R^{A5}$, $R^{B5}$, $R^{C5}$, $R^{A6}$, $R^{B6}$, and $R^{C6}$ are groups other than the group forming a prodrug, and
$R^1$ is hydrogen or a group selected from those represented by the following formulae:

a) -C(=O)-$P^{R0}$;
b) -C(=O)-L-$P^{R1}$; and

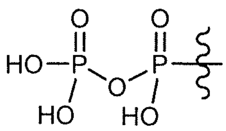
,
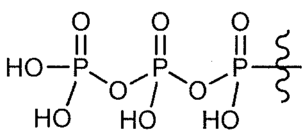

wherein
L is a linear or branched alkylene,
$P^{R0}$ is alkyl, or a carbocyclyl optionally substituted with substituent group B, and
$P^{R1}$ is a carbocyclyl optionally substituted with substituent group B,
wherein
the substituent group B: hydroxy and alkyl,

or a pharmaceutically acceptable salt thereof.
[15] The compound according to any one of [1] to [7], and [9] to [10],
wherein

$R^1$, $R^3$, $R^6$, $R^{A6}$, $R^{B6}$, and $R^{C6}$ are groups other than the group forming a prodrug, and
$R^{A5}$, $R^{B5}$, and $R^{C5}$ are each independently hydrogen or a group selected from those represented by the following formula:

a) -C(=O)-$P^{R0}$,

wherein $P^{R0}$s are each independently alkyl,

or a pharmaceutically acceptable salt thereof.
[16] The compound according to any one of [1], [2], [4] to [7], and [9] to [10],
wherein

$R^1$, $R^3$, $R^{A5}$, $R^{B5}$, $R^{C5}$, $R^{A6}$, $R^{B6}$, and $R^{C6}$ are groups other than the group forming a prodrug, and

$R^6$s are each independently hydrogen or a group selected from those represented by the following formula:

a) -C(=O)-$P^{R0}$,

wherein $P^{R0}$ is alkyl,

or a pharmaceutically acceptable salt thereof.
[17] A pharmaceutical composition comprising the compound according to any one of [1] to [16] or a pharmaceutically acceptable salt thereof.
[18] The pharmaceutical composition according to [17], wherein the pharmaceutical composition is an antiviral agent.
[19] An antiviral agent comprising the compound according to any one of [1] to [16] or a pharmaceutically acceptable salt thereof.
[20] A method for treating and/or preventing a viral infectious disease, comprising administering the compound according to any one of [1] to [16], or a pharmaceutically acceptable salt thereof.
[21] The compound according to any one of [1] to [16], or a pharmaceutically acceptable salt thereof, for use in treating and/or preventing a viral infectious disease.

[1'] A compound represented by Formula (I):

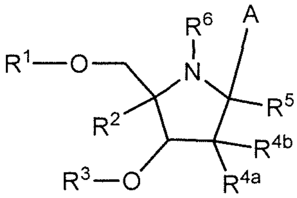

(I)

wherein

A is a group represented by any one of the following formulae:

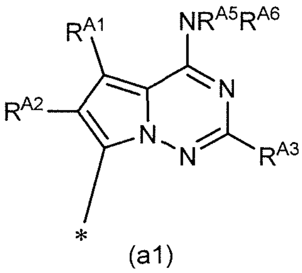
(a1)

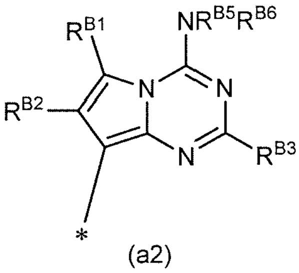
(a2)

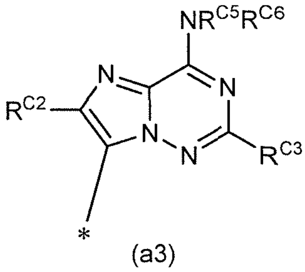
(a3)

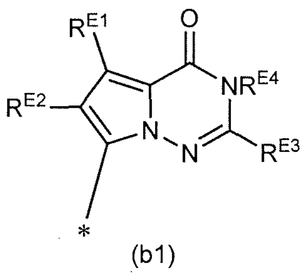
(b1)

wherein

$R^{A1}$, $R^{B1}$, and $R^{E1}$ are each independently hydrogen, halogen, hydroxy, - $B(OH)_2$, amino optionally substituted with substituent group $\gamma$, an aromatic heterocyclyl optionally substituted with substituent group $\alpha$, alkyl optionally substituted with substituent group $\beta$, or a group represented by any one of the following formulae: -C(=O)-N($R^{a1}$)($R^{a2}$), -C(=N$R^{a3}$)-$NH_2$, and -C(=NOH)-H,
wherein
$R^{a1}$ is hydrogen, $R^{a2}$ is hydrogen or alkyl optionally substituted with substituent group $\beta$, and $R^{a3}$ is hydrogen or hydroxy,
wherein

the substituent group $\alpha$: halogen and alkyl,
the substituent group $\beta$: cyano and hydroxy, and
the substituent group $\gamma$: alkyl,

$R^{A2}$, $R^{B2}$, $R^{C2}$, and $R^{E2}$ are each independently hydrogen,
$R^{A3}$, $R^{B3}$, $R^{C3}$, and $R^{E3}$ are each independently hydrogen, halogen, amino, or alkyl,
$R^{E4}$ is hydrogen,
$R^{A5}$, $R^{B5}$, and $R^{C5}$ are each independently hydrogen, alkyl, or a group forming a prodrug,
$R^{A6}$, $R^{B6}$, and $R^{C6}$ are each independently hydrogen, hydroxy, alkylcarbonyl, or a group forming a prodrug,

$R^1$ is hydrogen or a group forming a prodrug,
$R^2$ is hydrogen, halogen, C1-C3 alkyl, C2-C3 alkenyl, C2-C3 alkynyl, halo C1-C3 alkyl, halo C2-C3 alkenyl, or halo C2-C3 alkynyl,
$R^3$ is hydrogen or a group forming a prodrug,
$R^{4a}$ is hydrogen, hydroxy, or halogen,
$R^{4b}$ is hydrogen, halogen, C1-C3 alkyl, C1-C3 haloalkyl, C2-C3 alkenyl, or C2-C3 alkynyl,
$R^5$ is hydrogen, C1-C3 alkyl, C2-C3 alkenyl, or C2-C3 alkynyl, and
$R^6$ is hydrogen, C1-C3 alkyl, or a group forming a prodrug,
provided that the following compound is excluded:

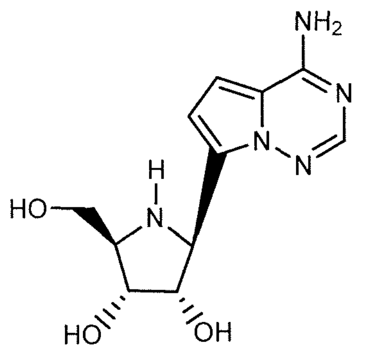

or a pharmaceutically acceptable salt thereof.
[2'] The compound according to [1'], represented by Formula:

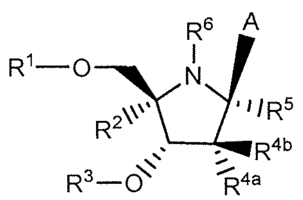

(I-1)

wherein

A is a group represented by any one of the following formulae:

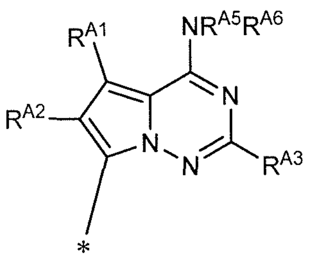

(a1)

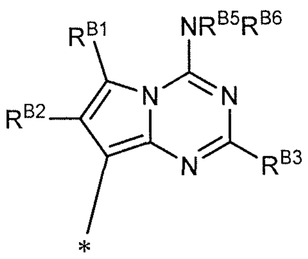

(a2)

wherein

$R^{A1}$ and $R^{B1}$ are each independently hydrogen, halogen, hydroxy, - $B(OH)_2$, amino, an aromatic heterocyclyl optionally substituted with substituent group $\alpha$, alkyl optionally substituted with substituent group $\beta$, or a

group represented by any of the following formulae: -C(=O)-N($R^{a1}$)($R^{a2}$), -C(=$NR^{a3}$)-$NH_2$, and -C(=NOH)-H, wherein $R^{a1}$ is hydrogen, $R^{a2}$ is hydrogen or alkyl optionally substituted with substituent group β, and $R^{a3}$ is hydrogen or hydroxy,
wherein

the substituent group α: halogen and alkyl, and
the substituent group β: cyano and hydroxy,

$R^{A2}$ and $R^{B2}$ are hydrogen,
$R^{A3}$ and $R^{B3}$ are each independently hydrogen, halogen, or alkyl,
$R^{A5}$ and $R^{B5}$ are each independently hydrogen, or a group forming a prodrug, and
$R^{A6}$ and $R^{B6}$ are each independently hydrogen, hydroxy, alkylcarbonyl, or a group forming a prodrug,

$R^1$ is hydrogen or a group forming a prodrug,
$R^2$ is hydrogen,
$R^3$ is hydrogen or a group forming a prodrug,
$R^{4a}$ is hydroxy,
$R^{4b}$ is hydrogen,
$R^5$ is hydrogen, and;
$R^6$ is hydrogen, C1-C3 alkyl, or a group forming a prodrug,

or a pharmaceutically acceptable salt of the compound.
[3'] The compound according to [2'], wherein

$R^{A6}$ and $R^{B6}$ are each independently hydrogen, hydroxy, or alkylcarbonyl,
$R^3$ is hydrogen, and
$R^6$ is hydrogen or C1-C3 alkyl,

or a pharmaceutically acceptable salt thereof.
[4'] The compound according to any one of [1'] to [3'],

wherein A is a group represented by either one of the following formulae:

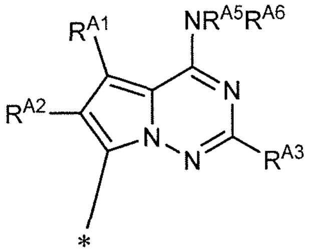
(a1)

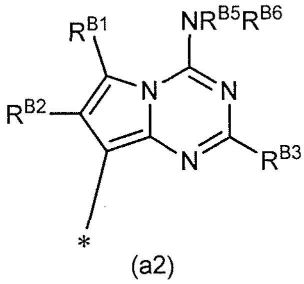
(a2)

wherein
$R^{A1}$ and $R^{B1}$ are each independently hydroxy, amino, an aromatic heterocyclyl optionally substituted with substituent group α, or a group represented by any of the following formulae: -C(=O)-N($R^{a1}$)($R^{a2}$), and -C(=$NR^{a3}$)-$NH_2$,
wherein $R^{a1}$ is hydrogen, $R^{a2}$ is hydrogen or cyanomethyl, and $R^{a3}$ is hydrogen or hydroxy,
wherein the substituent group α: fluorine and C1-C3 alkyl, and $R^{A3}$ and $R^{B3}$ are each independently hydrogen or fluorine, and
the other symbols are the same as those in [1'],

or a pharmaceutically acceptable salt thereof.
[5'] The compound according to any one of [1'] to [4'], wherein $R^{A1}$ and $R^{B1}$ are hydroxy, a 5-membered aromatic heterocyclyl, or a group represented by any of the following formulae: -C(=O)-$NH_2$, and -C(=NOH)-$NH_2$,
or a pharmaceutically acceptable salt thereof.
[6'] The compound according to any one of [1'] to [3'],

wherein

A is a group represented by either one of the following formulae:

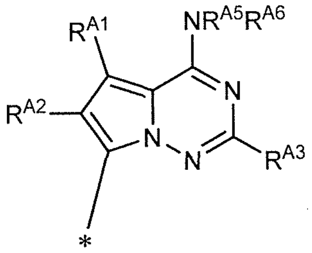
(a1)

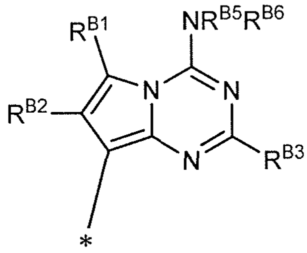
(a2)

wherein

$R^{A1}$ and $R^{B1}$ are each independently hydrogen, fluorine, hydroxy, - $B(OH)_2$, amino, an aromatic heterocyclyl optionally substituted with substituent group $\alpha$, cyanomethyl, C1-C3 alkyl substituted with hydroxy, or a group represented by the following formula: -C(=NOH)-H,
wherein the substituent group $\alpha$: C1-C3 alkyl, and
$R^{A3}$ and $R^{B3}$ are each independently hydrogen, fluorine, or methyl, and

the other symbols are the same as those in [1'],

or a pharmaceutically acceptable salt thereof.
[7'] The compound according to any one of [1'], [2'], [3'], and [6'], wherein $R^{A1}$ and $R^{B1}$ are fluorine, a 5- or 6-membered aromatic heterocyclyl, cyanomethyl, or a group represented by the following formula:

-C(=NOH)-H,

or a pharmaceutically acceptable salt thereof.
[8'] A compound selected from the group consisting of those represented by the following formulae:

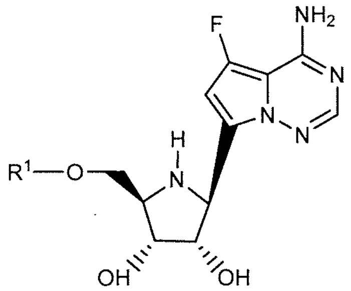

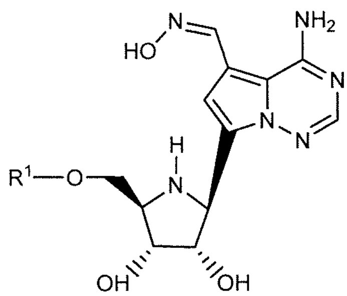

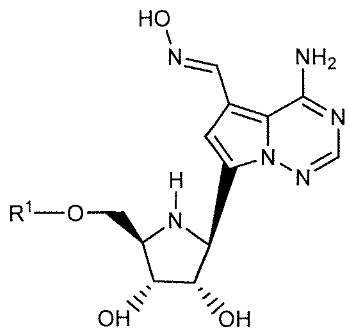

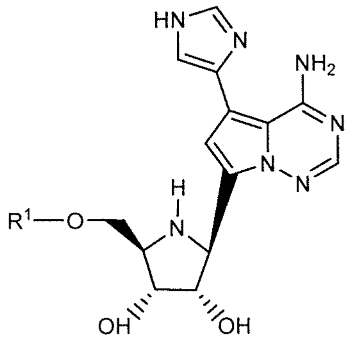

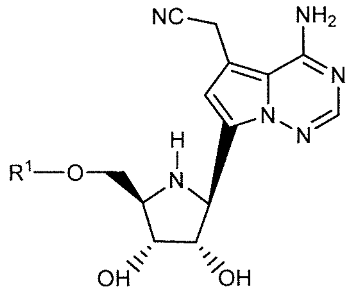

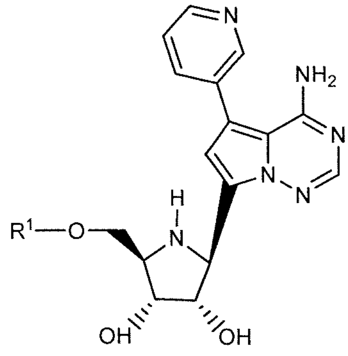

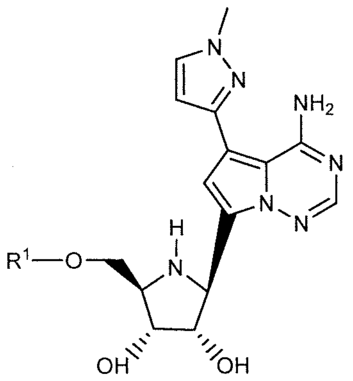

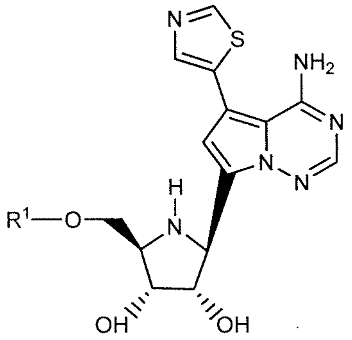

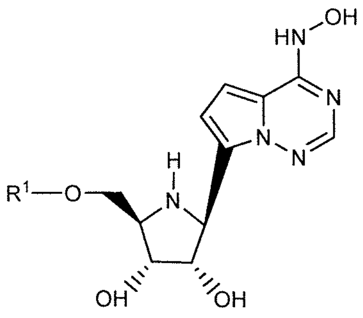

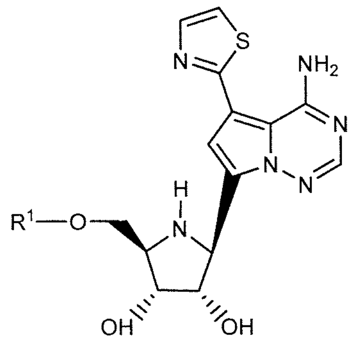

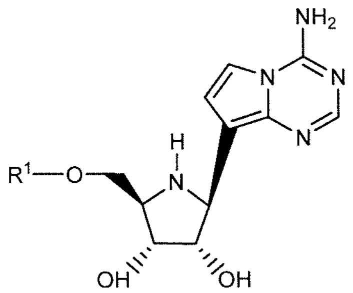

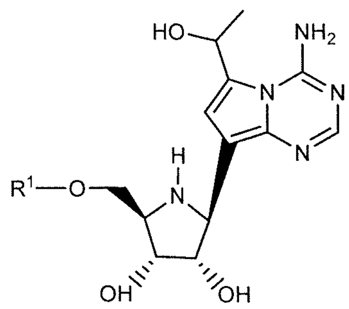

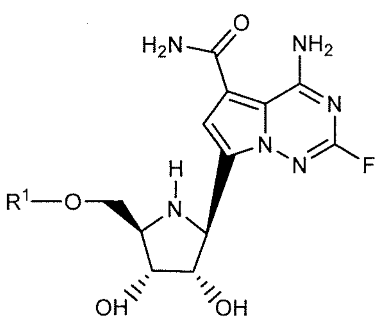

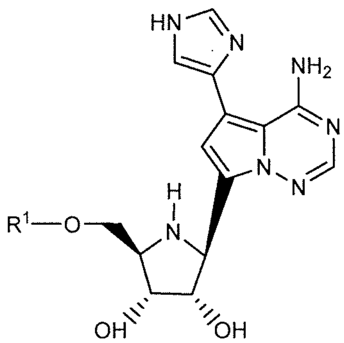

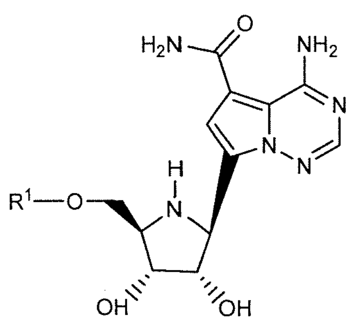

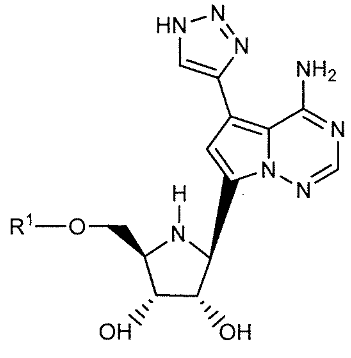

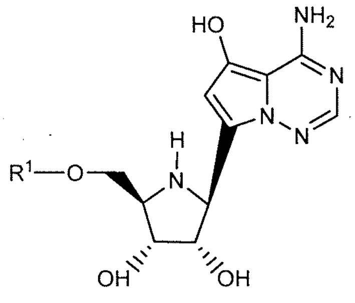

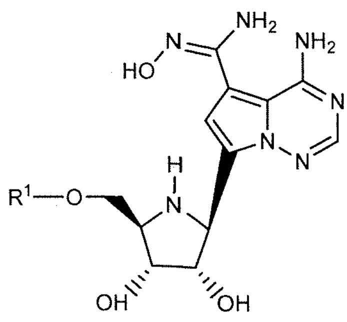

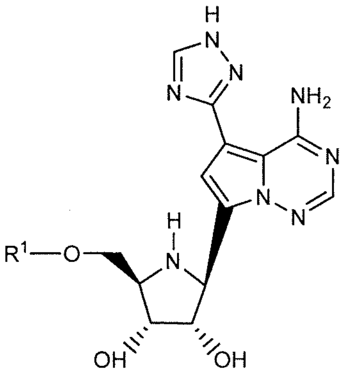

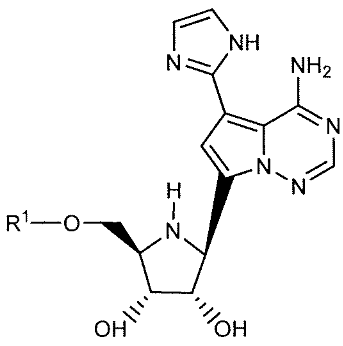

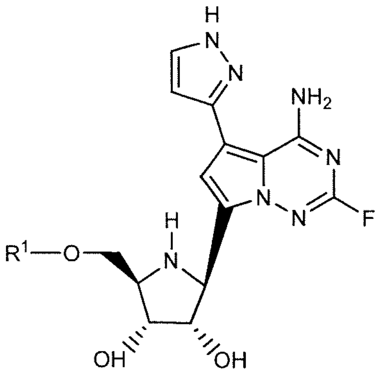

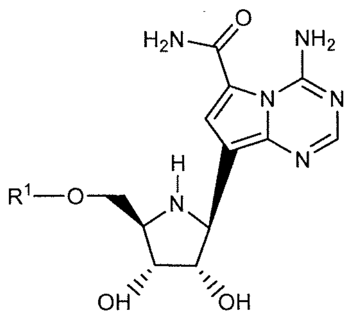

wherein $R^1$ is hydrogen or a group forming a prodrug, or a pharmaceutically acceptable salt thereof.

[9'] A compound represented by Formula (II):

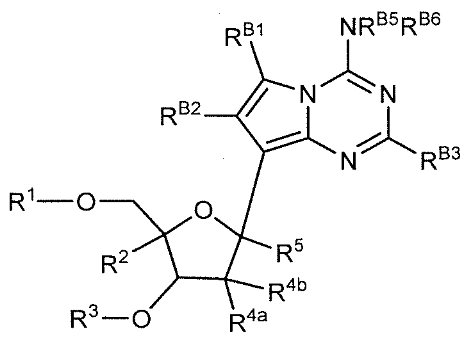

(II)

wherein

$R^{B1}$ is hydrogen, halogen, hydroxy, $-B(OH)_2$, amino optionally substituted with substituent group $\gamma$, an aromatic heterocyclyl optionally substituted with substituent group $\alpha$, alkyl optionally substituted with substituent group $\beta$, or a group represented by any one of the following formulae: $-C(=O)-N(R^{a1})(R^{a2})$, $-C(=NR^{a3})-NH_2$, and $-C(=NOH)-H$,

wherein

$R^{a1}$ is hydrogen, $R^{a2}$ is hydrogen or alkyl optionally substituted with substituent group $\beta$, and $R^{a3}$ is hydrogen or hydroxy,

wherein the substituent group $\alpha$: halogen and alkyl,
the substituent group $\beta$: cyano and hydroxy, and
the substituent group $\gamma$: alkyl,

$R^{B2}$ is hydrogen,
$R^{B3}$ is hydrogen, halogen, amino, or alkyl,
$R^{B5}$ is hydrogen, alkyl, or a group forming a prodrug,
$R^{B6}$ is hydrogen, hydroxy, alkylcarbonyl, or a group forming a prodrug,

$R^1$ is hydrogen or a group forming a prodrug,
$R^2$ is hydrogen, halogen, C1-C3 alkyl, C2-C3 alkenyl, C2-C3 alkynyl, halo C1-C3 alkyl, halo C2-C3 alkenyl, or halo C2-C3 alkynyl,
$R^3$ is hydrogen or a group forming a prodrug,
$R^{4a}$ is hydrogen, hydroxy, or halogen,
$R^{4b}$ is hydrogen, halogen, C1-C3 alkyl, C1-C3 haloalkyl, C2-C3 alkenyl, or C2-C3 alkynyl, and
$R^5$ is hydrogen or cyano,

or a pharmaceutically acceptable salt thereof.
[10'] A compound represented by Formula (II-a2-1):

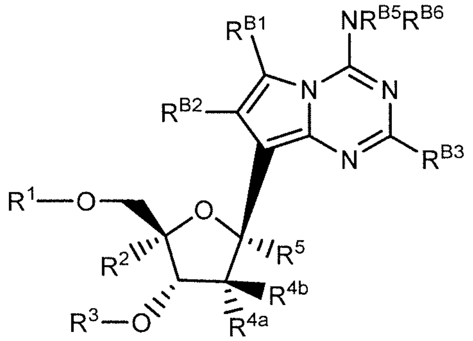

(II-a2-1)

wherein

$R^{B1}$ is hydrogen, halogen, hydroxy, $-B(OH)_2$, amino, an aromatic heterocyclyl optionally substituted with substituent group $\alpha$, alkyl optionally substituted with substituent group $\beta$, or a group represented by any one of the following formulae: - $C(=O)-N(R^{a1})(R^{a2})$, $-C(=NR^{a3})-NH_2$, and -C(=NOH)-H,
wherein $R^{a1}$ is hydrogen, $R^{a2}$ is hydrogen or alkyl optionally substituted with substituent group B, and $R^{a3}$ is hydrogen or hydroxy,

wherein the substituent group $\alpha$: halogen and alkyl, and
the substituent group $\beta$: cyano and hydroxy,

$R^{B2}$ is hydrogen,
$R^{B3}$ is hydrogen, halogen, or alkyl,
$R^{B5}$ is hydrogen or a group forming a prodrug,
$R^{B6}$ is hydrogen, hydroxy, alkylcarbonyl, or a group forming a prodrug,
$R^1$ is hydrogen or a group forming a prodrug,
$R^2$ is hydrogen or halogen,
$R^3$ is hydrogen or a group forming a prodrug,
$R^{4a}$ is hydroxy or halogen,
$R^{4b}$ is hydrogen or halogen, and
$R^5$ is hydrogen or cyano,

or a pharmaceutically acceptable salt thereof.
[11'] A compound selected from the group consisting of those represented by the following formulae:

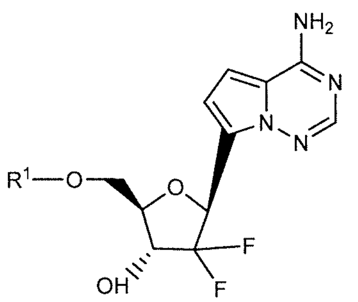

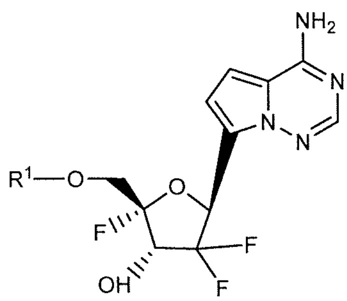

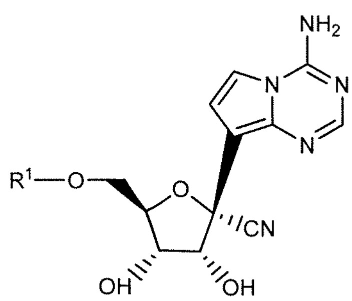

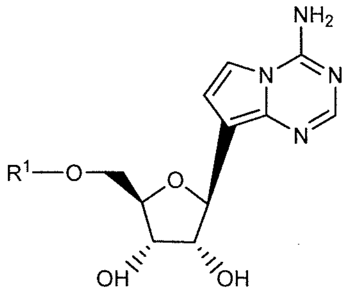
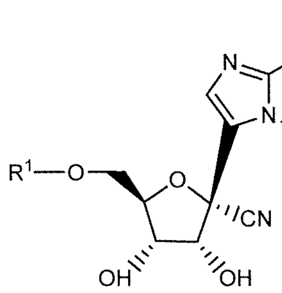
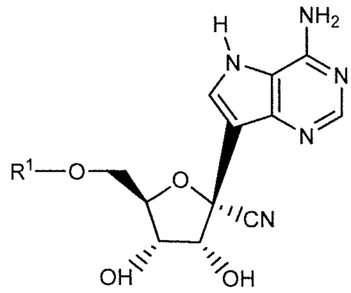

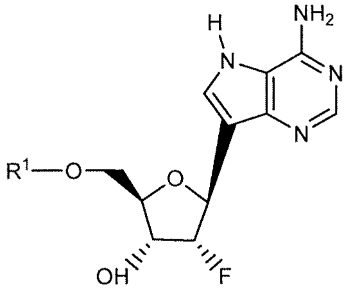

wherein $R^1$ is hydrogen or a group forming a prodrug,
or a pharmaceutically acceptable salt thereof.
[12'] The compound according to any one of [1'] to [11'],

wherein the groups forming the prodrugs of $R^1$ and $R^3$ are groups selected from those represented by the following formulae:

a) $-C(=O)-P^{R0}$;
b) $-C(=O)-L-P^{R1}$;
c) $-C(=O)-L-O-P^{R0}$;
d) $-C(=O)-L-S-P^{R0}$;
e) $-C(=O)-L-N(-P^{R2})_2$;
f) $-C(=O)-C(P^{R3})_2-NH_2$;
g) $-C(=O)-O-P^{R0}$;
h) $-C(P^{R4})_2-O-C(=O)-P^{R5}$;
i) $-C(P^{R4})_2-O-C(=O)-L-P^{R6}$;
j) $-C(P^{R4})_2-O-C(=O)-O-P^{R5}$;
k) $C(P^{R4})_2-O-C(=O)-O-L-P^{R6}$;
l) $-P(=O)(-OP^{R7})_2$;
m) $-P(=O)(-OP^{R7})-N(-P^{R8})_2$; and
n) $-P(=O)(-N(-P^{R8})_2)_2$,

the groups forming the prodrugs of $R^{A5}$, $R^{B5}$, $R^{C5}$, $R^{A6}$, $R^{B6}$, $R^{C6}$, and $R^6$ are groups selected from those represented by the following formulae:

a) $-C(=O)-P^{R0}$;
g) $-C(=O)-O-P^{R0}$;
o) $-C(=O)-O-L-P^{R1}$;
p) $-C(=O)-L-O-C(=O)-P^{R0}$; and
q) $-C(P^{R4})_2-P^{R1}$, or

the groups forming prodrugs of $R^1$ and $R^3$ may be taken together to form a group represented by the following formula: $-P(=O)(-OP^{R7})$, or alternatively,
the groups forming prodrugs of $R^1$ and $R^6$ may be taken together to form a group represented by the following formula: $-P(=O)(-OP^{R7})$, or $-P(=O)(-N(-P^{R8})_2)$,
wherein L's are each independently a linear or branched alkylene,

$P^{R0}$s are each independently alkyl optionally substituted with substituent group A, a carbocyclyl optionally substituted with substituent group B, or a heterocyclyl optionally substituted with substituent group B,
$P^{R1}$s are each independently a carbocyclyl optionally substituted with substituent group B, or a heterocyclyl optionally substituted with substituent group B,
$P^{R2}$s are each independently alkyl optionally substituted with substituent group A, a carbocyclyl optionally substituted with substituent group B, or a heterocyclyl optionally substituted with substituent group B, or
two $P^{R2}$s may be taken together with an adjacent atom to form a heterocycle optionally substituted with substituent group E,
$P^{R3}$s are each independently hydrogen or alkyl optionally substituted with substituent group C,
$P^{R4}$s are each independently hydrogen or alkyl,
$P^{R5}$s are each independently alkyl optionally substituted with substituent group A, a carbocyclyl optionally substituted with substituent group B, or a heterocyclyl optionally substituted with substituent group B,
$P^{R6}$s are each independently a carbocyclyl optionally substituted with substituent group B, or a heterocyclyl optionally substituted with substituent group B,
$P^{R7}$s are each independently hydrogen, alkyl optionally substituted with substituent group D, phenyl optionally substituted with substituent group E, or naphthyl optionally substituted with substituent group E, or
two $P^{R7}$s may be taken together with adjacent atoms to form a heterocycle optionally substituted with substituent group E, and
$P^{R8}$s are each independently hydrogen or alkyl optionally substituted with substituent group F,
wherein
the substituent group A: halogen,
the substituent group B: hydroxy, alkyl, and oxo,
the substituent group C: a carbocyclyl, a heterocyclyl, and alkylthio,
the substituent group D: a carbocyclyl, a heterocyclyl, alkyloxy, alkyloxycarbonyl, alkylcarbonyloxy, alkylthio, and alkylcarbonylthio,
the substituent group E: halogen, alkyl, and alkyloxy, and
the substituent group F: a carbocyclyl, a heterocyclyl, halogen, and alkyloxycarbonyl,

or a pharmaceutically acceptable salt thereof.
[13'] The compound according to any one of [1'] to [11'],

wherein $R^3$, $R^6$, $R^{A5}$, $R^{B5}$, $R^{C5}$, $R^{A6}$, $R^{B6}$, and $R^{C6}$ are groups other than the group forming a prodrug, and $R^1$ is hydrogen or a group selected from those represented by the following formulae:

a) $-C(=O)-P^{R0}$;
b) $-C(=O)-L-P^{R1}$;
f) $-C(=O)-C(P^{R3})_2-NH_2$;
g) $-C(=O)-O-P^{R0}$;
i) $-C(P^{R4})_2-O-C(=O)-L-P^{R6}$;
1) $-P(=O)(-OP^{R7})_2$; and
m) $-P(=O)(-OP^{R7})-N(-P^{R8})_2$,

wherein
L's are each independently a linear or branched alkylene,
$P^{R0}$s are each independently alkyl, a carbocyclyl optionally substituted with substituent group B, or a heterocyclyl optionally substituted with substituent group B,
$P^{R1}$s are each independently a carbocyclyl optionally substituted with substituent group B, or a heterocyclyl optionally substituted with substituent group B,
$P^{R4}$s are each independently hydrogen or alkyl,
$P^{R6}$s are each independently a carbocyclyl optionally substituted with substituent group B, or a heterocyclyl optionally substituted with substituent group B,
$P^{R7}$s are each independently hydrogen or a carbocyclyl, and
$P^{R8}$s are each independently hydrogen or alkyl optionally substituted with substituent group F,
wherein
the substituent group B: hydroxy, alkyl, and oxo, and
the substituent group F: alkyloxycarbonyl,

or a pharmaceutically acceptable salt thereof.

[14'] The compound according to [13'], wherein

$R^3$, $R^6$, $R^{A5}$, $R^{B5}$, $R^{C5}$, $R^{A6}$, $R^{B6}$, and $R^{C6}$ are groups other than the group forming a prodrug, and $R^1$ is hydrogen or a group selected from those represented by the following formulae:

a) -C(=O)-$P^{R0}$; and
b) -C(=O)-L-$P^{R1}$,

wherein
L is a linear or branched alkylene,
$P^{R0}$ is alkyl, or a carbocyclyl optionally substituted with substituent group B, and
$P^{R1}$ is a carbocyclyl optionally substituted with substituent group B,
wherein the substituent group B: hydroxy and alkyl,

or a pharmaceutically acceptable salt thereof.
[15'] The compound according to any one of [1'] to [7'], and [9'] to [10'],

wherein $R^1$, $R^3$, $R^6$, $R^{A6}$, $R^{B6}$, and $R^{C6}$ are groups other than the group forming a prodrug, and $R^{A5}$, $R^{B5}$, and $R^{C5}$ are each independently hydrogen or a group selected from those represented by the following formula:

a) -C(=O)-$P^{R0}$,

wherein $P^{R0}$s are each independently alkyl,

or a pharmaceutically acceptable salt thereof.
[16'] The compound according to any one of [1'], [2'], [4'] to [7'], and [9'] to [10'],

wherein $R^1$, $R^3$, $R^{A5}$, $R^{B5}$, $R^{C5}$, $R^{A6}$, $R^{B6}$, and $R^{C6}$ are groups other than the group forming a prodrug, and $R^6$s are each independently hydrogen or a group selected from those represented by the following formula:

a) -C(=O)-$P^{R0}$,

wherein $P^{R0}$ is alkyl,

or a pharmaceutically acceptable salt thereof.
[17'] A pharmaceutical composition comprising the compound according to any one of [1'] to [16'] or a pharmaceutically acceptable salt thereof.
[18'] The pharmaceutical composition according to [17'], wherein the pharmaceutical composition is an antiviral agent.
[19'] An antiviral agent comprising the compound according to any one of [1'] to [16'] or a pharmaceutically acceptable salt thereof.

The present invention further provides a method for treating or preventing a viral infectious disease using a prodrug compound. The present invention further provides an active compound of a prodrug compound. The active compound is useful as an antiviral agent, or an intermediate of the prodrug compound.

[EFFECT OF THE INVENTION]

The compound according to the present invention has a viral growth inhibitory action. A more preferred compound is a prodrug, and it is useful as a therapeutic agent and/or a preventive agent for a viral infectious disease, since after it is administered, it becomes an active compound having a viral growth inhibitory action in a body.

[MODE FOR CARRYING OUT THE INVENTION]

Hereinafter, the meaning of each term used in the present specification will be described. Unless particularly stated otherwise, each term is used in the same sense, either alone or in combination with other terms.
The term "consist of" means having only the constituent elements.

The term "comprise" means that elements are not limited to the constituent elements, and elements that are not described are not excluded.

Hereinafter, the present invention will be described while showing exemplary embodiments. Throughout the present specification, it should be understood that, unless particularly stated otherwise, an expression of a singular form also includes the concept of a plural form thereof. Therefore, it should be understood that, unless particularly stated otherwise, an article for a singular form (for example, in the case of English, "a", "an", "the", or the like) also includes the concept of a plural form thereof.

Furthermore, it should be understood that, unless particularly stated otherwise, the terms used in the present specification are used in the meanings normally used in the above-described art. Accordingly, unless otherwise defined, all terminologies and scientific and technical terms used in the present specification have the same meanings as commonly understood by those having ordinary skill in the art to which the present invention belongs. In a case of contradiction, priority is given to the present specification (including definitions).

"Halogen" encompasses a fluorine atom, a chlorine atom, a bromine atom, and an iodine atom. In particular, a fluorine atom and a chlorine atom are preferable.

The term "Alkyl" encompasses linear or branched hydrocarbon groups each having 1 to 15 carbon atoms, preferably 1 to 10 carbon atoms, more preferably 1 to 6 carbon atoms, even more preferably 1 to 4 carbon atoms, and most preferably 1 to 3 carbon atoms. Examples include methyl, ethyl, n-propyl, isopropyl, n-butyl, isobutyl, sec-butyl, tert-butyl, n-pentyl, isopentyl, neopentyl, n-hexyl, isohexyl, n-heptyl, isoheptyl, n-octyl, isooctyl, n-nonyl, and n-decyl.

Preferred embodiments of "alkyl" include methyl, ethyl, n-propyl, isopropyl, n-butyl, isobutyl, sec-butyl, tert-butyl, and n-pentyl. More preferred aspects include methyl, ethyl, n-propyl, isopropyl, and tert-butyl.

The term "Alkenyl" encompasses linear or branched hydrocarbon groups each having one or more double bonds at any position and having 2 to 15 carbon atoms, preferably 2 to 10 carbon atoms, more preferably 2 to 6 carbon atoms, even more preferably 2 to 4 carbon atoms, and most preferably 2 to 3 carbon atoms. Examples include vinyl, allyl, propenyl, isopropenyl, butenyl, isobutenyl, prenyl, butadienyl, pentenyl, isopentenyl, pentadienyl, hexenyl, isohexenyl, hexadienyl, heptenyl, octenyl, nonenyl, decenyl, undecenyl, dodecenyl, tridecenyl, tetradecenyl, and pentadecenyl.

Preferred embodiments of "alkenyl" include vinyl, allyl, propenyl, isopropenyl, and butenyl. More preferred embodiments include ethenyl, n-propenyl, and the like.

The term "alkynyl" encompasses linear or branched hydrocarbon groups each having one or more double bonds at any position and having 2 to 10 carbon atoms, preferably 2 to 8 carbon atoms, more preferably 2 to 6 carbon atoms, even more preferably 2 to 4 carbon atoms, and most preferably 2 to 3 carbon atoms. Alkynyl may further have a double bond at any position. Examples include ethynyl, propynyl, butynyl, pentynyl, hexynyl, heptynyl, octynyl, nonynyl, and decynyl.

Preferred embodiments of "alkynyl" include ethynyl, propynyl, butynyl, and pentynyl. More preferred embodiments include ethynyl, propynyl, and the like.

The term "alkylene" encompasses linear or branched divalent hydrocarbon groups each having 1 to 15 carbon atoms, preferably 1 to 10 carbon atoms, more preferably 1 to 6 carbon atoms, and even more preferably 1 to 4 carbon atoms. For example, it includes methylene, ethylene, trimethylene, propylene, tetramethylene, pentamethylene, hexamethylene and the like.

The term "carbocyclyl" means a carbocyclyl having 3 to 20 carbon atoms, preferably 3 to 16 carbon atoms, more preferably 4 to 12 carbon atoms, and encompasses an aromatic carbocyclyl and a non-aromatic carbocyclyl.

The term "aromatic carbocyclyl" means a cyclic aromatic hydrocarbon group which is monocyclic or polycyclic having two or more rings. Examples of the same include phenyl, naphthyl, anthryl, and phenanthryl.

Examples of an embodiment of the "aromatic carbocyclyl" include phenyl, 1-naphthyl, and 2-naphthyl. Another embodiment thereof includes phenyl.

"Aromatic carbocycle" means a ring derived from the above-described "aromatic carbocyclyl".

The term "non-aromatic carbocyclyl" means a cyclic saturated hydrocarbon group or a cyclic non-aromatic unsaturated hydrocarbon group, both having a single ring or two or more rings. The "non-aromatic carbocyclyl" which is polycyclic having two or more rings also encompasses a fused ring group wherein a non-aromatic carbocyclyl, which is monocyclic or polycyclic having two or more rings, is fused with a ring of the above-described "aromatic carbocyclyl".

Furthermore, the "non-aromatic carbocyclyl" also encompasses a bridged group or a group forming a spiro ring, such as follows.

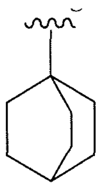 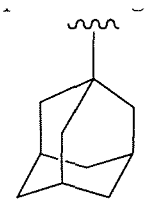 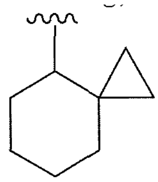

A non-aromatic carbocyclyl having a single ring preferably has 3 to 16 carbon atoms, more preferably 3 to 12 carbon atoms, and even more preferably 4 to 8 carbon atoms. Examples of the same include cyclopropyl, cyclobutyl, cyclopentyl, cyclohexyl, cycloheptyl, cyclooctyl, cyclononyl, cyclodecyl, cyclopropenyl, cyclobutenyl, cyclopentenyl, cyclohexenyl, cycloheptenyl, and cyclohexadienyl.

The non-aromatic carbocyclyl which is polycyclic having two or more rings preferably has 8 to 20 carbon atoms, and more preferably 8 to 16 carbon atoms. Examples of the same include indanyl, indenyl, acenaphthyl, tetrahydronaphthyl, and fluorenyl.

"Non-aromatic carbocycle" means a ring derived from the above-described "non-aromatic carbocyclyl".

The "aromatic heterocyclyl" encompasses an aromatic heterocyclyl and a non-aromatic heterocyclyl each of which has one or more rings having one or more, same or different heteroatom(s) selected optionally from O, S, and N.

The "heterocycle" means a ring derived from the above-described "heterocyclyl".

The term "aromatic heterocyclyl" means an aromatic cyclic group, which is monocyclic or polycyclic having two or more rings, having one or more, same or different heteroatom(s) selected optionally from O, S, and N.

An aromatic heterocyclyl having two or more rings also encompasses an aromatic heterocyclyl having a single ring or two or more rings, to which a ring in the "aromatic carbocyclyl" is fused, and the linking bond may be carried by any of the rings.

The aromatic heterocyclyl having a single ring is preferably a 5- to 8-membered ring, and more preferably a 5-membered or 6-membered ring. Examples of 5-membered aromatic heterocyclyl include pyrrolyl, imidazolyl, pyrazolyl, triazolyl, tetrazolyl, furyl, thienyl, isoxazolyl, oxazolyl, oxadiazolyl, isothiazolyl, thiazolyl, and thiadiazolyl. Examples of 6-membered aromatic heterocyclyl include pyridyl, pyridazinyl, pyrimidinyl, pyrazinyl, and triazinyl.

The aromatic heterocyclyl having two rings is preferably a 8- to 10-membered ring, and more preferably a 9-membered or 10-membered ring. Examples thereof include indolyl, isoindolyl, indazolyl, indolizinyl, quinolinyl, isoquinolinyl, cinnolinyl, phthalazinyl, quinazolinyl, naphthyridinyl, quinoxalinyl, purinyl, pteridinyl, benzimidazolyl, benzisoxazolyl, benzoxazolyl, benzoxadiazolyl, benzisothiazolyl, benzothiazolyl, benzothiadiazolyl, benzofuryl, isobenzofuryl, benzothienyl, benzotriazolyl, imidazopyridyl, triazolopyridyl, imidazothiazolyl, pyrazinopyridazinyl, oxazolopyridyl, and thiazolopyridyl. Examples of the 9-membered aromatic heterocyclyl include indolyl, isoindolyl, indazolyl, indolizinyl, purinyl, benzimidazolyl, benzisoxazolyl, benzoxazolyl, benzoxadiazolyl, benzisothiazolyl, benzothiazolyl, benzothiadiazolyl, benzotriazolyl, benzofuranyl, imidazopyridyl, triazolopyridyl, oxazolopyridyl, and thiazolopyridyl. Examples of the 10-membered aromatic heterocyclyl include quinolinyl, isoquinolinyl, cinnolinyl, phthalazinyl, quinazolinyl, naphthyridinyl, quinoxalinyl, pteridinyl, and pyrazinopyridazinyl.

The aromatic heterocyclyl having three or more rings is preferably a 13- to 15-membered ring. Examples of the same include carbazolyl, acridinyl, xanthenyl, phenothiazinyl, phenoxathiinyl, phenoxazinyl, and dibenzofuryl.

"Aromatic heterocycle" means a ring derived from the above-described "aromatic heterocyclyl".

The "non-aromatic heterocyclyl" means a non-aromatic cyclic group, which is monocyclic or polycyclic having two or more rings, having one or more, same or different heteroatom(s) selected optionally from O, S, and N. A non-aromatic heterocyclyl having two or more rings also includes a non-aromatic heterocyclyl having a single ring or two or more rings, to which a ring in each of the "aromatic carbocyclyl", "non-aromatic carbocyclyl", and/or "aromatic heterocyclyl" is fused, as well as a non-aromatic carbocyclyl having a single ring or two or more rings, to which a ring in the "aromatic heterocyclyl" is fused, and the linking bond may be carried by any of the rings.

Furthermore, the "non-aromatic heterocyclyl" also encompasses a bridged group or a group forming a spiro ring, such as follows.

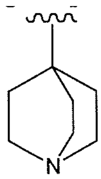 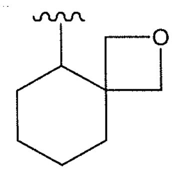

The non-aromatic heterocyclyl having a single ring is preferably a 3- to 8-membered ring, and more preferably

a 5-membered or 6-membered ring.

Examples of the 3-membered non-aromatic heterocyclyl include thiiranyl, oxiranyl and aziridinyl. Examples of 4-membered non-aromatic heterocyclyl include oxetanyl and azetidinyl. Examples of 5-membered non-aromatic heterocyclyl include oxathiolanyl, thiazolidinyl, pyrrolidinyl, pyrrolinyl, imidazolidinyl, imidazolinyl, pyrazolidinyl, pyrazolinyl, tetrahydrofuryl, dihydrothiazolyl, tetrahydroisothiazolyl, dioxolanyl, dioxolyl, and thiolanyl. Examples of 6-membered non-aromatic heterocyclyl include dioxanyl, thianyl, piperidyl, piperazinyl, morpholinyl, morpholino, thiomorpholinyl, thiomorpholino, dihydropyridyl, tetrahydropyridyl, tetrahydropyranyl, dihydroxazinyl, tetrahydropyridazinyl, hexahydropyrimidinyl, dioxazinyl, thiinyl, and thiazinyl. Examples of the 7-membered non-aromatic heterocyclyl include hexahydroazepinyl, tetrahydrodiazepinyl, and oxepanyl.

The non-aromatic heterocyclyl having two or more rings is preferably an 8- to 20-membered ring, more preferably an 8- to 13-membered ring, and even more preferably an 8- to 10-membered ring. Examples include indolinyl, isoindolinyl, chromanyl, and isochromanyl.

"Non-aromatic heterocycle" means a ring derived from the above-described "non-aromatic heterocyclyl".

The expression "optionally substituted with a substituent group a" herein means "may be substituted with one or more groups selected from a substituent group a". The same applies to the substituent groups β, γ, A, B, C, D, E, and F.

When the "carbocycle" and the "heterocycle" are substituted with "oxo", it means a ring in which two hydrogen atoms on the carbon atom are substituted as below.

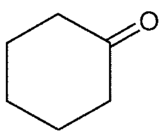
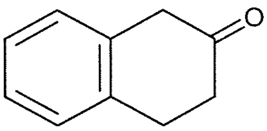
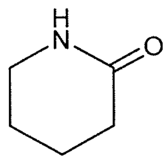
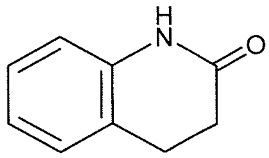

The "prodrug" in the present specification refers to, for example, a compound represented by the left-hand formula of the following reaction formula:

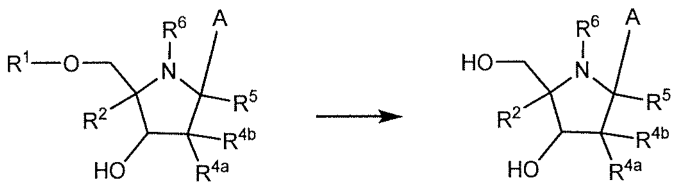

wherein $R^1$ is a group forming a prodrug; the symbols are the same as those described above,
or a pharmaceutically acceptable salt thereof, and means a compound which exhibits a viral RNA polymerase inhibitory activity or a viral growth inhibitory action by being converted into an active compound represented by the right-hand formula by a degradation reaction caused by a drug metabolizing enzyme, a hydrolyzing enzyme, gastric acid, intestinal bacteria, or the like under physiological conditions in vivo.

The prodrug more preferably means a compound having improved bioavailability and/or AUC (area under the blood concentration curve) upon in vivo administration as compared with the active compound.

Therefore, since the prodrug is efficiently absorbed into the body in the stomach, the intestine, or the like after administration to a living body (for example, oral administration), and is then converted into an active compound, the prodrug preferably exhibits a higher influenza therapeutic and/or preventive effect than the active compound.

The "group forming a prodrug" refers to, for example, the part of $-OR^1$ group ($R^1$ is a group forming a prodrug) or $-NR^6-R^5$ ($R^6$ is a group forming a prodrug) converted into -OH group or $-NH-R^5$ by a degradation reaction caused by a drug metabolizing enzyme, a hydrolyzing enzyme, gastric acid, intestinal bacteria, or the like under physiological conditions in vivo. The same applies to cases where $R^3$, $R^{A5}$, $R^{B5}$, $R^{C5}$, $R^{A6}$, $R^{B6}$, and $R^{C6}$ are groups forming a prodrug.

Examples of the group forming a prodrug include groups described in Prog. Med. 5: 2157-2161 (1985) and Supplied by The British Library - "The world's Knowledge".

The "group forming a prodrug" may be any group that the prodrug compound is converted into an active compound in vivo, and preferably includes, for example, a group selected from the following formulas.

The groups forming the prodrugs of $R^1$ and $R^3$ are groups selected from those represented by the following formulae:

a) $-C(=O)-P^{R0}$;

b) $-C(=O)-L-P^{R1}$;
c) $-C(=O)-L-O-P^{R0}$;
d) $-C(=O)-L-S-P^{R0}$;
e) $-C(=O)-L-N(-P^{R2})_2$;
f) $-C(=O)-C(P^{R3})_2-NH_2$;
g) $-C(=O)-O-P^{R0}$;
h) $-C(P^{R4})_2-O-C(=O)-P^{R5}$;
i) $-C(P^{R4})_2-O-C(=O)-L-P^{R6}$;
j) $-C(P^{R4})_2-O-C(=O)-O-P^{R5}$;
k) $-C(P^{R4})_2-O-C(=O)-O-L-P^{R6}$;
l) $-P(=O)(-OP^{R7})_2$;
m) $-P(=O)(-OP^{R7})-N(-P^{R8})_2$; and
n) $-P(=O)(-N(-P^{R8})_2)_2$.

The groups forming the prodrugs of $R^{A5}$, $R^{B5}$, $R^{C5}$, $R^{A6}$, $R^{B6}$, $R^{C6}$, and $R^6$ are groups selected from those represented by the following formulae:

a) $-C(=O)-P^{R0}$;
g) $-C(=O)-O-P^{R0}$;
o) $-C(=O)-O-L-P^{R1}$;
p) $-C(=O)-L-O-C(=O)-P^{R0}$; and
q) $-C(P^{R4})_2-P^{R1}$.

Alternatively,

the groups forming prodrugs of $R^1$ and $R^3$ may be taken together to form a group represented by the following formula: $-P(=O)(-OP^{R7})$, or
the groups forming prodrugs of $R^1$ and $R^6$ may be taken together to form a group represented by the following formula: $-P(=O)(-OP^{R7})$, or $-P(=O)(-N(-P^{R8})_2)$-.
L's are each independently a linear or branched alkylene,
$P^{R0}$s are each independently alkyl optionally substituted with substituent group A, a carbocyclyl optionally substituted with substituent group B, or a heterocyclyl optionally substituted with substituent group B,
$P^{R1}$s are each independently a carbocyclyl optionally substituted with substituent group B, or a heterocyclyl optionally substituted with substituent group B,
$P^{R2}$s are each independently alkyl optionally substituted with substituent group A, a carbocyclyl optionally substituted with substituent group B, or a heterocyclyl optionally substituted with substituent group B, or
two $P^{R2}$s may be taken together with an adjacent atom to form a heterocycle,
$P^{R3}$s are each independently hydrogen or alkyl optionally substituted with substituent group C,
$P^{R4}$s are each independently hydrogen or alkyl,
$P^{R5}$s are each independently alkyl optionally substituted with substituent group A, a carbocyclyl optionally substituted with substituent group B, or a heterocyclyl optionally substituted with substituent group B,
$P^{R6}$s are each independently a carbocyclyl optionally substituted with substituent group B, or a heterocyclyl optionally substituted with substituent group B,
$P^{R7}$s are each independently hydrogen, alkyl optionally substituted with substituent group D, phenyl optionally substituted with substituent group E, or naphthyl optionally substituted with substituent group E, or
two $P^{R7}$s may be taken together with adjacent atoms to form a heterocycle, and
$P^{R8}$s are each independently hydrogen or alkyl optionally substituted with substituent group F,
wherein the substituent group A: halogen,
the substituent group B: hydroxy, alkyl, and oxo,
the substituent group C: a carbocyclyl optionally substituted with substituent group G, a heterocyclyl optionally substituted with substituent group G, and alkylthio,
the substituent group D: a carbocyclyl optionally substituted with substituent group G, a heterocyclyl optionally substituted with substituent group G, a carbocyclylalkyloxy optionally substituted with substituent group G, alkyloxy, alkyloxycarbonyl, alkylcarbonyloxy, alkylthio, and alkylcarbonylthio,
the substituent group E: halogen, nitro, alkyl, and alkyloxy,
the substituent group F: a carbocyclyl, a heterocyclyl, halogen, and alkyloxycarbonyl, and
the substituent group G: halogen and cyano.

Embodiments and preferred embodiments of each substituent in the compound represented by Formula (I) or (II) are shown below. The following compounds having possible combination of the embodiments of each substituent are preferable.

A includes any one of the following formulae:

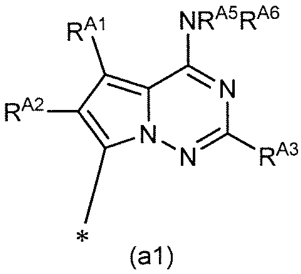
(a1)

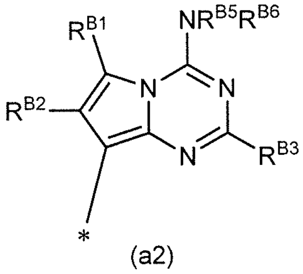
(a2)

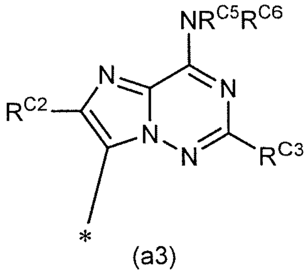
(a3)

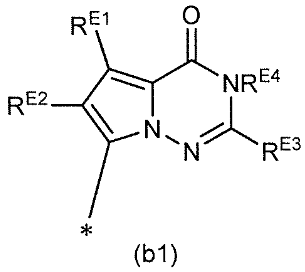
(b1)

An preferred embodiment of A includes the above-described (a1) or (a2).

A more preferred embodiment of A includes the above-described (a1).

$R^{A1}$, $R^{B1}$, and $R^{E1}$ include each independently hydrogen, halogen, hydroxy, - $B(OH)_2$, amino optionally substituted with substituent group $\gamma$, aromatic heterocyclyl optionally substituted with substituent group $\alpha$, non-aromatic heterocyclyl optionally substituted with substituent group $\alpha$, alkyl optionally substituted with substituent group B, or a group represented by any one of the following formulae: $-C(=O)-N(R^{a1})(R^{a2})$, $-C(=NR^{a3})-NH_2$, and -C(=NOH)-H,
wherein $R^{a1}$ is hydrogen, $R^{a2}$ is hydrogen or alkyl optionally substituted with substituent group $\beta$, and $R^{a3}$ is hydrogen or hydroxy.

The substituent groups are as follows:

the substituent group $\alpha$: halogen, hydroxy, alkyl, and hydroxyalkyl;
the substituent group $\beta$: cyano and hydroxy; and
the substituent group $\gamma$: alkyl.

Preferred embodiments of $R^{A1}$, $R^{B1}$, and $R^{E1}$ include each independently hydrogen, halogen, hydroxy, $-B(OH)_2$, amino, an aromatic heterocyclyl optionally substituted with substituent group $\alpha$, a non-aromatic heterocyclyl optionally substituted with substituent group $\alpha$, alkyl optionally substituted with substituent group B, or a group represented by any one of the following formulae: $-C(=O)-N(R^{a1})(R^{a2})$, $-C(=NR^{a3})-NH_2$, and -C(=NOH)-H,
wherein $R^{a1}$ is hydrogen, $R^{a2}$ is hydrogen or alkyl optionally substituted with substituent group B, and $R^{a3}$ is hydrogen or hydroxy.

The substituent groups are as follows:

the substituent group $\alpha$: halogen, hydroxy, alkyl, and hydroxyalkyl; and
the substituent group $\beta$: cyano and hydroxy.

Other preferred embodiments of $R^{A1}$, $R^{B1}$, and $R^{E1}$ include each independently hydroxy, amino, an aromatic heterocyclyl optionally substituted with substituent group a (fluorine, and C1-C3 alkyl), or a group represented by either one of the following formulae: $-C(=O)-N(R^{a1})(R^{a2})$, and $-C(=NR^{a3})-NH_2$,
wherein $R^{a1}$ is hydrogen, $R^{a2}$ is hydrogen or cyanomethyl, and $R^{a3}$ is hydrogen or hydroxy.

More preferable embodiments of $R^{A1}$, $R^{B1}$, and $R^{E1}$ include independently hydroxy, a 5-membered aromatic heterocyclyl, or a group represented by either one of the following formulae: $-C(=O)-NH_2$, and $-C(=NOH)-NH_2$.

Other preferred embodiments of $R^{A1}$, $R^{B1}$, and $R^{E1}$ include independently hydrogen, fluorine, hydroxy, $-B(OH)_2$, amino, an aromatic heterocyclyl optionally substituted with substituent group $\alpha$ (C1-C3 alkyl), cyanomethyl, C1-C3 alkyl substituted with hydroxy, or a group represented by the following formula: - C(=NOH).

More preferred embodiments of $R^{A1}$, $R^{B1}$, and $R^{E1}$ include independently fluorine, a 5- or 6-membered aromatic heterocyclyl, cyanomethyl, or a group represented by the following formula: -C(=NOH)-H.

$R^{A2}$, $R^{B2}$, $R^{C2}$, and $R^{E2}$ include each independently hydrogen.

$R^{A3}$, $R^{B3}$, $R^{C3}$, and $R^{E3}$ include each independently hydrogen, halogen, amino, or alkyl.

Preferred embodiments of $R^{A3}$, $R^{B3}$, $R^{C3}$, and $R^{E3}$ include each independently hydrogen, halogen, or alkyl.

Other preferred embodiments of $R^{A3}$, $R^{B3}$, $R^{C3}$, and $R^{E3}$ include each independently hydrogen or fluorine.

Other preferred embodiments of $R^{A3}$, $R^{B3}$, $R^{C3}$, and $R^{E3}$ include each independently hydrogen, fluorine, or methyl.

$R^{E4}$ include hydrogen.

$R^{A5}$, $R^{B5}$, and $R^{C5}$ include each independently hydrogen, alkyl, or a group forming a prodrug.

Preferred embodiments of $R^{A5}$, $R^{B5}$, and $R^{C5}$ include each independently hydrogen, or a group forming a prodrug.

More preferred embodiments of $R^{A5}$, $R^{B5}$, and $R^{C5}$ include each independently hydrogen.

Other preferred embodiments of $R^{A5}$, $R^{B5}$, and $R^{C5}$ include each independently a group forming a prodrug.

$R^{A6}$, $R^{B6}$, and $R^{C6}$ include each independently hydrogen, hydroxy, alkylcarbonyl, or a group forming a prodrug.

Preferred embodiments of $R^{A6}$, $R^{B6}$, and $R^{C6}$ include each independently hydrogen, hydroxy, or alkylcarbonyl.

More preferred embodiments of $R^{A6}$, $R^{B6}$, and $R^{C6}$ include each independently hydrogen or hydroxy.

Furthermore preferred embodiments of $R^{A6}$, $R^{B6}$, and $R^{C6}$ include each independently hydrogen.

$R^1$ includes each independently hydrogen, a group forming a prodrug, or a group that is selected from the group consisting of the following:

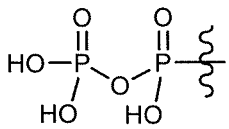
,
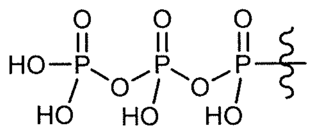

A preferred embodiment of $R^1$ includes each independently hydrogen.

Another preferred embodiment of $R^1$ includes each independently a group forming a prodrug.

$R^2$ includes each independently hydrogen, halogen, C1-C3 alkyl, C2-C3 alkenyl, C2-C3 alkynyl, halo C1-C3 alkyl, halo C2-C3 alkenyl, or halo C2-C3 alkynyl.

A preferred embodiment of $R^2$ includes each independently hydrogen.

$R^3$ includes each independently hydrogen, or a group forming a prodrug.

A preferred embodiment of $R^3$ includes each independently hydrogen.

$R^{4a}$ includes be hydrogen, hydroxy, or halogen,

A preferred embodiment of $R^{4a}$ includes hydroxy.

Another preferred embodiment of $R^{4a}$ includes hydroxy or halogen.

A more preferred embodiment of $R^{4a}$ includes hydroxy or fluorine.

$R^{4b}$ includes hydrogen, halogen, C1-C3 alkyl, C1-C3 haloalkyl, C2-C3 alkenyl, or C2-C3 alkynyl.

A preferred embodiment of $R^{4b}$ includes hydrogen.

Another preferred embodiment of $R^{4b}$ includes hydrogen or halogen.

A more preferred embodiment of $R^{4b}$ includes be hydrogen or fluorine.

$R^5$ of azasugar includes hydrogen, C1-C3 alkyl, C2-C3 alkenyl, or C2-C3 alkynyl.

A preferred embodiment of $R^5$ of azasugar includes hydrogen.

$R^5$ of sugar includes hydrogen or cyano.

A preferred embodiment of $R^5$ of sugar includes hydrogen.

Another preferred embodiment of $R^5$ of sugar includes cyano.

$R^6$ includes hydrogen, C1-C3 alkyl, or a group forming a prodrug.

A preferred embodiment of $R^6$ includes hydrogen or C1-C3 alkyl.

Another preferred embodiment of $R^6$ includes hydrogen, or a group forming a prodrug.

A group forming a prodrug can be formed in $R^1$, $R^3$, $R^6$, $R^{A5}$, $R^{B5}$, $R^{C5}$, $R^{A6}$, $R^{B6}$, Rcs.

In one embodiment, a group forming a prodrug can be formed only in $R^1$.

In one embodiment, a group forming a prodrug can be formed only in $R^6$.

In one embodiment, a group forming a prodrug can be formed only in $R^{A5}$, $R^{B5}$, or $R^{C5}$.

In another embodiment, a group forming a prodrug can be formed only in any one of $R^1$, $R^6$, $R^{A5}$, $R^{B5}$, and $R^{C5}$.

Examples of the group forming the prodrug of $R^1$ or $R^3$ include groups shown below:

a) $-C(=O)-P^{R0}$;
b) $-C(=O)-L-P^{R1}$;
c) $-C(=O)-L-O-P^{R0}$;
d) $-C(=O)-L-S-P^{R0}$;
e) $-C(=O)-L-N(-P^{R2})_2$;
f) $-C(=O)-C(P^{R3})_2-NH_2$;
g) $-C(=O)-O-P^{R0}$;
h) $-C(P^{R4})_2-O-C(=O)-P^{R5}$;
i) $-C(P^{R4})_2-O-C(=O)-L-P^{R6}$;
j) $-C(P^{R4})_2-O-C(=O)-O-P^{R5}$;

k) $-C(P^{R4})_2-O-C(=O)-O-L-P^{R6}$;
l) $-P(=O)(-OP^{R7})_2$;
m) $-P(=O)(-OP^{R7})-N(-P^{R8})_2$; and
n) $-P(=O)(-N(-P^{R8})_2)_2$,

wherein

L's are each independently a linear or branched alkylene,
$P^{R0}$s are each independently alkyl optionally substituted with substituent group A, a carbocyclyl optionally substituted with substituent group B, or a heterocyclyl optionally substituted with substituent group B,
$P^{R1}$s are each independently a carbocyclyl optionally substituted with substituent group B, or a heterocyclyl optionally substituted with substituent group B,
$P^{R2}$s are each independently alkyl optionally substituted with substituent group A, a carbocyclyl optionally substituted with substituent group B, or a heterocyclyl optionally substituted with substituent group B, or alternatively,
two $P^{R2}$s may be taken together with an adjacent atom to form a heterocycle optionally substituted with substituent group E,
$P^{R3}$s are each independently hydrogen or alkyl optionally substituted with substituent group C,
$P^{R4}$s are each independently hydrogen or alkyl,
$P^{R5}$s are each independently alkyl optionally substituted with substituent group A, a carbocyclyl optionally substituted with substituent group B, or a heterocyclyl optionally substituted with substituent group B,
$P^{R6}$s are each independently a carbocyclyl optionally substituted with substituent group B, or a heterocyclyl optionally substituted with a substituent group B,
$P^{R7}$s are each independently hydrogen, alkyl optionally substituted with substituent group D, phenyl optionally substituted with substituent group E, or naphthyl optionally substituted with substituent group E, or,
two $P^{R7}$s may be taken together with adjacent atoms to form a heterocycle optionally substituted with substituent group E, and
$P^{R8}$s are each independently hydrogen or alkyl optionally substituted with substituent group F,
wherein
the substituent group A: halogen,
the substituent group B: hydroxy, alkyl, and oxo,
the substituent group C: a carbocyclyl optionally substituted with substituent group G, a heterocyclyl optionally substituted with substituent group G, and alkylthio,
the substituent group D: a carbocyclyl optionally substituted with substituent group G, a heterocyclyl optionally substituted with substituent group G, a carbocyclylalkyloxy optionally substituted with substituent group G, alkyloxy, alkyloxycarbonyl, alkylcarbonyloxy, alkylthio, and alkylcarbonylthio,
the substituent group E: halogen, alkyl, and alkyloxy,
the substituent group F: a carbocyclyl, a heterocyclyl, halogen, and alkyloxycarbonyl, and
the substituent group G: halogen and cyano.

Preferred examples of the group forming the prodrug of $R^1$ or $R^3$ include groups shown below:

a) $-C(=O)-P^{R0}$;
b) $-C(=O)-L-P^{R1}$;
f) $-C(=O)-C(P^{R3})_2-NH_2$;
g) $-C(=O)-O-P^{R0}$;
i) $-C(P^{R4})_2-O-C(=O)-L-P^{R6}$;
l) $-P(=O)(-OP^{R7})_2$; and
m) $-P(=O)(-OP^{R7})-N(-P^{R8})_2$,
wherein
L's are each independently a linear or branched alkylene,
$P^{R0}$s are each independently alkyl, a carbocyclyl optionally substituted with substituent group B, or a heterocyclyl optionally substituted with substituent group B,
$P^{R1}$s are each independently a carbocyclyl optionally substituted with substituent group B, or a heterocyclyl optionally substituted with substituent group B,
$P^{R4}$s are each independently hydrogen or alkyl,
$P^{R6}$s are each independently a carbocyclyl optionally substituted with substituent group B, or a heterocyclyl optionally substituted with substituent group B,
$P^{R7}$s are each independently hydrogen, alkyl optionally substituted with substituent group D, or phenyl optionally

substituted with substituent group E, and
$P^{R8}$s are each independently hydrogen or alkyl optionally substituted with substituent group F,
wherein
the substituent group B: hydroxy, alkyl, and oxo,
the substituent group D: a carbocyclylalkyloxy optionally substituted with substituent group G, and alkyloxy,
the substituent group E: halogen, alkyl, and alkyloxy,
the substituent group F: alkyloxycarbonyl, and
the substituent group G: halogen and cyano.

Preferred examples of the group forming the prodrug of $R^1$ include groups shown below:

a) -C(=O)-$P^{R0}$; and
b) -C(=O)-L-$P^{R1}$,

wherein

L is a linear or branched alkylene,
$P^{R0}$ is alkyl, or a carbocyclyl optionally substituted with substituent group B, and
$P^{R1}$ is a carbocyclyl optionally substituted with substituent group B,
wherein the substituent group B: hydroxy, and alkyl.

Examples of the group forming the prodrugs of $R^{A5}$, $R^{B5}$, $R^{C5}$, $R^{A6}$, $R^{B6}$, $R^{C6}$, or $R^6$ include the following groups:

a) -C(=O)-$P^{R0}$;
g) -C(=O)-O-$P^{R0}$;
o) -C(=O)-O-L-$P^{R1}$;
p) -C(=O)-L-O-C(=O)-$P^{R0}$; and
q) -C($P^{R4}$)$_2$-$P^{R1}$,
wherein
L's are each independently a linear or branched alkylene,
$P^{R0}$s are each independently alkyl optionally substituted with substituent group A, a carbocyclyl optionally substituted with substituent group B, or a heterocyclyl optionally substituted with the substituent group B,
$P^{R1}$s are each independently a carbocyclyl optionally substituted with substituent group B, or a heterocyclyl optionally substituted with substituent group B, and
$P^{R4}$s are each independently hydrogen or alkyl,
wherein
the substituent group A: halogen, and
the substituent group B: hydroxy, alkyl, and oxo.

Preferred examples of the group forming the prodrugs of $R^{A5}$, $R^{B5}$, $R^{C5}$, $R^{A6}$, $R^{B6}$, $R^{C6}$, or $R^6$ include the following group:

a) -C(=O)-$P^{R0}$,

wherein $P^{R0}$s are each independently alkyl.
The preferred compound represented by Formula (I) is a compound selected from the group consisting of those represented by the following formulae:

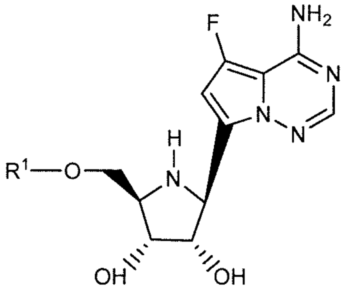
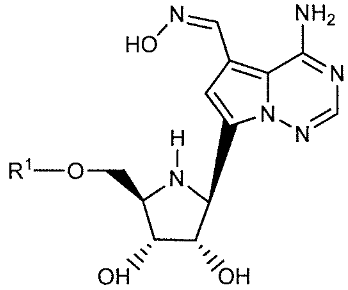
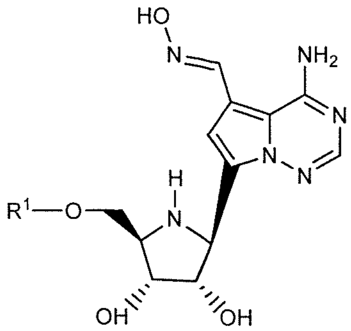

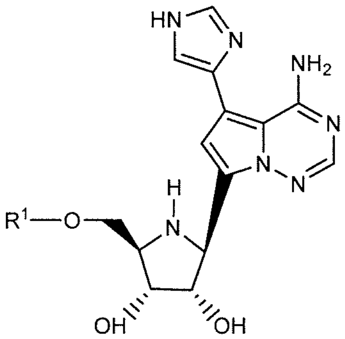
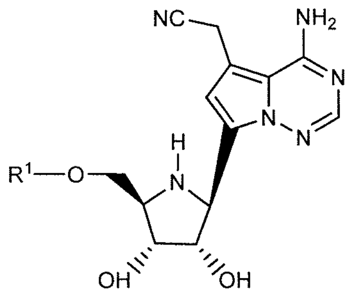
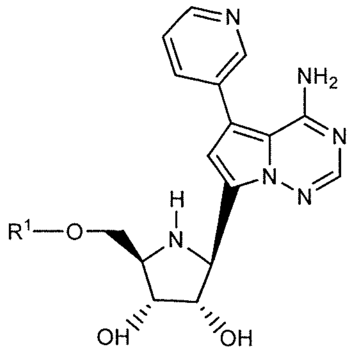

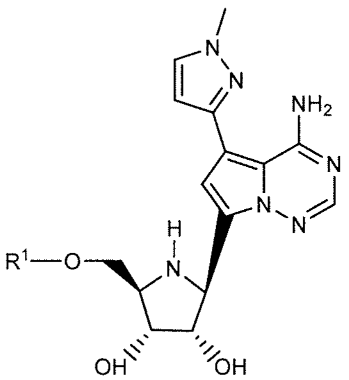
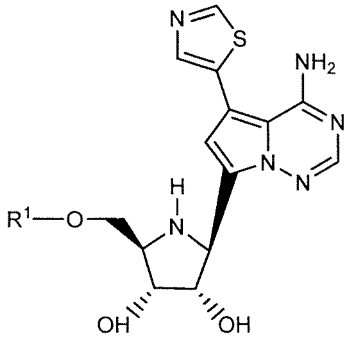
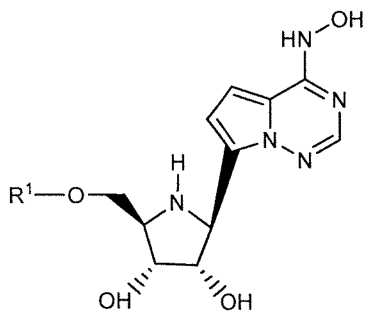

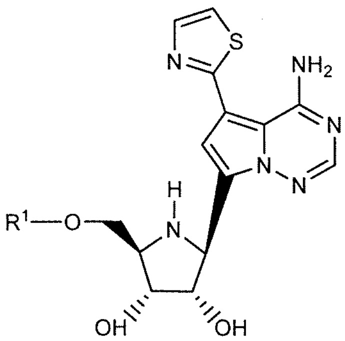
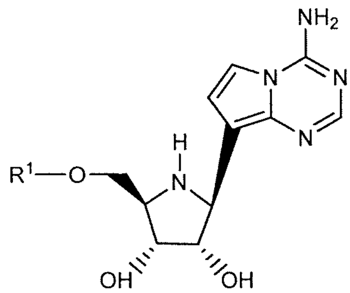
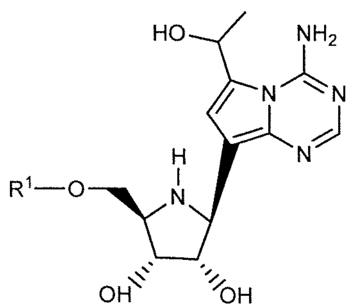

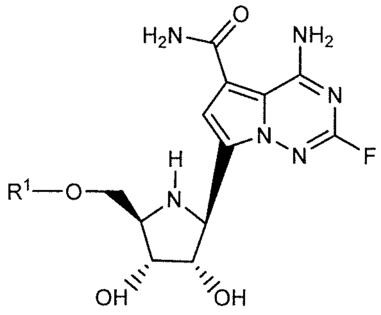
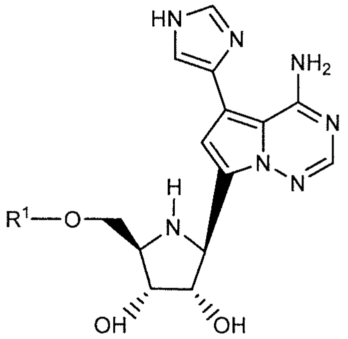
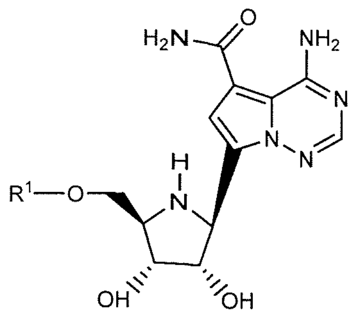

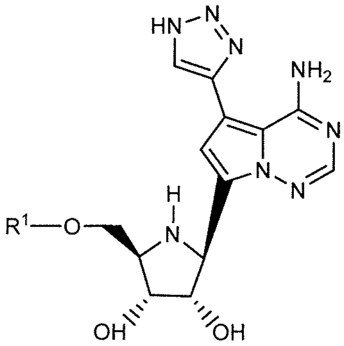
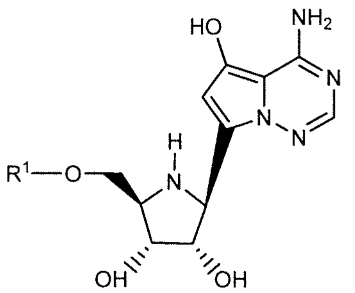
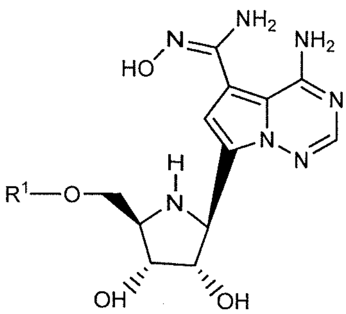

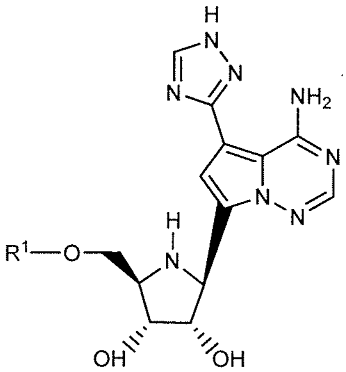
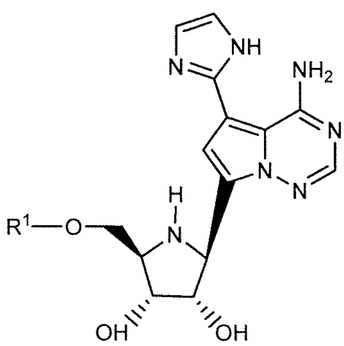
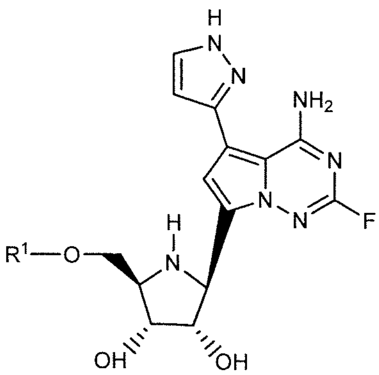

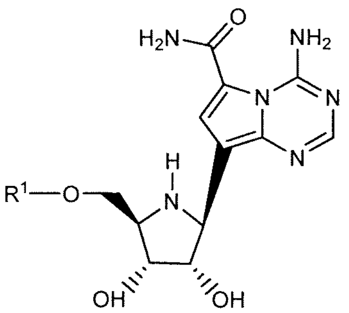
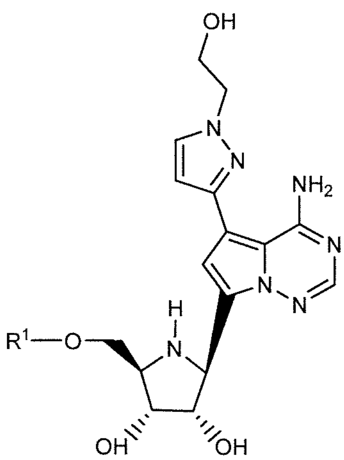
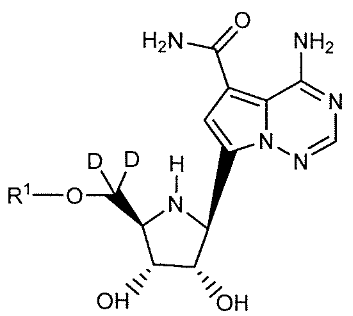

wherein $R^1$ is hydrogen, a group forming prodrug, or a group selected from the group consisting of the following:

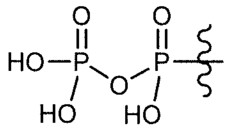
,
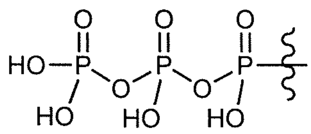

, or a pharmaceutically acceptable salt thereof.

More preferably, $R^1$ is hydrogen or a group selected from those represented by the following formulae:

a) $-C(=O)-P^{R0}$;
b) $-C(=O)-L-P^{R1}$;
c) $-C(=O)-L-O-P^{R0}$;
d) $-C(=O)-L-S-P^{R0}$;
e) $-C(=O)-L-N(-P^{R2})_2$;
f) $-C(=O)-C(P^{R3})_2-NH_2$;
g) $-C(=O)-O-P^{R0}$;
h) $-C(P^{R4})_2-O-C(=O)-P^{R5}$;
i) $-C(P^{R4})_2-O-C(=O)-L-P^{R6}$;
j) $-C(P^{R4})_2-O-C(=O)-O-P^{R5}$;
k) $-C(P^{R4})_2-O-C(=O)-O-L-P^{R6}$;
l) $-P(=O)(-OP^{R7})_2$;
m) $-P(=O)(-OP^{R7})-N(-P^{R8})_2$;
n) $-P(=O)(-N(-P^{R8})_2)_2$; and

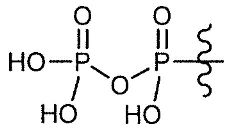
,
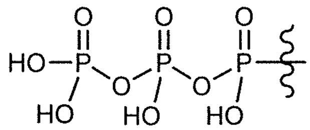

wherein

L's are each independently a linear or branched alkylene,
$P^{R0}$s are each independently alkyl optionally substituted with substituent group A, a carbocyclyl optionally substituted with substituent group B, or a heterocyclyl optionally substituted with substituent group B,
$P^{R1}$s are each independently a carbocyclyl optionally substituted with substituent group B, or a heterocyclyl optionally substituted with substituent group B,
$P^{R2}$s are each independently alkyl optionally substituted with substituent group A, a carbocyclyl optionally substituted with substituent group B, or a heterocyclyl optionally substituted with substituent group B,
or alternatively,
two $P^{R2}$s may be taken together with an adjacent atom to form a heterocycle optionally substituted with substituent group E,
$P^{R3}$s are each independently hydrogen or alkyl optionally substituted with substituent group C,
$P^{R4}$s are each independently hydrogen or alkyl,
$P^{R5}$s are each independently alkyl optionally substituted with substituent group A, a carbocyclyl optionally substituted with substituent group B, or a heterocyclyl optionally substituted with substituent group B,
$P^{R6}$s are each independently a carbocyclyl optionally substituted with substituent group B, or a heterocyclyl optionally substituted with substituent group B,
$P^{R7}$s are each independently hydrogen, alkyl optionally substituted with substituent group D, phenyl optionally substituted with substituent group E, or naphthyl optionally substituted with substituent group E,
or alternatively,
two $P^{R7}$s may be taken together with adjacent atoms to form a heterocycle optionally substituted with substituent group E, and
$P^{R8}$s are each independently hydrogen or alkyl optionally substituted with substituent group F,
wherein
the substituent group A: halogen,
the substituent group B: hydroxy, alkyl, and oxo,
the substituent group C: a carbocyclyl optionally substituted with substituent group G, a heterocyclyl optionally

substituted with substituent group G, and alkylthio,

the substituent group D: a carbocyclyl optionally substituted with substituent group G, a heterocyclyl optionally substituted with substituent group G, a carbocyclylalkyloxy optionally substituted with substituent group G, alkyloxy, alkyloxycarbonyl, alkylcarbonyloxy, alkylthio, and alkylcarbonylthio,

the substituent group E: halogen, alkyl, and alkyloxy,

the substituent group F: a carbocyclyl, a heterocyclyl, halogen, and alkyloxycarbonyl, and

the substituent group G: halogen and cyano.

Furthermore preferably, the above-described compound or pharmaceutically acceptable salt thereof is such that $R^1$ is hydrogen or a group selected from those represented by the following formulae:

a) $-C(=O)-P^{R0}$;
b) $-C(=O)-L-P^{R1}$;
f) $-C(=O)-C(P^{R3})_2-NH_2$;
g) $-C(=O)-O-P^{R0}$;
i) $-C(P^{R4})_2-O-C(=O)-L-P^{R6}$;
l) $-P(=O)(-OP^{R7})_2$; and
m) $-P(=O)(-OP^{R7})-N(-P^{R8})_2$; and

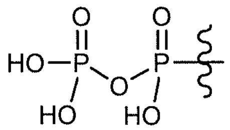
,
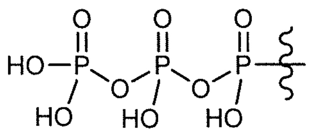

wherein

L's are each independently a linear or branched alkylene,

$P^{R0}$s are each independently alkyl, a carbocyclyl optionally substituted with substituent group B, or a heterocyclyl optionally substituted with substituent group B,

$P^{R1}$s are each independently a carbocyclyl optionally substituted with substituent group B, or a heterocyclyl optionally substituted with substituent group B,

$P^{R4}$s are each independently hydrogen or alkyl,

$P^{R6}$s are each independently a carbocyclyl optionally substituted with substituent group B, or a heterocyclyl optionally substituted with substituent group B,

$P^{R7}$s are each independently hydrogen, alkyl optionally substituted with substituent group D, or phenyl optionally substituted with substituent group E, and

$P^{R8}$s are each independently hydrogen or alkyl optionally substituted with substituent group F,

wherein

the substituent group B: hydroxy, alkyl, and oxo,

the substituent group D: a carbocyclylalkyloxy optionally substituted with substituent group G, and alkyloxy,

the substituent group E: halogen, alkyl, and alkyloxy;

the substituent group F: alkyloxycarbonyl, and

the substituent group G: halogen and cyano.

Most preferably, the above-described compound or pharmaceutically acceptable salt thereof is such that $R^1$ is hydrogen or a group selected from those represented by the following formulae:

a) $-C(=O)-P^{R0}$;
b) $-C(=O)-L-P^{R1}$; and

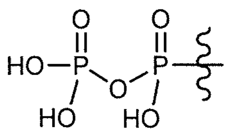
,
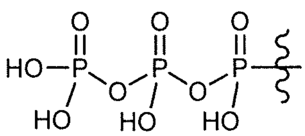

wherein

L is a linear or branched alkylene,
$P^{R0}$ is alkyl, or a carbocyclyl optionally substituted with substituent group B, and
$P^{R1}$ is a carbocyclyl optionally substituted with substituent group B,
wherein the substituent group B: hydroxy and alkyl.

Another preferred compound represented by Formula (I) is a compound selected from the group consisting of those represented by the following formulae, or a pharmaceutically acceptable salt thereof:

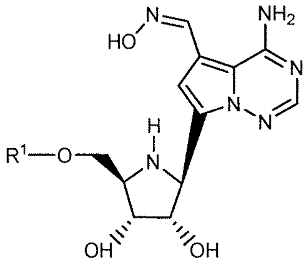
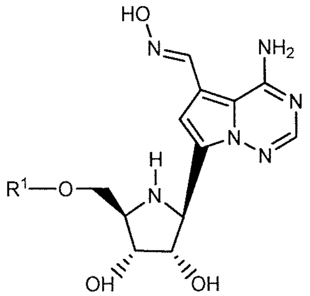
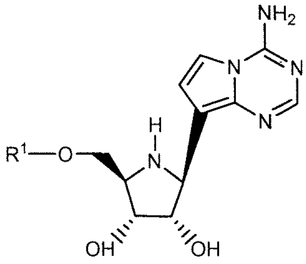
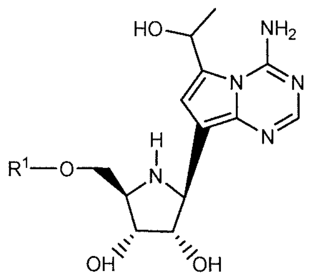

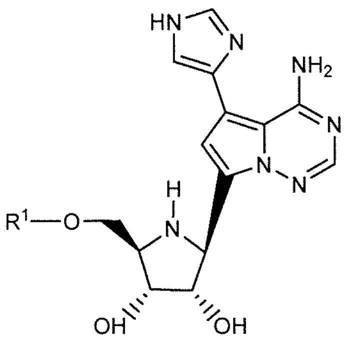
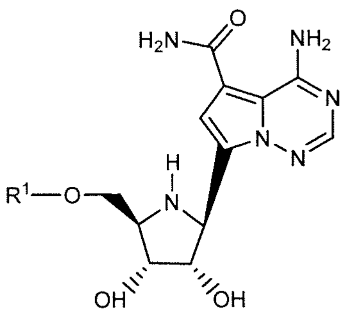
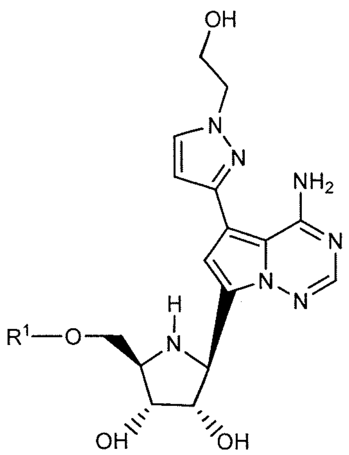

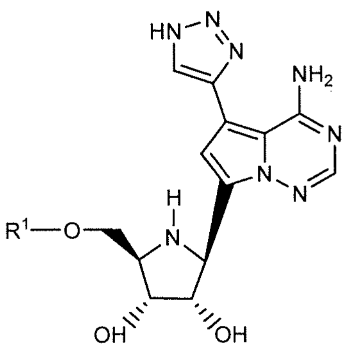
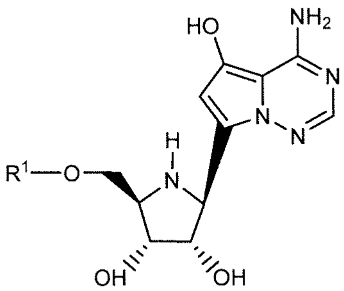
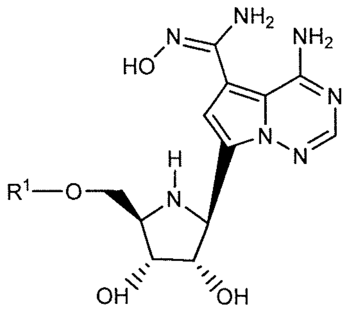

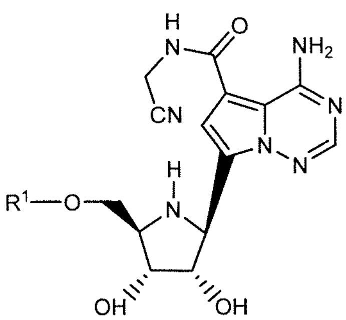
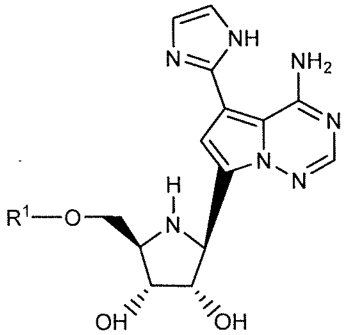
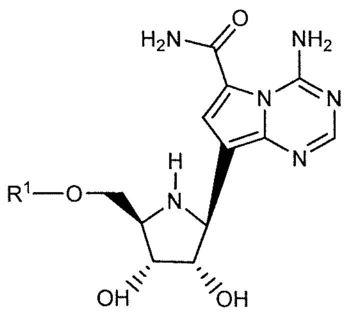

wherein $R^1$ is hydrogen, a group forming prodrug, or a group selected from the group consisting of the following:

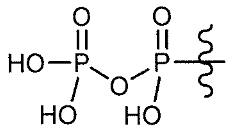 , 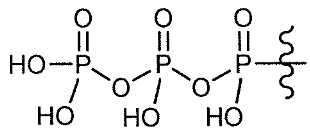

More preferably, $R^1$ is hydrogen or a group selected from those represented by the following formulae:

a) $-C(=O)-P^{R0}$;
b) $-C(=O)-L-P^{R1}$;
c) $-C(=O)-L-O-P^{R0}$;
d) $-C(=O)-L-S-P^{R0}$;
e) $-C(=O)-L-N(-P^{R2})_2$;
f) $-C(=O)-C(P^{R3})_2-NH_2$;
g) $-C(=O)-O-P^{R0}$;
h) $-C(P^{R4})_2-O-C(=O)-P^{R5}$;
i) $-C(P^{R4})_2-O-C(=O)-L-P^{R6}$;
j) $-C(P^{R4})_2-O-C(=O)-O-P^{R5}$;
k) $-C(P^{R4})_2-O-C(=O)-O-L-P^{R6}$;
l) $-P(=O)(-OP^{R7})_2$;
m) $-P(=O)(-OP^{R7})-N(-P^{R8})_2$;
n) $-P(=O)(-N(-P^{R8})_2)_2$; and

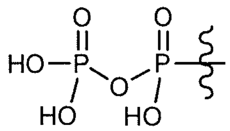 , 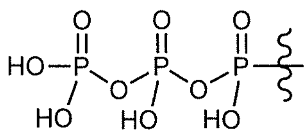

wherein

L's are each independently a linear or branched alkylene,
$P^{R0}$s are each independently alkyl optionally substituted with substituent group A, a carbocyclyl optionally substituted with substituent group B, or a heterocyclyl optionally substituted with substituent group B,
$P^{R1}$s are each independently a carbocyclyl optionally substituted with substituent group B, or a heterocyclyl optionally substituted with substituent group B,
$P^{R2}$s are each independently alkyl optionally substituted with substituent group A, a carbocyclyl optionally substituted with substituent group B, or a heterocyclyl optionally substituted with substituent group B,
or alternatively,
two $P^{R2}$s may be taken together with an adjacent atom to form a heterocycle optionally substituted with substituent group E,
$P^{R3}$s are each independently hydrogen or alkyl optionally substituted with substituent group C,
$P^{R4}$s are each independently hydrogen or alkyl,
$P^{R5}$s are each independently alkyl optionally substituted with substituent group A, a carbocyclyl optionally substituted with substituent group B, or a heterocyclyl optionally substituted with substituent group B,
$P^{R6}$s are each independently a carbocyclyl optionally substituted with substituent group B, or a heterocyclyl optionally substituted with substituent group B,
$P^{R7}$s are each independently hydrogen, alkyl optionally substituted with substituent group D, phenyl optionally substituted with substituent group E, or naphthyl optionally substituted with substituent group E,
or alternatively,
two $P^{R7}$s may be taken together with adjacent atoms to form a heterocycle optionally substituted with substituent group E, and
$P^{R8}$s are each independently hydrogen or alkyl optionally substituted with substituent group F,
wherein
the substituent group A: halogen,
the substituent group B: hydroxy, alkyl, and oxo,

the substituent group C: a carbocyclyl optionally substituted with substituent group G, a heterocyclyl optionally substituted with substituent group G, and alkylthio,

the substituent group D: a carbocyclyl optionally substituted with substituent group G, a heterocyclyl optionally substituted with substituent group G, a carbocyclylalkyloxy optionally substituted with substituent group G, alkyloxy, alkyloxycarbonyl, alkylcarbonyloxy, alkylthio, and alkylcarbonylthio,

the substituent group E: halogen, alkyl, and alkyloxy,

the substituent group F: a carbocyclyl, a heterocyclyl, halogen, and alkyloxycarbonyl, and

the substituent group G: halogen and cyano.

Furthermore preferably, the above-described compound or pharmaceutically acceptable salt thereof is such that $R^1$ is hydrogen or a group selected from those represented by the following formulae:

a) $-C(=O)-P^{R0}$;

b) $-C(=O)-L-P^{R1}$;

f) $-C(=O)-C(P^{R3})_2-NH_2$;

g) $-C(=O)-O-P^{R0}$;

i) $-C(P^{R4})_2-O-C(=O)-L-P^{R6}$;

l) $-P(=O)(-OP^{R7})_2$;

m) $-P(=O)(-OP^{R7})-N(-P^{R8})_2$; and

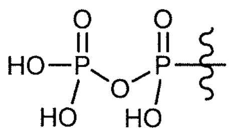
,
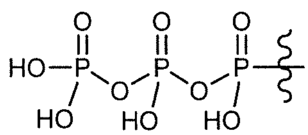

wherein

L's are each independently a linear or branched alkylene,

$P^{R0}$s are each independently alkyl, a carbocyclyl optionally substituted with substituent group B, or a heterocyclyl optionally substituted with substituent group B,

$P^{R1}$s are each independently a carbocyclyl optionally substituted with substituent group B, or a heterocyclyl optionally substituted with substituent group B,

$P^{R4}$s are each independently hydrogen or alkyl,

$P^{R6}$s are each independently a carbocyclyl optionally substituted with substituent group B, or a heterocyclyl optionally substituted with substituent group B,

$P^{R7}$s are each independently hydrogen, alkyl optionally substituted with substituent group D, or phenyl optionally substituted with substituent group E, and

$P^{R8}$s are each independently hydrogen or alkyl optionally substituted with substituent group F,

wherein

the substituent group B: hydroxy, alkyl, and oxo,

the substituent group D: a carbocyclylalkyloxy optionally substituted with substituent group G, and alkyloxy,

the substituent group E: halogen, alkyl, and alkyloxy;

the substituent group F: alkyloxycarbonyl, and

the substituent group G: halogen and cyano.

Most preferably, the above-described compound or pharmaceutically acceptable salt thereof is such that $R^1$ is hydrogen or a group selected from those represented by the following formulae:

a) $-C(=O)-P^{R0}$;

b) $-C(=O)-L-P^{R1}$; and

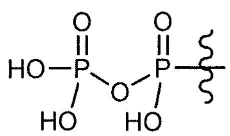
,
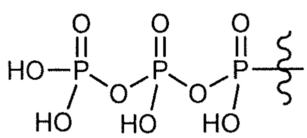

wherein

L is a linear or branched alkylene,
$P^{R0}$ is alkyl, or a carbocyclyl optionally substituted with substituent group B, and
$P^{R1}$ is a carbocyclyl optionally substituted with substituent group B,
wherein the substituent group B: hydroxy and alkyl.

The preferred compound represented by Formula (II) is a compound selected from the group consisting of those represented by the following formulae, or a pharmaceutically acceptable salt thereof:

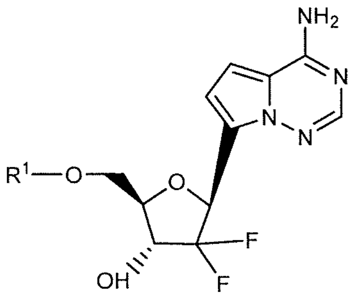
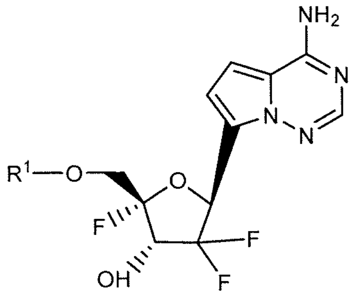
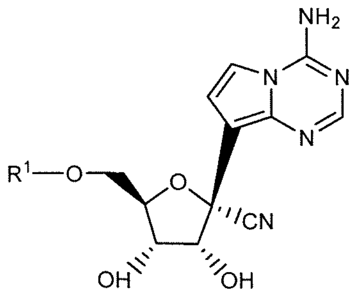
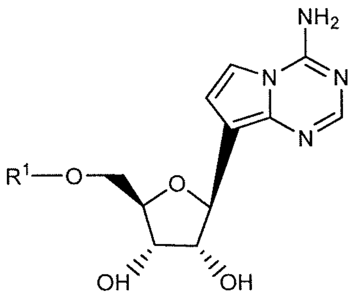
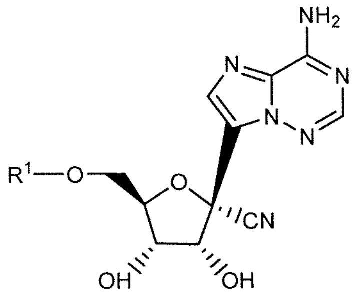
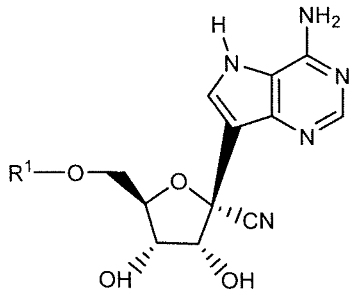
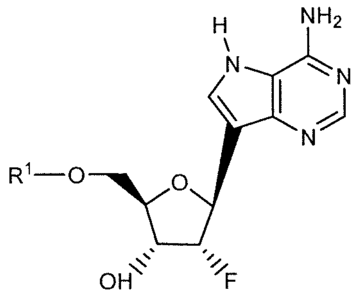

wherein $R^1$ is hydrogen, a group forming a prodrug, or a group selected from the group consisting of the following:

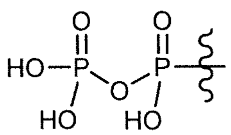 , 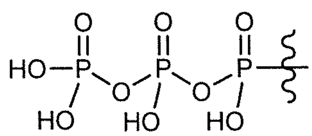 .

More preferably, $R^1$ is hydrogen or a group selected from those represented by the following formulae:

a) $-C(=O)-P^{R0}$;
b) $-C(=O)-L-P^{R1}$;
c) $-C(=O)-L-O-P^{R0}$;
d) $-C(=O)-L-S-P^{R0}$;
e) $-C(=O)-L-N(-P^{R2})_2$;

f) $-C(=O)-C(P^{R3})_2-NH_2$;
g) $-C(=O)-O-P^{R0}$;
h) $-C(P^{R4})_2-O-C(=O)-P^{R5}$;
i) $-C(P^{R4})_2-O-C(=O)-L-P^{R6}$;
j) $-C(P^{R4})_2-O-C(=O)-O-P^{R5}$;
k) $-C(P^{R4})_2-O-C(=O)-O-L-P^{R6}$;
l) $-P(=O)(-OP^{R7})_2$;
m) $-P(=O)(-OP^{R7})-N(-P^{R8})_2$;
n) $-P(=O)(-N(-P^{R8})_2)_2$; and

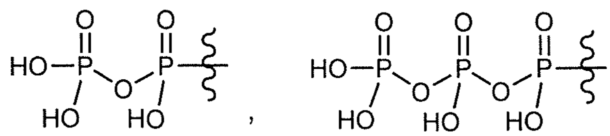

wherein

L's are each independently a linear or branched alkylene,
$P^{R0}$s are each independently alkyl optionally substituted with substituent group A, a carbocyclyl optionally substituted with substituent group B, or a heterocyclyl optionally substituted with substituent group B,
$P^{R1}$s are each independently a carbocyclyl optionally substituted with substituent group B, or a heterocyclyl optionally substituted with substituent group B,
$P^{R2}$s are each independently alkyl optionally substituted with substituent group A, a carbocyclyl optionally substituted with substituent group B, or a heterocyclyl optionally substituted with substituent group B,
or alternatively,
two $P^{R2}$s may be taken together with an adjacent atom to form a heterocycle optionally substituted with substituent group E,
$P^{R3}$s are each independently hydrogen or alkyl optionally substituted with substituent group C,
$P^{R4}$s are each independently hydrogen or alkyl,
$P^{R5}$s are each independently alkyl optionally substituted with substituent group A, a carbocyclyl optionally substituted with substituent group B, or a heterocyclyl optionally substituted with the substituent group B,
$P^{R6}$s are each independently a carbocyclyl optionally substituted with substituent group B, or a heterocyclyl optionally substituted with substituent group B,
$P^{R7}$s are each independently hydrogen, alkyl optionally substituted with substituent group D, phenyl optionally substituted with substituent group E, or naphthyl optionally substituted with substituent group E,
or alternatively,
two $P^{R7}$s may be taken together with adjacent atoms to form a heterocycle optionally substituted with substituent group E, and
$P^{R8}$s are each independently hydrogen or alkyl optionally substituted with substituent group F,
wherein
the substituent group A: halogen,
the substituent group B: hydroxy, alkyl, and oxo,
the substituent group C: a carbocyclyl optionally substituted with substituent group G, a heterocyclyl optionally substituted with substituent group G, and alkylthio,
the substituent group D: a carbocyclyl optionally substituted with substituent group G, a heterocyclyl optionally substituted with substituent group G, a carbocyclylalkyloxy optionally substituted with substituent group G, alkyloxy, alkyloxycarbonyl, alkylcarbonyloxy, alkylthio, and alkylcarbonylthio,
the substituent group E: halogen, alkyl, and alkyloxy,
the substituent group F: a carbocyclyl, a heterocyclyl, halogen, and alkyloxycarbonyl, and
the substituent group G: halogen and cyano.

Furthermore preferably, the above-described compound or pharmaceutically acceptable salt thereof is such that $R^1$ is hydrogen or a group selected from those represented by the following formulae:

a) $-C(=O)-P^{R0}$;
b) $-C(=O)-L-P^{R1}$;
f) $-C(=O)-C(P^{R3})_2-NH_2$;

g) -C(=O)-O-$P^{R0}$;
i) -C($P^{R4}$)$_2$-O-C(=O)-L-$P^{R6}$;
1) -P(=O)(-O$P^{R7}$)$_2$;
m) -P(=O)(-O$P^{R7}$)-N(-$P^{R8}$)$_2$; and

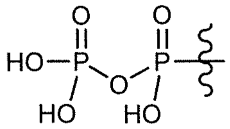 , 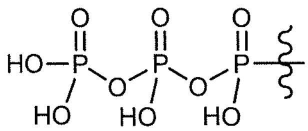

wherein

L's are each independently a linear or branched alkylene,
$P^{R0}$s are each independently alkyl, a carbocyclyl optionally substituted with substituent group B, or a heterocyclyl optionally substituted with substituent group B,
$P^{R1}$s are each independently a carbocyclyl optionally substituted with substituent group B, or a heterocyclyl optionally substituted with substituent group
B, $P^{R4}$s are each independently hydrogen or alkyl,
$P^{R6}$s are each independently a carbocyclyl optionally substituted with substituent group B, or a heterocyclyl optionally substituted with substituent group B,
$P^{R7}$s are each independently hydrogen, alkyl optionally substituted with substituent group D, or phenyl optionally substituted with substituent group E, and
$P^{R8}$s are each independently hydrogen or alkyl optionally substituted with substituent group F,
wherein
the substituent group B: hydroxy, alkyl, and oxo,
the substituent group D: a carbocyclylalkyloxy optionally substituted with substituent group G, and alkyloxy,
the substituent group E: halogen, alkyl, and alkyloxy;
the substituent group F: alkyloxycarbonyl, and
the substituent group G: halogen and cyano.

Most preferably, the above-described compound or pharmaceutically acceptable salt thereof is such that $R^1$ is hydrogen or a group selected from those represented by the following formulae:

a) -C(=O)-$P^{R0}$;
b) -C(=O)-L-$P^{R1}$; and

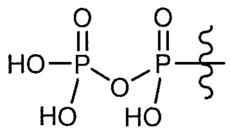 , 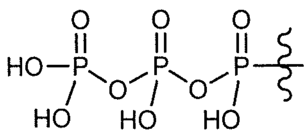

wherein

L is a linear or branched alkylene,
$P^{R0}$ is alkyl, or a carbocyclyl optionally substituted with substituent group B, and
$P^{R1}$ is a carbocyclyl optionally substituted with substituent group B,
wherein the substituent group B: hydroxy and alkyl.

The compounds represented by Formula (I) or (II) are not limited to specific isomers but include all possible isomers (e.g., keto-enol isomers, imine-enamine isomers, diastereoisomers, enantiomers, rotamers or the like), racemates or mixtures thereof.

One or more hydrogen atom, carbon atom and/or another atom of the compound represented by Formula (I) or (II) may be replaced with an isotope of the hydrogen atom, carbon atom and/or another atom. Examples of such isotopes include hydrogen, carbon, nitrogen, oxygen, phosphorus, sulfur, fluorine, iodine, and chlorine, as in the cases of $^{2}H$, $^{3}H$, $^{11}C$, $^{13}C$, $^{14}C$, $^{15}N$, $^{18}O$, $^{17}O$, $^{31}P$, $^{32}P$, $^{35}S$, $^{18}F$, $^{123}I$, and $^{36}Cl$, respectively. The compounds represented by Formula (I) or (II) include the compounds replaced with these isotopes. The compounds substituted with the isotopes

are also useful as pharmaceutical products and include all radiolabeled forms of the compounds represented by Formula (I) or (II). Furthermore, a "radiolabeling method" for producing the "radiolabeled forms" is also included in the present invention, and the "radiolabeled forms" are useful as tools for metabolic pharmacokinetics studies, studies on binding assay, and/or diagnostics.

A radiolabeled compound of the compounds represented by Formula (I) or (II) can be prepared using well-known methods in the art. For example, a tritium-labeled compound represented by Formula (I) or (II) can be prepared by introducing a tritium to a certain compound represented by Formula (I) or (II), through a catalytic dehalogenation reaction using a tritium. This method comprises reacting an appropriately-halogenated precursor of the compound represented by Formula (I) or (II) with tritium gas in the presence of an appropriate catalyst, such as Pd/C, and in the presence or absence of a base. Regarding other appropriate methods for preparing tritium-labeled compounds, "Isotopes in the Physical and Biomedical Sciences, Vol. 1, Labeled Compounds (Part A), Chapter 6 (1987)" can be referred to. A $^{14}$C-labeled compound can be prepared by using a raw material having $^{14}$C carbon.

Examples of the pharmaceutically acceptable salt of the compound represented by Formula (I) or (II) include salts of the compound represented by Formula (I) or (II) with alkali metal (for example, lithium, sodium, potassium, etc.), alkaline earth metal (for example, calcium, barium, etc.), magnesium, transition metal (for example, zinc, iron, etc.), ammonia, organic base (for example, trimethylamine, triethylamine, dicyclohexylamine, ethanolamine, diethanolamine, triethanolamine, meglumine, ethylenediamine, pyridine, picoline, quinoline, etc.) and amino acid or salts of the compound represented by Formula (I) with inorganic acid (for example, hydrochloric acid, sulfuric acid, nitric acid, carbonic acid, hydrobromic acid, phosphoric acid, hydroiodic acid, etc.), and organic acid (for example, formic acid, acetic acid, propionic acid, trifluoroacetic acid, citric acid, lactic acid, tartaric acid, oxalic acid, maleic acid, fumaric acid, succinic acid, mandelic acid, glutaric acid, malic acid, benzoic acid, phthalic acid, ascorbic acid, benzenesulfonic acid, p-toluenesulfonic acid, methanesulfonic acid, ethanesulfonic acid, trifluoroacetic acid, etc.). These salts can be formed according to methods that are conventionally carried out.

The compounds represented by Formula (I) or (II) or pharmaceutically acceptable salts thereof according to the present invention may form solvates (e.g., hydrates or the like), cocrystals and/or crystal polymorphs. The present invention encompasses those various solvates, cocrystals and crystal polymorphs. "Solvates" may be those wherein any numbers of solvent molecules (e.g., water molecules or the like) are coordinated with the compounds represented by Formula (I) or (II). When the compound represented by Formula (I) or (II) or a pharmaceutically acceptable salt thereof is left to stand in the atmosphere, moisture is absorbed, and adsorbed water may adhere thereto, or a hydrate may be formed. Recrystallization of the compounds represented by Formula (I) or (II) or pharmaceutically acceptable salts thereof may produce crystal polymorphs. The "Co-crystal" means that a compound represented by Formula (I) or (II), or a salt thereof and a counter molecule exist in the same crystal lattice, and it can include any number of counter molecules.

The compounds of the present invention have an RNA viral growth inhibitory action, and therefore, are useful as a therapeutic and/or preventive agent for diseases associated with the RNA virus. When the term "therapeutic agent and/or prophylactic agent" is used in the present invention, this also encompasses a symptom ameliorating agent. Examples of the disease associated with the RNA virus include viral infectious diseases.

In one embodiment, examples of the RNA virus include those of Orthomyxoviridae, Paramyxoviridae, Arenaviridae, Bunyaviridae, Flaviviviridae, Filoviridae, Togaviridae, Picornaviridae, and Coronaviridae.

As one embodiment, examples of the RNA virus include rhinovirus, hepatitis A virus, hepatitis C virus, poliovirus, measles virus, ebolavirus, coxsackie virus, West Nile virus, yellow fever virus, dengue virus, influenza A virus, influenza B virus, Lassa fever virus, lymphocytic choriomeningitis virus, Junin virus, machupo virus, guanarito virus, hantavirus, Rift Valley fever virus, La Crosse virus, California encephalitis virus, Crimean-Congo virus, Marburg virus, Japanese encephalitis virus, Kyasanur forest disease virus, Venezuelan equine encephalitis virus, Eastern equine encephalitis virus, Western equine encephalitis virus, Severe Acute Respiratory Syndrome (SARS) virus, parainfluenza virus, respiratory syncytial virus, puntatrovirus, tacaribe virus, pichinde virus, and coronavirus.

In one embodiment, examples of the RNA virus include influenza A virus, influenza B virus, and coronavirus.

(Method for producing compound of present invention)

The compounds represented by Formula (I) or (II) can be produced by, for example, the general synthesis method described below. Regarding extraction, purification, and the like, the treatments carried out in ordinary experiments of organic chemistry may be carried out.

The compounds of the present invention can be produced with reference to techniques known in the art.

Furthermore, abbreviations used in the present specification denote the following meanings.

Bn: benzyl
Boc: tert-butoxycarbonyl

Bz: benzoyl
DIEA: N,N-diisopropylethylamine
DMAP: 4-dimethylaminopyridine
DMB: 2,4-dimethoxybenzyl
DMF: dimethylformamide
Et: ethyl
HATU: O-(7-azabenzotriazol-1-yl)-N,N,N',N'-tetramethyluronium hexafluorophosphate
Me: methyl
$PdCl_2$(dppf): [1,1'-bis(diphenylphosphino)ferrocene]palladium (II) dichloride dichloromethane adduct
$PdCl_2(d_{tb}pf)$: [1,1'-bis(di-tert-butylphosphino)ferrocene]palladium (II) dichloride
$Pd(OH)_2$: palladium hydroxide
$Pd(PPh_3)_4$: tetrakis(triphenylphosphine)palladium
Ph: phenyl
SPhos Pd G3:. (2-dicyclohexylphosphino-2',6'-dimethoxybiphenyl)[2-(2'-amino-1,1'-biphenyl)]palladium (II) methanesulfonic acid
TBAF: tetrabutylammonium fluoride
TBDPS: tert-butyldiphenylsilyl
TBS: tert-butyldimethylsilyl
t-Bu: tert-butyl
TFA: trifluoroacetic acid
THF: tetrahydrofuran
TMS: trimethylsilyl
Ts: tosyl
M: mol/L
MMTr: p-methoxyphenyl diphenylmethyl
μM: μmol/L
nM: nmol/L
Incidentally, "wedge form" and "broken line" indicate the absolute configuration.

(Process 1)

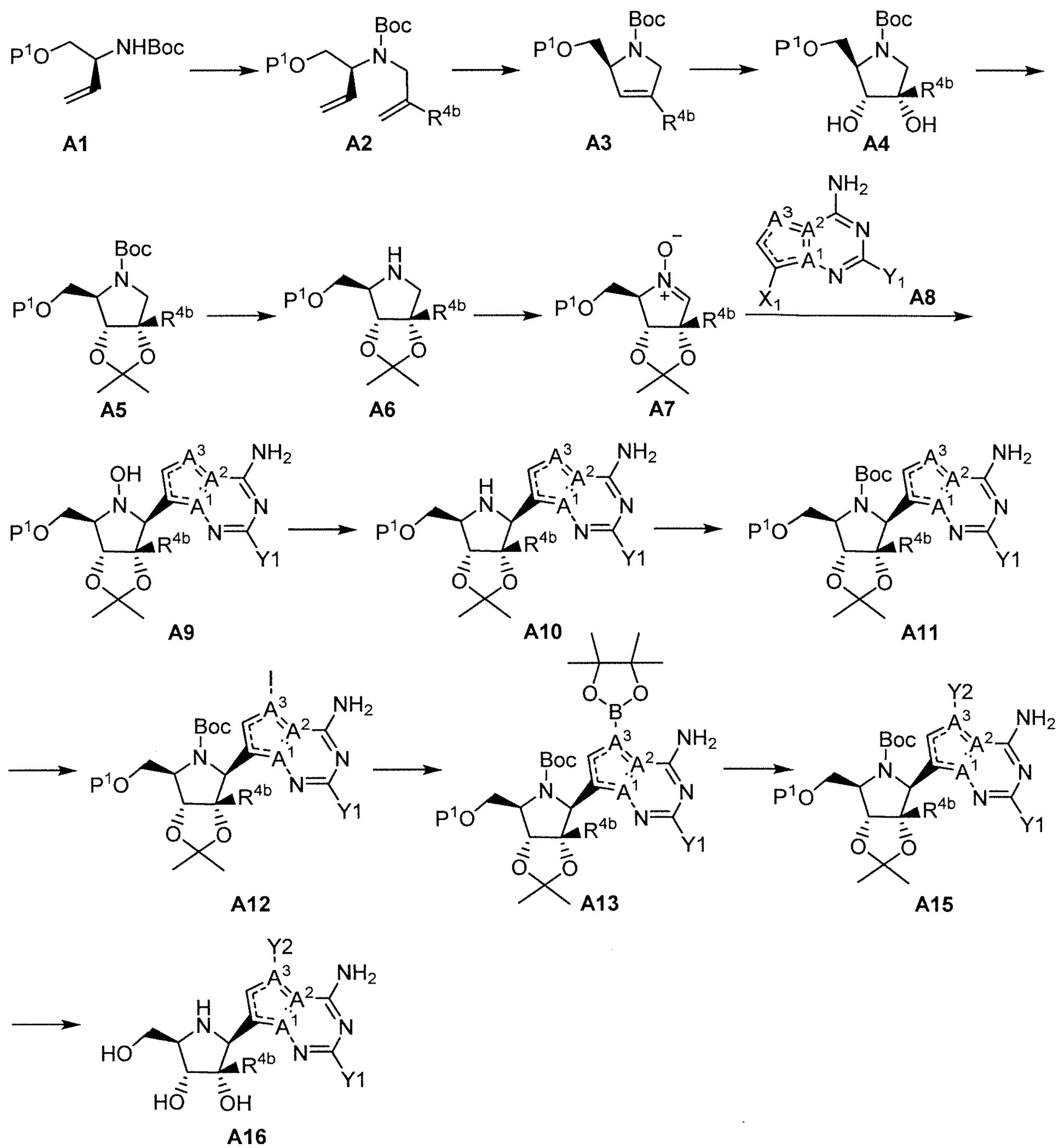

A1 A2 A3 A4

A5 A6 A7 A8

A9 A10 A11

A12 A13 A15

A16

wherein $P^1$ is a protecting group for OH; X1 is halogen; $A^1$ is carbon or nitrogen.; $A^2$ is carbon or nitrogen.; $A^3$ is carbon or nitrogen.; Y1 is $R^{A3}$, $R^{B3}$, or $R^{C3}$; Y2 is $R^{A1}$, or $R^{B1}$; other symbols are the same as those described above.)

Step 1

Compound A2 can be obtained by reacting Compound A1 with a base and allyl halide in the presence or absence of a catalyst.

Examples of the base include potassium tert-butoxide, sodium tert-butoxide, sodium hydride, sodium hydroxide, potassium hydroxide, potassium carbonate, and cesium carbonate, and the base can be used at 1 to 5 molar equivalents with respect to Compound A1.

Examples of the catalyst include tetrabutylammonium iodide, potassium iodide, and sodium iodide, and the catalyst can be used at 0.01 to 1 molar equivalents with respect to Compound A1 can be used.

The reaction temperature is -30°C to the reflux temperature of the solvent preferably 0°C to 40°C.

The reaction time is 0.5 to 72 hours, preferably 1 to 12 hours.

Examples of the reaction solvent include toluene, benzene, THF, dioxane, and DMF, and one of or a mixture of these can be used.

Step 2

Compound A3 can be obtained by reacting Compound A2 in the presence of a metal alkylidene complex catalyst.
The metal alkylidene complex catalyst is, for example, Grubbs first generation Grubbs second generation, or Schrock catalyst, and the catalyst can be used at 0.0001 to 1 molar equivalents with respect to compound A2.
The reaction temperature is 0°C to the reflux temperature of the solvent, preferably 0°C to 40°C.
The reaction time is 0.5 to 72 hours, preferably 1 to 24 hours.
Examples of the reaction solvent include dichloromethane, chloroform, toluene THF, dioxane, and DMF, and one of or a mixture of these can be used.

Step 3

Compound A4 can be obtained by reacting Compound A3 with osmium catalyst in the presence of an oxidizing agent.
Examples of the osmium catalyst include osmium tetroxide and potassium osmium (VI) acid dihydrate, and the catalyst can be used at 0.0001 to 1 molar equivalents of the same with respect to the compound A3.
Examples of the oxidizing agent include N-methylmorpholine oxide, trimethylamine oxide, and tert-butyl hydroperoxide, and the agent can be used at 2 to 10 molar equivalents of the same with respect to Compound A3.
The reaction temperature is 0°C to the reflux temperature of the solvent, preferably 10°C to 30°C.
The reaction time is 0.5 to 72 hours, preferably 1 to 12 hours.
Examples of the reaction solvent include dioxane, acetone, t-butyl alcohol, and water, and one of or a mixture of these can be used.

Step 4

Compound A5 can be obtained by reacting Compound A4 with 2,2-dimethoxypropane in the presence of an acid catalyst.
Examples of the acid catalyst include tosylic acid monohydrate, methanesulfonic acid, and acetic acid, and the catalyst can be used at 0.05 to 1 molar equivalents with respect to Compound A4.
The reaction temperature is 0°C to the reflux temperature of the solvent, preferably 10°C to 30°C.
The reaction time is 0.1 to 24 hours, preferably 0.5 to 4 hours.
Examples of the reaction solvent include acetone, toluene, DMF, and dioxane, and one of or a mixture of these can be used.

Step 5

Compound A6 can be obtained by reacting Compound A5 with Lewis acid.
Examples of the Lewis acid include zinc bromide, lithium bromide, trimethylsilyl iodide, and boron trifluoride-diethyl ether, and the Lewis acid can be used at 1 to 10 molar equivalents with respect to the compound A5.
The reaction temperature is 0°C to the reflux temperature of the solvent, preferably 10°C to 30°C.
The reaction time is 0.5 to 72 hours, preferably 1 to 12 hours.
Examples of the reaction solvent include dichloromethane, toluene, THF, acetonitrile, and water, and one of or a mixture of these can be used.

Step 6

Compound A7 can be obtained by reacting Compound A6 with an oxidizing agent in the presence or absence of a metal catalyst.
Examples of the metal catalyst include methyl rhenium trioxide, and sodium tungstate, and the catalyst can be used at 0.01 to 1 molar equivalents with respect to Compound A6.
Examples of the oxidizing agent include hydrogen peroxide water, 2-sulfonyloxaziridine, and dimethyldioxirane, and the oxidizing agent can be used at 1 to 5 molar equivalents with respect to Compound A6.
The reaction temperature is -50°C to 20°C, preferably -20°C to 0°C.
The reaction time is 0.5 to 24 hours, preferably 1 to 6 hours.
Examples of the reaction solvent include methanol, ethanol, isopropanol, acetone, acetonitrile, toluene, and

water, and one of or a mixture of these can be used.

Step 7

Nucleophile A8-2 can be adjusted by reacting Compound A8 with a silylating reagent in the presence of a base, followed by reaction with an organometallic reagent. Compound A9 can be obtained by reacting Compound A7 with Nucleophile A8-2.

Examples of the silylating reagent include trimethylsilyl chloride and 1,2-bis(chlorodimethylsilyl)ethane, and the silylating reagent can be used at 1 to 3 molar equivalents with respect to Compound A8.

Examples of the base include methyllithium, n-butyllithium, phenylmagnesium chloride and methylmagnesium chloride, and the base can be used at 2 to 3 molar equivalents with respect to Compound A8.

Examples of the organometallic reagent include n-butyllithium, isopropylmagnesium chloride, methylmagnesium chloride and ethylmagnesium bromide, and the organometallic reagent can be used at 1 to 2 molar equivalents with respect to Compound A8.

When an alkyllithium reagent is used as the base or the organometallic reagent, the reaction temperature is -100°C to 20°C, preferably -100°C to -50°C. When a Grignard reagent is used as the base or the organometallic reagent, the reaction temperature is -50°C to 20°C, preferably -30°C to -10°C.

The reaction time is 0.1 to 24 hours, preferably 0.5 to 3 hours.

Examples of the reaction solvent include THF, dioxane, toluene, and benzene, and one of or a mixture of these can be used.

Step 8

Compound A10 can be obtained by reacting Compound A9 with metal powder in the presence of an acid catalyst, or in the absence of any catalyst.

Examples of the metal powder include zinc, iron, and tin, and the metal powder can be used at 1 to 30 molar equivalents with respect to Compound A9.

Examples of the acid catalyst include ammonium chloride, acetic acid, and hydrochloric acid, and the acid catalyst can be used at 1 to 10 molar equivalents or as a solvent.

The reaction temperature is 0°C to the reflux temperature, preferably 20°C to 40°C.

The reaction time is 0.5 to 72 hours, preferably 1 to 12 hours.

Examples of the reaction solvent include methanol, ethanol, propanol, isopropanol, THF, DMF, acetic acid, and water, and one of or a mixture of these can be used.

Step 9

Compound A11 can be obtained by reacting Compound A10 with $Boc_2O$ in the presence or absence of a base.

Examples of the base include sodium hydroxide, sodium carbonate, sodium bicarbonate, potassium carbonate, calcium carbonate, cesium carbonate, pyridine, and triethylamine, and the base can be used at 1 to 5 molar equivalents relative to Compound A10.

The reaction temperature is -10°C to 80°C, preferably 10°C to 30°C.

The reaction time is 0.5 hours to 24 hours, and preferably 1 hour to 12 hours.

Examples of the reaction solvent include THF, dioxane, acetonitrile, dichloromethane, and water, and one of or a mixture of these can be used.

Step 10

Compound A12 can be obtained by reacting Compound A11 with an iodination reagent.

Examples of the iodination reagent include N-iodosuccinimide, N-iodosaccharin, and iodine, and the iodination reagent can be used at 1 to 5 molar equivalents with respect to Compound A11.

The reaction temperature is 0°C to the reflux temperature of the solvent, preferably 10°C to 30°C.

The reaction time is 0.5 hours to 24 hours, and preferably 1 hour to 12 hours.

Examples of the reaction solvent include THF, dioxane, acetonitrile, dichloromethane, acetic acid, and water, and one of or a mixture of these can be used.

Step 11

Compound A13 can be obtained by reacting Compound A12 with 4,4,5,5-tetramethyl-1,3,2-dioxaborolane in

the presence of a base and a metal catalyst.

Examples of the base include triethylamine, DIEA, potassium acetate, and potassium phosphate, and the base can be used at 1 to 5 molar equivalents with respect to Compound A12.

Examples of the metal catalyst include palladium acetate, bis(dibenzylideneacetone)palladium, $Pd(PPh_3)_4$, bis(triphenylphosphine)palladium (II) dichloride, SPhos Pd G3, and (2-dicyclohexylphosphino-2'-4'-6'-triisopropyl-1, 1'-biphenyl)[2-(2'-amino-1,1'-biphenyl)]palladium(II) methanesulfonate, and the metal catalyst can be used at 0.001 to 0.5 molar equivalents relative to Compound A12.

The reaction temperature is 0°C to the reflux temperature of the solvent, preferably 40°C to the reflux temperature of the solvent.

The reaction time is 0.5 hours to 24 hours, and preferably 1 hour to 12 hours.

Examples of the reaction solvent include THF, dioxane, acetonitrile, toluene, and DMF, and one of or a mixture of these can be used.

Step 12

Compound A15 can be obtained by reacting Compound A13 with aryl halide A14 in the presence of a metal catalyst and a base.

Examples of the metal catalyst include palladium acetate, bis(dibenzylideneacetone)palladium, $Pd(PPh_3)_4$, bis(triphenylphosphine)palladium (II) dichloride, and bis(tri-tert-butylphosphine)palladium, and the metal catalyst can be used at 0.001 to 0.5 molar equivalents with respect to Compound A13.

Examples of the base include lithium hydroxide, sodium hydroxide, potassium hydroxide, potassium tert-butoxide, sodium tert-butoxide, sodium carbonate, potassium bicarbonate, sodium bicarbonate, sodium phosphate, sodium hydrogen phosphate, potassium phosphate, and potassium hydrogen phosphate, and the base can be used at 1 to 10 molar equivalents with respect to Compound A13.

The aryl halide A14 can be used at 1 to 10 molar equivalent(s) with respect to Compound A13.

The reaction temperature may be 20°C to reflux temperature of solvent, and if necessary, by a microwave irradiation.

The reaction time is 0.1 hours to 48 hours, and preferably 0.5 hours to 12 hours.

Examples of the reaction solvent include THF, toluene, DMF, dioxane, and water, and one of or a mixture of these can be used. The reaction solvent can be used at 5 to 10 mol equivalents with respect to Compound A15.

Step 13

Compound A16 can be obtained by reacting Compound A15 with an acid, or a Lewis acid.

Examples of the acid include hydrochloric acid and sulfuric acid, and examples of the Lewis acid include boron trichloride, boron tribromide, trimethylsilyl iodide, and dimethylboron bromide. The acid or the Lewis acid can be used at 5 molar equivalents to a solvent amount with respect to Compound A15.

The reaction temperature is -20°C to the reflux temperature of the solvent, preferably 0°C to 50°C.

The reaction time is 0.5 hours to 72 hours, and preferably 1 hour to 24 hours.

Examples of the reaction solvent include methanol, dichloromethane, dichloroethane, toluene, and water, and one of or a mixture of these can be used.

(Process 2)

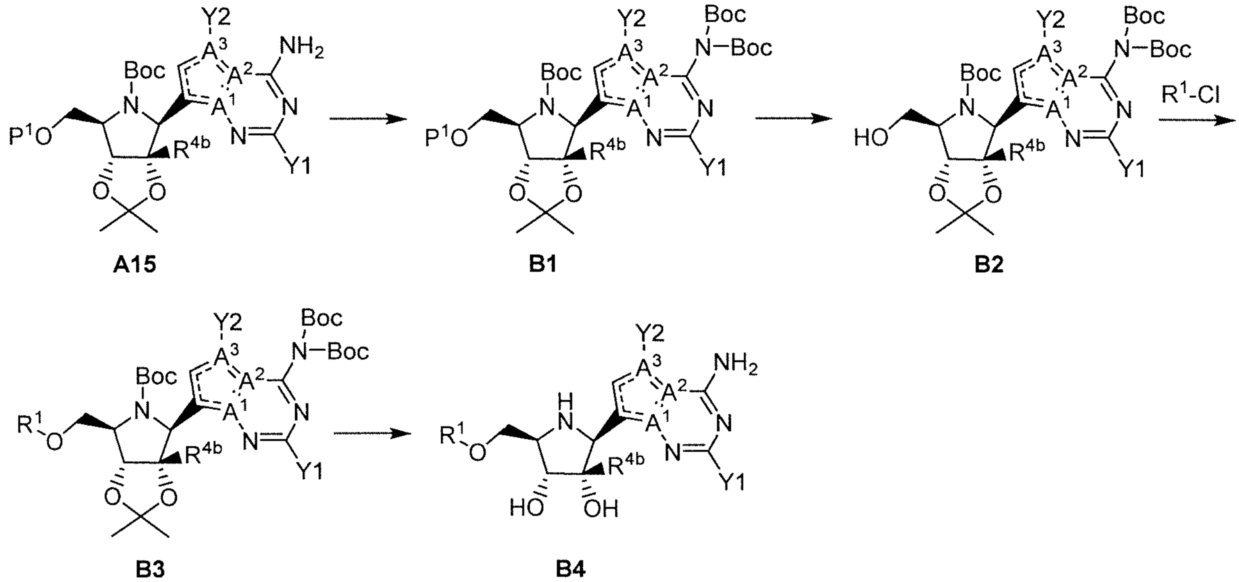


A15 B1 B2

B3 B4

wherein the symbols are the same as those described above, respectively.

Step 1

Compound B1 can be obtained by reacting Compound A15 with $Boc_2O$ in the presence of a base and 4-dimethylaminopyridine.

Examples of the base include sodium hydroxide, sodium carbonate, sodium bicarbonate, potassium carbonate, calcium carbonate, cesium carbonate, pyridine, and triethylamine, and the base can be used at 2 to 10 molar equivalents relative to Compound A15.

The reaction temperature is 20°C to the reflux temperature of the solvent, preferably 50°C to 90°C.

The reaction time is 0.5 hours to 72 hours, and preferably 1 hour to 24 hours.

Examples of the reaction solvent include THF, dioxane, acetonitrile, and DMF, and one of or a mixture of these can be used.

Step 2 ($P^1$ = Bn group)

Compound B2 can be obtained by reacting Compound B1 with hydrogen in the presence of a metal catalyst.

Examples of the metal catalyst include $Pd(OH)_2$, and palladium carbon, and the metal catalyst can be used at 0.01 to 1 molar equivalent(s) with respect to Compound B1.

The reaction temperature is 0°C to the reflux temperature of the solvent, preferably 10°C to 30°C.

The reaction time is 0.5 hours to 24 hours, and preferably 0.5 to 6 hours.

Examples of the reaction solvent include THF, methanol, ethanol, ethyl acetate, and DMF, and one of or a mixture of these can be used.

Step 2' ($P^1$ = TBDPS group)

Compound B2 can be obtained by reacting compound B1 with tetrabutylammonium fluoride.

The reaction temperature is 0°C to the reflux temperature of the solvent, preferably 10°C to 30°C.

The reaction time is 0.5 hours to 24 hours, and preferably 0.5 to 6 hours.

Examples of the reaction solvent include THF, dioxane, acetonitrile, ethyl acetate, and DMF, and one of or a mixture of these can be used.

Step 3

Compound B3 can be obtained by reacting Compound B2 with $R^1$-Cl in the presence of a base.

Examples of the base include pyridine, triethylamine, DIEA, sodium bicarbonate, sodium carbonate, and potassium phosphate, and the base can be used at 1 to 10 molar equivalents with respect to Compound B2.

The reaction temperature is 0°C to the reflux temperature of the solvent, preferably 20°C to 80°C.
The reaction time is 0.5 hours to 24 hours, and preferably 0.5 to 12 hours.
Examples of the reaction solvent include 1,2-dichloromethane, THF, dioxane, acetonitrile, and DMF, and one of or a mixture of these can be used.

Step 4

Compound B4 can be obtained by reacting Compound B3 with an acid.
Examples of the acid include trifluoroacetic acid, chloric acid, and sulfuric acid, and the acid can be used at 10 molar equivalents to a solvent amount with respect to

Compound B3.

The reaction temperature is 0°C to the reflux temperature of the solvent, preferably 10°C to 30°C.
The reaction time is 0.5 hours to 72 hours, and preferably 1 hour to 24 hours.
Examples of the reaction solvent include water, toluene, benzene, dichloromethane, and 1,2-dichloromethane, and one of or a mixture of these can be used.
The compounds of the present invention have a viral growth inhibitory action, and therefore, are useful as a therapeutic and/or preventive agent for viral infectious diseases.
Furthermore, the compound of the present invention has utility as a pharmaceutical product, and preferably, the compound of the present invention has any one or a plurality of the following excellent features:

a) The compound has weak inhibitory action against CYP enzymes (for example, CYP1A2, CYP2C9, CYP2C19, CYP2D6, CYP3A4, etc.);
b) The compound shows excellent pharmacokinetics such as high bioavailability or moderate clearance;
c) The compound has high metabolic stability;
d) The compound does not show irreversible inhibitory action against CYP enzymes (for example, CYP3A4) in the range of the concentration of the measurement conditions described herein;
e) The compound does not have mutagenesis;
f) The compound has a low risk of cardiovascular systems;
g) The compound shows high solubility;
h) The compound has low toxicity to cells;
i) The compound has large gap between toxicity to cells and viral proliferation inhibition; and
j) The compound has good lung migration.

Examples of a virus growth inhibitor include embodiments having $EC_{50}$ of 100 μM or less, preferably 10 μM or less, and more preferably 1 μM or less, for example, in a CPE suppression effect confirmation test described below.
The pharmaceutical composition of the present invention can be administered by either an oral method or a parenteral method. Examples of a parenteral administration method include percutaneous, subcutaneous, intravenous, intraarterial, intramuscular, intraperitoneal, transmucosal, inhalation, transnasal, ocular instillation, ear instillation, and intravaginal administration.
In the case of oral administration, the pharmaceutical composition may be prepared into any dosage form that is commonly used, such as a solid preparation for internal use (for example, a tablet, a powder preparation, a granular preparation, a capsule, a pill, or a film preparation), or a liquid preparation for internal use (for example, a suspension, an emulsion, an elixir, a syrup, a limonade, a spirit preparation, an aromatic water preparation, an extraction, a decoction, or a tincture) and administered. The tablet may be a dragee, a film-coated tablet, an enteric-coated tablet, a sustained release tablet, a troche, a sublingual tablet, a buccal tablet, a chewable tablet, or an orally disintegrating tablet; the powder preparation and granular preparation may be dry syrups; and the capsule may be a soft capsule, a microcapsule, or a sustained release capsule.
In the case of parenteral administration, the pharmaceutical composition can be suitably administered in any dosage form that is commonly used, such as an injectable preparation, an infusion, or a preparation for external use (for example, an eye drop, a nasal drop, an ear drop, an aerosol, an inhalant, a lotion, an impregnating agent, a liniment, a gargling agent, an enema, an ointment, a plaster, a jelly, a cream, a patch, a poultice, a powder preparation for external use, or a suppository). The injectable preparation may be an emulsion of O/W type, W/O type, O/W/O type, W/O/W type, or the like.
A pharmaceutical composition can be obtained by mixing an effective amount of the compound of the present invention with various pharmaceutical additives appropriate for the dosage form, such as an excipient, a binder, a disintegrating agent, and a lubricating agent, as necessary. Furthermore, the pharmaceutical composition can be prepared into a pharmaceutical composition for use for a child, an elderly, a patient with a serious case, or a surgical operation, by appropriately changing the effective amount of the compound of the present invention, the dosage form, and/or various pharmaceutical additives. For example, pediatric pharmaceutical compositions may be administered to patients who are neonates (younger than 4 weeks old after the birth), infants (4 weeks old to younger than 1 year old after the birth), children (1 year old or older and younger than 7 years old), infant children (7 years old or older and younger than 15 years old), or 15 to 18 years old. For example, the geriatric pharmaceutical compositions may be administered to patients who are 65 years old or older.

It is desirable to set the amount of administration of the pharmaceutical composition of the present invention, after considering the age and body weight of the patient, the type and degree of the disease, the route of administration, and the like; however, in the case of oral administration, the amount of administration is usually 0.05 to 100 mg/kg/day and is preferably in the range of 0.1 to 10 mg/kg/day. Although varying depending on the administration route, the dose in the case of parenteral administration is within a range of usually 0.005 to 10 mg/kg/day, preferably 0.01 to 1 mg/kg/day. This may be administered once a day or several times a day.

The compound of the present invention may be used in combination with, for example, another therapeutic agent for viral infectious diseases (the therapeutic agent includes an approved drug and a drug that is under development or to be developed in the future) (hereinafter, referred to as concomitant drug), for the purpose of enhancing the action of the compound, reducing the amount of administration of the compound, or the like. At this time, the timing of administration for the compound of the present invention and the concomitant drug is not limited, and these may be administered simultaneously to the target of administration or may be administered with a time difference. Further, the compound of the present invention and the concomitant medicament may be administered as two or more kinds of different compositions containing each active ingredient or as a single formulation containing both active ingredient.

The amount of administration of the concomitant drug can be appropriately selected based on the clinically used dosage. Furthermore, the blending ratio of the compound of the present invention and the concomitant drug can be appropriately selected according to the target of administration, the route of administration, the target disease, symptoms, combination, and the like. For example, when the target of administration is a human, 0.01 to 100 parts by weight of the concomitant drug may be used with respect to 1 part by weight of the compound of the present invention.

[EXAMPLES]

Hereinafter, the present invention will be described in more detail by way of Examples, Reference Examples, and Test Examples; however, the present invention is not intended to be limited by these.

(Method for identifying compound)

The NMR analysis obtained in each Example was performed at 400 MHz, and measurement was made using DMSO-$d_6$, $CDCl_3$ MeOH, and $D_2O$. Furthermore, when NMR data are shown, there are occasions in which all the measured peaks are not described. MS (m/z) can be measured under the following measurement conditions 1 or measurement conditions 2, though the measurement conditions are not limited to these.

(Measurement conditions 1)

Column: ACQUITY UPLC (registered trademark) BEH C18 (1.7 μm i.d.2.1 × 50 mm) (Waters)
Flow rate: 0.8 mL/min
UV detection wavelength: 254 nm
Mobile phase: [A] was 0.1% formic acid-containing aqueous solution, and [B] was 0.1% formic acid-containing acetonitrile solution.
Gradient: A linear gradient of 5% to 100% solvent [B] was carried out for 3.5 minutes, and then 100% solvent [B] was maintained for 0.5 minutes.

(Measurement conditions 2)

Column: ACQUITY UPLC (registered trademark) BEH C18 (1.7 μm i.d. 2.1 × 50 mm) (Waters)
Flow rate: 0.55 mL/min
UV detection wavelength: 254 nm

Mobile phase: [A] was 0.1% formic acid-containing aqueous solution, and [B] was 0.1% formic acid-containing acetonitrile solution.

Gradient: A linear gradient of 5% to 100% solvent [B] was carried out for 3 minutes, and then 100% solvent [B] was maintained for 0.5 minutes.

Note that, herein, the description MS (m/z) indicates the value observed in the mass spectrometry.

Example 1

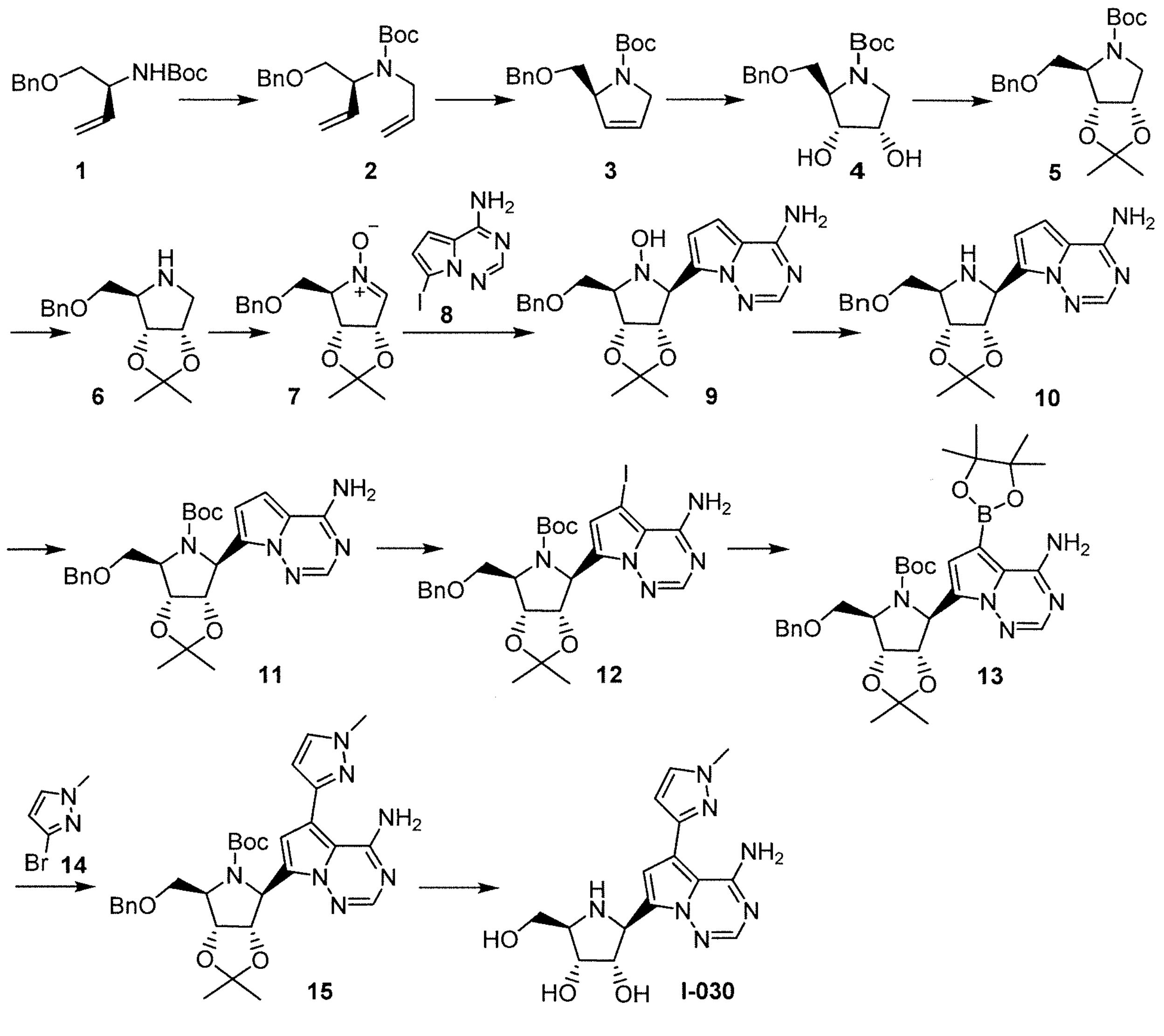


Step 1

Under a nitrogen atmosphere, a toluene solution (45 mL) of Compound 1 (26.6 g, 96.0 mol) was added to a suspension of t-BuOK (16.21 g, 144 mol) in toluene (240 mL), and the mixture was stirred at room temperature for 1 hour. Allyl bromide (17.47 g, 144 mmol) and tetrabutylammonium iodide (3.56 g, 9.63 mmol) were added to the reaction solution, and the mixture was stirred at room temperature for 3 hours. An ammonium chloride aqueous solution was added to the reaction solution, and extraction with toluene was performed. The organic layer was washed with water and concentrated under a reduced pressure. The residue obtained was purified by silica gel column chromatography (hexane-ethyl acetate) to obtain Compound 2 (28.74 g, yield 94%).

1H-NMR ($CDCl_3$) δ: 1.44 (9H, s), 3.58-3.70 (2H, m), 3.76 (2H, brs), 4.32-4.89 (3H, m), 5.00-5.26 (4H, m), 5.76-5.95 (2H, m), 7.27-7.37 (5H, m).

Step 2

First generation Grabb's catalyst (2.13 g, 2.59 mmol) was added to a dichloromethane (391 mL) solution of Compound 2 (41.12 g, 130 mmol) under a nitrogen atmosphere, and the mixture was stirred at room temperature for 9 hours and then allowed to stand overnight. The reaction solution was concentrated under a reduced pressure, and the residue obtained was purified by silica gel column chromatography (hexane-ethyl acetate) to obtain Compound 3 (35.10 g, yield 93%).

1H-NMR ($CDCl_3$) δ: 1.42 (4.5H, s), 1.47 (4.5H, s), 3.44 (0.5H, t, J = 7.9 Hz), 3.61 (0.5H, t, J = 7.8 Hz), 3.76 (0.5H, dd, J = 8.9, 2.6 Hz), 3.83 (0.5H, dd, J = 9.2, 2.3 Hz), 3.99-4.29 (2H, m), 4.49-4.60 (2.5H, m), 4.69 (0.5H, brs), 5.78-5.93 (2H, m), 7.23-7.38 (5H, m).

Step 3

Potassium osmium (VI) acid dihydrate (0.89 g, 2.42 mmol) and N-methylmorpholine oxide (42.5 g, 362 mmol) were added to an acetone (490 mL)-water (70 mL) solution of Compound 3 (35.1 g, 121 mmol) at room temperature, and the mixture was stirred at room temperature for 4.5 hours. An aqueous solution (176 mL) of sodium bisulfite (35.1 g) was added to the reaction solution, and an insoluble matter was filtered. Filtrate was concentrated under a reduced pressure, and residue was extracted with ethyl acetate. The organic layer was concentrated under a reduced pressure. The residue obtained was purified by silica gel column chromatography (hexane-ethyl acetate) to obtain Compound 4 (39.83 g, yield 97%).

1H-NMR ($CDCl_3$) δ: 1.40 (4.5H, s), 1.44 (4.5H, s), 2.85 (0.5H, brs), 2.95 (0.5H, brs), 3.05 (0.5H, brs), 3.13 (0.5H, brs), 3.32-3.68 (2H, m), 3.69-3.90 (2H, m), 4.18 (1H, brs), 4.31 (1H, t, J = 4.6 Hz), 4.51 (2H, dd, J = 15.9, 13.1 Hz), 7.25-7.38 (5H, m).

Step 4

2,2-dimethoxypropane (15.2 mL, 124 mmol) and tosylic acid monohydrate (589 mg, 3.10 mmol) were added to an acetone (102 mL) solution of Compound 4 (20.4 g, 62.0 mmol), and the mixture was stirred at room temperature for 20 minutes. To the reaction solution, 3% sodium bicarbonate water was added, and extraction with ethyl acetate was performed. The organic layer was washed with saline and dried over anhydrous magnesium sulfate, and thereafter the solvent was concentrated under a reduced pressure to obtain Compound 5 (23.5 g, yield 96%).

1H-NMR ($CDCl_3$) δ: 1.31 (3H, s), 1.41 (3H, s), 1.46 (9H, s), 3.49-3.82 (4H, m), 4.05 (0.5H, s), 4.20 (0.5H, s), 4.49 (2H, s), 4.67-4.78 (2H, m), 7.23-7.38 (5H, m).

Step 5

Zinc bromide (12.02 g, 53.4 mmol) was added to a chloroform (50 mL) solution of Compound 5 (10.0 g, 26.7 mmol), and the mixture was stirred overnight at room temperature overnight. To the reaction solution, 5% sodium bicarbonate water was added, and filtration with KC flock and extraction with chloroform were performed. The organic layer was washed with 5% sodium bicarbonate water and filtered with KC flock. The organic layer was dried over anhydrous magnesium sulfate, and the solvent was distilled off under a reduced pressure to obtain Compound 6 (7.08 g, yield 98%).

1H-NMR ($CDCl_3$) δ: 1.31 (3H, s), 1.47 (3H, s), 2.97-3.09 (2H, m), 3.35-3.51 (3H, m), 4.52 (2H, s), 4.57-4.71 (2H, m), 7.24-7.39 (5H, m).

Step 6

To a suspension of methyltrioxorhenium (739 mg, 2.97 mmol) in methanol (822 mL), 30% aqueous hydrogen peroxide (60.6 mL, 593 mmol) was added dropwise at - 20°C, and the mixture was stirred for 15 minutes. A methanol solution (90 mL) of Compound 6 (82.2 g, 297 mmol) was added dropwise to the reaction solution at -20°C, and the mixture was stirred for 1.5 hours. A 20% sodium thiosulfate aqueous solution was added to the reaction solution, extraction with chloroform was performed, then the organic layer was dried with anhydrous magnesium sulfate, and the solvent was distilled off under a reduced pressure. The residue obtained was purified by silica gel column chromatography (hexane-ethyl acetate) to obtain Compound 7 (54.3 g, yield 66%).

1H-NMR (DMSO-D6) δ: 1.32 (3H, s), 1.37 (3H, s), 3.76 (1H, dd, J = 10.3, 2.3 Hz), 3.90 (1H, dd, J = 10.3, 3.0 Hz), 4.01-4.04 (2H, m), 4.50 (2H, s), 4.86 (1H, d, J = 6.3 Hz), 5.17 (1H, d, J = 6.3 Hz), 7.10 (1H, s), 7.26-7.39 (4H, m).

Step 7

Under a nitrogen atmosphere, trimethylsilyl chloride (31.5 mL, 247 mmol) was added to a THF (298 mL) solution of Compound 8 (29.7 g, 107 mmol) at room temperature, and the mixture was stirred at room temperature for 10 minutes. A 2 M phenylmagnesium chloride THF solution (123 mL, 247 mmol) was added dropwise to the reaction solution at -30°C, and the mixture was stirred at 0°C for 10 minutes. Furthermore, a turbo Grignard reagent (1.3 M THF solution, 99 mL, 129 mmol) was added dropwise to the reaction solution at -30°C, and the mixture was stirred for 10 minutes. Compound 7 (30.7 g, 118 mmol) was added to the obtained reaction solution, and the mixture was stirred at -20°C for 30 minutes. A 10% citric acid aqueous solution (300 mL) was added to the reaction solution, and extraction with ethyl acetate was performed. The organic layer was washed with 10% citric acid aqueous solution and water, and was dried over anhydrous magnesium sulfate. The solvent was distilled off under a reduced pressure. The precipitated solid was collected by filtration with ethyl acetate-hexane to obtain Compound 9 (38.6 g, yield 87%).

1H-NMR ($CDCl_3$) $\delta$: 1.35 (3H, s), 1.57 (3H, s), 3.42 (1H, brs), 3.91 (2H, d, J = 4.1 Hz), 4.47 (1H, d, J = 5.6 Hz), 4.61 (2H, dd, J = 15.6, 11.7 Hz), 4.76 (1H, t, J = 6.6 Hz), 5.21 (1H, t, J = 6.6 Hz), 5.75 (2H, brs), 6.35 (1H, d, J = 4.4 Hz), 6.72 (1H, d, J = 4.3 Hz), 7.06 (1H, s), 7.30-7.44 (5H, m), 8.04 (1H, brs).

Step 8

Zinc powder (15.6 g, 238 mmol) was added to an acetic acid (100 mL) solution of Compound 9 (10.9 g, 26.5 mmol) at room temperature, and the mixture was stirred at room temperature for 2 hours. The reaction solution was filtered through celite, washed with ethyl acetate, and then the filtrate was concentrated under a reduced pressure. Ethyl acetate and water were added to the residue obtained, and the mixture was neutralized with sodium bicarbonate to adjust the pH to 8. The aqueous layer was extracted with ethyl acetate, and the obtained organic layer was washed with saturated saline solution, then dried over anhydrous magnesium sulfate. The solvent was distilled off under a reduced pressure to obtain Compound 10 (10.5 g, yield 100%).

1H-NMR ($CDCl_3$) $\delta$: 1.36 (3H, s), 1.58 (3H, s), 3.51 (1H, q, J = 4.8 Hz), 3.58-3.65 (1H, m), 3.66-3.73 (1H, m), 4.56 (2H, dd, J = 16.1, 12.0 Hz), 4.61-4.68 (2H, m), 4.97 (1H, t, J = 6.2 Hz), 5.46 (2H, brs), 6.56 (1H, d, J = 4.4 Hz), 6.68 (1H, d, J = 4.4 Hz), 7.27-7.38 (5H, m), 7.90 (1H, s).

Step 9

$Boc_2O$ (22.2 g, 102 mmol) was added dropwise to a THF (382 mL) solution of Compound 10 (38.2 g, 96.7 mmol) at room temperature, and the mixture was stirred for 1 hour. N-ethylpiperazine (1.84 mL, 14.5 mmol) was added to the reaction solution at room temperature, and the mixture was stirred for 1 hour. Ethyl acetate (382 mL) and a 2.4% citric acid aqueous solution (382 mL, 48.4 mmol) were added to the reaction solution to adjust the pH to 3. The organic layer was washed with saturated saline solution, 5% sodium bicarbonate water, and saturated saline solution, then dried over anhydrous magnesium sulfate. The solvent was distilled off under a reduced pressure to obtain Compound 11 (46.7 g, yield 94%).

1H-NMR ($CDCl_3$) $\delta$: 1.14-1.49 (12H, m), 1.59 (3H, s), 3.54-3.84 (2H, m), 4.24-4.45 (1H, m), 4.52-4.64 (2H, m), 4.81 (1H, d, J = 5.8 Hz), 4.85-4.95 (1H, m), 5.43 (2H, s), 5.48-5.63 (1H, m), 6.41-6.61 (2H, m), 7.25-7.38 (5H, m), 7.92 (1H, s).

Step 10

N-iodosuccinimide (28.5 g, 127 mmol) was added to a DMF (400 mL) solution of Compound 11 (39.1 g, 79.0 mmol), and the mixture was stirred at room temperature for 1 hour. A 10% sodium thiosulfate aqueous solution (400 mL) was added dropwise to the reaction solution at 0°C, and the mixture was stirred for 20 minutes. Ethyl acetate was added to the reaction solution, and extraction was performed. The organic layer was washed with 0.5% saline and then dried over anhydrous magnesium sulfate, and the solvent was distilled off under a reduced pressure to obtain Compound 12 (48.6 g, yield 98%).

1H-NMR ($CDCl_3$) $\delta$: 1.17-1.48 (12H, m), 1.58 (3H, s), 3.59-3.90 (2H, m), 4.24-4.42 (1H, m), 4.56 (2H, dd, J = 16.1, 12.0 Hz), 4.75-4.87 (2H, m), 5.39-5.59 (1H, m), 6.02 (2H, brs), 6.62-6.78 (1H, m), 7.23-7.40 (5H, m), 7.87 (1H, s).

Step 11

Under a nitrogen atmosphere, 4,4,5,5-tetramethyl-1,3,2-dioxaborolane (0.525 mL, 3.62 mmol), triethylamine (0.836 mL, 6.03 mmol), and SPhos Pd G3 (94.0 mg, 0.121 mmol) were added to a toluene (15 mL) solution of Compound 12 (1.5 g, 2.41 mmol), and the mixture was heated and refluxed for 5 hours. The reaction solution was concentrated

under a reduced pressure, and the thus obtained residue was purified by silica gel column chromatography (hexane-ethyl acetate) to obtain Compound 13 (1.13 g, yield 75%).

1H-NMR ($CDCl_3$) δ: 1.20-1.44 (24H, m), 1.58 (3H, s), 3.60-3.84 (2H, m), 4.24-4.46 (1H, m), 4.58 (2H, dd, J = 27.5, 12.0 Hz), 4.82 (2H, dd, J = 23.8, 5.6 Hz), 5.40-5.65 (1H, m), 6.95 (1H, s), 7.23-7.40 (5H, m), 7.95 (1H, s).

Step 12

Compound 14 (1.23 g, 7.63 mmol), $PdCl_2$ (dtbpf) (249 mg, 0.381 mmol), and a 1 M potassium phosphate aqueous solution (5.72 mL, 5.72 mmol) were added to a THF (23.7 mL) solution of Compound 13 (2.37 g, 3.81 mmol), and the mixture was stirred at 50°C overnight. A 10% citric acid aqueous solution was added to the reaction solution, and extraction with ethyl acetate was performed. The organic layer was washed with saturated saline solution and dried over anhydrous magnesium sulfate, and thereafter the solvent was distilled off under a reduced pressure. The residue obtained was purified by silica gel column chromatography (hexane-ethyl acetate) to obtain Compound 15 (1.70 g, yield 77%).

1H-NMR ($CDCl_3$) δ: 1.18-1.49 (12H, m), 1.61 (3H, s), 3.62-3.89 (2H, m), 3.90 (3H, s), 4.25-4.44 (1H, m), 4.55-4.66 (2H, m), 4.79-4.85 (1H, m), 4.86-4.99 (1H, m), 5.47-5.62 (2H, m), 6.06-6.35 (1H, m), 6.76 (1H, s), 7.23-7.37 (6H, m), 7.81 (1H, s), 10.21 (1H, s).

Step 13

A 1 M boron trichloride dichloromethane solution (0.625 mL, 0.625 mmol) was added to Compound 15 (60 mg, 0.104 mmol) at room temperature, and the mixture was stirred for 2 hours. To the reaction solution, 2 mL of methanol was added at 0°C, and the mixture was stirred at room temperature for 3 hours. The reaction solution was concentrated under a reduced pressure, and the residue obtained was solidified with ethyl acetate-methanol to obtain Compound I-030 (38 mg, 97% yield).

1H-NMR (MeOD) δ: 3.80-3.91 (3H, m), 4.03 (3H, s), 4.35-4.40 (1H, m), 4.90-4.93 (1H, m), 5.13 (1H, d, J = 8.3 Hz), 6.89 (1H, d, J = 2.4 Hz), 7.54 (1H, s), 7.78 (1H, d, J = 2.3 Hz), 8.15 (1H, s).

Example 2

11

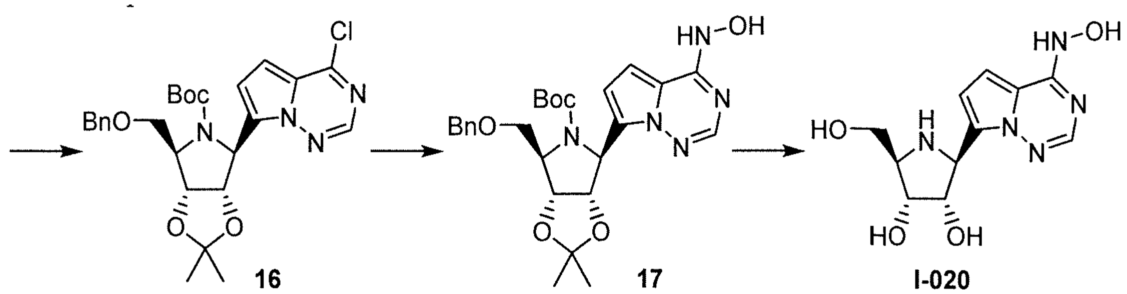

16 17 I-020

Step 1

Trimethylsilyl chloride (0.206 mL, 1.61 mmol) and tert-butyl nitrite (0.480 mL, 4.04 mmol) were added to a THF (10 mL) solution of Compound 11 (400 mg, 0.807 mmol) at 0°C, and the mixture was stirred at room temperature for 1 hour and a half. Saturated sodium bicarbonate water was added to the reaction solution, and extraction with ethyl acetate was performed. The organic layer was washed with saturated saline solution and dried over anhydrous magnesium sulfate, and thereafter the solvent was distilled off under a reduced pressure. The residue obtained was purified by silica gel column chromatography (hexane-ethyl acetate) to obtain Compound 16 (170 mg, yield 41%).

1H-NMR ($CDCl_3$) δ: 1.12-1.50 (12H, m), 1.61 (3H, s), 3.60-3.75 (1.5H, m), 3.80-3.91 (0.5H, m), 4.28-4.46 (1H, m), 4.49-4.61 (2H, m), 4.74-4.90 (2H, m), 5.49-5.64 (1H, m), 6.80-6.91 (2H, m), 7.20-7.25 (2H, m), 7.27-7.34 (4H, m).

Step 2

A 50 wt% hydroxylamine aqueous solution (224 mg, 3.40 mmol) was added to an ethanol (4 mL) solution of Compound 16 (175 mg, 0.340 mmol), and the mixture was stirred at 80°C for 1 hour and a half. The reaction solution

was concentrated under a reduced pressure, and the thus obtained residue was purified by silica gel column chromatography (hexane-ethyl acetate) to obtain Compound 17 (152 mg, yield 87%).

1H-NMR ($CDCl_3$) δ: 1.21-1.47 (12H, m), 1.57 (3H, s), 3.47-3.82 (2H, m), 4.21-4.41 (1H, m), 4.57 (2H, s), 4.79 (2H, dd, J = 23.3, 5.8 Hz), 5.29-5.57 (1H, m), 6.07-6.25 (1H, m), 6.47 (1H, s), 7.22-7.36 (6H, m).

Step 3

Compound I-020 was synthesized in the same manner as Step 13 in Example 1.

1H-NMR (MeOD) δ: 3.12 (1H, q, J = 4.7 Hz), 3.68-3.78 (2H, m), 4.03 (1H, t, J = 5.6 Hz), 4.23 (1H, t, J = 6.5 Hz), 4.40 (1H, d, J = 7.0 Hz), 6.37 (1H, d, J = 4.1 Hz), 6.54 (1H, brs), 7.39 (1H, s).

Example 3

13

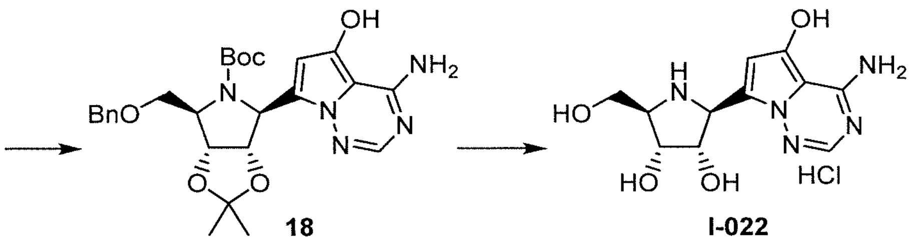

18

I-022

Step 1

To a 1,4 dioxane (0.250 ml) solution of Compound 13 (50.0 mg, 0.0804 mmol), 5-bromo-1H-imidazole (23.7 mg, 0.161 mmol) and $PdCl_2$ (dtbpf) (5.24 mg, 8.04 μmol) were added, and the mixture was stirred at 100°C for 5 hours. A 10% citric acid aqueous solution was added to the reaction solution, and extraction with ethyl acetate was performed. The organic layer was dried over anhydrous magnesium sulfate, and thereafter the solvent was distilled off under a reduced pressure. The thus obtained residue was purified by silica gel column chromatography (hexane-ethyl acetate) to obtain Compound 18 (9.20 mg, yield 22%).

1H-NMR (MeOD) δ: 7.38 (s, 1H), 7.23-7.16 (m, 5H), 5.93-5.87 (m, 1H), 5.22 (s, 1H), 4.85-4.71 (m, 1H), 4.64 (d, J = 5.4 Hz, 1H), 4.47 (dd, J = 16.6, 12.4 Hz, 2H), 4.16 (dd, J = 5.4, 5.4 Hz, 1H), 3.57-3.49 (m, 2H), 1.42-1.12 (m, 15H).

Step 2

Compound I-022 was synthesized in the same manner as Step 13 in Example 1.

1H-NMR (MeOD) δ: 7.88-7.86 (m, 1H), 6.53-6.52 (m, 1H), 5.05 (d, J = 8.4 Hz, 1H), 4.74 (dd, J = 8.0, 4.3 Hz, 1H), 4.32 (dd, J = 4.0, 4.0 Hz, 1H), 3.91-3.80 (m, 3H).

Example 4

12

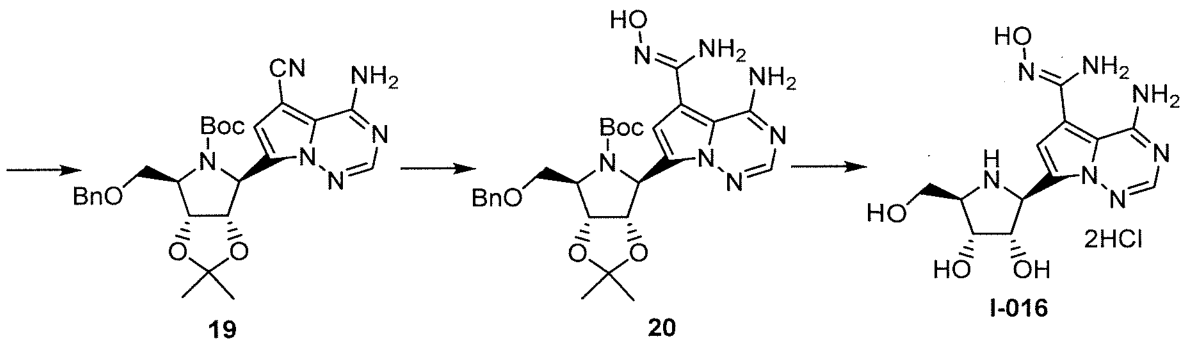

19

20

I-016

Step 1

Zinc cyanide (II) (226 mg, 1.93 mmol) and $Pd(PPh_3)_4$ (112 mg, 0.096 mmol) were added to a DMF (3 mL) solution of Compound 12 (300 mg, 0.483 mmol), and the mixture was stirred at 120°C for 4 hours. Water was added to the reaction solution, and extraction with ethyl acetate was performed. The organic layer was washed with saturated saline solution, and the organic layer was dried over anhydrous magnesium sulfate. The solvent was distilled off under a reduced pressure. The residue obtained was purified by silica gel column chromatography (hexane-ethyl acetate) to obtain Compound 19 (133 mg, yield 53%).

1H-NMR ($CDCl_3$) δ: 1.21 (4.5H, s), 1.36 (3H, s), 1.43 (4.5H, s), 1.59 (3H, s), 3.68-3.91 (2H, m), 4.29-4.40 (1H, m), 4.53 (2H, dd, J = 17.0, 11.6 Hz), 4.76-4.77 (2H, m), 5.46-5.49 (1H, m), 5.85 (2H, brs), 6.81-6.84 (1H, m), 7.24-7.36 (5H, m), 8.07 (1H, s).

Step 2

Hydroxylamine hydrochloride (71.4 mg, 1.028 mmol) was added to an ethanol (2 mL) solution of Compound 19 (107 mg, 0.206 mmol), and the mixture was stirred at 80°C for 2 hours. The solvent was distilled off under a reduced pressure, and the residue obtained was purified by silica gel column chromatography (hexane-ethyl acetate) to obtain Compound 20 (90 mg, yield 79%).

1H-NMR ($CDCl_3$) δ: 1.17 (5.5H, brs), 1.37 (3.5H, brs), 1.43 (3H, brs), 1.61 (3H, brs), 3.68-4.05 (2H, m), 4.29-4.87 (7H, m), 5.55-5.57 (1H, m), 6.74-6.77 (1H, m), 7.30-7.33 (5H, m), 7.80-7.86 (1H, m).

Step 3

$Pd(OH)_2$ (100 mg, 0.712 mmol) was added to a THF (2 mL) solution of Compound 20 (50 mg, 0.090 mmol) under a hydrogen atmosphere, and the mixture was stirred at room temperature for 4 hours. The reaction solution was filtered through celite, and the solvent was distilled off under a reduced pressure.

The residue obtained (28 mg, 0.060 mmol) was dissolved in a methanol (3 mL) solution of 10% hydrochloric acid, and was allowed to stand overnight. The obtained solid was collected by filtration and washed with ethanol, to obtain Compound I-016 (28 mg).

1H-NMR ($D_2O$) δ: 3.86-3.87 (1H, m), 3.92-3.93 (2H, m), 4.45 (1H, t, J = 4.3 Hz), 4.86-4.90 (2H, m), 5.12 (1H, d, J = 8.5 Hz), 7.24 (1H, s), 7.94 (1H, s).

Example 5

20

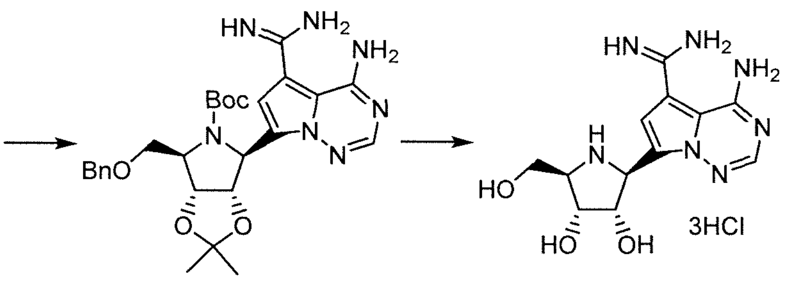

21

I-017

Step 1

Raney Nickel (32 mg, 0.542 mmol) and ammonium chloride (58 mg, 1.084 mmol) were added to an ethanol (18 mL) solution of Compound 20 (300 mg, 0.542 mmol), and the mixture was stirred at room temperature for 5 hours under a hydrogen atmosphere. The reaction solution was filtered through celite, and the solvent was distilled off under a reduced pressure. The residue obtained was purified by silica gel column chromatography (ethyl acetate-methanol) to obtain Compound 21 (125 mg, yield 43%).

MS (m/z) = 538 $[M+H]^+$

Step 2

Compound I-017 was synthesized in the same manner as Step 3 in Example 4.

1H-NMR ($D_2O$) δ: 3.81-3.83 (1H, m), 3.90-3.91 (2H, m), 4.41 (1H, dd, J = 5.0, 3.7 Hz), 4.85 (2H, dd, J = 8.3, 5.1 Hz), 5.09 (1H, t, J = 4.6 Hz), 7.40 (1H, s), 8.15 (1H, s).

Example 6

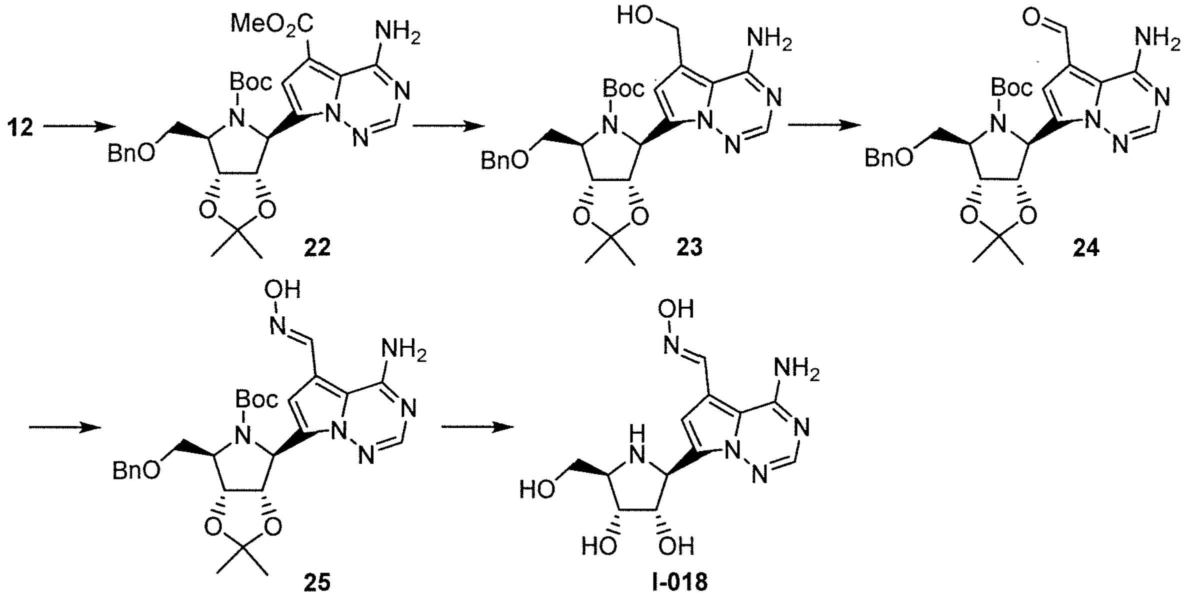

12 22 23 24 25 I-018

Step 1

$PdCl_2$ (dppf) (394 mg, 0.483 mmol) and potassium acetate (948 mg, 9.65 mmol) were added to a DMF (30 mL)-methanol (30 mL) solution of Compound 12 (3.00 g, 4.83 mmol), and the mixture was stirred at 95°C for 3 hours under a carbon monoxide atmosphere. Water was added to the reaction solution, and extraction with ethyl acetate was performed. The organic layer was washed with saturated saline solution, and dried over anhydrous magnesium sulfate. The solvent was distilled off under a reduced pressure. The residue obtained was purified by silica gel column chromatography (hexane-ethyl acetate) to obtain Compound 22 (1975 mg, yield 74%).

1H-NMR ($CDCl_3$) δ: 1.23-1.25 (5H, m), 1.36 (3H, brs), 1.42 (4H, brs), 1.59 (3H, brs), 3.74-3.82 (5H, m), 4.34-4.38 (1H, m), 4.57 (2H, dd, J = 19.4, 12.0 Hz), 4.78-4.79 (1H, m), 4.84 (1H, brs), 5.45-5.47 (1H, m), 6.21 (1H, brs), 7.05 (1H, brs), 7.26 (5H, brs), 7.95 (1H, s), 9.45 (1H, brs).

Step 2

Lithium aluminum hydride (1187 mg, 31.3 mmol) was added to THF (20 mL) of Compound 22 (1732 mg, 3.13 mmol) under ice cooling, and the mixture was stirred for 2 hours. Sodium sulfate decahydrate (20.16 g, 62.6 mmol) was added to the reaction solution, and the mixture was stirred at room temperature for 4 hours. After the reaction mixture was filtered through celite, the solvent was distilled off under a reduced pressure to obtain Compound 23 (1510 mg, yield 92%).

1H-NMR ($CDCl_3$) δ: 1.21-1.28 (5.5H, m), 1.36 (3.5H, brs), 1.43 (3H, brs), 1.63 (3H, brs), 3.62-3.92 (2H, m), 4.24-4.43 (1H, m), 4.49-4.75 (4H, m), 4.85-4.90 (2H, m), 5.50 (1H, brs), 6.27-6.47 (3H, m), 7.25-7.28 (5H, m), 7.86 (1H, brs).

Step 3

Manganese dioxide (2.19 g, 25.2 mmol) was added to a THF (30 mL) solution of Compound 23 (1.33 g, 2.52 mmol), and the mixture was stirred at 65°C for 6 hours. The reaction solution was filtered through celite, and the solvent was distilled off under a reduced pressure. The residue obtained was purified by silica gel column chromatography

(hexane-ethyl acetate) to obtain Compound 24 (1.12 mg, yield 85%).
MS (m/z) = 524 $[M+H]^+$

Step 4

Hydroxylamine hydrochloride (229 mg, 3.29 mmol) and sodium acetate (270 mg, 3.29 mmol) were added to an ethanol (2 mL) solution of Compound 24 (345 mg, 0.659 mmol), and the mixture was stirred at room temperature for 4 hours. Water was added to the reaction solution, extraction with dichloromethane was performed, and thereafter, the solvent was distilled off under a reduced pressure. The residue obtained was purified by silica gel column chromatography (hexane-ethyl acetate) to obtain Compound 25 (200 mg, yield 56%).

1H-NMR ($CDCl_3$) δ: 1.22 (5.5H, brs), 1.35 (4.5H, brs), 1.44 (4H, brs), 1.59 (3H, brs), 3.68-3.92 (2H, m), 4.29-4.35 (1H, m), 4.56 (2H, dd, J = 20.5, 11.9 Hz), 4.79-4.85 (2H, m), 5.46-5.48 (1H, m), 6.52-6.59 (1H, m), 7.30-7.32 (4H, m), 7.86-7.88 (2H, m).

Step 5

Compound 25 (200 mg, 0.371 mmol) was dissolved in a methanol (5 mL) solution of 10% hydrochloric acid, and was allowed to stand overnight. The reaction solution was concentrated.

The residue obtained was dissolved in dichloromethane (2 mL), and a 1 M boron trichloride dichloromethane solution (3.71 mL, 3.71 mmol) was added thereto at -78°C, then the mixture was stirred at room temperature for 1 hour. Methanol (5 mL) was added to the reaction solution at -78°C, the temperature was raised to room temperature, and then the solvent was distilled off under a reduced pressure. The residue obtained was purified by amino silica gel column chromatography (ethyl acetate-methanol) to obtain Compound 1-018 (59 mg, yield 52%).

1H-NMR (MeOD) δ: 3.08 (1H, q, J = 4.7 Hz), 3.58-3.62 (2H, m), 3.92 (1H, t, J = 5.5 Hz), 4.18 (1H, t, J = 6.6 Hz), 4.39 (1H, d, J = 7.0 Hz), 6.73 (1H, s), 7.69 (1H, s), 8.06 (1H, s).

Example 7

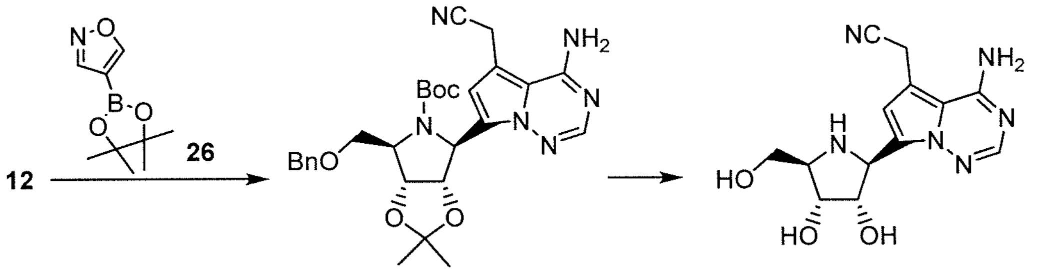
12
26

27 I-028

Step 1

Compound 26 (157 mg, 0.805 mmol), $PdCl_2$ (dtbpf) (10.49 mg, 0.016 mmol), and potassium carbonate (66.7 mg, 0.483 mmol) were added to a THF (2 mL)-water (0.2 mL) solution of Compound 12 (100 mg, 0.161 mmol), and the mixture was stirred at 100°C for 3 hours. Water was added to the reaction solution, and extraction with dichloromethane was performed. The organic layer was subjected to distillation under a reduced pressure, and the residue obtained was purified by silica gel column chromatography (hexane-ethyl acetate) to obtain Compound 27 (54 mg, yield 63%).

1H-NMR ($CDCl_3$) δ: 1.22 (4.5H, brs), 1.36 (3H, brs), 1.45 (4.5H, s), 1.59 (3H, brs), 3.62-3.98 (4H, m), 4.26-4.36 (1H, m), 4.58 (2H, tt, J = 18.1, 5.8 Hz), 4.81 (2H, brs), 5.33-5.33 (2H, m), 5.51-5.53 (1H, m), 6.49-6.52 (1H, m), 7.27-7.32 (5H, m), 7.91 (1H, s).

Step 2

Compound I-028 was synthesized in the same manner as Step 13 in Example 1.

1H-NMR (MeOD) δ: 3.20 (1H, q, J = 4.7 Hz), 3.69-3.77 (2H, m), 4.04 (1H, t, J = 5.5 Hz), 4.25 (2H, s), 4.30 (1H, t, J = 6.5 Hz), 4.54 (1H, d, J = 7.2 Hz), 6.80 (1H, s), 7.81 (1H, s).

Example 8

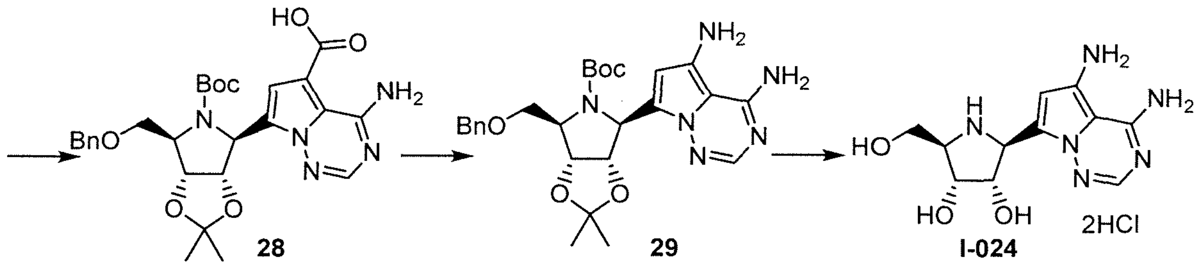

22 → 28 → 29 → I-024

2HCl

Step 1

A 4 M lithium hydroxide aqueous solution (0.0313 ml, 0.226 mmol) was added to a THF (1.00 ml) solution of Compound 22 (100 mg, 0.109 mmol), and the mixture was stirred at 80°C overnight. Methanol (1 ml) was added to the reaction solution, and the mixture was stirred at 80°C for 2 hours. A 10% citric acid aqueous solution was added to the reaction solution, and extraction with ethyl acetate was performed. The organic layer was dried over anhydrous magnesium sulfate, and thereafter the solvent was distilled off under a reduced pressure. The residue obtained was purified by silica gel column chromatography (hexane-ethyl acetate) to obtain Compound 28 (65 mg, yield 67%).

1H-NMR (DMSO-D6) δ: 9.40 (brs, 1H), 8.25 (brs, 1H), 8.04 (brs, 1H), 7.32-7.27 (m, 5H), 6.95-6.87 (m, 1H), 5.29 (d, J = 1.5 Hz, 1H), 4.77 (dd, J = 5.8, 1.5 Hz, 1H), 4.70-4.67 (m, 1H), 4.54 (s, 2H), 4.19 (dd, J = 5.8, 5.8 Hz, 1H), 3.63-3.60 (m, 2H), 1.45-1.14 (m, 15H).

Step 2

Diphenylphosphoryl azide (80.0 μl, 0.371 mmol) and triethylamine (51.4 μl, 0.371 mmol) were added to a toluene (1.00 ml) solution of Compound 28 (100 mg, 0.185 mmol), and the mixture was stirred at 100°C for 2 hours. Water was added to the reaction solution, and extraction with ethyl acetate was performed. The organic layer was dried over anhydrous magnesium sulfate, and thereafter the solvent was distilled off under a reduced pressure. The residue obtained was purified by silica gel column chromatography (chloroform-methanol) to obtain compound 29 (36.8 mg, yield: 39%).

1H-NMR (MeOD) δ: 7.38 (s, 1H), 7.25-7.15 (m, 5H), 5.97 (brs, 1H), 5.23 (d, J = 1.8 Hz, 1H), 4.65 (d, J = 5.0 Hz, 1H), 4.47 (s, 2H), 4.15 (dd, J = 5.0, 5.0 Hz, 1H), 3.61-3.53 (m, 1H), 1.42-1.12 (m, 15H).

Step 3

Compound I-024 was synthesized in the same manner as Step 13 in Example 1.

1H-NMR (MeOD) δ: 7.76-7.75 (m, 1H), 6.48-6.46 (m, 1H), 4.98 (d, J = 8.2 Hz, 1H), 4.74-4.70 (m, 1H), 4.30 (dd, J = 3.8, 3.8 Hz, 1H), 3.89-3.78 (m, 3H).

Example 9

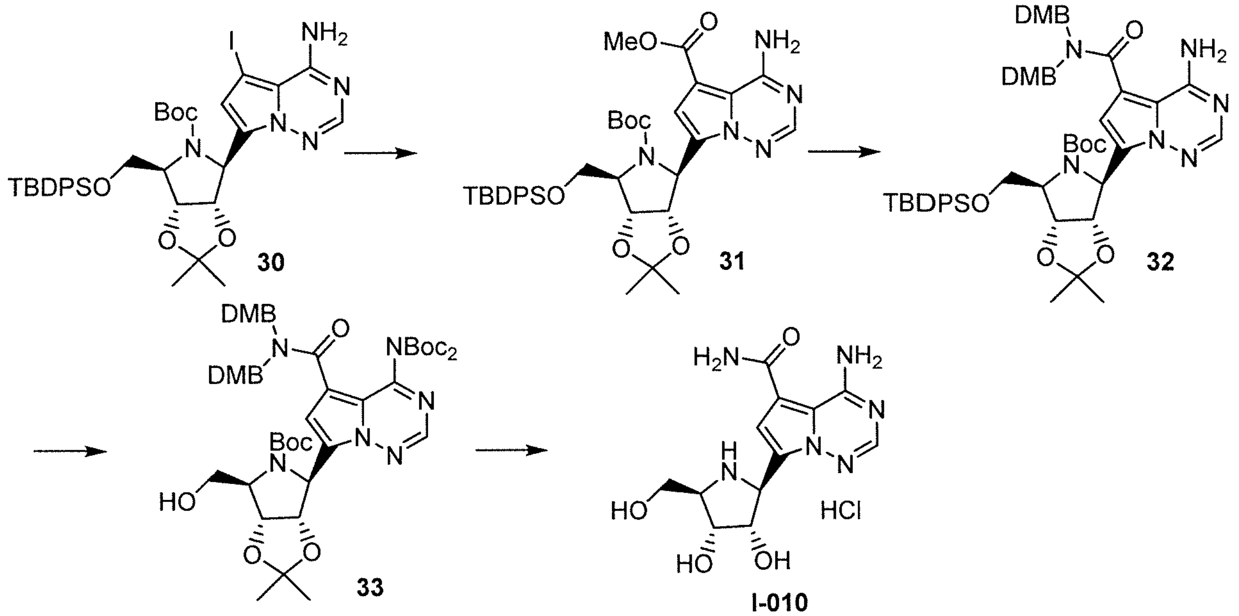

30 31 32 33 I-010

Step 1

$PdCl_2$ (dppf) (3.18 g, 3.9 mmol) and potassium acetate (7.65 g, 78.0 mmol) were added to a DMF (300 mL)-methanol (60 mL) solution of Compound 30 (30.0 g, 39.0 mmol), and the mixture was stirred at 90°C for 5 hours under a carbon monoxide atmosphere. Water was added to the reaction solution, and extraction with ethyl acetate was performed. The organic layer was washed with water and saturated saline solution, and dried over anhydrous sodium sulfate. The solvent was distilled off under a reduced pressure. The residue obtained was purified by silica gel column chromatography (hexane-ethyl acetate) to obtain Compound 31 (14.0 g, yield 51.2%).
MS (m/z) = 702 $[M+H]^+$

1H-NMR ($CDCl_3$) δ: 1.06 (9H, brs), 1.21-1.45 (15H, m), 3.65 (1.6H, s), 3.70 (1.4H, s), 3.77-4.01 (2H, m), 4.31-4.45 (0.9H, m), 4.60 (0.4H, brs), 4.68-4.80 (0.8H, m), 4.85 (0.4H, brs), 5.42 (0.4H, brs), 5.52 (0.4H, brs), 5.83 (0.7H, brs), 7.01 (0.5H, brs), 7.12 (0.5H, brs), 7.29-7.46 (6H, m), 7.58-7.69 (3.7H, m), 7.91 (0.3H, brs), 8.00 (0.3H, brs), 9.47 (0.7H, s).

Step 2

Potassium tert-butoxide (11.2 g, 100 mmol) was added to a tert-butanol (252 mL)-water (28 ml) solution of Compound 31 (14.0 g, 20.0 mmol) under ice cooling, and the mixture was stirred at 60°C for 110 minutes. Under ice cooling, a 2 M hydrochloric acid aqueous solution was added to make the mixture acidic, and extraction with ethyl acetate was performed. The organic layer was washed with saturated saline solution, and dried over anhydrous sodium sulfate. The solvent was distilled off under a reduced pressure.

Residue was dissolved in DMF (90 ml), bis (2,4-dimethoxybenzyl)amine (12.7 g, 40.0 mmol) and HATU (15.22 g, 40.0 mmol) were added to the reaction solution, and the mixture was stirred at room temperature for 4 hours under a nitrogen atmosphere. Water was added to the reaction solution, and extraction with ethyl acetate was performed. The organic layer was washed with water and saturated saline solution, and dried over anhydrous sodium sulfate. The solvent was distilled off under a reduced pressure. The residue obtained was purified by silica gel column chromatography (hexane-ethyl acetate) to obtain Compound 32 (16.7 g, yield 84.5%).
MS (m/z) = 688 $[M+H]^+$

1H-NMR ($CDCl_3$) δ: 1.02 (9H, s), 1.14 (3H, brs), 1.19-1.40 (9H, m), 1.54 (3H, s), 3.50-3.94 (13H, m), 4.18-4.96 (7H, m), 5.24-5.63 (2H, m), 6.11 (0.6H, s), 6.25-6.55 (4.4H, m), 6.63-6.85 (1H, m), 7.09 (1H, brs), 7.29-7.48 (6H, m), 7.57-7.70 (4.2H, m), 7.82 (0.8H, s).

Step 3

$Boc_2O$ (13.75 ml, 59.2 mmol), triethylamine (8.21 ml, 59.2 mmol) and DMAP (620 mg, 5.07 mmol) were added to a THF (120 ml) solution of Compound 32 (16.7 g, 16.92 mmol), and the mixture was refluxed for 7 hours. The solvent was distilled off under a reduced pressure.

Residue was dissolved in THF (126 ml). TBAF (1 M THF solution, 20.28 ml, 20.28 mmol) was added to the reaction solution, and the mixture was stirred at room temperature for 3 hours. The solvent was distilled off under a reduced pressure, and the residue obtained was purified by silica gel column chromatography (hexane-ethyl acetate) to obtain Compound 33 (9.49 g, yield 59.2%).

1H-NMR ($CDCl_3$) δ:1.08 (s, 6H), 1.36 (s, 27H), 2.88-2.99 (m, 1H), 3.46-3.70 (m, 2H), 3.55 (s, 3H), 3.62 (s, 3H), 3.81 (s, 3H), 3.83 (s, 3H), 4.12-4.20 (m, 1H), 4.27 (s, 2H), 4.42-4.85 (m, 4H), 5.51 (s, 1H), 6.32 (s, 1H), 6.39 (s, 1H), 6.53 (d, J = 8.4 Hz, 1H), 6.60 (d, J = 8.4 Hz, 1H), 6.81 (s, 1H), 7.12 (d, J = 8.4 Hz, 1H), 7.51 (d, J = 8.4 Hz, 1H), 8.50 (s, 1H).

Step 4

TFA (25.3 mL, 329 mmol) and water (2.71 mL, 151 mmol) were added to Compound 33 (1300 mg, 1.370 mmol), and the mixture was stirred at room temperature overnight. Toluene was added to the reaction solution, and the solvent was distilled off under a reduced pressure. Residue was purified by amino column chromatography (ethyl acetate-methanol) to obtain Compound I-010 (304 mg, yield 72%).

1H-NMR (DMSO-d6) δ:2.62-2.79 (m, 1H), 3.05 (ddd, J = 4.8, 4.8, 4.8 Hz, 1H), 3.40 (ddd, J = 5.2, 4.8, 10.8 Hz, 1H), 3.48 (ddd, J = 4.8, 5.2, 10.8 Hz, 1H), 3.74 (ddd, J = 5.2, 5.2, 5.2 Hz, 1H), 3.98 (ddd, J = 6.0, 5.2, 5.2 Hz, 1H), 4.39-4.49 (m, 2H), 4.65 (d, J = 5.6 Hz, 1H), 4.70 (d, J = 6.0 Hz, 1H), 7.29 (s, 1H), 7.47 (brs, 1H), 7.90 (s, 1H), 7.92-8.01 (m, 1H), 8.06 (brs, 1H), 10.37-10.45 (m, 1H).

Example 10

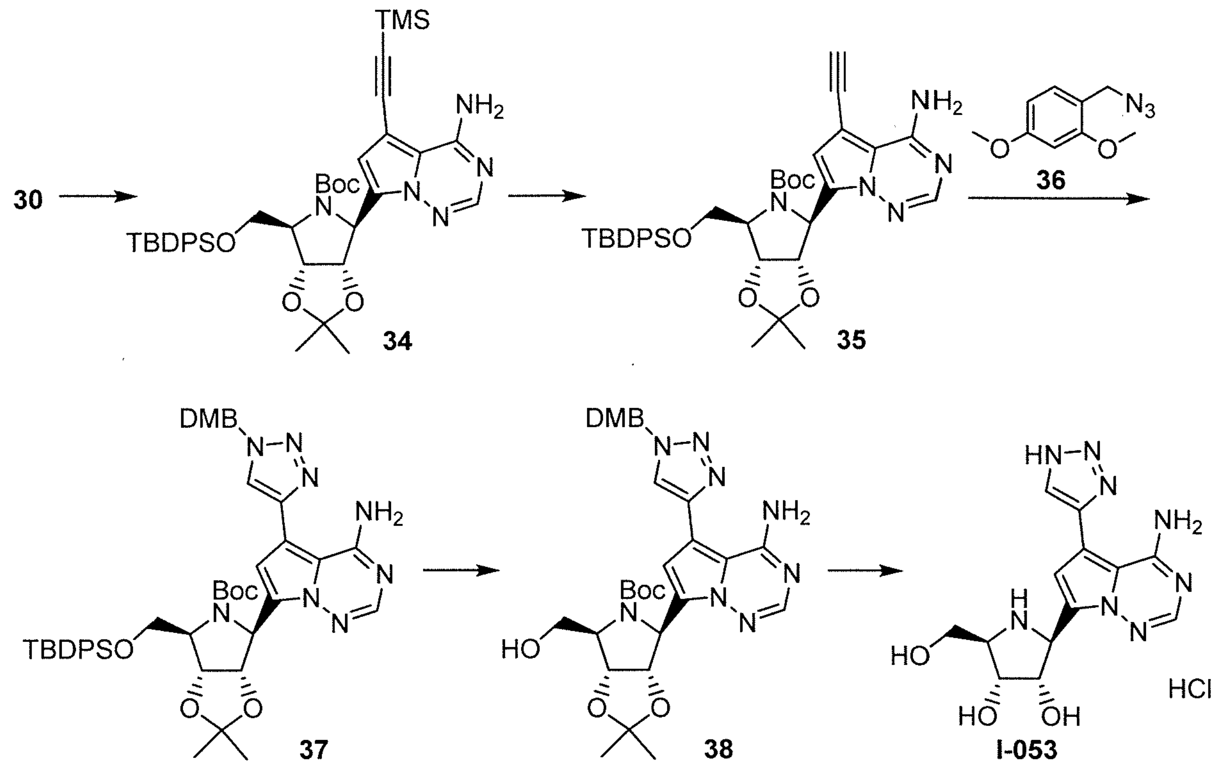

30 34 35 36 37 38 I-053

Step 1

Trimethylsilylacetylene (5.0 mL, 36 mmol), bistriphenylphosphine palladium dichloride (1.8 g, 2.6 mmol), copper iodide (0.99 g, 5.2 mmol), and triethylamine (10.8 mL, 78 mmol) were added to a DMF (200 mL) solution of Compound 30 (20 g, 26 mmol), and the mixture was stirred at room temperature for 2 hours and a half under a nitrogen atmosphere. Water was added to the reaction solution, and extraction with ethyl acetate was performed. The organic layer was dried over anhydrous sodium sulfate, and the solvent was distilled off under a reduced pressure. The residue obtained was purified by silica gel column chromatography (hexane-ethyl acetate) to obtain Compound 34 (9.0 mg, yield 47%).

1H-NMR ($CDCl_3$) δ: 0.23 (9H, s), 1.07 (9H, s), 1.26 (3H, s), 1.31-1.40 (9H, m), 1.56 (3H, s), 3.69-3.83 (2H, m),

4.38 (1H, d, J = 25.1 Hz), 4.71 (2H, t, J = 45.7 Hz), 5.45 (1H, d, J = 42.2 Hz), 6.65 (1H, d, J = 29.1 Hz), 7.35-7.42 (6H, m), 7.64-7.69 (4H, m), 7.87 (1H, d, J = 20.8 Hz).
MS (m/z) = 741 $[M+H]^+$

Step 2

Potassium carbonate (3.3 g, 24 mmol) was added to a methanol (180 mL) solution of Compound 34 (9.0 g, 12 mmol), and the mixture was stirred at room temperature for 2 hours. The reaction solution was concentrated under a reduced pressure, water was added to the reaction solution, and extraction with ethyl acetate was performed. The organic layer was dried over anhydrous sodium sulfate, and the solvent was distilled off under a reduced pressure. The residue obtained was purified by silica gel column chromatography (hexane-ethyl acetate) to obtain Compound 35 (7.0 mg, yield 86%).

1H-NMR ($CDCl_3$) δ: 1.06 (9H, s), 1.26-1.40 (15H, m), 1.57 (3H, s), 3.29 (1H, s), 3.68-3.85 (2H, m), 4.37 (1H, d, J = 29.1 Hz), 4.73 (2H, t, J = 43.2 Hz), 5.43 (1H, d, J = 45.4 Hz), 6.65 (1H, d, J = 31.9 Hz), 7.34-7.42 (6H, m), 7.63-7.68 (4H, m), 7.87 (1H, d, J = 14.1 Hz).
MS (M/Z) = 669 $[M+H]^+$

Step 3

Compound 36 (6.1 g, 31.4 mmol), DIEA (1.83 mL, 10.5 mmol), and copper iodide (0.20 g, 1.05 mmol) were added to a THF (70 mL) solution of Compound 35 (7.0 g, 10.5 mmol), and the mixture was stirred at 70°C for 4 hours and a half. The reaction solution was concentrated under a reduced pressure, and the thus obtained residue was purified by silica gel column chromatography (hexane-ethyl acetate) to obtain Compound 37 (5.7 g, yield 63%).

1H-NMR ($CDCl_3$) δ: 1.01 (9H, s), 1.26 (3H, s), 1.35-1.40 (9H, m), 1.59 (3H, s), 3.74-4.02 (8H, m), 4.37 (1H, d, J = 23.8 Hz), 4.83 (2H, t, J = 67.9 Hz), 5.35 (2H, d, J = 22.3 Hz), 5.47 (1H, s), 6.41-6.63 (3H, m), 6.92-7.05 (1H, m), 7.27-7.37 (6H, m), 7.58-7.90 (6H, m).
MS (m/z) = 862 $[M+H]^+$

Step 4

A 1 M TBAF THF solution (8.7 mL, 8.7 mmol) was added to a THF (50 mL) solution of Compound 37 (5.0 g, 5.8 mmol), and the mixture was stirred at room temperature for 2 hours. Water was added to the reaction solution, and extraction with ethyl acetate was performed. The organic layer was dried over anhydrous sodium sulfate, and the solvent was distilled off under a reduced pressure. The residue obtained was purified by silica gel column chromatography (hexane-ethyl acetate) to obtain Compound 38 (2.94 mg, yield 81%).

MS (m/z) = 623 $[M+H]^+$
1H-NMR ($CDCl_3$) δ: 1.17 (3H, s), 1.35-1.39 (9H, m), 1.61 (3H, s), 3.70-3.77 (1H, m), 3.83 (3H, s), 3.86 (3H, s), 4.16 (1H, d, J = 6.3 Hz), 4.28 (1H, d, J = 46.4 Hz), 4.63-5.18 (3H, m), 5.48 (2H, s), 5.80 (1H, s), 6.50-6.52 (2H, m), 6.71 (1H, d, J = 38.8 Hz), 7.26 (1H, s), 7.66 (1H, s), 7.86 (1H, s), 10.68 (1H, d, J = 55.5 Hz).

Step 5

TFA (36 mL) and water (4 mL) were added to Compound 38 (2.0 g, 3.2 mmol), and the mixture was stirred at room temperature for 2 hours. The reaction solution was concentrated under a reduced pressure, toluene was added to residue, and was concentrated under a reduced pressure. Ethyl acetate-methanol (2 : 1) solvent was added to the residue, and the mixture was filtered. The filtrate was concentrated under a reduced pressure, methanol and amino silica gel were added to the residue obtained, and the residue was filtered. The obtained solid was suspended in THF, and the supernatant suspension and the precipitated amino silica gel were separated by decantation. The mother liquor obtained by filtration and the supernatant suspension obtained by decantation were combined, and then the solvent was distilled off under a reduced pressure. The residue was again suspended in methanol, then a 4 M ethyl acetate hydrochloride solution was added thereto, and the mixture was filtered. The solvent in the filtrate was distilled off under a reduced pressure, an ethyl acetate-methanol (2 : 1) solution was added to the residue, and the resulting solid was collected by filtration to obtain compound I-053 (739 mg, 62% yield).

1H-NMR ($D_2O$) δ: 3.93 (3H, s), 4.48 (1H, s), 4.93 (1H, dd, J = 8.0, 4.6 Hz), 5.15 (1H, d, J = 8.3 Hz), 7.36 (1H, s), 7.98 (1H, s), 8.30 (1H, s).
MS (m/z) = 333 $[M+H]^+$

Example 11

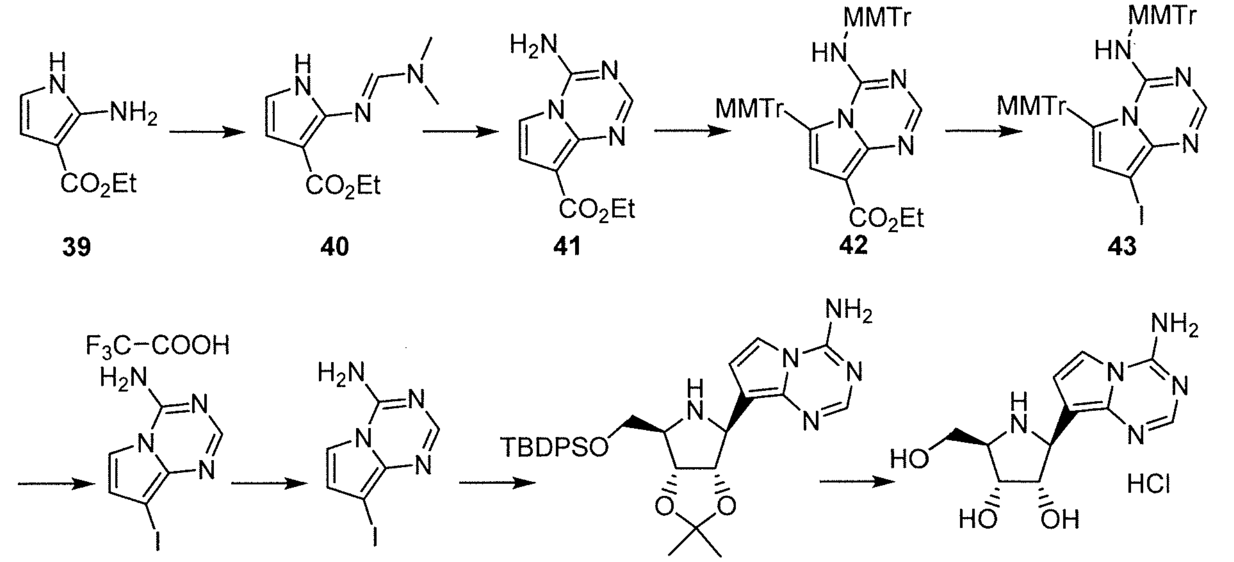

39 40 41 42 43

44 45 46 I-012

Step 1

N,N-dimethylformamide dimethyl acetal (61.2 mL, 457 mmol) was added to a toluene (329 mL) solution of Compound 39 (47.0 g, 305 mmol), and the mixture was stirred for 30 minutes under reflux. Water was added to the reaction solution, and extraction with ethyl acetate was performed. The organic layer was washed with saturated saline solution, and dried over anhydrous sodium sulfate. The solvent was distilled off under a reduced pressure. The residue obtained was purified by silica gel column chromatography (hexane-ethyl acetate) to obtain Compound 40 (51.0 mg, yield 80%).

1H-NMR ($CDCl_3$) δ: 1.31 (t, J = 7.2 Hz, 3H), 3.02 (s, 3H), 3.06 (s, 3H), 4.20 (q, J = 7.1 Hz, 2H), 6.34 (t, J = 2.8 Hz, 1H), 6.47 (t, J = 3.1 Hz, 1H), 8.27 (s, 1H).

Step 2

To Compound 40 (50.5 g, 241 mmol), 1-amidinopyrazole hydrochloride (88.4 g, 603 mmol), acetonitrile (1.01 L) and DIEA (211 mL, 1.21 mol) were added, and the mixture was stirred at 50°C overnight. The reaction solution was allowed to cool, and then the precipitated solid was collected by filtration to obtain Compound 41 (49.0 g, yield 99%).

1H-NMR (DMSO-$D_6$) δ: 1.24-1.30 (m, 3H), 4.23 (q, J = 7.1 Hz, 2H), 7.14 (d, J = 3.6 Hz, 1H), 7.46 (d, J = 3.6 Hz, 1H), 8.04 (d, J = 2.9 Hz, 1H), 8.40 (brs, 1H).

Step 3

Triethylamine (36.9 mL, 267 mmol) and 4-monomethoxytrityl chloride (46.4 g, 150 mmol) were added to a suspension of Compound 41 (10.0 g, 48.5 mmol) in dichloromethane (250 mL), and the mixture was stirred at room temperature for 4.5 hours. A saturated ammonium chloride aqueous solution was added to the reaction solution, and extraction with dichloromethane was performed. The organic layer was washed with saturated saline solution, and dried over anhydrous sodium sulfate. The solvent was distilled off under a reduced pressure. The residue obtained was purified by silica gel column chromatography (hexane-ethyl acetate) to obtain Compound 42 (36.4 mg, yield 100%).

1H-NMR ($CDCl_3$) δ: 1.32 (t, J = 7.0 Hz, 3H), 3.76 (s, 3H), 3.79 (s, 3H), 4.34 (q, J = 7.0 Hz, 2H), 6.47-6.70 (m, 11H), 6.94-7.43 (m, 19H), 7.61 (s, 1H).

Step 4

A 4 M lithium hydroxide aqueous solution (121 mL, 485 mmol) was added to a THF (182 mL)-ethanol (182 mL)

solution of Compound 42 (36.4 g, 48.5 mmol), and the mixture was stirred under reflux for 6 hours. Concentrated hydrochloric acid (38.4 mL, 461 mmol) was added to the reaction solution at 0°C, citric acid was further added until the pH of the reaction solution reached 4, and extraction with ethyl acetate was performed. The organic layer was washed with water and saturated saline solution, and dried over anhydrous sodium sulfate. The solvent was distilled off under a reduced pressure.

To a DMF (94.0 mL) solution of the residue obtained (9.43 g, 13.1 mmol), sodium bicarbonate (4.39 g, 52.2 mmol) and N-iodosuccinimide (3.08 g, 13.7 mmol) were added at 0°C, and the mixture was stirred at room temperature for 1 hour. A 10% citric acid aqueous solution was added to the reaction solution, and extraction with ethyl acetate was performed. The organic layer was washed with water and saturated saline solution, and dried over anhydrous sodium sulfate. The solvent was distilled off under a reduced pressure. The obtained residue was purified by silica gel column chromatography (hexane-ethyl acetate) to obtain Compound 43 (8.54 g, Yield 81%).

1H-NMR ($CDCl_3$) δ: 3.76 (s, 3H), 3.79 (s, 3H), 6.27-6.68 (m, 11H), 6.93-7.45 (m, 20H).

Steps 6, 7

To Compound 43 (16.1 g, 20.0 mmol), 1,2 dichloroethane (242 mL) and TFA (40.3 mL, 523 mmol) were added, and the mixture was stirred under reflux for 4.5 hours. Hexane (400 mL) and water were added to the reaction solution, and extraction was performed. Potassium carbonate (83.0 g, 600 mmol) was added to the aqueous layer, and the precipitated solid was collected by filtration to obtain Compound 44 (4.18 g).

Potassium carbonate (1.85 g, 13.4 mmol) was added to a DMF (10.0 mL) solution of Compound 44 (1.00 g), and the mixture was stirred at room temperature for 10 minutes. Water was added to the reaction solution, and the precipitated solid was collected by filtration to obtain Compound 45 (828 mg).

1H-NMR (DMSO-$D_6$) δ: 6.95 (d, J = 3.3 Hz, 1H), 7.54 (d, J = 3.5 Hz, 1H), 7.85 (s, 1H), 8.21 (brs, 2H).

Steps 8, 9

Using Compound 45, Compound 46 was synthesized in the same manner as Steps 7, 8 in Example 1.

Compound 46 (307 mg, 0.566 mmol) was dissolved in a methanol (10 mL) solution of 5% hydrochloric acid, and was stirred at 50°C for 2 hours. The reaction solution was concentrated under a reduced pressure, and the residue obtained was purified by amino silica gel column chromatography (chloroform-methanol) to obtain Compound I-012 (67.4 mg, 45% yield).

1H-NMR (DMSO-$D_6$) δ: 2.93 (1H, q, J = 4.4 Hz), 3.41-3.53 (2H, m), 3.73-3.86 (2H, m), 4.16 (1H, d, J = 6.5 Hz), 4.45 (1H, d, J = 4.8 Hz), 4.58-4.67 (2H, m), 6.80 (1H, d, J = 3.3 Hz), 7.36 (1H, d, J = 3.0 Hz), 7.72 (1H, s), 7.97 (2H, brs).

Example 12

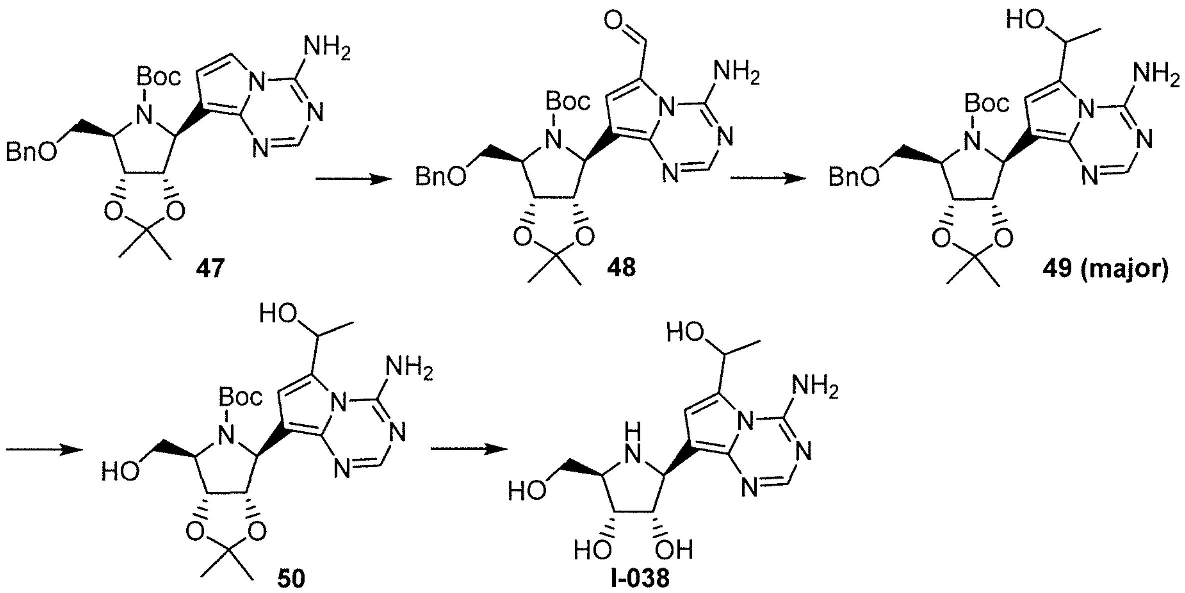

47 48 49 (major)

50 I-038

Step 1

Phosphoryl chloride (1.35 mL, 14.5 mmol) was added to DMF (14.4 mL) at 0°C under a nitrogen atmosphere, and the mixture was stirred for 30 minutes. A DMF (14.4 mL) solution of Compound 47 (1.44 g, 2.91 mmol) was added to the reaction solution, and the mixture was stirred at room temperature for 1.5 hours. A 1 M sodium hydroxide aqueous solution (14.5 mL, 14.5 mmol) was added to the reaction solution at 0°C, and the mixture was stirred at room temperature for 2.5 hours. Water was added to the reaction solution, and extraction with ethyl acetate was performed. The organic layer was washed with water and saturated saline solution, and dried over anhydrous sodium sulfate. The solvent was distilled off under a reduced pressure. The residue obtained was purified by silica gel column chromatography (hexane-ethyl acetate) to obtain Compound 48 (1.10 mg, yield 72%).
1H-NMR ($CDCl_3$) δ: 1.32-1.44 (m, 12H), 1.57 (s, 3H), 3.62-3.90 (m, 2H), 4.26-4.45 (m, 2H), 4.55 (d, J = 11.5 Hz, 1H), 4.87-5.01 (m, 2H), 5.21-5.28 (m, 1H), 5.98 (s, 1H), 7.21-7.29 (m, 5H), 7.44-7.47 (m, 1H), 8.03 (s, 1H), 8.63-8.80 (m, 1H), 10.80 (s, 1H).

Step 2

A 3 M methylmagnesium chloride THF solution (489 μL, 1.47 mmol) was added to a THF (1.54 mL) solution of Compound 48 (154 mg, 0.293 mmol) under a nitrogen atmosphere at 0 °C, and the mixture was stirred at 0°C for 1 hour. A saturated ammonium chloride aqueous solution was added to the reaction solution, and extraction with ethyl acetate was performed. The organic layer was washed with saturated saline solution, and dried over anhydrous sodium sulfate. The solvent was distilled off under a reduced pressure. The residue obtained was purified by silica gel column chromatography (hexane-ethyl acetate) to obtain Compound 49 (87.7 mg, yield 55%), which is a single enantiomer.
1H-NMR ($CDCl_3$) δ: 1.38-1.42 (m, 12H), 1.56-1.61 (m, 6H), 3.52-3.68 (m, 2H), 4.24-4.37 (m, 1H), 4.45-4.58 (m, 2H), 4.93-4.95 (m, 1H), 5.12-5.29 (m, 3H), 6.64-6.66 (m, 1H), 7.18-7.25 (m, 5H), 7.75-7.77 (m, 1H).

Step 3

In a THF (1.18 mL) solution of Compound 49 (59.0 mg, 0.109 mmol), palladium hydroxide-activated carbon (59.0 mg) was stirred under a hydrogen atmosphere at room temperature for 1 hour. The reaction solution was filtered through celite, and the filtrate was subjected to distillation under a reduced pressure. The obtained residue was purified by silica gel column chromatography (chloroform-methanol) to obtain Compound 50 (49.1 mg, yield 100%).
1H-NMR ($CDCl_3$) δ: 1.16 (s, 3H), 1.35 (s, 3H), 1.39 (s, 6H), 1.61 (s, 3H), 1.67-1.75 (m, 3H), 2.82-2.84 (m, 1H), 3.64-3.71 (m, 1H), 4.18-4.28 (m, 2H), 4.77-4.83 (m, 3H), 5.27-5.29 (m, 1H), 6.72-6.89 (m, 1H), 7.72-7.76 (m, 1H).

Step 4

A 4 M hydrochloric acid dioxane solution (855 μL, 3.42 mmol) and water (95.0 μL) were added to Compound 50 (47.5 mg, 0.106 mmol), and the mixture was stirred at room temperature for 40 minutes. Toluene was added to the reaction solution, and distillation under a reduced pressure was performed. The residue obtained was purified by amino column chromatography (chloroform-methanol) to obtain Compound I-038 (single enantiomer, 5.10 mg, yield 16%).
1H-NMR (MeOD) δ: 1.61 (d, J = 6.5 Hz, 3H), 3.18-3.21 (m, 1H), 3.76 (d, J = 4.3 Hz, 2H), 4.04-4.08 (m, 2H), 4.33-4.35 (m, 1H), 5.19-5.24 (m, 1H), 6.82 (s, 1H), 7.68 (s, 1H).

Example 13

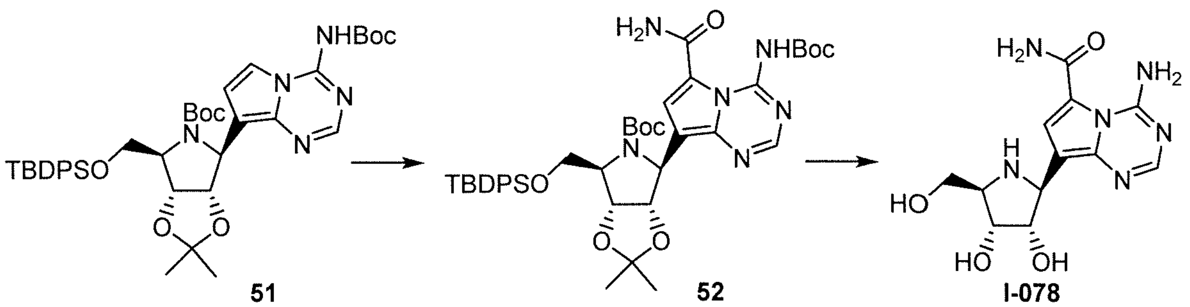

51 52 I-078

Step 1

To a THF (2.9 ml) solution of Compound 51 (290.7 mg, 0.391 mmol), chlorosulfonyl isocyanate (37.4 µl, 0.43 mmol) was added, and the mixture was stirred at 50°C for 30 minutes. Water was added to the reaction solution, and extraction with ethyl acetate was performed. The organic layer was dried over anhydrous magnesium sulfate. The solvent was distilled off under a reduced pressure. The residue obtained was purified by silica gel column chromatography (hexane-ethyl acetate) to obtain Compound 52 (103.2 mg, yield 34%).

1H-NMR ($CDCl_3$) δ: 13.89 (1H, s), 8.32-8.20 (1H, m), 7.63-7.59 (4H, m), 7.44-7.32 (7H, m), 5.30 (2H, s), 4.99 (1H, s), 4.84-4.75 (1H, brm), 4.40-4.30 (2H, brm), 3.97-3.85 (1H, brm), 3.71 (1H, s), 1.59 (9H, s), 1.57 (9H, s), 1.26 (6H, t, J = 7.2 Hz), 1.05 (9H, s).

Step 2

A 1 M TBAF THF solution (262 µl, 0.262 mmol) was added to a THF (1 ml) solution of Compound 52 (103.2 mg, 0.131 mmol), and the mixture was stirred for 29 hours. A 10% citric acid aqueous solution was added to the reaction solution, and extraction with ethyl acetate was performed. The organic layer was washed with water and then dried over anhydrous magnesium sulfate, and the solvent was distilled off under a reduced pressure. The residue obtained was purified by amino silica gel column chromatography (ethyl acetate-hexane-methanol).

Compound obtained (41.6 mg, 0.076 mmol) was dissolved in TFA (749 µl) and water (83 µl), and stirred at room temperature for 30 minutes. The solvent was distilled off under a reduced pressure, and the residue obtained was purified by amino column chromatography (ethyl acetate-methanol) to obtain Compound I-078 (9.8 mg, yield 42%).

1H-NMR (MeOD) δ: 7.86 (1H, s), 7.71 (1H, s), 4.30 (1H, d, J = 7.5 Hz), 4.12 (1H, t, J = 6.2 Hz), 4.06 (1H, t, J = 6.2 Hz), 3.76 (2H, d, J = 4.5 Hz), 3.18 (1H, s).

Example 14

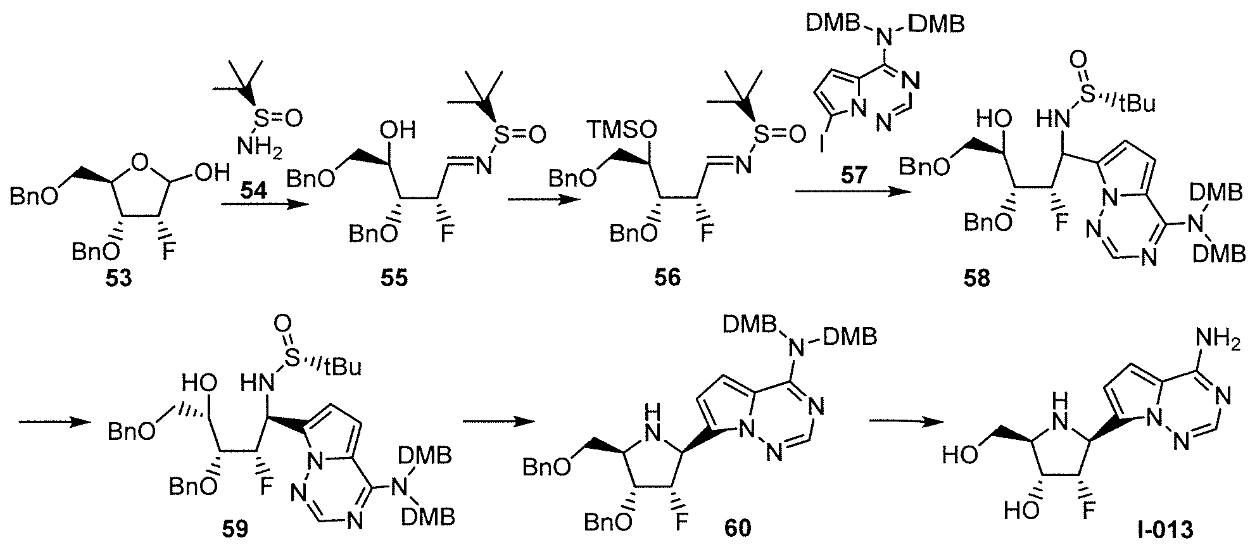


Step 1

Compound 54 (10.94 g, 90 mmol) and copper sulfate anhydrous (43.2 g, 271 mmol) were added to a toluene (200 mL) solution of Compound 53 (10 g, 30.1 mmol), and the mixture was stirred at 90°C for 24 hours. The reaction solution was filtered, and the solvent of the filtrate was distilled off under a reduced pressure. The residue obtained was purified by silica gel column chromatography (hexane-ethyl acetate) to obtain Compound 55 (7.16 mg, yield 55%).

1H-NMR ($CDCl_3$) δ: 1.08 (3.5H, s), 1.17 (4.5H, s), 3.47-3.57 (1H, m), 3.60-3.73 (1H, m), 4.16-4.19 (1H, m), 4.24-4.34 (2H, m), 4.51-4.69 (4H, m), 4.86-5.09 (1H, m), 5.24-5.34 (1H, m), 7.20-7.37 (11H, m).

Step 2

Imidazole (3.36 g, 49.3 mmol) and trimethylsilyl chloride (4.2 mL, 32.9 mmol) were added to a dichloromethane (70 mL) solution of Compound 55 (7.16 g, 16.44 mmol), and the mixture was stirred at room temperature for 2 hours. Water was added to the reaction solution, and extraction with ethyl acetate was performed. The organic layer was washed with saturated saline solution and dried over anhydrous magnesium sulfate, and thereafter the solvent was concentrated under a reduced pressure. The residue obtained was purified by silica gel column chromatography (hexane-ethyl acetate) to obtain Compound 56 (4.08 mg, yield 49%).

1H-NMR ($CDCl_3$) δ: 0.12 (9H, s), 1.18 (9H, s), 3.51-3.58 (2H, m), 3.97-4.15 (2H, m), 4.51 (2H, s), 4.58 (1H, d, J = 11.4 Hz), 4.73 (1H, d, J = 11.3 Hz), 5.47-5.59 (1H, m), 7.24-7.36 (10H, m), 8.17 (1H, dd, J = 11.3, 3.6 Hz).

Steps 3, 4

A 1.3 M isopropylmagnesium chloride lithium chloride complex THF solution (6.49 mL, 8.44 mmol) was added to a THF (40 mL) solution of Compound 57 (4.73 g, 8.44 mmol) under ice cooling, and the mixture was stirred for 30 minutes. A THF (10 mL) solution of Compound 56 (4.08 g, 8.04 mmol) was added to the reaction solution under ice cooling, and the mixture was stirred at room temperature for 5 hours. A saturated ammonium chloride aqueous solution was added to the reaction solution, and extraction with ethyl acetate was performed. The organic layer was washed with saturated saline solution and dried over anhydrous magnesium sulfate, and thereafter the solvent was concentrated under a reduced pressure. The residue obtained was dissolved in THF (25 mL), 1 M TBAF (24.11 mL, 24.11 mmol) was added thereto, and the mixture was stirred at room temperature for 30 minutes. The solvent was concentrated under a reduced pressure, and the residue obtained was purified by silica gel column chromatography (hexane-ethyl acetate) to obtain Compound 58 (2.02 g, yield 29%).

To a THF (5 mL) solution of Compound 58 (990 mg, 1.14 mmol), 3-chlorobenzoic acid (891 mg, 5.69 mmol) and triphenylphosphine (1791 mg, 6.83 mmol) were added, and diisopropyl azodicarboxylate (1.11 mL, 5.69 mmol) was added thereto under ice cooling, then the mixture was stirred at room temperature for 6 hours. The solvent was concentrated under a reduced pressure, and the residue obtained was roughly purified by silica gel column chromatography (hexane-ethyl acetate). The crude purified product was dissolved in a 7 M ammonia solution in methanol (5 mL), and the solution was stirred at 120 °C for 8 hours in a sealed tube. The solvent of the reaction solution was concentrated under a reduced pressure, and the residue obtained was roughly purified by silica gel column chromatography (hexane-ethyl acetate). The diastereomers were separated by supercritical fluid chromatography (SFC) to obtain Compound 59 (141 mg, yield 14%).

SFC Sorting Condition

Preparative column (IC-IC, manufactured by Daicel Corporation), flow: 30 mL/min, mobile phase: MeOH 75% (+ 0.1% DEA), sample: 10 mg/mL (MeOH/$CHCl_3$ = 1/1), injection amount: 10 mg, detection wavelength: 220 nm, BP: 8 MPa

1H-NMR ($CDCl_3$) δ: 1.15 (9H, s), 2.74 (1H, d, J = 7.5 Hz), 3.42 (1H, dd, J = 9.2, 6.7 Hz), 3.53 (1H, dd, J = 9.7, 6.0 Hz), 3.77-3.78 (11H, m), 4.03-4.07 (1H, m), 4.25 (1H, d, J = 11.2 Hz), 4.39-4.57 (4H, m), 4.92 (4H, dd, J = 34.0, 16.8 Hz), 5.08-5.23 (1H, m), 5.48 (1H, ddd, J = 23.7, 8.6, 4.7 Hz), 6.40-6.44 (5H, m), 6.57-6.58 (1H, m), 7.06-7.33 (16H, m), 7.96 (1H, s).

Steps 7, 8

Triphenylphosphine (170 mg, 0.648 mmol), imidazole (44 mg, 0.648 mmol) and iodine (123 mg, 0.486 mmol) were added to a toluene (2 mL) solution of Compound 59 (141 mg, 0.162 mmol), and the mixture was stirred at room temperature overnight. A 10% sodium thiosulfate aqueous solution (10 mL) was added to the reaction solution, and extraction with ethyl acetate was performed. The organic layer was washed with saturated saline solution and dried over anhydrous magnesium sulfate, and thereafter the solvent was concentrated under a reduced pressure. The residue obtained was purified by silica gel column chromatography (hexane-ethyl acetate) to obtain Compound 60 (121 mg, yield 99%).

With use of Compound 60, Compound 1-013 was synthesized in the same manner as Step 4 in Example 9.

1H-NMR (MeOD) δ: 3.08-3.12 (1H, m), 3.65 (1H, dd, J = 11.5, 4.2 Hz), 3.72 (1H, dd, J = 11.4, 3.3 Hz), 4.10 (1H, ddd, J = 19.1, 8.3, 4.8 Hz), 4.73-4.75 (1H, m), 4.91 (1H, ddd, J = 54.8, 4.7, 3.3 Hz), 6.61 (1H, d, J = 4.5 Hz), 6.76 (1H, d, J = 5.1 Hz), 7.70 (1H, s).

Example 15

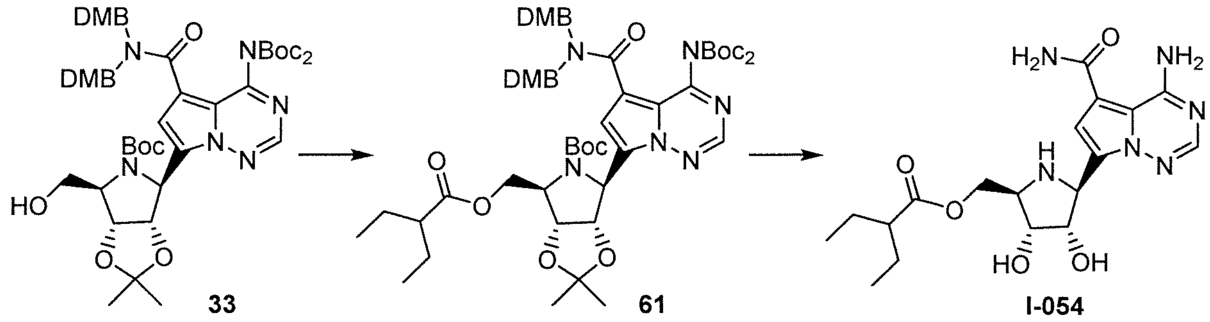

33 61 I-054

Step 1

Triethylamine (1.46 ml, 10.54 mmol) and 2-ethylbutanoyl chloride (1.08 ml, 7.90 mmol) were added to a 1,2-dichloroethane (50 ml) solution of Compound 33 (5.0 g, 5.27 mmol), and the mixture was stirred at 80°C for 4 hours under a nitrogen atmosphere. The solvent of the reaction solution was distilled off under a reduced pressure, and the reaction solution was dissolved in ethyl acetate. The organic layer was washed with water, a saturated sodium bicarbonate aqueous solution, and saturated saline solution, and dried over anhydrous sodium sulfate. The solvent was distilled off under a reduced pressure. The residue obtained was purified by silica gel column chromatography (hexane-ethyl acetate) to obtain Compound 61 (5.31 g, yield 96.2%).

1H-NMR ($CDCl_3$) δ:0.88-0.94 (m, 6H), 1.06 (s, 6H), 1.38 (s, 27H), 1.58-1.69 (m, 4H), 2.21-2.30 (m, 1H), 3.55 (s, 3H), 3.61 (s, 3H), 3.80 (s, 6H), 3.90-4.05 (m, 1H), 4.10-4.38 (m, 4H), 4.46-4.95 (m, 4H), 5.45-5.67 (m, 1H), 6.28-6.37 (m, 2H), 6.51-6.62 (m, 2H), 6.72-6.84 (m, 1H), 7.06-7.18 (m, 1H), 7.50 (d, J = 8.4 Hz, 1H), 8.54 (s, 1H).

Step 2

Compound I-054 was synthesized in the same manner as Step 4 in Example 9.

1H-NMR (DMSO-$d_6$) δ:0.82 (t, J = 7.4 Hz, 6H), 1.45-1.57 (m, 4H), 2.18-2.22 (m, 1H), 2.99 (br, 1H), 3.74 (q, J = 5.4 Hz, 1H), 3.95-4.01 (m, 2H), 4.19 (dd, J = 10.9, 5.0 Hz, 1H), 4.49 (br, 1H), 4.84 (dd, J = 15.2, 5.6 Hz, 2H), 7.27 (s, 1H), 7.45 (br, 1H), 7.89 (s, 1H), 7.96 (br, 1H), 8.08 (br, 1H), 10.39 (br, 1H).

Example 16

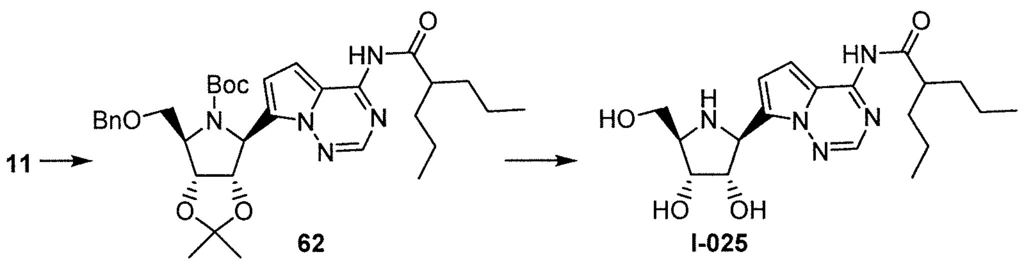

11 62 I-025

Step 1

To a dichloromethane (2 ml) solution of Compound 11 (200.5 mg, 0.40 mmol), 2-propylvaleryl chloride (83 μl, 0.485 mmol), triethylamine (112 μl, 0.809 mmol) and DMAP (9.9 mg, 0.081 mmol) were added, and the mixture was stirred at room temperature for 18 hours. A saturated ammonium chloride aqueous solution was added to the reaction solution, extraction with ethyl acetate was performed, and the solvent was distilled off under a reduced pressure. The residue obtained was purified by silica gel column chromatography (hexane-ethyl acetate) to obtain Compound 62 (179.1

mg, yield 71%).

1H-NMR ($CDCl_3$) δ: 8.12 (1H, s), 7.76 (1H, s), 7.29 (4H, s), 7.19 (1H, d, J = 4.4 Hz), 6.74 (1H, s), 5.59 (1H, d, J = 37.4 Hz), 4.84 (1H, s), 4.77 (1H, d, J = 5.9 Hz), 4.55 (2H, s), 4.37 (1H, d, J = 44.9 Hz), 3.74 (2H, s), 2.64 (1H, s), 1.74-1.73 (1H, m), 1.55 (9H, s), 1.36 (8H, brs), 1.27-1.24 (6H, brm), 0.94 (6H, t, J = 8.0 Hz).

Step 2

Compound 1-025 was synthesized in the same manner as Steps 3, 4 in Example 12.

1H-NMR (MeOD) δ: 0.98 (6H, t, J = 7.3 Hz), 1.39-1.49 (4H, m), 1.50-1.57 (2H, m), 1.72-1.77 (2H, m), 2.78-2.83 (1H, m), 3.24 (1H, q, J = 4.7 Hz), 3.74-3.75 (2H, m), 4.07 (1H, dd, J = 5.5, 5.1 Hz), 4.34 (1H, dd, J = 7.0, 5.1 Hz), 4.68 (1H, d, J = 7.0 Hz), 6.99 (1H, d, J = 4.6 Hz), 7.20 (1H, d, J = 4.6 Hz), 8.20 (1H, s).

Example 17

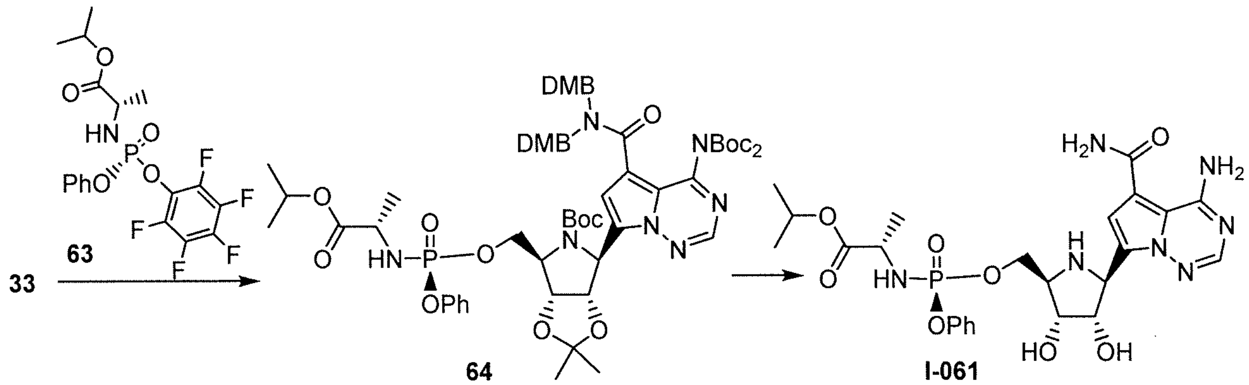

A 1 M tert-butylmagnesium chloride THF solution (0.659 mL, 0.659 mmol) was added dropwise to a THF (1.75 mL) solution of Compound 33 (250 mg, 0.263 mmol) at 0°C under a nitrogen atmosphere, and the mixture was stirred for 20 minutes. A THF (1.75 mL) solution of Compound 63 (155 mg, 0.342 mmol) was added to the reaction solution, and the mixture was stirred at room temperature for 1.5 hours. A saturated ammonium chloride aqueous solution was added to the reaction solution, and extraction with ethyl acetate was performed. The organic layer was washed with saturated saline solution, and dried over anhydrous sodium sulfate. The solvent was distilled off under a reduced pressure. The residue obtained was purified by silica gel column chromatography (hexane-ethyl acetate) to obtain Compound 64 (225 mg, yield 70%).

With use of Compound 64, Compound 1-061 was synthesized in the same manner as Step 4 in Example 9.

1H-NMR (DMSO-$D_6$) δ: 1.12-1.22 (m, 9H), 3.76-3.81 (m, 2H), 3.92-4.00 (m, 2H), 4.05-4.13 (m, 2H), 4.51 (d, J = 4.8 Hz, 1H), 4.82-4.88 (m, 3H), 5.93 (dd, J = 12.5, 10.0 Hz, 1H), 7.15-7.37 (m, 7H), 7.48 (s, 1H), 7.90 (s, 1H), 7.98 (s, 1H), 8.05 (s, 1H), 10.40 (s, 1H).

Example 18

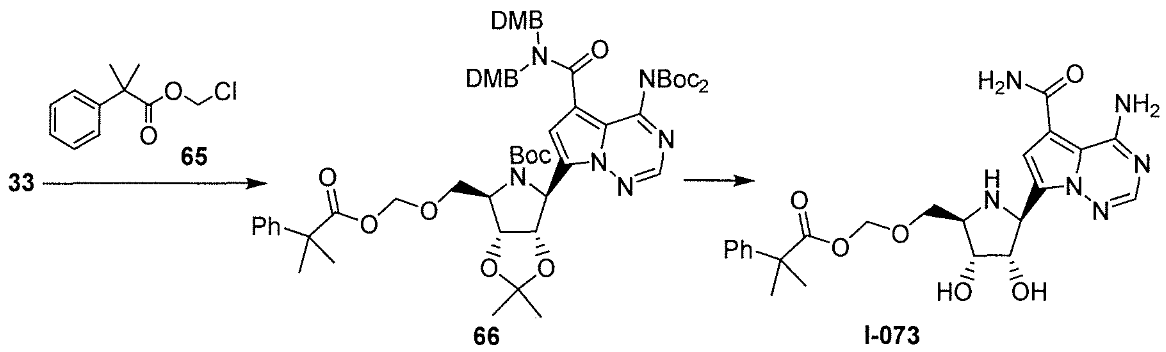

Step 1

Sodium hydride (30 mg, 0.74 mmol), Compound 65 (118 mg, 0.55 mmol), and sodium iodide (28 mg, 0.18 mmol) were added to a DMF (3.5 mL) solution of Compound 33 (350 mg, 0.37 mmol), and the mixture was stirred at 50°C for 2 hours. Water was added to the reaction solution, and extraction with ethyl acetate was performed. The organic layer was dried over anhydrous sodium sulfate, and the solvent was distilled off under a reduced pressure. The residue obtained was purified by silica gel column chromatography (hexane-ethyl acetate) to obtain Compound 66 (235 mg, yield 57%).
MS (m/z) = 1126 $[M+H]^+$

Step 2

Compound I-073 was synthesized in the same manner as Step 4 in Example 9.

1H-NMR (DMSO-$D_6$) δ: 1.51 (6H, s), 3.15-3.17 (1H, m), 3.46 (1H, dd, J = 9.6, 6.6 Hz), 3.57 (1H, dd, J = 9.7, 4.8 Hz), 3.65 (1H, dd, J = 10.7, 5.3 Hz), 3.93 (1H, q, J = 5.6 Hz), 4.45 (1H, d, J = 5.5 Hz), 4.76 (1H, d, J = 5.3 Hz), 4.79 (1H, d, J = 5.8 Hz), 5.24 (1H, d, J = 6.1 Hz), 5.28 (1H, d, J = 6.1 Hz), 7.20-7.24 (2H, m), 7.31-7.32 (4H, m), 7.48 (1H, s), 7.90 (1H, s), 7.98 (1H, s), 8.07 (1H, s), 10.41 (1H, d, J = 3.3 Hz).
MS (m/z) = 485 $[M+H]^+$

Example 19

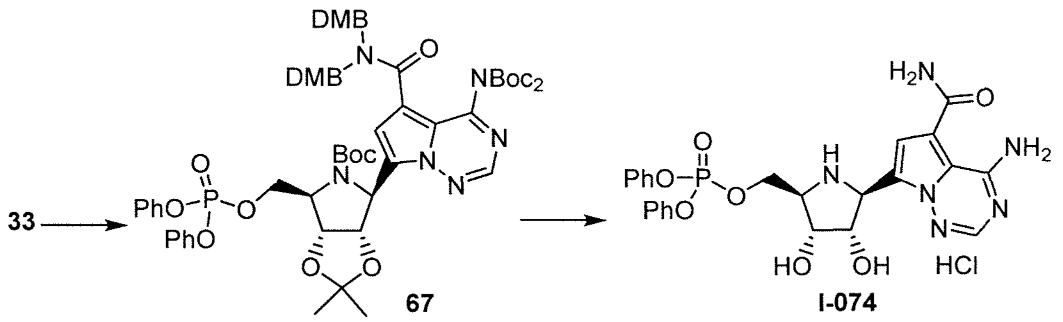

Step 1

Triethylamine (0.0313 ml, 0.226 mmol) and diphenylphosphoryl chloride (33.4 mg, 0.124 mmol) were added to a dichloromethane (1.0 ml) solution of Compound 33 (100 mg, 0.113 mmol), and the mixture was stirred at 40°C for 3 hours. The reaction solution was added to water, and extraction with ethyl acetate was performed. The organic layer was dried over anhydrous magnesium sulfate. The solvent was distilled off under a reduced pressure. The residue obtained was purified by silica gel column chromatography (hexane-ethyl acetate) to obtain Compound 67 (122 mg, yield 97%).

1H-NMR ($CDCl_3$) δ: 8.49 (s, 1H), 7.52-6.31 (m, 17H), 5.54-5.42 (m, 1H), 4.92-4.88 (m, 1H), 4.70-4.28 (m, 8H), 3.81-3.71 (m, 6H), 3.58-3.53 (m, 6H), 2.21-0.81 (m, 33H).

Step 2

Zinc bromide (0.0313 ml, 0.226 mmol) was added to a dichloroethane (1.22 ml) solution of Compound 67 (122 mg, 0.109 mmol), and the mixture was stirred at 60°C for 1 hour. Methanol and a saturated sodium bicarbonate aqueous solution were added to the reaction solution, and extraction with ethyl acetate was performed. The organic layer was dried over anhydrous sodium sulfate, and then the solvent was distilled off under a reduced pressure. The residue obtained was purified by silica gel column chromatography (ethyl acetate-methanol).

A 10% hydrochloric acid methanol solution (2.00 ml) was added to Compound obtained (77 mg, 0.133 mmol), and the mixture was stirred at room temperature through the night. The solvent was distilled off under a reduced pressure, and the residue obtained was purified by silica gel column chromatography (ethyl acetate-methanol) to obtain Compound I-074 (8.50 mg, yield 11%).

1H-NMR (MeOD) δ: 7.70 (s, 1H), 7.27-7.26 (m, 5H), 7.14-7.11 (m, 6H), 4.50 (d, J = 5.3 Hz, 1H), 4.41-4.28 (m, 2H), 4.17 (dd, J = 5.5, 2.7 Hz, 1H), 3.94 (dd, J = 5.5, 2.8 Hz, 1H), 3.42-3.38 (m, 1H).

Example 20

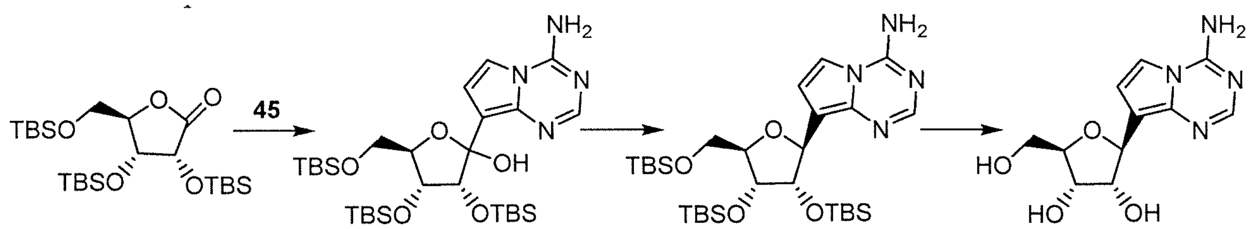


**68** **69** **70** **I-014**

Steps 1, 2

To a THF (2.50 mL) suspension of Compound 68 (132 mg, 0.509 mmol), chlorotrimethylsilane (130 μL, 1.02 mmol) was added under a nitrogen atmosphere, and the mixture was stirred at room temperature for 10 minutes. To the reaction solution, a 2 M phenylmagnesium chloride THF solution (535 μL, 1.07 mmol) was added dropwise at 0°C, and the mixture was stirred for 10 minutes. A 1.3 M isopropylmagnesium chloride/lithium chloride complex THF solution (509 μL, 0.662 mmol) was added dropwise to the reaction solution, and the mixture was stirred for 10 minutes. A THF (2.50 mL) solution of Compound 45 (250 mg, 0.509 mmol) was added to the reaction solution at -20°C, and the mixture was stirred at room temperature for 1 hour. A saturated ammonium chloride aqueous solution was added to the reaction solution, and extraction with ethyl acetate was performed. The organic layer was washed with saturated saline solution, and dried over anhydrous sodium sulfate. The solvent was distilled off under a reduced pressure. The residue obtained was purified by silica gel column chromatography (hexane-ethyl acetate) to obtain Compound 69 (77.7 mg, yield 24%).

Triethylsilane (98.0 μL, 0.616 mmol) and boron trifluoride diethyl ether complex (78.0 μL, 0.616 mmol) were added to a dichloromethane (1.54 mL) solution of Compound 69 (77.0 mg, 0.123 mmol) at -78°C under a nitrogen atmosphere, and the mixture was stirred at 0°C for 20 minutes. A saturated sodium bicarbonate aqueous solution was added to the reaction solution, and extraction with dichloromethane was performed. The organic layer was washed with saturated saline solution, and dried over anhydrous sodium sulfate. The solvent was distilled off under a reduced pressure. The residue obtained was purified by silica gel column chromatography (hexane-ethyl acetate) to obtain Compound 70 (33.6 mg, yield 45%).

1H-NMR ($CDCl_3$) δ: -0.29 (s, 3H), -0.10 (s, 3H), 0.08 (s, 3H), 0.09 (s, 3H), 0.11 (s, 6H), 0.76 (s, 9H), 0.93 (s, 9H), 0.94 (s, 9H), 3.75 (dd, J = 11.0, 3.5 Hz, 1H), 3.83 (dd, J = 10.8, 4.5 Hz, 1H), 4.03 (q, J = 3.7 Hz, 1H), 4.26 (dd, J = 4.5, 3.6 Hz, 1H), 4.35 (dd, J = 6.5, 4.5 Hz, 1H), 5.28 (d, J = 6.8 Hz, 1H), 5.54 (brs, 2H), 6.86 (d, J = 3.5 Hz, 1H), 6.99 (d, J = 3.3 Hz, 1H), 7.93 (s, 1H).

Step 3

Compound I-014 was synthesized in the same manner as Step 4 in Example 10.

1H-NMR (DMSO-$D_6$) δ: 3.45-4.10 (m, 7H), 4.67-4.90 (m, 2H), 6.80 (d, J = 2.8 Hz, 0.4H), 6.84 (d, J = 2.8 Hz, 0.6H), 7.37-7.39 (m, 1H), 7.77 (s, 1H), 8.06 (brs, 2H).

Example 21

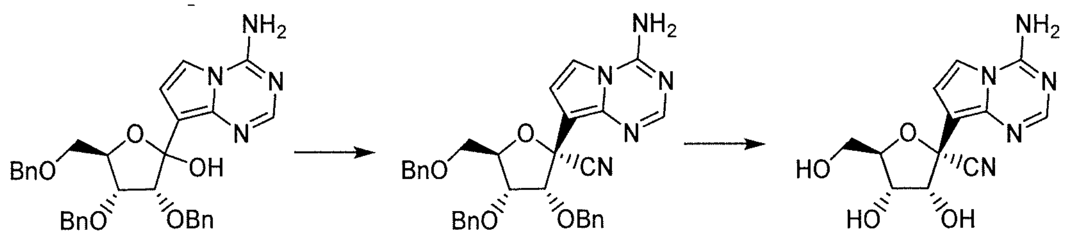

71 72 I-046

Step 1

Under a nitrogen atmosphere, trifluoromethanesulfonic acid (225 μL, 2.53 mmol) and trimethylsilyl cyanide (728 μL, 5.43 mmol) were added to a dichloromethane (20.0 mL) solution of Compound 71 (1.00 g, 1.81 mmol) synthesized in the same manner as in Step 1 of Example 20 at 0°C, and the mixture was stirred at room temperature for 30 minutes. A saturated sodium bicarbonate aqueous solution was added to the reaction solution, and extraction with dichloromethane was performed. The organic layer was washed with saturated saline solution, and dried over anhydrous sodium sulfate. The solvent was distilled off under a reduced pressure. The residue obtained was purified by silica gel column chromatography (hexane-ethyl acetate) to obtain Compound 72 (630 mg, 62% yield).

1H-NMR ($CDCl_3$) δ: 3.60 (dd, J = 10.8, 4.5 Hz, 1H), 3.70 (dd, J = 10.8, 4.0 Hz, 1H), 4.11 (t, J = 5.1 Hz, 1H), 4.45-4.55 (m, 4H), 4.64 (d, J = 12.0 Hz, 1H), 4.74 (dd, J = 20.5, 11.9 Hz, 2H), 5.25 (d, J = 5.3 Hz, 1H), 5.56 (brs, 2H), 6.77 (d, J = 3.5 Hz, 1H), 7.12 (d, J = 3.5 Hz, 1H), 7.19-7.32 (m, 15H), 7.86 (s, 1H).

Step 2

Under a nitrogen atmosphere, trifluoromethanesulfonic acid (18.1 μL, 0.204 mmol) and a 1 M boron trichloride dichloromethane solution (459 μL, 0.459 mmol) were added to a dichloromethane (2.29 mL) solution of Compound 72 (57.3 mg, 0.102 mmol) at 0°C, and the mixture was stirred at room temperature for 30 minutes. Triethylamine (191 μL, 1.38 mmol) and ethanol were added to the reaction solution, and the solvent was distilled off under a reduced pressure. The residue obtained was purified by amino column chromatography (chloroform-methanol) to obtain Compound I-046 (9.2 mg, yield 31%).

1H-NMR (MeOD) δ: 3.71 (dd, J = 12.5, 2.5 Hz, 1H), 3.86 (dd, J = 12.5, 2.3 Hz, 1H), 4.28-4.30 (m, 2H), 4.69 (d, J = 4.8 Hz, 1H), 7.06 (d, J = 3.5 Hz, 1H), 7.34 (d, J = 3.5 Hz, 1H), 7.83 (s, 1H).

Example 23

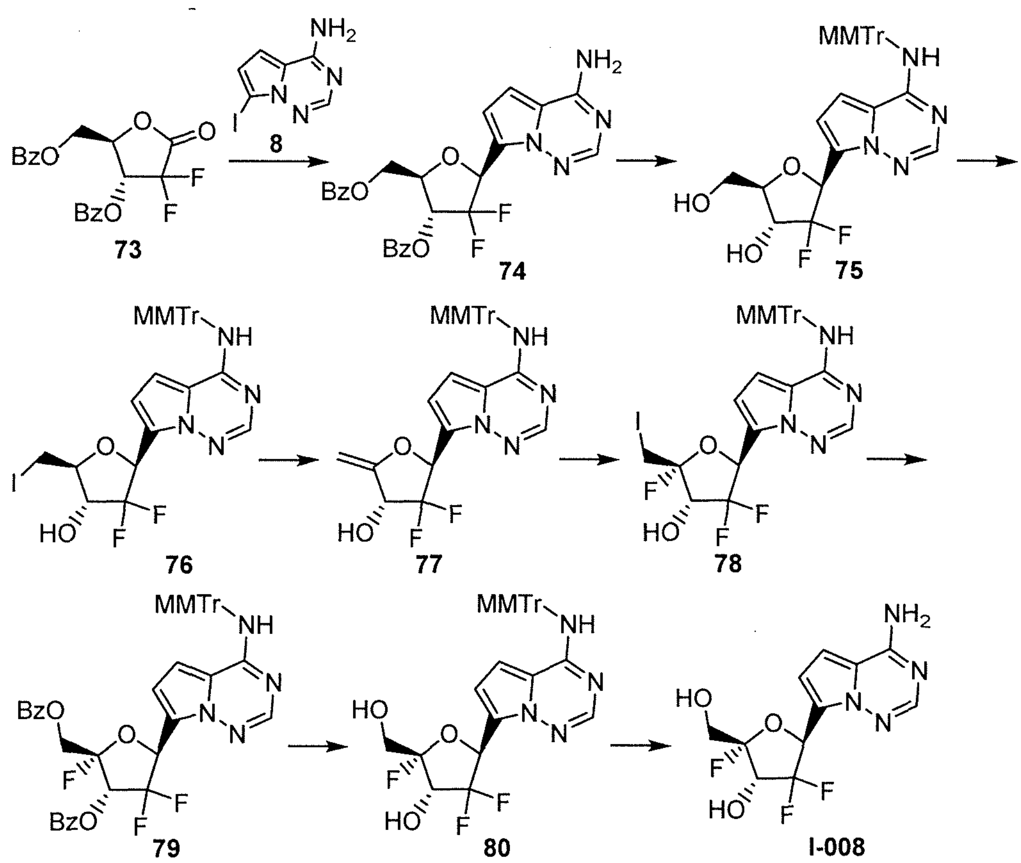

8 73 74 75 76 77 78 79 80 I-008

Step 1

Under a nitrogen atmosphere, chlorotrimethylsilane (10.4 g, 96.0 mmol) and a 2 M phenylmagnesium chloride THF solution (47.8 mL, 96.0 mmol) were added dropwise to a THF (156 mL) solution of Compound 73 (11.9 g, 45.7 mmol) at -20°C, and the mixture was stirred at 0°C for 30 minutes. A 1.3 M isopropylmagnesium chloride/lithium chloride complex THF solution (38.3 mL, 49.8 mmol) was added dropwise to the reaction solution at -20°C, and the mixture was stirred for 20 minutes. A THF (78.0 mL) solution of Compound 8 (15.6 g, 41.5 mmol) was added to the reaction solution, and the mixture was stirred at -20°C for 1 hour. The reaction solution was added to a 10% citric acid aqueous solution, and extraction with ethyl acetate was performed. The organic layer was dried over anhydrous sodium sulfate, and then the solvent was distilled off under a reduced pressure. The residue obtained was purified by silica gel column chromatography (chloroform-methanol).

TFA (212 mL) and triethylsilane (33.2 mL, 208 mmol) were added to the purified product (21.2 g), and the mixture was stirred under reflux for 1 hour. A saturated sodium bicarbonate aqueous solution were added to the reaction solution, and extraction with ethyl acetate was performed. The organic layer was washed with saturated sodium bicarbonate aqueous solution, water, and saturated saline solution, dried over anhydrous sodium sulfate, and then the solvent was distilled off under a reduced pressure. The residue obtained was purified by silica gel column chromatography (chloroform-methanol) to obtain Compound 74 (12.6 g, 2-step yield: 62%).

1H-NMR ($CDCl_3$) δ: 4.58-4.78 (m, 3H), 5.49 (brs, 2H), 5.75 (dd, J = 14.3, 5.2 Hz, 1H), 5.90 (dd, J = 18.1, 7.0 Hz, 1H), 6.65 (d, J = 4.4 Hz, 1H), 6.91-6.93 (m, 1H), 7.39-7.64 (m, 6H), 7.98 (s, 1H), 8.06 (d, J = 7.8 Hz, 2H), 8.11 (d, J = 8.0 Hz, 2H).

Step 2

Triethylamine (5.05 mL, 36.4 mmol) and 4-monomethoxytrityl chloride (8.99 g, 29.1 mmol) were added to a dichloromethane (36.0 mL) solution of Compound 74 (3.60 g, 7.28 mmol), and the mixture was stirred at room temperature for 2.5 hours. A saturated ammonium chloride aqueous solution was added to the reaction solution, and extraction with dichloromethane was performed. The organic layer was washed with saturated saline solution, and dried over anhydrous sodium sulfate. The solvent was distilled off under a reduced pressure. The residue obtained was purified by silica gel column chromatography (hexane-ethyl acetate).

Methanol (51.7 mL), dichloromethane (6.00 mL), and a 28% sodium methoxide methanol solution (2.97 mL, 14.8 mmol) were added to the purified product (5.17 g, 6.74 mmol), and the mixture was stirred at room temperature for 45 minutes. Acetic acid (848 μL, 14.8 mmol) was added to the reaction solution, and distillation under a reduced pressure was performed. Water was added to the residue, and extraction with ethyl acetate was performed. The organic layer was washed with saturated saline solution, and dried over anhydrous sodium sulfate. The solvent was distilled off under a reduced pressure. The residue obtained was purified by silica gel column chromatography (hexane-ethyl acetate) to obtain Compound 75 (3.50 g, yield 93%).

1H-NMR ($CDCl_3$) δ: 2.50 (brs, 1H), 2.93 (brs, 1H), 3.79 (s, 3H), 3.82-3.86 (m, 1H), 3.98-4.05 (m, 2H), 4.61-4.68 (m, 1H), 5.60 (t, J = 13.1 Hz, 1H), 6.44 (s, 1H), 6.61 (d, J = 4.5 Hz, 1H), 6.78-6.85 (m, 3H), 7.19-7.21 (m, 2H), 7.27-7.30 (m, 10H), 7.67 (s, 1H).

Step 3

To a THF (135 mL) solution of Compound 75 (7.42 g, 13.3 mmol), triphenylphosphine (9.33 g, 35.6 mmol) and imidazole (2.75 g, 40.4 mmol) were added, iodine (8.21 g, 32.3 mmol) was added at 0°C, and then, the mixture was stirred at room temperature for 1.5 hours. A 10% sodium thiosulfate aqueous solution and a saturated sodium bicarbonate aqueous solution were added to the reaction solution, and extraction with ethyl acetate was performed. The organic layer was washed with a 10% citric acid aqueous solution, water, and saturated saline solution, dried over anhydrous sodium sulfate, and then the solvent was distilled off under a reduced pressure. The residue obtained was purified by silica gel column chromatography (hexane-ethyl acetate) to obtain Compound 76 (7.71 g, yield 87%).

1H-NMR ($CDCl_3$) δ: 2.44-2.46 (m, 1H), 3.43 (dd, J = 5.7, 2.6 Hz, 2H), 3.78 (s, 3H), 3.94-3.98 (m, 1H), 4.24-4.31 (m, 1H), 5.76 (dd, J = 16.3, 9.3 Hz, 1H), 6.39 (s, 1H), 6.61 (d, J = 4.4 Hz, 1H), 6.81 (d, J = 8.8 Hz, 2H), 6.86-6.88 (m, 1H), 7.19 (d, J = 8.8 Hz, 2H), 7.26-7.29 (m, 10H), 7.68 (s, 1H).

Step 4

Diazabicycloundecene (8.74 mL, 58.0 mmol) was added to a THF (116 mL) solution of Compound 76 (7.75 g, 11.6 mmol), and the mixture was stirred at 50°C for 5 hours. To the reaction solution, 2M hydrochloric acid was added, and extraction with ethyl acetate was performed. The organic layer was washed with water and saturated saline solution, and dried over anhydrous sodium sulfate. The solvent was distilled off under a reduced pressure. The residue obtained was purified by silica gel column chromatography (hexane-ethyl acetate) to obtain Compound 77 (6.01 g, yield 96%).

1H-NMR ($CDCl_3$) δ: 2.26 (d, J = 7.5 Hz, 1H), 3.78 (s, 3H), 4.45-4.46 (m, 1H), 4.63-4.64 (m, 1H), 4.92-4.98 (m, 1H), 5.99 (t, J = 11.0 Hz, 1H), 6.41 (s, 1H), 6.60 (d, J = 4.4 Hz, 1H), 6.78-6.82 (m, 3H), 7.18-7.20 (m, 2H), 7.26-7.29 (m, 10H), 7.69 (s, 1H).

Step 5

N-iodosuccinimide (2.85 g, 12.7 mmol) and triethylamine-hydrogen trifluoride (1.72 mL, 10.5 mmol) were added to an acetonitrile (57.0 mL) solution of Compound 77 (5.70 g, 10.5 mmol) at 0°C, and the mixture was stirred at room temperature for 1 hour. A saturated sodium bicarbonate aqueous solution and a sodium thiosulfate aqueous solution were added to the reaction solution, and extraction with ethyl acetate was performed. The organic layer was washed with water and saturated saline solution, and dried over anhydrous sodium sulfate. The solvent was distilled off under a reduced pressure. The residue obtained was purified by silica gel column chromatography (hexane-ethyl acetate) to obtain Compound 78 (4.30 g, yield 59%).

1H-NMR ($CDCl_3$) δ: 2.65 (d, J = 12.4 Hz, 1H), 3.50-3.57 (m, 1H), 3.61-3.66 (m, 1H), 3.78 (s, 3H), 4.92-5.05 (m, 1H), 5.82 (dd, J = 16.2, 5.6 Hz, 1H), 6.41 (s, 1H), 6.58 (d, J = 4.4 Hz, 1H), 6.77 (d, J = 4.7 Hz, 1H), 6.80-6.82 (m, 2H), 7.18-7.21 (m, 2H), 7.26-7.29 (m, 10H), 7.64 (s, 1H).

Step 6

Benzoyl chloride (881 μL, 7.59 mmol) was added to a pyridine (30.4 mL) solution of Compound 78 (4.34 g, 6.32 mmol) at 0°C, and the mixture was stirred at room temperature for 2 hours. The reaction solution was concentrated, water was added to the concentrated solution, and extraction with ethyl acetate was performed. The organic layer was washed with 0.4 M hydrochloric acid, a saturated sodium bicarbonate aqueous solution and saturated saline solution, dried over anhydrous sodium sulfate, and then the solvent was distilled off under a reduced pressure.

DMF (232 mL), sodium benzoate (8.44 g, 58.6 mmol), and 15-crown-5-ether (12.9 g, 58.6 mmol) were added to the residue obtained (4.63 g, 5.86 mmol), and the mixture was stirred at 100°C for 3 days. Water was added to the reaction solution, and extraction with ethyl acetate was performed. The organic layer was washed with saturated sodium

bicarbonate aqueous solution, water, and saturated saline solution, dried over anhydrous sodium sulfate, and then the solvent was distilled off under a reduced pressure. The residue obtained was purified by silica gel column chromatography (hexane-ethyl acetate) to obtain Compound 79 (2.79 g, yield 56%).

1H-NMR ($CDCl_3$) δ: 3.80 (s, 3H), 4.63-4.76 (m, 2H), 6.10 (t, J = 11.3 Hz, 1H), 6.26-6.35 (m, 1H), 6.43 (s, 1H), 6.61 (d, J = 4.5 Hz, 1H), 6.81-6.83 (m, 3H), 7.21 (d, J = 8.8 Hz, 2H), 7.28-7.31 (m, 12H), 7.45-7.51 (m, 3H), 7.62 (t, J = 7.4 Hz, 1H), 7.66 (s, 1H), 7.96 (d, J = 7.8 Hz, 2H), 8.13 (d, J = 7.8 Hz, 2H).

Step 7

A 7 M ammonia methanol solution (55.0 mL, 385 mmol) was added to Compound 79 (2.79 g, 3.56 mmol), and the mixture was stirred at room temperature for 2.5 hours. The solvent of the reaction solution was distilled off under a reduced pressure, and the obtained residue was purified by silica gel column chromatography (hexane-ethyl acetate) to obtain Compound 80 (1.86 g, yield 90%).

1H-NMR ($CDCl_3$) δ: 2.63-2.70 (m, 2H), 3.78 (s, 3H), 3.85-3.88 (m, 2H), 5.03-5.17 (m, 1H), 5.77 (dd, J = 17.0, 5.1 Hz, 1H), 6.46 (s, 1H), 6.59 (d, J = 4.7 Hz, 1H), 6.74 (d, J = 4.7 Hz, 1H), 6.81 (d, J = 8.8 Hz, 2H), 7.19 (d, J = 8.8 Hz, 2H), 7.27-7.29 (m, 10H), 7.63 (s, 1H).

Step 8

TFA (648 μL, 8.41 mmol) and triethylsilane (1.34 mL, 8.41 mmol) were added to a dichloromethane (19.4 mL) solution of Compound 80 (970 mg, 1.68 mmol), and the mixture was stirred at room temperature for 30 minutes. The solvent of the reaction solution was distilled off under a reduced pressure, and the obtained residue was purified by amino column chromatography (chloroform-methanol) to obtain Compound 1-008 (370 mg, yield 72%).

1H-NMR ($CD_3OD$) δ: 3.75-3.76 (m, 2H), 4.65-4.76 (m, 1H), 5.92 (dd, J = 15.3, 6.0 Hz, 1H), 6.81 (d, J = 4.7 Hz, 1H), 6.86 (d, J = 4.7 Hz, 1H), 7.78 (s, 1H).

Example 24

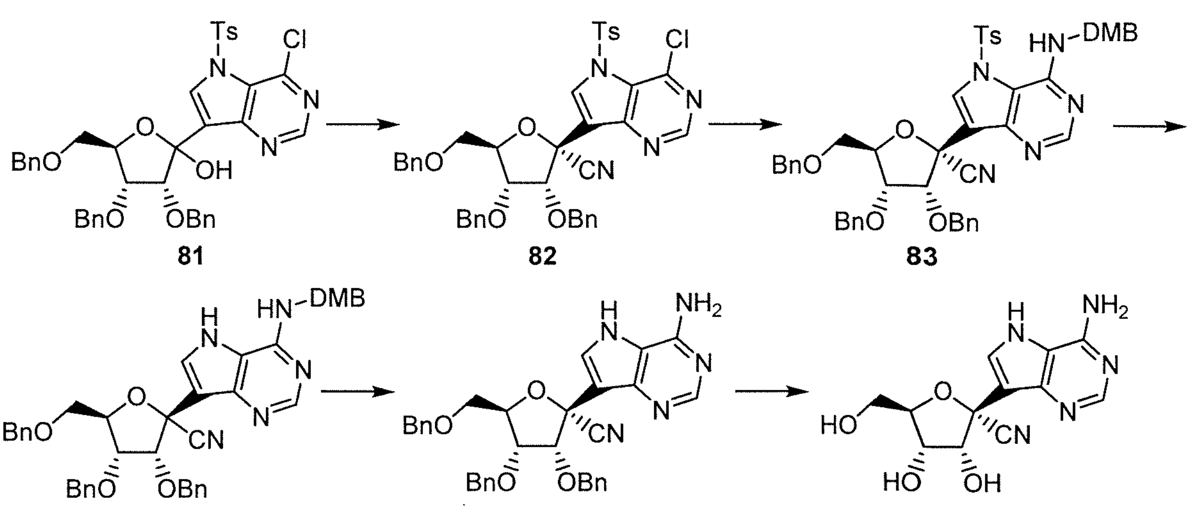

81 82 83

84 85 I-048

Steps 1, 2

To a dichloromethane (15 mL) solution of Compound 81 (734 mg, 1.01 mmol) synthesized in the same manner as Step 1 of Example 20, cyanotrimethylsilane (0.948 mL, 7.07 mmol) was added under ice cooling, and the mixture was stirred for 10 minutes. Trimethylsilyl trifluoromethanesulfonate (1.64 mL, 9.10 mmol) was added to the reaction solution under ice cooling, and the mixture was stirred for 1 hour. Saturated sodium bicarbonate water was added to the reaction solution, extraction with dichloromethane was performed, and the solvent was distilled off under a reduced pressure. The residue obtained was purified by silica gel column chromatography (hexane-ethyl acetate) to obtain Compound 82 (340 mg, yield 46%).

To a 1,4 dioxane (2.9 mL) solution of Compound 82 (287 mg, 0.39 mmol), 2,4 dimethoxybenzylamine (71.8

mg, 0.429 mmol) and potassium carbonate (162 mg, 1.17 mmol) were added, and the mixture was stirred at 80°C for 4 hours. Water was added to the reaction solution, extraction with dichloromethane was performed, and the solvent was distilled off under a reduced pressure. The residue obtained was purified by silica gel column chromatography (hexane-ethyl acetate) to obtain Compound 83 (259 mg, yield 77%).

1H-NMR ($CDCl_3$) δ: 2.29 (3H, s), 3.60 (1H, dd, J = 10.7, 4.2 Hz), 3.78 (1H, dd, J = 10.6, 3.7 Hz), 3.83 (3H, s), 3.92 (3H, s), 4.01 (1H, t, J = 5.5 Hz), 4.33 (1H, d, J = 11.9 Hz), 4.52 (4H, ddd, J = 29.3, 18.4, 8.4 Hz), 4.71 (2H, s), 4.77 (2H, d, J = 5.4 Hz), 4.86 (1H, d, J = 5.0 Hz), 6.47 (1H, dd, J = 8.3, 1.7 Hz), 6.55 (1H, s), 7.04 (2H, d, J = 8.3 Hz), 7.14 (3H, d, J = 5.3 Hz), 7.27 (13H, dt, J = 19.2, 7.1 Hz), 7.47 (2H, d, J = 8.2 Hz), 7.94 (1H, t, J = 5.4 Hz), 8.00 (1H, s), 8.33 (1H, s).

Steps 3, 4, 5

A THF solution (1.50 mL, 1.50 mmol) of 1 M TBAF was added to a THF (2.6 mL) solution of Compound 83 (259 mg, 0.30 mmol), and the mixture was stirred at room temperature for 5 hours. Water was added to the reaction solution, extraction with dichloromethane was performed, and the solvent was distilled off under a reduced pressure. The residue obtained was purified by silica gel column chromatography (hexane-ethyl acetate) to obtain Compound 84 (155 mg, yield 73%).

A mixed solution of TFA-water (9: 1, 1 mL) was added to Compound 84 (152 mg, 0.214 mmol), and the mixture was allowed to stand overnight. The solvent of the reaction solution was distilled off under a reduced pressure. The residue obtained was purified by silica gel column chromatography (hexane-ethyl acetate) to obtain Compound 85 (101 mg, yield 84%).

A dichloromethane solution (0.48 mL, 0.48 mmol) of 1 M boron trichloride was added to a dichloromethane (0.54 mL) solution of Compound 85 (27 mg, 0.048 mmol) at -78°C, and the mixture was stirred at room temperature for 30 minutes. Methanol (1 mL) was added to the reaction solution, and the solvent was distilled off under a reduced pressure. The obtained residue was purified by silica gel column chromatography (chloroform-methanol) to obtain Compound I-048 (6 mg, 43% yield).

1H-NMR (MeOD) δ: 3.62 (1H, d, J = 12.5 Hz), 3.79 (1H, d, J = 12.3 Hz), 4.24 (2H, t, J = 4.3 Hz), 4.65 (1H, d, J = 4.8 Hz), 7.61 (1H, s), 8.01 (1H, s).

Example 25

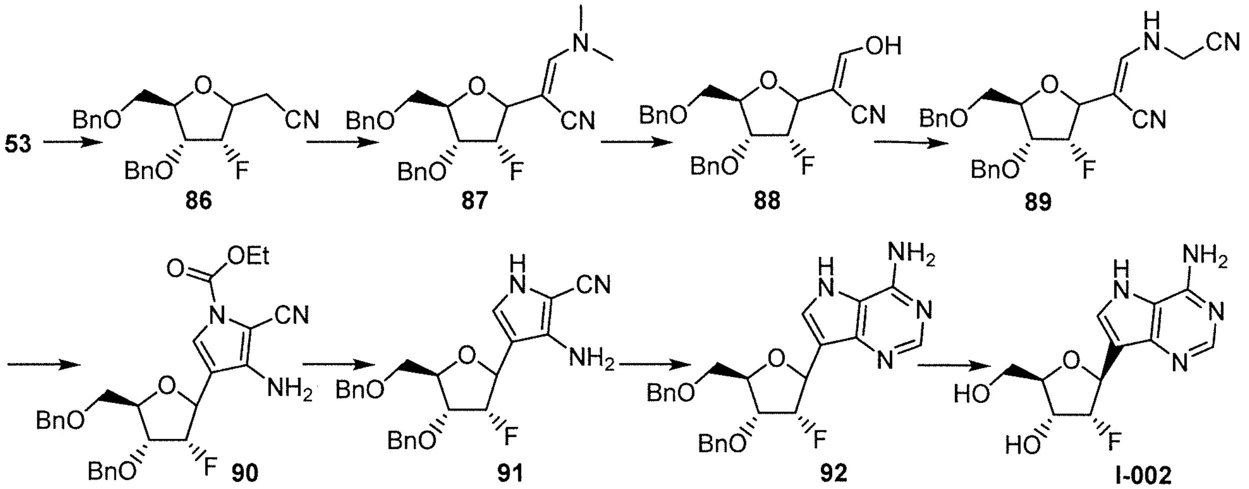

53 86 87 88 89 90 91 92 I-002

Step 1

Diethyl cyanomethylphosphonate (25.7 mL, 159 mmol) was added to a THF (308 mL) solution of potassium tert-butoxide (17.8 g, 159 mmol) at 0°C, and the mixture was stirred for 10 minutes. A THF (132 mL) solution of Compound 53 (44.0 g, 132 mmol) was added to the reaction solution at 0°C, and the mixture was stirred at room temperature for 1.5 hours. Methyl tert-butyl ether and a saturated ammonium chloride aqueous solution were added to the reaction solution, and extraction with methyl tert-butyl ether was performed. The organic layer was washed with water and saturated saline solution, and dried over anhydrous sodium sulfate. The solvent was distilled off under a reduced pressure. The residue obtained was purified by silica gel column chromatography (hexane-ethyl acetate) to obtain Compound 86

(42.1 g, 87% yield, dr = 1 : 1).

1H-NMR ($CDCl_3$) δ: 2.67-2.69 (m, 0.75H), 2.74-2.75 (m, 1.25H), 3.52-3.57 (m, 1H), 3.64-3.72 (m, 1H), 4.01-4.21 (m, 2H), 4.31-4.35 (m, 0.5H), 4.37-4.40 (m, 0.5H), 4.47-4.59 (m, 3H), 4.71 (t, J = 11.7 Hz, 1H), 4.77 (t, J = 4.3 Hz, 0.25H), 4.90 (t, J = 4.3 Hz, 0.25H), 4.94-4.96 (m, 0.25H), 5.07-5.10 (m, 0.25H), 7.30-7.35 (m, 10H).

Step 2

Tert-butoxybis(dimethylamino)methane (52.3 mL, 253 mmol) was added to a THF (450 mL) solution of Compound 86 (45.0 g, 127 mmol), and the mixture was stirred at 40°C overnight. A saturated ammonium chloride aqueous solution was added to the reaction solution, and extraction with ethyl acetate was performed. The organic layer was washed with saturated saline solution, and dried over anhydrous sodium sulfate. The solvent was distilled off under a reduced pressure. The residue obtained was purified by silica gel column chromatography (hexane-ethyl acetate) to obtain Compound 87 (44.5 g, 86% yield, dr = 1 : 1).

1H-NMR ($CDCl_3$) δ: 3.05 (s, 3H), 3.11 (s, 3H), 3.55-3.62 (m, 1H), 3.66-3.72 (m, 1H), 3.98-4.04 (m, 0.5H), 4.10-4.16 (m, 0.5H), 4.22-4.24 (m, 1H), 4.44-4.60 (m, 4H), 4.67-4.74 (m, 1H), 4.75-4.77 (m, 0.25H), 4.87-4.90 (m, 0.5H), 5.02 (t, J = 4.3 Hz, 0.25H), 6.58 (s, 0.5H), 6.62 (s, 0.5H), 7.28-7.36 (m, 10H).

Steps 3 to 6

Water (269 mL) and acetic acid (269 mL) were added to a THF (269 mL) solution of Compound 87 (53.7 g, 131 mmol), and stirred overnight at room temperature. Water was added to the reaction solution, and extraction with ethyl acetate was performed. A sodium carbonate aqueous solution was added to the organic layer until the pH reached 8, and extraction with ethyl acetate was performed. The organic layer was washed with saturated saline solution and dried over anhydrous sodium sulfate, then the solvent was distilled off under a reduced pressure to obtain Compound 88 (51.0 g).

Water (51 mL), sodium acetate (22.5 g, 274 mmol), and aminoacetonitrile hydrochloride (25.3 g, 274 mmol) were added to a methanol (510 mL) solution of Compound 88 (51.0 g, 130 mmol), and the mixture was stirred at room temperature for 6 hours. The reaction solution was subjected to distillation under a reduced pressure, a saturated ammonium chloride aqueous solution was added to the concentrated solution, and extraction with ethyl acetate was performed. The organic layer was washed with a 5% sodium bicarbonate aqueous solution and saturated saline solution, and dried over anhydrous magnesium sulfate, then the solvent was distilled off under a reduced pressure to obtain Compound 89 (54.5 g).

Ethyl chloroformate (18.5 mL, 193 mmol) and diazabicycloundecene (77.6 mL, 514 mmol) were added to a dichloromethane (542 mL) solution of Compound 89 (54.2 g, 129 mmol) under a nitrogen atmosphere at 0°C, and the mixture was stirred at room temperature for 5 hours. To the reaction solution, 2M hydrochloric acid was added, and extraction with chloroform was performed. The organic layer was washed with water, a saturated sodium bicarbonate aqueous solution, and saturated saline solution, and dried over anhydrous magnesium sulfate, then the solvent was distilled off under a reduced pressure to obtain Compound 90 (80.5 g).

Potassium carbonate (53.3 g, 386 mmol) was added to an ethanol (480 mL) solution of Compound 90 (80.5 g, 129 mmol), and the mixture was stirred at room temperature for 2.5 hours. The solvent was distilled off under a reduced pressure, water was added to the concentrated solution, and extraction with ethyl acetate was performed. The organic layer was washed with saturated saline solution and dried over anhydrous magnesium sulfate, and thereafter the solvent was distilled off under a reduced pressure. The residue obtained was purified by silica gel column chromatography (hexane-ethyl acetate) to obtain Compound 91 (34.2 g, 4-step yield 62%, dr (α : 6) = 1 : 3) was obtained.

1H-NMR ($CDCl_3$) δ: 3.59-3.64 (m, 1H), 3.76 (dd, J = 10.8, 2.5 Hz, 0.25H), 3.83 (dd, J = 10.5, 2.3 Hz, 0.75H), 3.91 (brs, 0.5H), 4.04 (brs, 1.5H), 4.13-4.16 (m, 1H), 4.23-4.28 (m, 1H), 4.45-4.61 (m, 3H), 4.71 (d, J = 11.5 Hz, 0.25H), 4.78 (d, J = 11.8 Hz, 0.75H), 4.91-4.93 (m, 0.5H), 4.98-4.99 (m, 0.5H), 5.03-5.06 (m, 1H), 6.66 (d, J = 3.3 Hz, 0.75H), 6.72 (d, J = 3.3 Hz, 0.25H), 7.24-7.38 (m, 10H), 7.75 (brs, 0.75H), 7.88 (brs, 0.25H).

Step 7

Formamidine acetate (42.2 g, 405 mmol) was added to an ethanol (342 mL) suspension of Compound 91 (34.2 g, 81.0 mmol), and the mixture was stirred under reflux for 2.5 hours. The reaction solution was subjected to distillation under a reduced pressure, water was added to the concentrated solution, and extraction with ethyl acetate was performed. The organic layer was washed with a saturated sodium bicarbonate aqueous solution and saturated saline solution, dried over anhydrous magnesium sulfate, and then the solvent was distilled off under a reduced pressure. Ethyl acetate-diisopropyl ether was added to the obtained residue to precipitate a solid, which was then collected by filtration to obtain Compound 92 (34.5 g, 95% yield, dr (a : 8) = 1 : 2).

1H-NMR (MeOD) δ: 3.64-3.88 (m, 2H), 4.19-4.48 (m, 2H), 4.51-4.63 (m, 3H), 4.71-4.77 (m, 1H), 5.13-5.18 (m, 0.5H), 5.26-5.31 (m, 0.5H), 5.49-5.56 (m, 1H), 7.28-7.38 (m, 10H), 7.52 (s, 0.7H), 7.61 (s, 0.3H), 8.13 (s, 0.7H), 8.16 (s, 0.3H).

Step 8

Acetic acid (5 mL) and palladium-carbon (3.00 g, 2.82 mmol) were added to a THF (20 mL) solution of Compound 92 (1.50 g, 3.34 mmol), and the mixture was stirred overnight at room temperature under a hydrogen atmosphere. The reaction solution was filtered through celite, toluene was added thereto, and distillation under a reduced pressure was performed. The residue obtained was purified by silica gel column chromatography (ethyl acetate-methanol) to obtain Compound I-002 (450 mg, 50% yield).

1H-NMR (MeOD) δ: 3.75 (dd, J = 12.5, 3.0 Hz, 1H), 3.94 (dd, J = 12.5, 2.0 Hz, 1H), 4.06-4.09 (m, 1H), 4.44 (dt, J = 13.1, 5.0 Hz, 1H), 5.07 (t, J = 4.5 Hz, 0.5H), 5.21 (t, J = 4.5 Hz, 0.5H), 5.34 (d, J = 4.5 Hz, 0.5H), 5.39 (d, J = 4.5 Hz, 0.5H), 7.62 (s, 1H), 8.15 (s, 1H).

Example 26

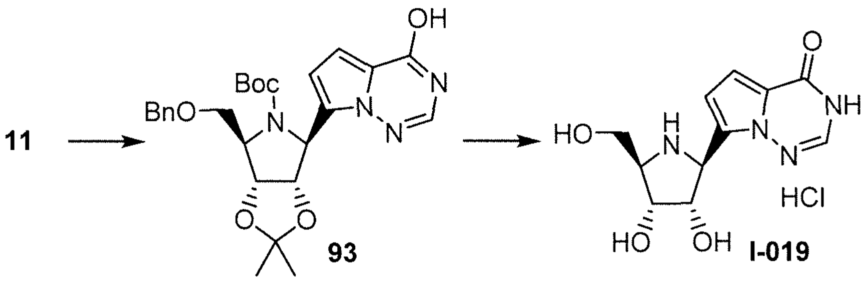

Step 1

Trimethylsilyl chloride (0.206 mL, 1.61 mmol) and tert-butyl nitrite (0.480 mL, 4.04 mmol) were added to a THF (10 mL) solution of Compound 11 (400 mg, 0.807 mmol) at 0°C, and the mixture was stirred at room temperature for 1 hour and a half. Saturated sodium bicarbonate water was added to the reaction solution, and extraction with ethyl acetate was performed. The organic layer was washed with saturated saline solution and dried over anhydrous magnesium sulfate, and thereafter the solvent was distilled off under a reduced pressure. The residue obtained was purified by silica gel column chromatography (hexane-ethyl acetate) to obtain Compound 93 (85 mg, yield 21%).

1H-NMR ($CDCl_3$) δ: 1.16-1.49 (12H, m), 1.59 (3H, s), 3.53-3.90 (2H, m), 4.25-4.43 (1H, m), 4.57 (2H, s), 4.73-4.86 (2H, m), 5.34-5.60 (1H, m), 6.37-6.51 (1H, m), 6.97 (1H, d, J = 4.5 Hz), 7.26-7.37 (5H, m), 7.53 (1H, d, J = 3.5 Hz), 9.66-9.88 (1H, m).

Step 2

Compound I-019 was synthesized in the same manner as Step 13 in Example 1.

1H-NMR (MeOD) δ: 3.64 (1H, dd, J = 8.3, 3.8 Hz), 3.70-3.74 (2H, m), 4.21 (1H, t, J = 4.0 Hz), 4.66 (1H, dd, J = 8.0, 4.5 Hz), 4.94 (1H, d, J = 8.0 Hz), 6.73 (1H, d, J = 4.5 Hz), 6.95 (1H, d, J = 4.5 Hz), 7.74 (1H, s).

The following compounds were synthesized according to the above general synthesis method and the method described in Examples. The structure and physical properties (LC/MS data, NMR spectrum) are shown in the tables below. "No." represents compound number, "Structure" means a chemical structure, and "MS" represents a mass in LC/MS (liquid chromatography/mass spectrometry).

Incidentally, in the structural formula, "wedge form" and "broken line" indicate the configuration.

[Table 1]

| No. | Structure | NMR or MS |
|---|---|---|
| I-001 | 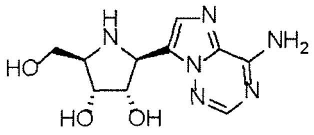 | 1H-NMR (D2O) δ: 3.32 (1H, q, J = 5.1 Hz), 3.69-3.78 (2H, m), 4.15 (1H, t, J = 5.1 Hz), 4.48 (1H, dtd, J = 7.7, 5.8, 2.9 Hz), 4.55 (1H, d, J = 8.3 Hz), 7.64 (1H, s), 8.07 (1H, s). |
| I-003 | 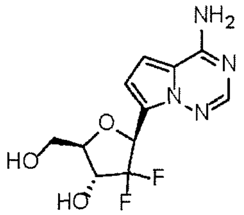 | 1H-NMR (DMSO-D6) δ: 3.57-3.62 (m, 1H), 3.65-3.68 (m, 1H), 3.78-3.81 (m, 1H), 4.09-4.14 (m, 1H), 4.98-5.01 (m, 1H), 5.56 (dd, J = 14.3, 10.8 Hz, 1H), 6.13-6.15 (m, 1H), 6.75 (d, J = 4.0 Hz, 1H), 6.90 (d, J = 4.5 Hz, 1H), 7.81-7.87 (m, 3H). |
| I-004 | 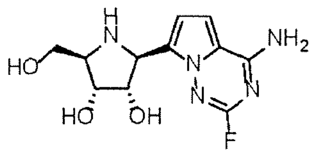 | 1H-NMR (MeOD) δ: 3.34-3.37 (1H, m), 3.60-3.61 (1H, m), 3.81-3.82 (2H, m), 4.27 (1H, t, J = 4.1 Hz), 4.66-4.68 (1H, m), 6.83 (1H, d, J = 4.8 Hz), 6.98 (1H, d, J = 4.5 Hz). |
| I-005 | 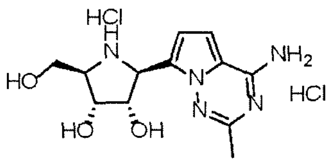 | 1H-NMR (MeOD) δ: 3.81-3.82 (1H, m), 3.87-3.88 (2H, m), 4.35 (1H, t, J = 4.0 Hz), 4.81 (1H, dd, J = 8.2, 4.6 Hz), 5.10 (1H, d, J = 8.0 Hz), 7.05 (1H, d, J = 4.3 Hz), 7.33-7.35 (1H, m). |
| I-006 | 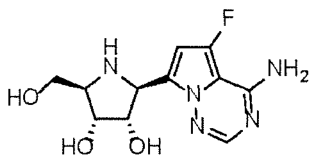 | 1H-NMR (MeOD) δ: 3.18 (1H, q, J = 4.6 Hz), 3.65-3.75 (2H, m), 4.00 (1H, t, J = 5.4 Hz), 4.22 (1H, t, J = 6.4 Hz), 4.51 (1H, d, J = 7.0 Hz), 6.49 (1H, s), 7.69 (1H, s). |
| I-007 | 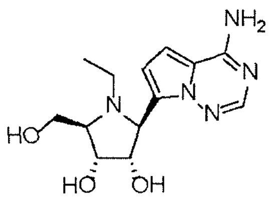 | 1H-NMR ($CD_3OD$) δ: 7.74 (s, 1H), 6.85 (d, J = 4.4 Hz, 1H), 6.68 (d, J = 4.4 Hz, 1H), 4.44-4.37 (m, 2H), 4.05-4.03 (m, 1H), 3.63 (d, J = 2.9 Hz, 2H), 2.97-2.96 (m, 1H), 2.75-2.60 (m, 2H), 0.88 (t, J = 7.1Hz, 3H). |
| I-009 | 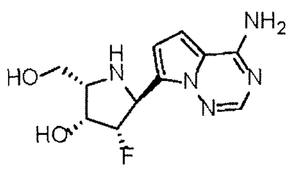 | 1H-NMR (MeOD) δ: 3.49 (1H, dd, J = 11.5, 6.1 Hz), 3.60 (1H, ddd, J = 10.9, 6.8, 1.3 Hz), 3.76 (1H, dd, J = 11.0, 6.0 Hz), 4.37 (1H, dt, J = 8.6, 4.1 Hz), 4.78-4.81 (1H, m), 5.24 (1H, ddd, J = 53.2, 6.4, 4.3 Hz), 6.59 (1H, d, J = 4.5 Hz), 6.76 (1H, d, J = 4.5 Hz), 7.70 (1H, s). |
| I-011 | 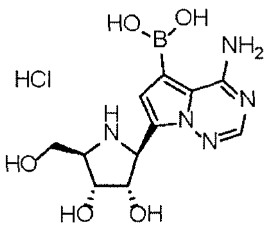 | 1H-NMR (MeOD) δ: 8.25-8.24 (m, 1H), 7.54-7.53 (m, 1H), 5.13 (d, J = 8.2 Hz, 1H), 4.88-4.84 (m, 1H), 4.37 (dd, J = 3.6, 3.6 Hz, 1H), 3.92-3.82 (m, 3H). |

(continued)

| No. | Structure | NMR or MS |
|---|---|---|
| I-015 | 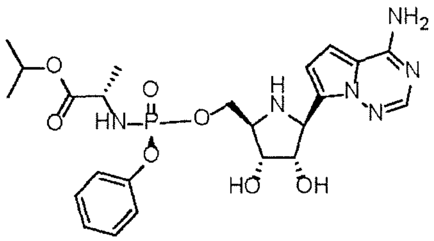 | 1H-NMR (MeOD) δ: 1.20 (d, J = 6.5 Hz, 3H), 1.22 (d, J = 6.5 Hz, 3H), 1.33 (d, J = 7.3 Hz, 3H), 3.36-3.40 (m, 1H), 3.90-3.94 (m, 1H), 4.07 (t, J = 5.6 Hz, 1H), 4.20-4.36 (m, 3H), 4.57 (d, J = 6.5 Hz, 1H), 4.92-4.98 (m, 1H), 6.69 (d, J = 4.5 Hz, 1H), 6.87 (d, J = 4.5 Hz, 1H), 7.17-7.20 (m, 1H), 7.23-7.25 (m, 2H), 7.35 (t, J = 7.8 Hz, 2H), 7.79 (s, 1H). |

[Table 2]

| No. | Structure | NMR or MS |
|---|---|---|
| I-019 | 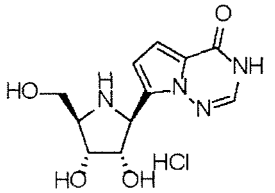 | 1H-NMR (MeOD) δ: 3.64 (1H, dd, J = 8.3, 3.8 Hz), 3.70-3.74 (2H, m), 4.21 (1H, t, J = 4.0 Hz), 4.66 (1H, dd, J = 8.0, 4.5 Hz), 4.94 (1H, d, J = 8.0 Hz), 6.73 (1H, d, J = 4.5 Hz), 6.95 (1H, d, J = 4.5 Hz), 7.74 (1H, s). |
| I-021 | 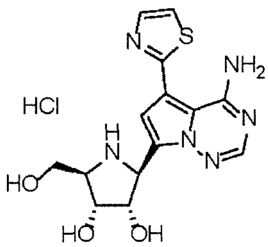 | 1H-NMR (MeOD) δ: 10.17 (br s, 0.6H), 9.28-9.24 (m, 0.4H), 8.61-8.57 (m, 0.6H), 8.26-8.22 (m, 1.0H), 8.08-8.05 (m, 0.4H), 7.36-7.31 (m, 0.6H), 7.26-7.23 (m, 0.4H), 5.17 (d, J = 8.2 Hz, 1.0H), 4.92-4.83 (m, 1.0H), 4.36 (dd, J = 6.0, 3.0 Hz, 1.0H), 3.89-3.84 (m, 3.0H). |
| I-023 | 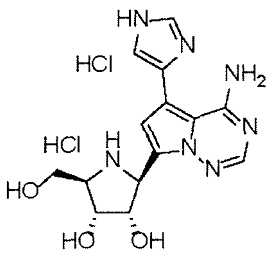 | 1H -NMR (MeOD) δ: 9.00 (s, 0.2H), 8.17-8.07 (m, 1.8H), 7.82-7.76 (m, 1.0H), 7.46 (s, 1.0H), 5.13 (d, J = 8.0 Hz, 1.0H), 4.92-4.86 (m, 1.0H), 4.38 (dd, J = 4.0, 4.0 Hz, 1.0H), 3.93-3.85 (m, 3.0H). |
| I-026 | 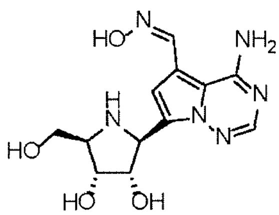 | MS(m/z) = 309 |
| I-027 | 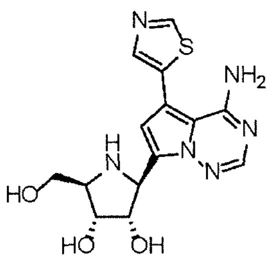 | 1H-NMR ($CD_3OD$) δ: 3.82-3.85 (m, 4H), 4.32-4.34 (m, 1H), 5.15 (d, J = 8.3 Hz, 1H), 7.23 (s, 1H), 8.04 (s, 1H), 8.23 (s, 1H), 9.24 (s, 1H). |

(continued)

| No. | Structure | NMR or MS |
|---|---|---|
| I-029 | HCl 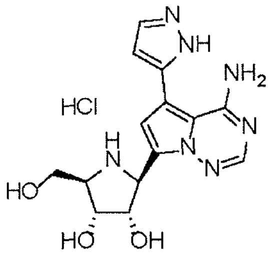 | 1H-NMR (MeOD) δ: 8.15 (s, 1H), 7.86 (d, J = 2.4 Hz, 1H), 7.57 (s, 1H), 6.94 (d, J = 2.4 Hz, 1H), 5.14 (d, J = 8.2 Hz, 1H), 4.93-4.90 (m, 1H), 4.38 (dd, J = 4.3, 3.0 Hz, 1H), 3.93-3.83 (m, 3H). |
| I-031 | 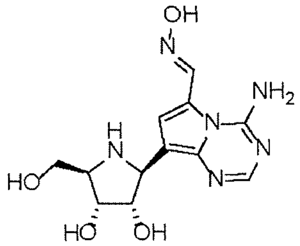 | 1H-NMR (MeOD) δ: 3.13-3.17 (m, 1H), 3.74 (d, J = 4.3 Hz, 2H), 4.01-4.07 (m, 2H), 4.26 (d, J = 6.8 Hz, 1H), 7.12 (s, 1H), 7.73 (s, 1H), 8.09 (s, 1H). |

Table 3]

| No. | Structure | NMR or MS |
|---|---|---|
| I-032 | HCl 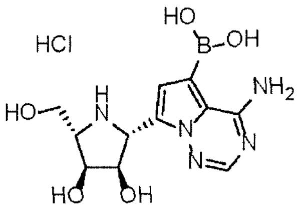 | 1H-NMR (MeOD) δ: 8.23-8.22 (m, 1H), 7.53-7.52 (m, 1H), 5.13 (d, J = 8.2 Hz, 1H), 4.91-4.83 (m, 1H), 4.37 (dd, J = 4.2, 2.1Hz, 1H), 3.92-3.82 (m, 3H). |
| I-033 | HCl HCl 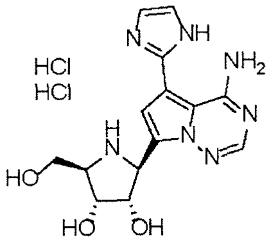 | 1H-NMR (MeOD) δ: 8.16 (s, 1H), 7.57 (s, 1H), 7.31 (s, 2H), 5.14 (d, J = 8.5 Hz, 1H), 4.93-4.91 (m, 1H), 4.38 (dd, J = 4.3, 2.7 Hz, 1H), 3.93-3.85 (m, 3H). |
| I-034 | 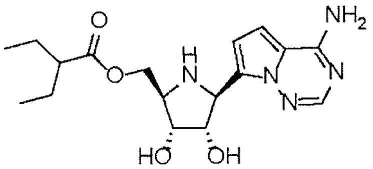 | 1H-NMR ($CD_3OD$) δ: 1.05 (t, J = 7.4 Hz, 6H), 1.68-1.83 (m, 4H), 2.43 (tt, J = 8.6, 3.9 Hz, 1H), 3.54-3.59 (m, 1H), 4.17 (t, J = 5.8 Hz, 1H), 4.37-4.45 (m, 2H), 4.48 (t, J = 6.7 Hz, 1H), 4.66 (d, J = 6.7 Hz, 1H), 6.84 (d, J = 4.4 Hz, 1H), 7.01 (d, J = 4.4 Hz, 1H), 7.92 (s, 1H). |
| I-035 | 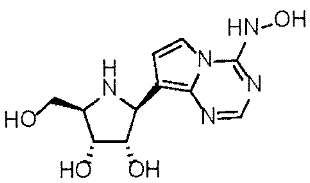 | 1H-NMR (MeOD) δ: 3.13-3.16 (m, 1H), 3.77 (d, J = 4.3 Hz, 2H), 4.01-4.07 (m, 2H), 4.26 (d, J = 6.5 Hz, 1H), 6.57 (d, J = 3.3 Hz, 1H), 7.08 (d, J = 3.5 Hz, 1H), 7.42 (s, 1H). |

(continued)

| No. | Structure | NMR or MS |
|---|---|---|
| I-036 | 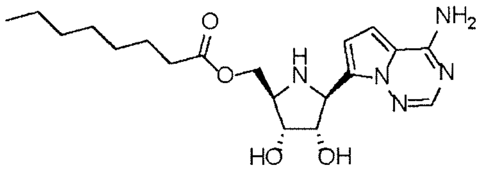 | 1H-NMR ($CD_3OD$) δ: 0.86 (t, J = 6.9 Hz, 3H), 1.25-1.28 (m, 8H), 1.56-1.63 (m, 2H), 2.34 (t, J = 7.4 Hz, 2H), 3.39-3.42 (m, 1H), 4.03-4.04 (m, 1H), 4.20 (dd, J = 11.7, 6.0 Hz, 1H), 4.30-4.36 (m, 2H), 4.56 (dd, J = 6.0, 3.2 Hz, 1H), 6.69 (d, J = 4.7 Hz, 1H), 6.85 (d, J = 4.4 Hz, 1H), 7.77 (s, 1H). |
| I-037 | 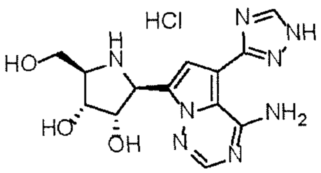 | 1H-NMR (MeOD) δ: 3.86-3.92 (3H, m), 4.38 (1H, dd, J = 4.4, 2.9 Hz), 4.92-4.94 (1H, m), 5.16 (1H, d, J = 8.2 Hz), 7.76 (1H, s), 8.24 (1H, s), 8.69 (1H, s). |
| I-039 | 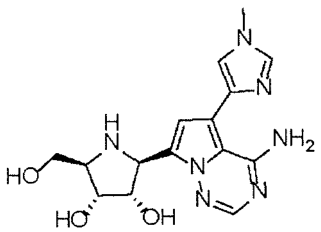 | 1H-NMR (MeOD) δ: 8.05-8.05 (br m, 1H), 7.83 (br s, 1H), 7.74 (br s, 1H), 7.39-7.37 (br m, 1H), 5.10 (d, J = 8.3 Hz, 1H), 4.92-4.86 (m, 1H), 4.37 (dd, J = 4.2, 3.1Hz, 1H), 3.92-3.82 (m, 6H). |
| I-040 | 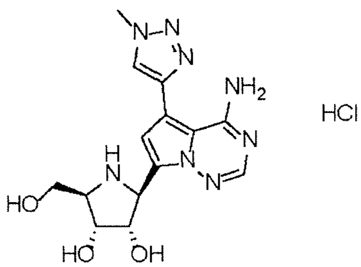 | 1H-NMR (D2O) δ: 8.24 (1H, s), 7.89 (1H, s), 7.22 (1H, s), 5.05 (1H, d, J = 8.3 Hz), 4.81 (1H, dd, J = 8.3, 4.9 Hz), 4.35 (1H, s), 4.07 (3H, s), 3.82 (3H, s). |

[Table 4]

| No. | Structure | NMR or MS |
|---|---|---|
| I-041 | 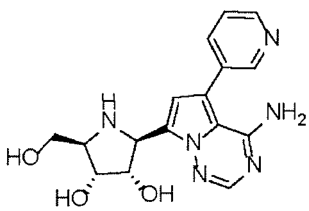 | 1H-NMR (MeOD) δ: 9.20 (br s, 1H), 8.98-8.97 (m, 1H), 8.85-8.83 (m, 1H), 8.33-8.22 (m, 2H), 7.43 (br s, 1H), 5.20 (d, J = 8.3 Hz, 1H), 4.93-4.87 (m, 1H), 4.38 (dd, J = 3.6, 3.6 Hz, 1H), 3.93-3.86 (m, 3H). |
| I-042 | 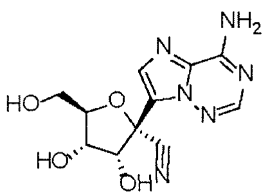 | 1H-NMR (D2O) δ: 3.81 (2H, ddd, J = 25.5, 12.7, 3.7 Hz), 4.39 (1H, dd, J = 5.3, 4.0 Hz), 4.44 (1H, dd, J = 7.3, 4.0 Hz), 4.98 (1H, d, J = 5.6 Hz), 7.86 (1H, s), 8.16 (1H, s). |

(continued)

| No. | Structure | NMR or MS |
|---|---|---|
| I-043 | 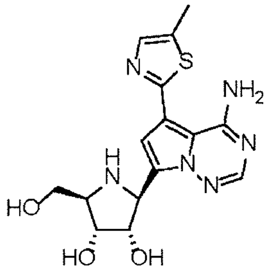 | 1H-NMR (MeOD) δ: 8.18 (1H, s), 7.65 (1H, s), 7.54 (1H, s), 5.12 (1H, d, J = 8.4 Hz), 4.86-4.84 (1H, br m), 4.37 (1H, t, J = 3.5 Hz), 3.91-3.86 (3H, m), 2.59 (3H, s). |
| I-044 | 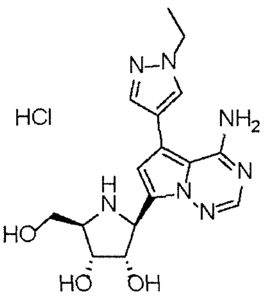 | 1H-NMR (DMSO-d6)δ: 1.43 (3H, t, J = 6.8 Hz), 3.17 (1H, s), 3.57 (1H, s), 3.62-3.75 (2H, m), 4.10-4.27 (3H, m), 4.50-4.67 (1H, m), 4.75-4.96 (1H, m), 7.00 (1H, s), 7.63 (1H, s), 7.99 (1H, s), 8.18 (1H, s), 8.76 (1H, s), 10.43 (1H,s). |
| I-045 | 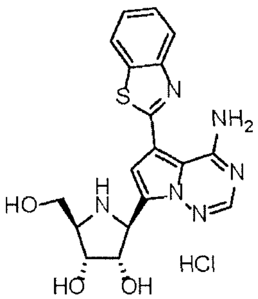 | 1H-NMR (DMSO-d6) δ : 3.65-3.70 (m, 1H), 3.78-3.83 (m, 2H), 4.24-4.29 (m, 1H), 4.63-4.69 (m, 1H), 5.02-5.07 (m, 1H), 7.43 (t, J = 8.0Hz, 1H), 7.51 (s, 1H), 7.53 (t, J = 8.0Hz, 1H), 7.95 (d, J = 8.0Hz, 1H), 8.04 (s, 1H), 8.05 (d, J = 8.0Hz, 1H). |
| I-047 | 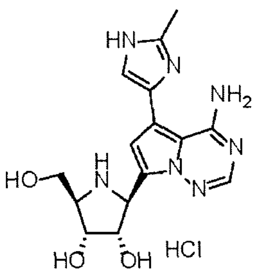 | 1H-NMR (MeOD) δ: 3.81-3.91 (3H, m), 4.37 (1H, t, J = 3.5 Hz), 4.83 (3H, s), 4.85-4.90 (1H, m), 5.14 (1H, d, J = 8.3 Hz), 7.35 (1H, s), 7.66 (1H, s), 8.19 (1H, s). |
| I-049 | 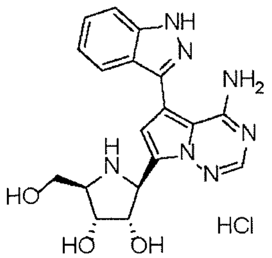 | 1H-NMR (MeOD) δ: 3.86-3.95 (m, 3H), 4.44 (dd, J = 4.3, 2.1 Hz, 1H), 4.96 (dd, J = 8.0, 4.4 Hz, 2H), 5.23 (d, J = 8.0 Hz, 1H), 7.36 (t, J = 7.7 Hz, 1H), 7.54 (t, J = 7.8 Hz, 1H), 7.66 (d, J = 8.7 Hz, 1H), 7.85 (s, 1H), 8.08 (s, 1H), 8.28 (d, J = 8.0 Hz, 1H). |

[Table 5]

| No. | Structure | NMR or MS |
|---|---|---|
| I-050 | 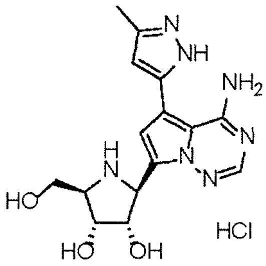 | 1H-NMR (MeOD) δ: 2.40 (s, 3H), 3.82-3.92 (m, 3H), 4.37 (dd, J = 3.8, 1.9 Hz, 1H), 4.90-4.92 (m, 1H), 5.12 (d, J = 8.3 Hz, 1H), 6.64 (s, 1H), 7.46 (s, 1H), 8.09 (s, 1H). |
| I-051 | 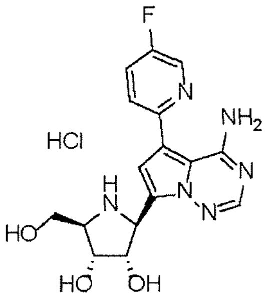 | 1H-NMR (D2O) δ: 8.35 (1H, s), 7.86 (2H, br s), 7.61 (1H, br s), 7.38 (1H, s), 5.05 (1H, d, J = 8.3 Hz), 4.57 (1H, dd, J = 11.3, 8.3 Hz), 4.38 (1H, d, J = 4.8 Hz), 3.82 (3H, s). |
| I-052 | 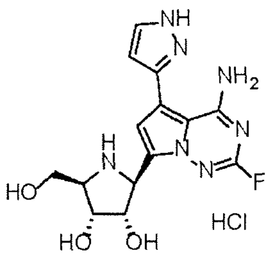 | 1H-NMR (MeOD) δ: 3.72-3.90 (m, 3H), 4.35 (t, J = 4.0 Hz, 1H), 4.82-4.85 (m, 1H), 5.01 (d, J = 8.0 Hz, 1H), 6.72 (d, J = 2.5 Hz, 1H), 7.23 (s, 1H), 7.72 (d, J = 2.5 Hz, 1H). |
| I-055 | 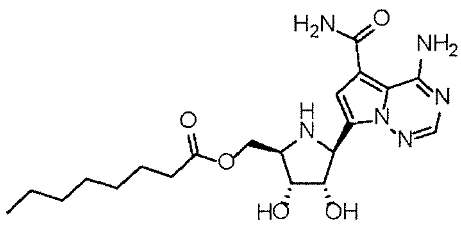 | 1H-NMR (DMSO-D6) δ: 0.84 (3H, t, J = 6.9 Hz), 1.23 (8H, t, J = 2.5 Hz), 1.51 (2H, t, J = 7.0 Hz), 2.29 (2H, t, J = 7.4 Hz), 3.01 (1H, s), 3.26-3.30 (1H, m), 3.72 (1H, q, J = 5.6 Hz), 3.93-3.97 (2H, m), 4.17 (1H, dd, J = 10.8, 4.5 Hz), 4.49 (1H, d, J = 4.5 Hz), 4.82 (1H, d, J = 5.8 Hz), 4.86 (1H, d, J = 5.8 Hz), 7.27 (1H, s), 7.47 (1H, s), 7.90 (1H, s), 7.98 (1H, d, J = 3.5 Hz), 8.09 (1H, s), 10.40 (1H, d, J = 3.5 Hz). |
| I-056 | 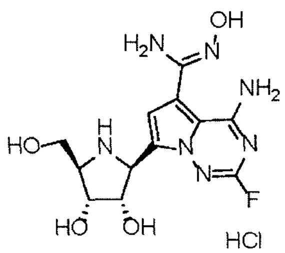 | 1H-NMR (MeOD) δ: 3.80-3.86 (m, 3H), 4.34 (dd, J = 4.3, 3.1 Hz, 3H), 4.81-4.84 (m, 1H), 5.03 (d, J = 8.3 Hz, 1H), 7.31 (s, 1H). |
| I-057 | 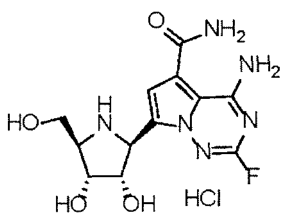 | 1H-NMR (MeOD) δ: 3.71-3.86 (m, 3H), 4.32 (t, J = 4.0 Hz, 1H), 4.80 (dd, J = 7.8, 4.6 Hz, 1H), 4.97 (d, J = 7.9 Hz, 1H). |

(continued)

| No. | Structure | NMR or MS |
|---|---|---|
| I-058 | 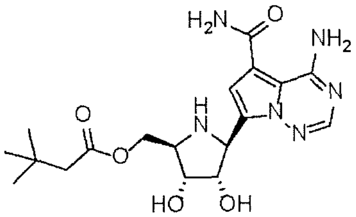 | 1H-NMR (DMSO-D6) δ: 0.98 (9H, s), 2.20 (2H, s), 3.00 (1H, s), 3.26-3.30 (2H, m), 3.73 (1H, q, J = 5.5 Hz), 3.92-3.98 (2H, m), 4.16 (1H, dd, J = 10.9, 4.9 Hz), 4.50 (1H, d, J = 4.8 Hz), 4.82 (1H, d, J = 5.8 Hz), 4.86 (1H, d, J = 5.8 Hz), 7.27 (1H, s), 7.47 (1H, s), 7.90 (1H, s), 7.97 (1H, d, J = 3.3 Hz), 8.09 (1H, s), 10.40 (1H, d, J = 3.3 Hz). |

[Table 6]

| No. | Structure | NMR or MS |
|---|---|---|
| I-059 | 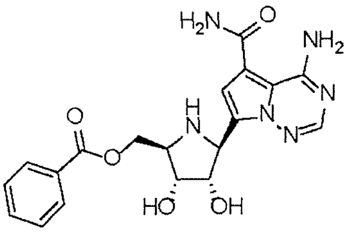 | 1H-NMR (DMSO-d6) δ : 3.41-3.49 (m, 1H), 3.85 (ddd, J = 6.0, 6.0, 6.0 Hz, 1H), 3.99 (ddd, J = 5.2 5.2, 5.2 Hz, 1H), 4.24 (dd, J = 6.8, 10.8 Hz, 1H), 4.43 (dd, J = 4.4, 10.8 Hz, 1H), 4.51-4.57 (m, 1H), 4.88-4.93 (m, 2H), 7.30 (s, 1H), 7.46 (br-s, 1H), 7.53 (dd, J = 8.0, 8.0 Hz, 1H), 7.66 (tdd, J = 8.0, 1.2, 1.2 Hz, 1H), 7.88 (s, 1H), 7.94-7.99 (m, 1H), 8.01 (dd, J = 8.0, 1.2 Hz, 2H), 8.08 (br-s, 1H), 10.37-10.44 (m, 1H). |
| I-060 | 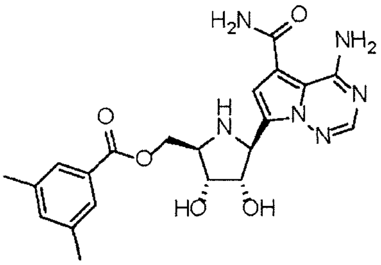 | 1H-NMR (DMSO-d6) δ : 2.32 (s, 6H), 2.38-2.48 (m, 1H), 3.77-3.85 (m, 1H), 3.93-3.99 (m, 1H), 4.19 (dd, J = 7.2, 10.8 Hz, 1H), 4.40 (dd, J = 4.8, 10.8 Hz, 1H), 4.51-4.57 (m, 1H), 4.84-4.94 (m, 2H), 7.25-7.30 (m, 2H), 7.43 (br-s, 1H), 7.61 (s, 2H), 7.87 (s, 1H), 7.91-7.99 (m, 1H), 8.05 (br-s, 1H), 10.35-10.43 (m, 1H). |
| I-062 | 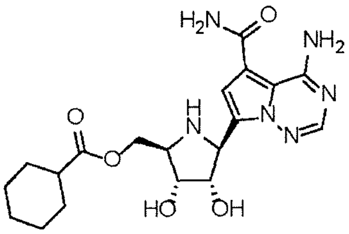 | 1H-NMR (DMSO-d6) δ : 1.10-1.41 (m, 6H), 1.61-1.69 (m, 2H), 1.76-1.86 (m, 2H), 2.24-2.35 (m, 1H), 2.92-3.14 (m, 1H), 3.23-3.31 (m, 1H), 3.70-3.77 (m, 1H), 3.90-4.00 (m, 2H), 4.16 (dd, J = 10.8, 4.8 Hz, 1H), 4.49 (d, J = 4.8 Hz, 1H), 4.81 (d, J = 6.0 Hz, 1H), 4.86 (d, J = 5.6 Hz, 1H), 7.27 (s, 1H), 7.47 (br-s, 1H), 7.89 (s, 1H), 7.93-8.01 (m, 1H), 8.09 (br-s, 1H), 10.36-10.45 (m, 1H). |
| I-063 | 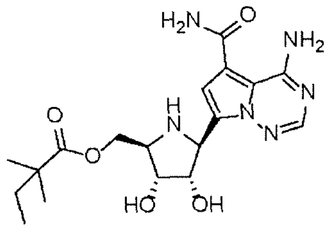 | 1H-NMR (CD3OD)δ: 0.85 (3H, t, J = 7.5 Hz), 1.18 (6H, s), 1.60 (2H, q), 3.38 (1H, q, J = 5.3 Hz), 4.01 (1H, t, J = 5.6 Hz), 4.18-4.27 (2H, m), 4.34 (1H, t, J = 6.3 Hz), 4.49 (1H, d, J = 6.5 Hz), 7.23 (1H, s), 7.84 (1H, s). |
| I-064 | 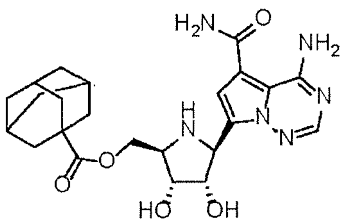 | 1H-NMR (CD3OD)δ: 1.72, 1.81 (6H, ABq, J = 12.3 Hz), 1.88-1.92 (6H, m), 1.97-2.03 (3H, m), 3.37 (1H, q, J = 5.0 Hz), 4.04 (1H, t, J = 5.8 Hz), 4.17-4.27 (2H, m), 4.37 (1H, t, J = 5.9 Hz), 4.51 (1H, d, J = 6.0 Hz), 7.23 (1H, s), 7.84 (1H,s). |

(continued)

| No. | Structure | NMR or MS |
|---|---|---|
| I-065 | 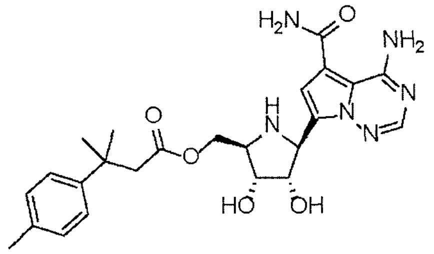 | 1H-NMR (MeOD) δ: 7.90 (1H, s), 7.24 (2H, d, J = 8.1 Hz), 7.22 (1H, s), 7.02 (2H, d, J = 8.1 Hz), 4.51 (1H, d, J = 6.0 Hz), 4.26 (1H, t, J = 6.0 Hz), 4.13 (1H, dd, J = 11.4, 4.1 Hz), 4.00 (1H, dd, J = 11.4, 6.5 Hz), 3.83 (1H, t, J = 5.7 Hz), 3.24-3.22 (1H, m), 2.68 (2H, s), 2.19 (3H, s), 1.44 (6H, s). |
| I-066 | 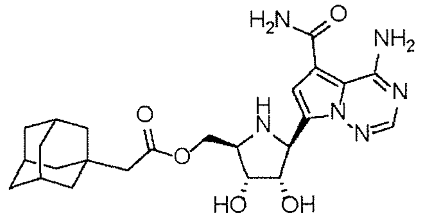 | 1H-NMR (MeOD) δ: 7.88 (1H, s), 7.25 (1H, s), 4.55 (1H, d, J = 6.1 Hz), 4.36 (1H, t, J = 6.1 Hz), 4.27 (1H, dd, J = 11.3, 4.4 Hz), 4.17 (1H, dd, J = 11.3, 6.0 Hz), 4.04 (1H, t, J = 5.7 Hz), 3.41 (1H, dd, J = 10.2, 5.7 Hz), 2.12 (2H, s), 1.95 (3H, s), 1.75 (3H, d, J = 12.2 Hz), 1.66 (9H, d, J = 12.2 Hz). |

[Table 7]

| No. | Structure | NMR or MS |
|---|---|---|
| I-067 | 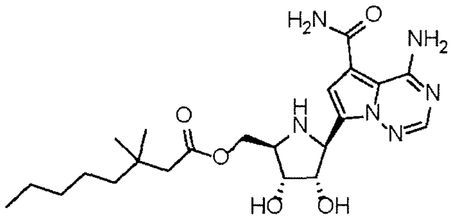 | 1H-NMR (MeOD) δ: 7.88 (1H, s), 7.25 (1H, s), 4.55 (1H, d, J = 6.3 Hz), 4.35 (1H, t, J = 6.0 Hz), 4.28 (1H, dd, J = 11.4, 4.4 Hz), 4.18 (1H, dd, J = 11.4, 6.0 Hz), 4.02 (1H, t, J = 5.4 Hz), 3.42 (1H, t, J = 5.4 Hz), 2.26 (2H, s), 1.30-1.26 (8H, m), 1.01 (6H, s), 0.89 (3H, t, J = 7.1 Hz). |
| I-068 | 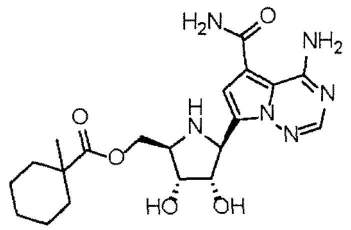 | 1H-NMR (DMSO-D6) δ: 1.10 (3H, s), 1.16-1.29 (6H, m), 1.43-1.50 (2H, m), 1.92-1.96 (2H, m), 3.02 (1H, s), 3.26-3.30 (1H, m), 3.75 (1H, q, J = 5.6 Hz), 3.95-4.04 (2H, m), 4.17 (1H, dd, J = 10.9, 4.7 Hz), 4.48 (1H, d, J = 4.8 Hz), 4.82 (1H, d, J = 5.8 Hz), 4.87 (1H, d, J = 5.5 Hz), 7.27 (1H, s), 7.47 (1H, s), 7.89 (1H, s), 7.98 (1H, d, J = 3.1 Hz), 8.09 (1H, s), 10.41 (1H, d, J = 3.0 Hz). |
| I-069 | 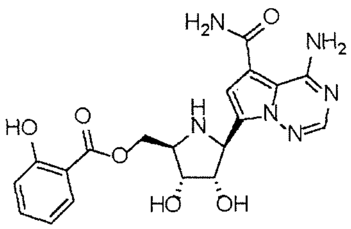 | 1H-NMR (DMSO-d6) δ : 3.44-3.51 (m, 1H), 3.82-3.88 (m, 1H), 3.99 (ddd, J = 4.8, 4.8 4.8 Hz, 1H), 4.29 (dd, J = 6.4, 10.8 Hz, 1H), 4.47 (dd, J = 4.8, 10.8 Hz, 1H), 4.52-4.57 (m, 1H), 4.90-4.96 (m, 2H), 6.94 (t, J = 7.2 Hz, 1H), 6.98 (d, J = 8.4 Hz, 1H), 7.30 (s, 1H), 4.46 (br-s, 1H), 7.53 (dd, J =7.2, 1.6 Hz, 1H), 7.86-7.90 (m, 2H), 7.95-7.99 (m, 2H), 8.07 (br-s, 1H), 10.36-10.45 (m, 1H), 10.53 (br-s, 1H). |
| I-070 | 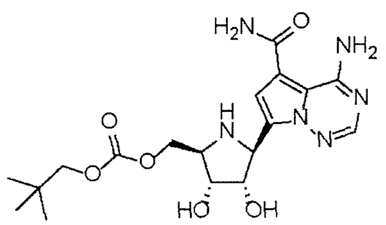 | 1H-NMR (MeOD) δ: 0.95 (s, 9H), 3.42-3.48 (m, 1H), 3.84 (dd, J = 12.0, 10.2 Hz, 2H), 4.00 (t, J = 5.6 Hz, 1H), 4.21 (dd, J = 11.0, 6.4 Hz, 1H), 4.27-4.36 (m, 2H), 4.58 (d, J = 6.0 Hz, 1H), 7.23 (s, 1H), 7.86 (s, 1H). |

(continued)

| No. | Structure | NMR or MS |
|---|---|---|
| I-071 | 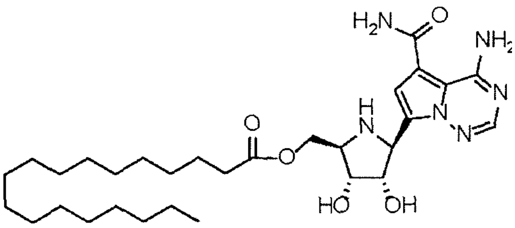 | 1H-NMR (MeOD) δ: 7.87 (1H, s), 7.24 (1H, s), 4.61 (1H, br s), 4.55 (1H, d, J = 6.5 Hz), 4.33-4.30 (2H, m), 4.19 (1H, dd, J = 11.8, 6.5 Hz), 4.01 (1H, t, J = 5.8 Hz), 3.40-3.39 (2H, m), 2.37 (2H, t, J = 7.3 Hz), 1.65-1.62 (2H, br m), 1.28 (26H, d, J = 6.8 Hz), 0.91 (3H, t, J = 6.8 Hz). |
| I-072 | 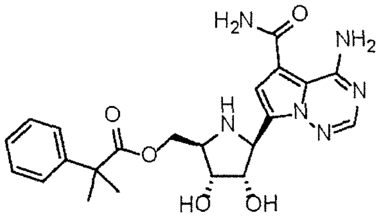 | 1H-NMR (CD3OD)δ: 1.58 (3H, s), 1.59 (3H, s), 3.30-3.35 (1H, m), 3.90 (1H, t, J = 5.6 Hz), 4.17-4.27 (3H, m), 4.43 (1H, d, J = 6.5 Hz), 7.13-7.19 (2H, m), 7.23-7.29 (2H, m), 7.31-7.35 (2H, m), 7.77 (1H, s). |
| I-075 | 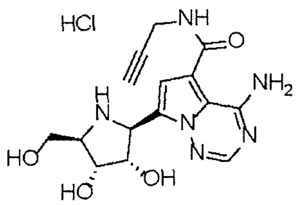 | MS(m/z) = 347 |
| I-076 | 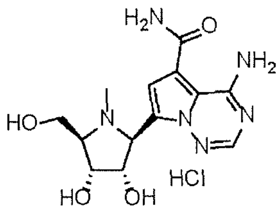 | 1H-NMR (D2O) δ: 3.07 (3H, s), 3.77 (1H, q, J = 4.1 Hz), 4.04 (2H, dd, J = 4.0, 1.1 Hz), 4.54 (1H, t, J = 4.8 Hz), 4.88 (1H, dd, J = 8.2, 5.0 Hz), 5.11 (1H, d, J = 8.3 Hz), 7.49 (1H, s), 8.02 (1H, s). |

[Table 8]

| No. | Structure | NMR or MS |
|---|---|---|
| I-077 | 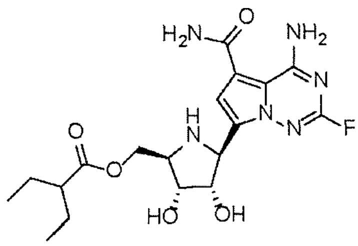 | 1H-NMR (DMSO-d6) δ : 0.83 (t, J = 7.6 Hz, 6H), 1.40-1.59 (m, 5H), 2.15-2.25 (m, 1H), 2.97-3.08 (m, 1H), 3.69-3.76 (m, 1H), 3.90-4.01 (m, 2H), 4.18 (dd, J = 5.2, 10.8 Hz, 1H), 4.39-4.45 (m, 1H), 4.85 (d, J = 6.0 Hz, 1H), 4.89 (d, J = 6.0 Hz, 1H), 7.30 (s, 1H), 7.58 (br-s, 1H), 8.17 (br-s, 1H), 8.61-8.67 (m, 1H), 10.90-10.96 (m, 1H). |
| I-079 | 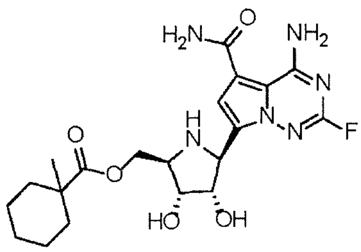 | 1H-NMR (DMSO-d6) δ : 1.10 (s, 3H), 1.14-1.35 (m, 6H), 1.39-1.54 (m, 4H), 1.89-1.98 (m, 2H), 2.96-3.07 (m, 1H), 3.70-3.77 (m, 1H), 3.90-4.00 (m, 2H), 4.17 (dd, J = 4.8, 10.8 Hz, 1H), 4.38-4.45 (m, 1H), 4.84 (d, J = 5.6 Hz, 1H), 4.88 (d, J = 6.0 Hz, 1H), 7.29 (s, 1H), 7.57 (br-s, 1H), 8.16 (br-s, 1H), 8.60-8.66 (m, 1H), 10.90-10.95 (m, 1H). |

(continued)

| No. | Structure | NMR or MS |
|---|---|---|
| I-080 | 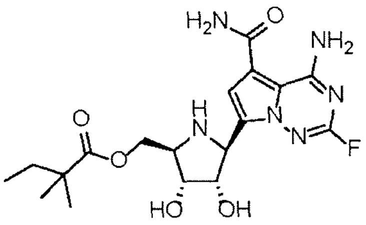 | 1H-NMR (MeOD) δ: 0.84 (t, J = 7.5 Hz, 3H), 1.17 (s, 6H), 1.60 (q, J = 7.5 Hz, 2H), 3.38 (dd, J = 10.5, 5.6 Hz, 1H), 4.00 (t, J = 5.7 Hz, 1H), 4.20 (ddd, J = 24.8, 11.4, 5.2 Hz, 2H), 4.30 (t, J = 6.0 Hz, 1H), 4.46 (d, J = 6.4 Hz, 1H), 7.22 (s, 1H). |
| I-081 | 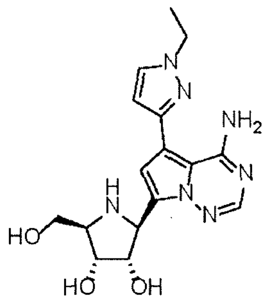 HCl | 1H-NMR (D2O) δ: 1.40 (3H, t, J = 7.3 Hz), 3.81-3.88 (3H, m), 4.19 (2H, q, J = 7.3 Hz), 4.36-4.41 (1H, m), 4.85 (1H, dd, J = 8.4, 4.8 Hz), 5.09 (1H, d, J = 8.5 Hz), 6.71 (1H, d, J = 2.4 Hz), 7.32 (1H, s), 7.69 (1H, d, J = 2.4 Hz), 7.93 (1H, s). |
| I-082 | 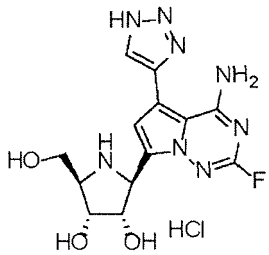 HCl | 1H-NMR (MeOD) δ: 3.76-3.81 (1H, m), 3.86-3.90 (2H, m), 4.37-4.40 (1H, m), 5.01 (1H, d, J = 8.0 Hz), 7.31 (1H, s), 8.29 (1H, s). |
| I-083 | 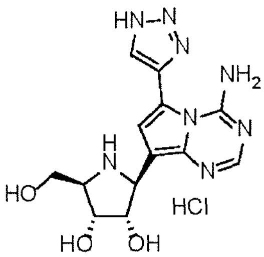 HCl | 1H-NMR (D2O) δ: 3.75-3.80 (1H, m), 3.84-3.89 (2H, m), 4.32-4.36 (1H, m), 4.62 (1H, dd, J = 8.5, 4.9 Hz), 4.95 (1H, d, J = 8.5 Hz), 7.27 (1H, s), 7.84 (1H, s), 8.17 (1H, s). |
| I-084 | HCl 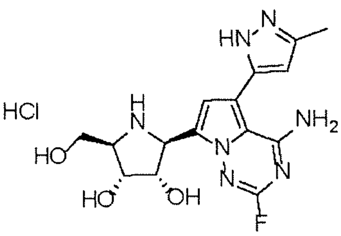 | 1H-NMR (MeOD) δ: 2.37 (3H, s), 3.74-3.79 (1H, m), 3.84-3.89 (2H, m), 4.37 (1H, t, J = 4.1 Hz), 4.83-4.88 (1H, m), 5.03 (1H, d, J = 8.0 Hz), 6.47 (1H, s), 7.19 (1H, s). |

Table 9]

| No. | Structure | NMR or MS |
|---|---|---|
| I-085 | 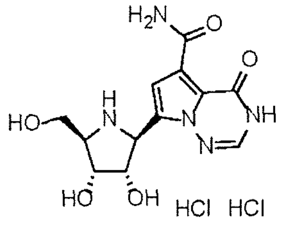 | 1H-NMR (D2O) δ: 3.77-3.86 (3H, m), 4.33-4.37 (1H, m), 4.81 (1H, dd, J = 8.5, 4.8 Hz), 5.03 (1H, d, J = 8.5 Hz), 7.25 (1H, s), 7.93 (1H, s). |
| I-086 | 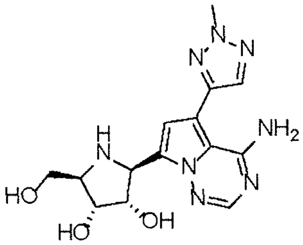 | 1H-NMR (MeOD) δ: 3.81-3.90 (3H, m), 4.33 (3H, s), 4.36-4.40 (1H, m), 4.89-4.94 (1H, m), 5.14 (1H, d, J = 8.3 Hz), 7.61-7.65 (1H, m), 8.16-8.20 (1H, m), 8.24-8.28 (1H, m). |
| I-087 | 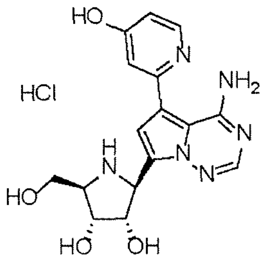 | 1H-NMR (MeOD) δ: 3.81-3.91 (3H, m), 4.36-4.41 (1H, m), 4.90-4.95 (1H, m), 5.13 (1H, d, J = 8.3 Hz), 7.45 (1H, dd, J = 8.8, 3.0 Hz), 7.69 (1H, s), 8.06 (1H, d, J = 9.0 Hz), 8.14 (1H, s), 8.23 (1H, d, J = 2.8 Hz). |
| I-088 | 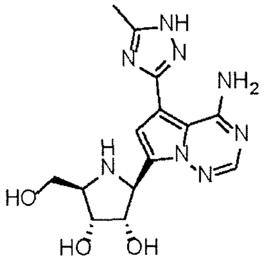 | 1H-NMR (D2O) δ: 2.44 (3H, s), 3.79-3.88 (3H, m), 4.31-4.38 (1H, m), 4.82-4.89 (1H, m), 5.09 (1H, d, J = 8.5 Hz), 7.45 (1H, s), 8.00 (1H, s). |
| I-089 | 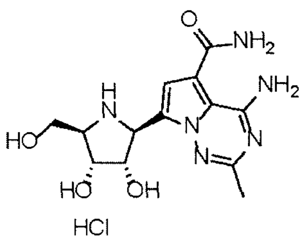 | 1H-NMR (DMSO-D6) δ: 2.34 (3H, s), 3.53-3.62 (1H, m), 3.69 (2H, d, J = 4.9 Hz), 4.20 (1H, t, J = 4.1 Hz), 4.54-4.60 (1H, m), 4.80-4.88 (1H, m), 7.56 (1H, s), 7.69 (1H, br s), 8.23 (1H, br s), 8.43 (1H, br s), 8.57 (1H, br s), 10.26 (1H, br s), 10.79 (1H, br s). |
| I-090 | 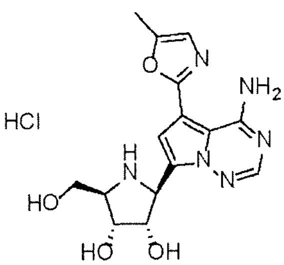 | 1H-NMR (D2O) δ: 2.30 (3H, s), 3.79-3.86 (3H, m), 4.33-4.39 (1H, m), 4.83 (1H, dd, J = 8.5, 4.8 Hz), 5.07 (1H, d, J = 8.5 Hz), 6.86 (1H, s), 7.42 (1H, s), 7.97 (1H, s). |

(continued)

| No. | Structure | NMR or MS |
|---|---|---|
| I-091 | 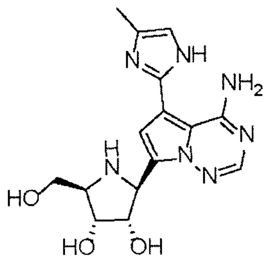 | 1H-NMR (MeOD) δ: 2.16 (3H, br s), 3.09 (1H, q, J = 4.7 Hz), 3.58-3.68 (2H, m), 3.95 (1H, t, J = 5.6 Hz), 4.23 (1H, t, J = 6.5 Hz), 4.40 (1H, d, J = 7.0 Hz), 6.56-6.73 (1H, m), 6.95 (1H, s), 7.62 (1H, s). |

[Table 10]

| No. | Structure | NMR or MS |
|---|---|---|
| I-092 | 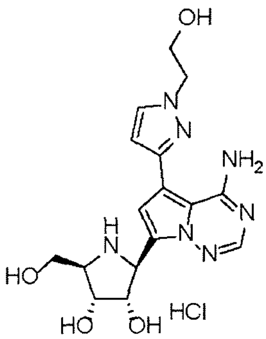 | 1H-NMR (D2O) δ: 3.82-3.89 (3H, m), 3.92 (2H, t, J = 5.1 Hz), 4.30 (2H, t, J = 5.0 Hz), 4.38-4.41 (1H, m), 4.86 (1H, dd, J = 8.4, 4.8 Hz), 5.10 (1H, d, J = 8.4 Hz), 6.76 (1H, d, J = 2.4 Hz), 7.35 (1H, s), 7.72 (1H, d, J = 2.4 Hz), 7.94 (1H,s). |
| I-093 | 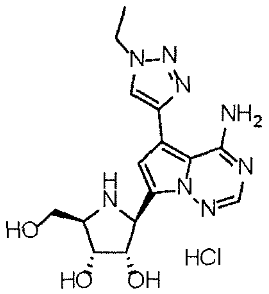 | 1H-NMR (D2O) δ: 1.50 (3H, t, J = 7.4 Hz), 3.81-3.89 (3H, m), 4.37-4.42 (1H, m), 4.46 (2H, q, J = 7.4 Hz), 4.85 (1H, dd, J = 8.5, 4.8 Hz), 5.09 (1H, d, J = 8.5 Hz), 7.30 (1H, s), 7.97 (1H, s), 8.38 (1H, s). |
| I-094 | 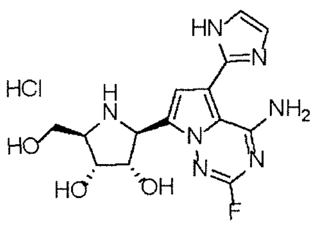 | 1H-NMR (MeOD) δ: 3.77-3.94 (3H, m), 4.34-4.39 (1H, m), 4.91-4.94 (1H, m), 5.09 (1H, d, J = 8.3 Hz), 7.36 (1H, s), 7.66 (2H, s). |
| I-095 | 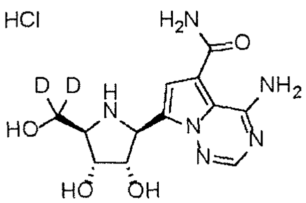 | 1H-NMR (MeOD) δ: 3.82 (1H, d, J = 3.1 Hz), 4.37 (1H, dd, J = 4.3, 3.2 Hz), 4.85-4.88 (1H, m), 5.12 (1H, d, J = 8.3 Hz), 7.67 (1H, s), 8.21 (1H, s). |

(continued)

| No. | Structure | NMR or MS |
|---|---|---|
| I-096 | 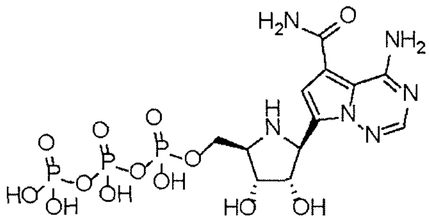 | 1H-NMR (D2O) δ: 3.94-4.01 (1H, m), 4.32-4.39 (2H, m), 4.53-4.59 (2H, m), 5.14 (1H, d, J = 8.0 Hz), 7.59 (1H, s), 7.98 (1H, s). 31 P-NMR (D2O) δ: -22.68 (1H, t, J = 19.6 Hz), -11.62 (1H, d, J = 19.6 Hz), -8.28 (1H, d, J = 21.5 Hz). |

Biological Test Examples for the compounds of the present invention will be described below.

The compound represented by the formula (I) or (II) according to the present invention may be any compound as long as it has an RNA viral growth inhibitory action and inhibits growth of an RNA virus.

For example, in the evaluation method described below, $EC_{50}$ is preferably 100 μM or less, more preferably 10 μM or less, and still more preferably 1 μM or less.

Test Example 1: CPE suppression effect confirmation test (SARS-CoV-2, Vero E6/TMPRSS2 cells)

<Material>

- · 2% FBS/MEM (prepared by adding 1% penicillin/streptomycin and inactivated FBS to MEM)
- · 10% FBS/DMEM (prepared by adding 1% penicillin/streptomycin, 1 mg/mL Geneticin and inactivated FBS to DMEM)
- · Vero E6/TMPRSS2 cells
- · CellTiter-Glo (registered trademark) 2.0 Reagent (Promega)
- · Plate reader

<Operation procedure (antiviral activity evaluation)>

· Dilution and dispensing of test substance

A 3- to 5-fold serial dilution series of test substances was prepared in DMSO, diluted with 2% FBS/MEM so that the concentration of DMSO was 2%, and dispensed into a 96 well plate (50 pL/well, DMSO final concentration 0.5%).

· Dilution and dispensing of SARS-CoV-2

SARS-CoV-2 (hCoV -19/Japan/TY/WK- 521/2020 strain) was diluted to an appropriate concentration with 2% FBS/MEM and added to the plate into which the test substance was dispensed (50 μL/well).

· Dilution and dispensing of cells

The cells prepared to an appropriate number were seeded at 100 pL/well to the plate into which the test substance and the virus were dispensed. Thereafter, the cells were cultured in a $CO_2$ incubator at 37°C for 3 days.

. Dispensing of CellTiter-Glo ® 2.0 Reagent

After completion of the culture, cell morphology, presence or absence of crystals, and the like were observed with naked eyes and under a microscope. The supernatant, 100 μL, was removed from the plate in such a manner that cells are not sucked, and 60 μL of CellTiter-Glo (registered trademark) 2.0 Reagent was added. After being mixed by a plate mixer, the mixture was allowed to stand for 10 to 20 minutes.

· Measurement of luminescence intensity

An appropriate amount was dispensed into a 96 well white plate from the plate left standing, and the luminescence intensity was measured with a plate reader.

<Operation procedure (cytotoxic activity evaluation)>

· Dilution and dispensing of test substance

A 3- to 5-fold serial dilution series of test substances was prepared in DMSO, diluted with 2% FBS/MEM so that the concentration of DMSO was 2%, and dispensed into a 96 well plate (50 μL/well, DMSO final concentration 0.5%).

· Dilution and dispensing of cells

The cells prepared to an appropriate number were seeded at 100 μL/well to the plate into which the test substance was dispensed. Thereafter, the cells were cultured in a $CO_2$ incubator at 37°C for 3 days.

· Dispensing of CellTiter-Glo (registered trademark) 2.0 Reagent

Same as those described in the section for evaluation of antiviral activity

· Measurement of luminescence intensity

Same as those described in the section for evaluation of antiviral activity

<Calculation of each measurement item value>

· Calculation of 50% CPE inhibitory concentration ($EC_{50}$); Calculation was performed using Microsoft Excel, TIBCO Spotfire, or a program having equivalent calculation processing capability based on the following calculation formula.
· $EC_{50}$ = 10^Z

Z = (50% - High%) / (High% - Low%) × {log(High conc.) - log(Low conc.)} + log(High conc.)

· Calculation of 50% cell proliferation inhibitory concentration ($CC_{50}$); Calculation was performed using Microsoft Excel, TIBCO Spotfire, or a program having equivalent calculation processing capability based on the following calculation formula.

$$CC_{50} = 10\hat{}Z$$

Z = (50% - High%) / (High% - Low%) × {log(High conc.) - log(Low conc.)} + log(High conc.)

· Calculation of Selectivity Index (SI)

$$SI = CC_{50}/EC_{50}$$

Test Example 2: CPE suppression effect confirmation test (influenza virus)

<Material>

· 0.5% BSA/MEM (prepared by adding 1% kanamycin and BSA solution to MEM)
· RPMI 2650 cells; cells were prepared to 2 × $10^6$ cells/mL with 0.5% BSA/MEM, and seeded on a 96 well plate at 100 μL/well.
· Trypsin solution; trypsin powder dissolved in DPBS and filtered through a 0.45 μm filter was used.

· Resazurin Reagent; The resazurin powder was dissolved in DPBS so as to be 2.4 mg/mL and filtered through a 0.45 um filter, and the solution was diluted with DPBS so as to be 0.24 mg/mL immediately before the assay and used.
· 10% SDS solution
· Plate reader

<Operation procedure (antiviral activity evaluation)>

· Dilution and dispensing of cells

On the day before the inoculation of influenza virus (hereinafter, referred to as Flu), cells prepared to an appropriate number were seeded in a 96-well plate at 100 μL/well.Thereafter, the cells were cultured in a $CO_2$ incubator at 37°C.

· Dilution and dispensing of influenza virus

Flu (A/WSN/33 (H1N1) strain) was previously diluted with 0.5% BSA/MEM to an appropriate concentration. The cells seeded on the 96 well plate were washed with MEM, and the Flu solution was inoculated at 100 μL/well.Thereafter, the cells were cultured in a $CO_2$ incubator at 37°C for 1 hour.

· Preparation of 2 μg/mL trypsin/0.5% BSA/MEM

Before dilution of a test substance, a trypsin solution was added to 0.5% BSA/MEM to prepare 2 μg/mL trypsin/0.5% BSA/MEM.

· Dilution and dispensing of test substance

A 3- to 5-fold serial dilution series of test substances was prepared in DMSO in advance, and diluted with 2 μg/mL trypsin/0.5% BSA/MEM so that the final concentration of DMSO was 0.5% (100 to 200 pL/well). One hour after inoculation with Flu, the cells were washed with MEM, and then cultured in a test substance-added medium for 3 to 6 days. Double measurement was performed for each drug.

· Dispensing of resazurin solution

A resazurin solution was prepared with MEM so as to have a final concentration of 0.024 mg/mL. After completion of the culture, cell morphology, presence or absence of crystals, and the like were observed with naked eyes and under a microscope. The supernatant was removed from the plate in such a manner that cells are not sucked, and a resazurin solution was added (110 μL/well). The mixture was mixed with a plate mixer and then cultured in a $CO_2$ incubator at 37°C for 2 to 4 hours. After completion of the culture, 10 μL of a 10% SDS solution was dispensed into each well to inactivate the virus.

• Measurement of fluorescence intensity

Fluorescence intensity of the mixed 96-well plate was measured using a plate reader at two wavelengths: Ex531 nm/Em590 nm.

<Operation procedure (cytotoxic activity evaluation)>

• Dilution and dispensing of cells

On the day before the compound treatment, cells prepared to an appropriate number were seeded in a 96 well plate at 100 μL/well. Thereafter, the cells were cultured in a $CO_2$ incubator at 37°C.

· Preparation of 2 μg/mL trypsin/0.5% BSA/MEM

Same as those described in the section for evaluation of antiviral activity

· Dilution and dispensing of test substance

A 3- to 5-fold serial dilution series of test substances was prepared in DMSO in advance, and diluted with 2 μg/mL trypsin/0.5% BSA/MEM so that the final concentration of DMSO was 0.5% (100 to 200 μL/well). The cells were washed with MEM, and then cultured in a test substance-added medium for 3 to 6 days. Double measurement was performed for each drug.

• Dispensing of resazurin solution

Same as those described in the section for evaluation of antiviral activity

• Measurement of fluorescence intensity

Same as those described in the section for evaluation of antiviral activity

<Calculation of each measurement item value>

· Calculation of 50% CPE inhibitory concentration ($EC_{50}$); Calculation was performed using Microsoft Excel, TIBCO Spotfire, or a program having equivalent calculation processing capability based on the following calculation formula.
· $EC_{50}$ = 10^Z

Z = (50% - High%) / (High% - Low%) × {log(High conc.) - log(Low conc.)} + log(High conc.)

· Calculation of 50% cell proliferation inhibitory concentration ($CC_{50}$); Calculation was performed using Microsoft Excel, TIBCO Spotfire, or a program having equivalent calculation processing capability based on the following calculation formula.

$$CC_{50} = 10\text{^}Z$$

Z = (50% - High%) / (High% - Low%) × {log(High conc.) - log(Low conc.)} + log(High conc.)

• Calculation of Selectivity Index (SI)

$$SI = CC_{50}/EC_{50}$$

The compounds of the present invention were tested essentially as described above. The results are shown in the following tables.

[Table 11]

| No. | SARS-CoV-2 EC50μM | SARS-CoV-2 CC50μM | SARS-CoV-2 SI | No. | Influenza EC50μM | Influenza CC50μM | Influenza SI |
|---|---|---|---|---|---|---|---|
| I-001 | >0.800 | 0.977 | <1.22 | I-001 | 1.05 | 70.5 | 67.1 |
| I-010 | 0.18 | 448 | 2488 | I-002 | 4.77 | >500 | >105 |
| I-016 | 157 | >500 | >318 | I-003 | 22.6 | >500 | >22.1 |
| I-017 | 44.9 | >500 | >11.1 | I-004 | 243 | >500 | >2.06 |
| I-022 | 1.03 | 129 | 125 | I-005 | 235 | >500 | >2.13 |

(continued)

| No. | SARS-CoV-2 EC50μM | SARS-CoV-2 CC50μM | SARS-CoV-2 SI | No. | Influenza EC50μM | Influenza CC50μM | Influenza SI |
|---|---|---|---|---|---|---|---|
| I-023 | 0.17 | 157 | 923 | I-006 | 0.316 | 5.38 | 17 |
| I-024 | 49.3 | 189 | 3.83 | I-007 | 473 | >500 | 1.06 |
| I-029 | 34.7 | 316 | 9.01 | I-008 | 7.01 | 432 | 61.6 |
| I-033 | 3.74 | >500 | >134 | I-009 | 406 | >500 | 1.23 |
| I-037 | 1.97 | 17 | 8.63 | I-011 | 6.7 | 75.1 | 11.2 |
| I-039 | 83.1 | >500 | >6.02 | I-012 | 1.78 | >500 | >281 |
| I-040 | 84.3 | >500 | >5.93 | 1-013 | 251 | >500 | >1.99 |
| 1-042 | 75.1 | >500 | >6.66 | 1-014 | 0.188 | 13.1 | 69.7 |
| I-044 | 22.6 | 444 | 16.6 | I-018 | 1.06 | >500 | >472 |
| I-045 | 3.78 | 96 | 25.4 | I-019 | 65.6 | >500 | >7.62 |
| I-046 | 31.1 | 485 | 15.6 | I-020 | 20.2 | >500 | >24.8 |
| I-047 | 48.2 | >500 | >10.4 | I-021 | 23.9 | >500 | >20.9 |
| I-048 | 40.9 | >500 | >12.2 | I-022 | 105 | >500 | >4.76 |
| I-049 | 69.6 | 397 | 5.7 | I-023 | 0.983 | 51.8 | 52.7 |
| I-050 | 45.4 | >500 | >11.0 | I-024 | 47.9 | 310 | 6.47 |
| 1-051 | 81.5 | >500 | >6.13 | I-026 | 0.965 | >500 | >518 |
| I-052 | 6.27 | >500 | >79.7 | I-027 | 10.3 | >500 | >48.5 |
| I-053 | 0.465 | 315 | 677 | I-028 | 4.95 | 102 | 20.6 |
| I-056 | 28.2 | >500 | >17.7 | I-030 | 7.65 | >500 | >65.4 |
| I-057 | 0.131 | 56.8 | 434 | I-031 | 343 | >500 | > 1.46 |
| I-075 | 2.7 | >500 | >185 | I-032 | 361 | >500 | >1.38 |
| I-076 | 16.3 | >500 | >30.7 | I-035 | 375 | >500 | >1.33 |
| I-078 | 3.23 | >500 | >155 | I-037 | >4.00 | 6.81 | <1.70 |
| | | | | I-038 | 4.41 | >500 | >113 |
| | | | | I-041 | 6.56 | >500 | >76.2 |
| | | | | I-043 | 203 | >500 | >2.46 |

Test Example 3: CPE suppression effect confirmation test (SARS-CoV-2, 293TAT cells)

- 2% FBS/MEM (prepared by adding 1% penicillin/streptomycin and inactivated FBS to MEM)
- 10% FBS/DMEM (prepared by adding 1% penicillin/streptomycin and inactivated FBS to DMEM)
- HEK293T/ACE2-TMPRSS2 cells
- CellTiter-Glo (registered trademark) 2.0 Reagent (Promega)
- Plate reader

<Operation procedure (antiviral activity evaluation)>

· Dilution and dispensing of test substance

A 3- to 5-fold serial dilution series of test substances was prepared in DMSO, diluted with 2% FBS/MEM so that the concentration of DMSO was 2%, and dispensed into a 96 or 384 well plate (50 μL/well or 40 μL/well, DMSO final concentration 0.5%).

· Dilution and dispensing of SARS-CoV-2

SARS-CoV-2 (hCoV -19/Japan/TY/WK- 521/2020 strain) was diluted to an appropriate concentration with 2% FBS/MEM and added to the plate into which the test substance was dispensed (50 μL/well or 10 μL/well).

• Dilution and dispensing of cells

The cells prepared to an appropriate number were seeded at 100 μL/well or 10 μL/well to the plate into which the test substance and the virus were dispensed. Thereafter, the cells were cultured in a $CO_2$ incubator at 37°C for 3 days.

• Dispensing of CellTiter-Glo (registered trademark) 2.0 Reagent

After completion of the culture, cell morphology, presence or absence of crystals, and the like were observed with naked eyes and under a microscope. The supernatant, 100 μL, was removed from the 96-well plate in such a manner that cells are not sucked, and 60 μL of CellTiter-Glo (registered trademark) 2.0 Reagent was added. Alternatively, CellTiter-Glo (registered trademark) 2.0 Reagent was added at 15 μL/well to a 384 well plate. After being mixed by a plate mixer, the mixture was allowed to stand for 10 to 30 minutes.

· Measurement of luminescence intensity

An appropriate amount was dispensed into a 96 well white plate from the plate left standing, and the luminescence intensity was measured with a plate reader. Alternatively, the luminescence intensity of the 384 well plate was measured directly on a plate reader.

<Operation procedure (cytotoxic activity evaluation)>

• Dilution and dispensing of test substance

A 3- to 5-fold serial dilution series of test substances was prepared in DMSO, diluted with 2% FBS/MEM so that the concentration of DMSO was 2%, and dispensed into a 96 or 384/well well plate (50 μL/well or 40 pL/well, DMSO final concentration 0.5%).

· Dilution and dispensing of cells

The cells prepared to an appropriate number were seeded at 100 μL/well or 40 μL/well to the plate into which the test substance was dispensed. Thereafter, the cells were cultured in a $CO_2$ incubator at 37°C for 3 days.

• Dispensing of CellTiter-Glo (registered trademark) 2.0 Reagent

Same as those described in the section for evaluation of antiviral activity

• Measurement of luminescence intensity

Same as those described in the section for evaluation of antiviral activity

<Calculation of each measurement item value>

• Calculation of 50% CPE inhibitory concentration ($EC_{50}$); Calculation was performed using Microsoft Excel, TIBCO Spotfire, or a program having equivalent calculation processing capability based on the following calculation formula.
• $EC_{50}$ = 10^Z

Z = (50% - High%) / (High% - Low%) × {log(High conc.) - log(Low conc.)} + log(High conc.)

• Calculation of 50% cell proliferation inhibitory concentration ($CC_{50}$); Calculation was performed using Microsoft Excel, TIBCO Spotfire, or a program having equivalent calculation processing capability based on the following

calculation formula.

$$CC_{50} = 10^{\wedge}Z$$

Z = (50% - High%) / (High% - Low%) × {log(High conc.) - log(Low conc.)} + log(High conc.)

• Calculation of Selectivity Index (SI)

$$SI = CC_{50}/EC_{50}$$

The compounds of the present invention were tested essentially as described above. The results are shown in the following tables.

[Table 12]

| No. | SARS-CoV-2 EC50μM | SARS-CoV-2 CC50μM | SARS-CoV-2 SI |
|---|---|---|---|
| I-081 | 1.38 | 306 | 222 |
| I-082 | 3.97 | 150 | 37.8 |
| I-083 | 5.2 | 45.3 | 8.71 |
| I-084 | 5.33 | 257 | 48.2 |
| I-085 | 5.51 | 337 | 61.2 |
| I-086 | 11.6 | 225 | 19.4 |
| I-087 | 18.9 | 75 | 3.97 |
| I-088 | 30.4 | >500 | >16.4 |
| I-089 | 35.3 | >500 | >14.2 |
| I-090 | 38.1 | >500 | >13.1 |
| I-091 | 218 | >250 | >2.29 |
| I-092 | 0.317 | >500 | >1577 |
| I-093 | 211 | >500 | >2.37 |
| I-094 | 49.1 | >500 | >10.2 |
| I-095 | 0.0228 | 62.9 | 2759 |

Test Example 4: Comparison with prior art

Comparison with the prior art compounds was performed according to Test Examples 1 and 2.

[Table 13]

| | 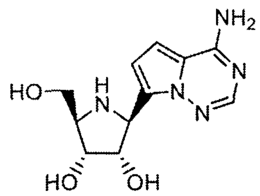 | 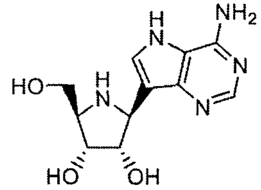 | 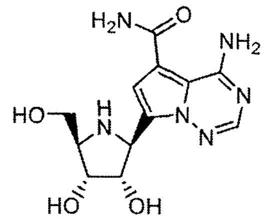 | 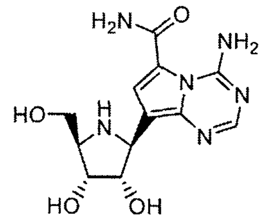 |
|---|---|---|---|---|
| Compound No. | WO2014078778 Example5_30g | Galidesivir | I-010 | I-078 |
| SARC-CoV-2_Vero EC50μM | >4.00 | >100 | 0.18 | 3.23 |
| SARC-CoV-2_Vero SI | <0.41 | - | 2488 | >155 |
| SARS-CoV-2_ 293T-AT EC50μM | >4.00 | >20.0 | 0.0395 | 1.86 |
| SARS-CoV-2_ 293T-AT SI | <1.34 | <5.3 | 1050 | 53 |

From the above results, it was found that among the compounds of the present invention having azasugar, compounds having specific substituent groups in $R^{A1}$ and $R^{B1}$ exhibit high activity against SARS-CoV-2, have a large SI value, and have a large deviation between cytotoxicity and activity.

[Table 14]

| | | | |
|---|---|---|---|
| | 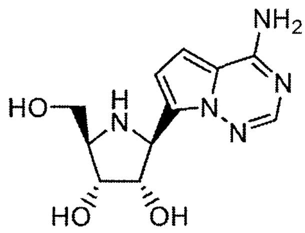 | 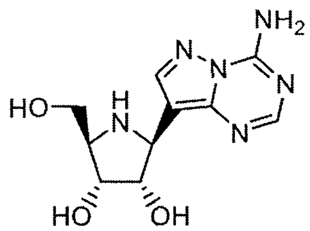 | 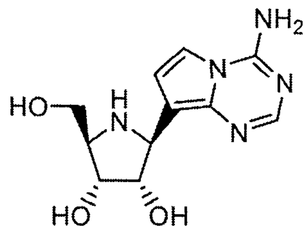 |
| Compound No. | WO2014078778 Example5_30g | WO2006002231 Example 13_9-4 | I-012 |
| Influenza EC50μM | 0.471 | 87.8 | 1.78 |
| Influenza SI | 73.7 | 4.69 | >281 |

From the above results, it was found that specific compounds among the compounds of the present invention having azasugar exhibit high activity against influenza viruses, have a large SI value, and have a large deviation between cytotoxicity and activity.

[Table 15]

| | | |
|---|---|---|
| | 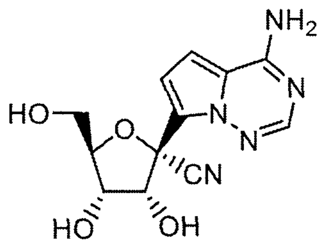 | 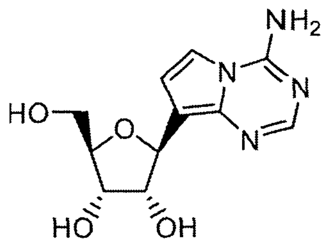 |
| Compound No. | GS-441524 | I-014 |
| Influenza EC50μM | >500 | 0.188 |

From the above results, specific compounds among the compounds of the present invention having ribose exhibited high activity against influenza viruses.

Test Example 5: Lung distribution test

Materials and methods for experiments

(1) Animals used: SD rats are used.
(2) Rearing conditions: the SD rats are allowed to freely take solid feed and sterilized tap water.
(3) Dose and grouping setting: a given dose is orally administered. Grouping is set as below.
Oral administration: 1 to 100 mg/kg (n = 2 to 3)
(4) The test sample is orally administered in a form of solution or suspension.
(5) Administration method: the test sample is forcedly administered into the stomach through an oral probe for the oral administration.
(6) Evaluation items: Blood and lung are collected, and the concentrations of the compound of the present invention in plasma and lung are measured using LC/MS/MS.

The compounds of the present invention were tested essentially as described above.

(Result)

Lung concentrations of Compound I-057 as an active form and Compound I-077 as a prodrug form thereof 8 hours after they were orally administered (25 mg/kg) are shown below.
In addition, lung concentrations of other prodrug forms 8 hours after they were orally administered (25 mg/kg) are shown below.

[Table 16]

| Compound | Active form concentration ng/g | Prodrug form concentration ng/g | Compound | Active form concentration ng/g | Prodrug form concentration ng/g |
|---|---|---|---|---|---|
| I-057 | 955 | - | I-063 | 6712 | 3.85 |
| I-077 | 2946 | Below detection limit | I-068 | 6245 | Below detection limit |
| I-054 | 6480 | Below detection limit | I-069 | 3882 | Below detection limit |
| I-058 | 2691 | 12.7 | I-072 | 915 | 6.41 |

Test Example 6: (BA test)

Materials and methods for experiments to evaluate oral absorption

(1) Animals: Mice or rats are used.
(2) Rearing conditions: the mice or the rats are allowed to freely take solid feed and sterilized tap water.
(3) Dose and grouping setting: a given dose is orally administered and intravenously administered. Grouping is set as below. (Dosage can be changed per compound)

Oral administration 2 to 60 μmol/kg or 1 to 30 mg/kg (n = 2 to 3)
Intravenous administration 1 to 20 μmol/kg or 0.5 to 10 mg/kg (n = 2 to 3)

(4) Preparation of dosing solution: Oral administration is performed by orally administering the test sample as a solution or a suspension. Intravenous administration is performed after solubilization.
(5) Administration method: For the oral administration, the test sample is forcedly administered into the stomach through an oral probe. For intravenous administration, the test sample is administered to the tail vein through a syringe with an injection needle.
(6) Evaluation item: blood is collected over time, and the concentration of the compound of the present invention in plasma is measured using LC/MS/MS.
(7) Statistical analysis: an area under concentration in plasma-time curve (AUC) is calculated as to change in the concentration of the compound of the present invention in plasma by the moment analysis method, and the bioavailability (BA) of the compound of the present invention is calculated from the dose ratio and AUC ratio between the oral administration group and the intravenous administration group.

The dilution concentration or the dilution solvent are changed as necessary.
The compounds of the present invention were tested essentially as described above.

Test Example 7: Clearance Evaluation Test

Materials and methods for experiments

(1) Animals used: SD rats are used.
(2) Rearing conditions: the SD rats are allowed to freely take solid feed and sterilized tap water.
(3) Dose and grouping setting: a given dose is intravenously administered. Grouping is set as below.
Intravenous administration 1 μmol/kg (n = 2)

(4) Preparation of dosing solution: the test sample is solubilized using a solvent of dimethyl sulfoxide/propylene glycol = 1/1 and administered.
(5) Administration method: the test sample is administered to the tail vein through a syringe with an injection needle.
(6) Evaluation item: blood is collected over time, and the concentration of the compound of the present invention in plasma is measured using LC/MS/MS.
(7) Statistical analysis: Total body clearance (CLtot) was calculated as to change in the concentration of the compound of the present invention in plasma by the moment analysis method. The dilution concentration or the dilution solvent are changed as necessary.

The compounds of the present invention were tested essentially as described above.

Test Example 8: (CYP3A4 (MDZ) MBI test)

This test as to the inhibition of CYP3A4 by the compound of the present invention is to evaluate mechanism based inhibition (MBI) ability from enhancement in inhibitory effect, caused by a metabolism reaction, of the compound of the present invention. The inhibition of CYP3A4 is evaluated in pooled human liver microsomes by using the 1-hydroxylation reaction of midazolam (MDZ) as an index.

Reaction conditions are as follows: substrate, 10 μmol/L MDZ; prereaction time, 0 or 30 minutes; substrate metabolism reaction time, 2 minutes; reaction temperature, 37°C; pooled human liver microsomes, 0.5 mg/mL for the prereaction, and 0.05 mg/mL (10-fold dilution) for the reaction; concentrations of the compound of the present invention for the prereaction, 1, 5, 10, and 20 μmol/L (4 points) or 0.83, 5, 10, and 20 μmol/L (4 points).

The pooled human liver microsomes and a solution of the compound of the present invention are added according to the recipe of the prereaction described above into a K-Pi buffer solution (pH 7.4) as a prereaction solution in a 96-well plate. A portion thereof is transferred to another 96-well plate so as to be diluted by 1/10 with a K-Pi buffer solution containing the substrate. A coenzyme NADPH is added thereto to start the reaction serving as an index (0-min preincubation). After reaction for a given time, the reaction is terminated by the addition of a solution of methanol/acetonitrile = 1/1 (V/V). NADPH is also added to the remaining prereaction solution to start prereaction (30-min preincubation). After prereaction for a given time, a portion thereof is transferred to another plate so as to be diluted by 1/10 with a K-Pi buffer solution containing the substrate, to start the reaction serving as an index. After reaction for a given time, the reaction is terminated by the addition of a solution of methanol/acetonitrile = 1/1 (V/V). Each plate where the index reaction has been performed is centrifuged at 3000 rpm for 15 minutes. Then, midazolam 1-hydroxide in the centrifugation supernatants is quantified by LC/MS/MS. The dilution concentration or the dilution solvent are changed as necessary.

Only a solvent DMSO used for dissolving the compound is added to the reaction solution instead of the compound of the present invention, and the mixture is used as a control (100%). Remaining activity (%) at the time of the addition of the compound of the present invention at each concentration is calculated, and IC is calculated by inverse estimation based on a logistic model using the concentrations and the rates of suppression. Shifted IC value is calculated as "IC of preincubation at 0 mini IC of preincubation at 30min". When a shifted IC is 1.5 or more, this is defined as positive. When a shifted IC is 1.0 or less, this is defined as negative.

The compounds of the present invention were tested essentially as described above.

Test Example 9: Metabolism Stability Test

Using pooled human liver microsomes or pooled rat liver microsomes, a compound of the present invention is reacted for a constant time, and a remaining rate is calculated by comparing a reacted sample and an unreacted sample, thereby, a degree of metabolism in liver is assessed.

A reaction is performed (oxidative reaction) at 37°C for 0 minute or 30 minutes in the presence of 1 mmol/L NADPH in 0.2 mL of a buffer (50 mmol/L Tris-HCl pH 7.4, 150 mmol/L potassium chloride, 10 mmol/L magnesium chloride) containing 0.5 mg protein/mL of human liver microsomes or rat liver microsomes. After the reaction, 50 μL of the reaction solution is added to 100 μL of a methanol/acetonitrile = 1/1 (v/v), mixed and centrifuged at 3000 rpm for 15 minutes. The compound of the present invention in the centrifuged supernatant is quantified by LC/MS/MS or solid-phase extraction (SPE)/MS. The ratio of the amount of the compound after the reaction, with respect to the amount of the compound at 0 minutes of the reaction defined as 100%, is shown as the residual rate. Hydrolysis reaction is performed in the absence of NADPH, and glucuronidation reaction is performed in the presence of 5 mmol/L UDP-glucuronic acid instead of NADPH. Then, the same operation is carried out. The dilution concentration or the dilution solvent are changed as necessary.

The compounds of the present invention were tested essentially as described above.

Test Example 10: Fluctuation Ames Test

The compound of the present invention is evaluated for its mutagenicity.

Cryopreserved *Salmonella typhimurium* (TA98 strain and TA100 strain), 20 pL, was inoculated to 10 mL of a liquid nutrient medium (2.5% Oxoid nutrient broth No. 2) and shake-precultured at 37°C for 10 hours. The bacterial solution of the TA98 strain, 7.70 to 8.00 mL, is centrifuged (2000 × g, 10 min) to remove the culture solution. The bacterium is suspended in Micro F buffer solution ($K_2HPO_4$: 3.5 g/L, $KH_2PO_4$: 1 g/L, $(NH_4)_2SO_4$: 1 g/L, trisodium citrate dihydrate: 0.25 g/L, and $MgSO_4 \cdot 7H_20$: 0.1 g/L) in the same volume as that of the bacterial solution used in the centrifugation and added to 120 mL of an exposure medium (Micro F buffer solution containing biotin: 8 μg/mL, histidine: 0.2 μg/mL, and glucose: 8 mg/mL). To 120 to 130 mL of an exposure medium, 3.10 to 3.42 mL of a bacterial solution of the TA100 strain was added, to prepare a test bacterial solution. 12 μL each of a DMSO solution of the compound of the present invention (several serial dilutions from maximum dose 50 mg/mL at 2- to 3-fold common ratio), DMSO as a negative control, and 50 μg/mL of a 4-nitroquinoline-1-oxide DMSO solution for the TA98 strain and 0.25 μg/mL of a 2-(2-furyl)-3-(5-nitro-2-furyl)acrylamide DMSO solution for the TA100 strain under non-metabolic activation conditions or 40 μg/mL of a 2-aminoanthracene DMSO solution for the TA98 strain and 20 μg/mL of a 2-aminoanthracene DMSO solution for the TA100 strain under metabolic activation conditions as a positive control, and 588 μL of the test bacterial solution (a mixed solution of 498 μL of the test bacterial solution and 90 μL of S9 mix under metabolic activation conditions) are mixed, and the mixture is shake-cultured at 37°C for 90 minutes. The bacterial solution, 460μL, exposed to the compound of the present invention is mixed with 2300 μL of an indicator medium (Micro F buffer containing biotin: 8 μg/mL, histidine: 0.2 μg/mL, glucose: 8 mg/mL, and bromocresol purple: 37.5 μg/mL), and the mixture is dispensed into 48 wells/dose of a microplate at 50 μL/well and statically cultured at 37°C for 3 days. The color of the solution in a well containing a bacterium that has acquired growth ability due to a mutation in amino acid (histidine) synthase gene is changed from purple to yellow depending on pH change. Therefore, yellow-colored wells with bacterial growth among the 48 wells per dose are counted and evaluated by comparison with the negative control group. Negativity to mutagenicity is indicated by (-), and positivity thereto is indicated by (+). The dilution concentration or the dilution solvent are changed as necessary.

The compounds of the present invention were tested essentially as described above.

Test Example 11: (hERG test)

For the purpose of evaluating the compound of the present invention for the risk of electrocardiogram QT interval prolongation, the effect of the compound of the present invention on delayed rectifier $K^+$ current ($I_{Kr}$) which plays an important role in a ventricular repolarization process is studied using CHO cells caused to express a human ether-a-go-go related gene (hERG) channel.

The cells are kept at a membrane potential of -80 mV by the whole cell patch clamp method using a fully automated patch clamp system (QPatch; Sophion Bioscience A/S), given a leak potential of -50 mV, and then given depolarization stimulation of +20 mV for 2 seconds and further repolarization stimulation of -50 mV for 2 seconds. $I_{Kr}$ induced by this procedure is recorded. An extracellular fluid (NaCl: 145 mmol/L, KCl: 4 mmol/L, $CaCl_2$: 2 mmol/L, $MgCl_2$: 1 mmol/L, glucose: 10 mmol/L, HEPES (4-(2-hydroxyethyl)-1-piperazineethanesulfonic acid): 10 mmol/L, pH = 7.4) adjusted to 0.1% with dimethyl sulfoxide is used as a vehicle to apply the vehicle or the extracellular fluid containing the compound of the present invention dissolved at a concentration of interest to the cells under conditions of room temperature for 7 minutes or longer. From the obtained $I_{Kr}$, the absolute value of the maximum tail current based on the current value at the membrane potential where the cells have been kept is measured using analytical software (QPatch Assay software; Sophion Bioscience A/S). The maximum tail current after the application of the compound of the present invention with respect to the maximum tail current after the application of the vehicle is further calculated as the rate of inhibition to evaluate the influence of the compound of the present invention on $I_{Kr}$. The dilution concentration or the dilution solvent are changed as necessary.

Test Example 12: Ames Test

Mutagenicity of the compound of the present invention was evaluated by an Ames test with *Salmonella typhimurium* TA98 strain, TA100 strain, TA1535 strain, and TA1537 strain (or TA97 strain, or alternatively, TA97a strain) and an *Escherichia coli* WP2uvrA strain as test bacterial strains. 0.1 mL of a DMSO solution of the compound of the present invention is mixed with 0.5 mL of S9 mix under metabolic activation conditions or 0.5 mL of a phosphate buffer solution and 0.1 mL of each test bacterial solution under non-metabolic activation conditions, and the mixture is overlaid on a minimum glucose agar plate together with 2 mL of soft agar for overlay containing histidine and biotin, or tryptophan. At the same time, the same procedure was performed for a negative control substance (DMSO) and a positive control substance (2-(2-furyl)-3-(5-nitro-2-furyl)acrylamide, sodium azide, 9-aminoacridine, or 2-aminoanthracene). After culture

at 37°C for 48 hours, revertant colonies that have appeared are counted and evaluated by comparison with the negative control group. When the number of revertant colonies increases in a concentration-dependent manner and becomes twice or more the number of colonies of the negative control group, positivity (+) is determined. The dilution concentration or the dilution solvent are changed as necessary.

The compounds of the present invention were tested essentially as described above.

Test Example 13: (Solubility test)

The solubility of the compound of the present invention is determined under 1% DMSO addition conditions. With DMSO, 10 mmol/L solution of the compound is prepared. The solution of the compound of the present invention, 2 μL each, is added to a JP-1 fluid and a JP-2 fluid, 198 μL each. The mixture is shaken for 1 hour at a room temperature, and the mixture is vacuum-filtered. The filtrate is 10- or 100-fold diluted with methanol/water = 1/1 (v/v) or acetonitrile/methanol/water = 1/1/2 (v/v/v), and the compound concentration in the filtrate is measured with LC/MS or Solid-Phase Extraction (SPE) /MS by the absolute calibration method. The dilution concentration or the dilution solvent are changed as necessary.

The recipe of the JP-1 fluid is as follows.

Water is added to 2.0 g of sodium chloride and 7.0 mL of hydrochloric acid to bring the amount to 1000 mL.

The composition of the JP-2 fluid is as below.

3.40 g of potassium dihydrogen phosphate and 3.55 g of dibasic sodium phosphate anhydrous are dissolved in water to bring the amount to 1000 mL, and to 1 volume of the resultant, 1 volume of water is added.

Test Example 14: Teratogenicity

The risk of developmental anomaly of the compound of the present invention is evaluated.

Embryos are removed from pregnant rats at 9.5 days of gestation and transferred to dishes containing HBSS (Hanks' Balanced Salt Solution). A glass vial is dispensed with 1 mL of culture medium at the desired concentration (obtained by dissolving the compound of the present invention in 100% DMSO solution and diluted with rat serum to a final concentration of 0.1%), and one rat embryo is placed in each glass vial. The glass vial is attached to a rotary fetus culturing device, and rotary culturing is performed at 37°C, 20 rpm, and an optimal oxygen concentration (Start of culture to 18 hours of culture: 5% oxygen, 5% carbon dioxide, 90% nitrogen; 18 to 26 hours of culture: 20% oxygen, 5% carbon dioxide, 75% nitrogen; 26 to 42 hours of culture: 40% oxygen, 5% carbon dioxide, 55% nitrogen, 42 to 48 hours of culture: 95% oxygen, 5% carbon dioxide). After 48 hours, embryos are removed and morphology is observed. The morphology of the embryo is quantified using Total Morphological Score* (TMS), and the risk of developmental anomaly of each compound of the present invention is evaluated in comparison with a control group.

*TMS is obtained by scoring the degree of growth of cultured embryos, and 17 items (yolk sac circulation, allantoic membrane, flexure, heart, caudal neural tube, hindbrain, midbrain, forebrain, ear, eye, nose, branchial arch, maxillary protrusion, mandibular protrusion, forelimb, hind limb, number of body segments) are scored with a maximum of 5 points, and a total score is a growth score. The normal TMS of rat 11.5 day embryos was 40, indicating that the lower the TMS, the less the embryos developed normally, and the higher the risk of developmental anomaly of the exposed inventive compounds.

The compounds of the present invention were tested essentially as described above.

The following formulation examples are merely examples and not intended to limit the scope of the invention.

The compound of the present invention can be administered as a pharmaceutical composition by any conventional route, in particular enterally, for example, orally, for example, in the form of tablets or capsules, or parenterally, for example, in the form of injectable solutions or suspensions, topically, for example, in the form of lotions, gels, ointments or creams, or in a nasal or suppository form. Pharmaceutical compositions comprising a compound of the present invention in free form or in a pharmaceutically acceptable salt form in association with at least one pharmaceutically acceptable carrier or diluent can be manufactured in a conventional manner by mixing, granulating or coating methods. For example, the oral composition can be a tablet, a granular preparation, or a capsule, each containing an excipient, a disintegrating agent, a binder, a lubricating agent, and the like, as well as an active ingredient and the like. Furthermore, the composition for injection can be prepared as a solution or a suspension, may be sterilized, and may contain a preservative, a stabilizer, a buffering agent, and the like.

[INDUSTRIAL APPLICABILITY]

The compound according to the present invention has a viral RNA polymerase inhibitory activity or a viral growth inhibitory action, and is considered to be useful as a therapeutic agent and/or a preventive agent for a disease or a condition associated with RNA virus.

## Claims

1. A compound represented by Formula (I):

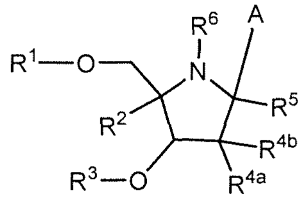

(I)

wherein

A is a group represented by any one of the following formulae:

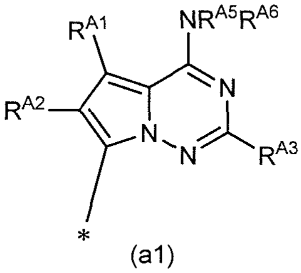
(a1)

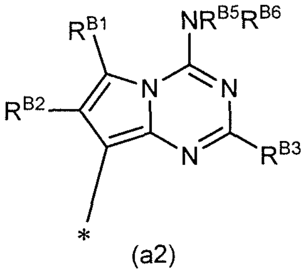
(a2)

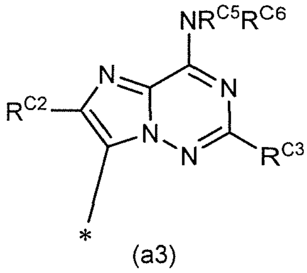
(a3)

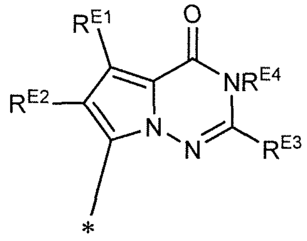
(b1)

wherein

$R^{A1}$, $R^{B1}$, and $R^{E1}$ are each independently hydrogen, halogen, hydroxy,$-B(OH)_2$, amino optionally substituted with substituent group γ, an aromatic heterocyclyl optionally substituted with substituent group α, a non-aromatic heterocyclyl optionally substituted with substituent group α, alkyl optionally substituted with substituent group β, or a group represented by any of the following formulae: $-C(=O)-N(R^{a1})(R^{a2})$, $-C(=NR^{a3})-NH_2$, and -C(=NOH)-H, wherein
$R^{a1}$ is hydrogen, $R^{a2}$ is hydrogen or alkyl optionally substituted with substituent group β, and $R^{a3}$ is hydrogen or hydroxy,
wherein

the substituent group α: halogen, hydroxy, alkyl, and hydroxyalkyl,
the substituent group β: cyano and hydroxy, and
the substituent group γ: alkyl,

$R^{A2}$, $R^{B2}$, $R^{C2}$, and $R^{E2}$ are each independently hydrogen,
$R^{A3}$, $R^{B3}$, $R^{C3}$, and $R^{E3}$ are each independently hydrogen, halogen, amino, or alkyl,
$R^{E4}$ is hydrogen,
$R^{A5}$, $R^{B5}$, and $R^{C5}$ are each independently hydrogen, alkyl, or a group forming a prodrug, and
$R^{A6}$, $R^{B6}$, and $R^{C6}$ are each independently hydrogen, hydroxy, alkylcarbonyl, or a group forming a prodrug,

$R^1$ is hydrogen, a group forming prodrug, or a group selected from the group consisting of the following:

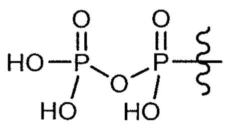
,
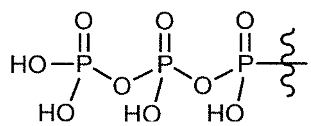

$R^2$ is hydrogen, halogen, C1-C3 alkyl, C2-C3 alkenyl, C2-C3 alkynyl, halo C1-C3 alkyl, halo C2-C3 alkenyl, or halo C2-C3 alkynyl,
$R^3$ is hydrogen or a group forming a prodrug,
$R^{4a}$ is hydrogen, hydroxy, or halogen,

$R^{4b}$ is hydrogen, halogen, C1-C3 alkyl, C1-C3 haloalkyl, C2-C3 alkenyl, or C2-C3 alkynyl,
$R^5$ is hydrogen, C1-C3 alkyl, C2-C3 alkenyl, or C2-C3 alkynyl, and
$R^6$ is hydrogen, C1-C3 alkyl, or a group forming a prodrug,
provided that the following compound is excluded:

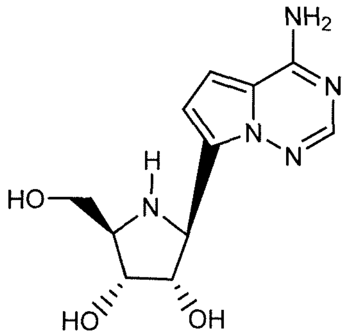

or a pharmaceutically acceptable salt thereof.

**2.** The compound according to claim 1, represented by Formula:

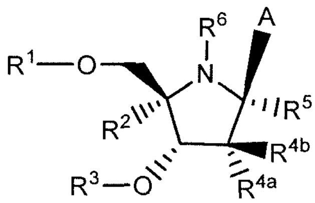
(I-1)

wherein

A is a group represented by either one of the following formulae:

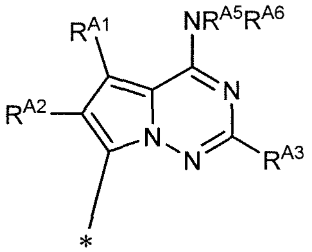
(a1)

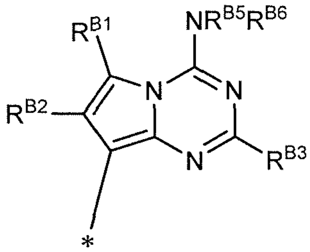
(a2)

wherein

$R^{A1}$ and $R^{B1}$ are each independently hydrogen, halogen, hydroxy, - $B(OH)_2$, amino, an aromatic heterocyclyl optionally substituted with substituent group $\alpha$, a non-aromatic heterocyclyl optionally substituted with substituent group $\alpha$, alkyl optionally substituted with substituent group $\beta$, or a group represented by any of the following formulae: -C(=O)-N($R^{a1}$)($R^{a2}$), C(=N$R^{a3}$)-$NH_2$, and -C(=NOH)-H,
wherein $R^{a1}$ is hydrogen, $R^{a2}$ is hydrogen or alkyl optionally substituted with substituent group B, and $R^{a3}$ is hydrogen or hydroxy,
wherein

the substituent group $\alpha$: halogen, hydroxy, alkyl, and hydroxyalkyl, and
the substituent group $\beta$: cyano and hydroxy,

$R^{A2}$ and $R^{B2}$ are hydrogen,

$R^{A3}$ and $R^{B3}$ are each independently hydrogen, halogen, or alkyl,
$R^{A5}$ and $R^{B5}$ are each independently hydrogen, or a group forming a prodrug, and
$R^{A6}$ and $R^{B6}$ are each independently hydrogen, hydroxy, alkylcarbonyl, or a group forming a prodrug,

$R^1$ is hydrogen, a group forming prodrug, or a group selected from the group consisting of the following:

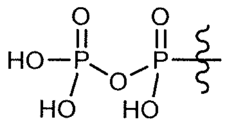
,
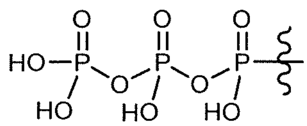

$R^2$ is hydrogen,
$R^3$ is hydrogen or a group forming a prodrug,
$R^{4a}$ is hydroxy,
$R^{4b}$ is hydrogen,
$R^5$ is hydrogen, and
$R^6$ is hydrogen, C1-C3 alkyl, or a group forming a prodrug,

or a pharmaceutically acceptable salt thereof.

**3.** The compound according to claim 2, wherein

$R^{A6}$ and $R^{B6}$ are each independently hydrogen, hydroxy, or alkylcarbonyl,
$R^3$ is hydrogen, and
$R^6$ is hydrogen or C1-C3 alkyl,

or a pharmaceutically acceptable salt thereof.

**4.** The compound according to any one of claims 1 to 3, wherein A is a group represented by either one of the following formulae:

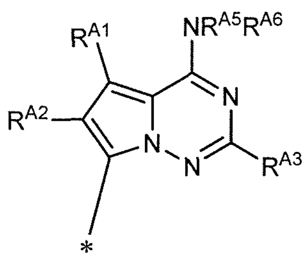
(a1)

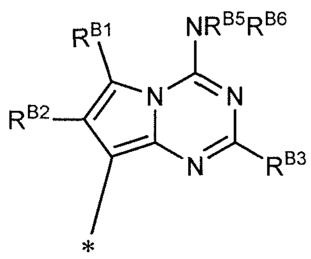
(a2)

wherein

$R^{A1}$ and $R^{B1}$ are each independently hydroxy, amino, an aromatic heterocyclyl optionally substituted with substituent group $\alpha$, or a group represented by any of the following formulae: -C(=O)-N($R^{a1}$)($R^{a2}$), and -C(=N$R^{a3}$)-$NH_2$,
wherein $R^{a1}$ is hydrogen, $R^{a2}$ is hydrogen or cyanomethyl, and $R^{a3}$ is hydrogen or hydroxy,
wherein the substituent group $\alpha$: fluorine and C1-C3 alkyl, and
$R^{A3}$ and $R^{B3}$ are each independently hydrogen or fluorine,

or a pharmaceutically acceptable salt thereof.

**5.** The compound according to any one of claims 1 to 4, wherein $R^{A1}$ and $R^{B1}$ are hydroxy, a 5-membered aromatic heterocyclyl, or a group represented by either one of the following formulae: -C(=O)-$NH_2$, and -C(=NOH)-$NH_2$, or a pharmaceutically acceptable salt thereof.

6. The compound according to any one of claims 1 to 3,

wherein A is a group represented by either one of the following formulae:

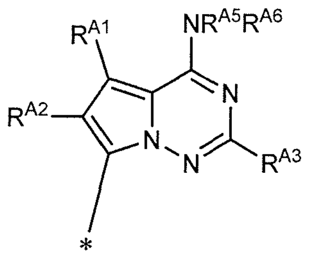
(a1)

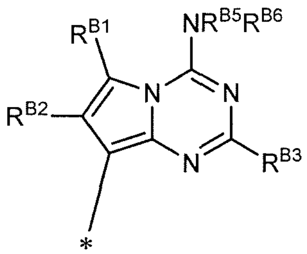
(a2)

wherein
$R^{A1}$ and $R^{B1}$ are each independently hydrogen, fluorine, hydroxy, $-B(OH)_2$, amino, an aromatic heterocyclyl optionally substituted with substituent group $\alpha$, cyanomethyl, C1-C3 alkyl substituted with hydroxy, or a group represented by the following formula: -C(=NOH)-H, wherein the substituent group $\alpha$: C1-C3 alkyl, and
$R^{A3}$ and $R^{B3}$ are each independently hydrogen or fluorine, or methyl,

or a pharmaceutically acceptable salt thereof.

7. The compound according to any one of claims 1, 2, 3 and 6, wherein $R^{A1}$ and $R^{B1}$ are fluorine, a 5- or 6-membered aromatic heterocyclyl, cyanomethyl, or a group represented by the following formula: -C(=NOH)-H, or a pharmaceutically acceptable salt thereof.

8. A compound selected from the group consisting of those represented by the following formulae:

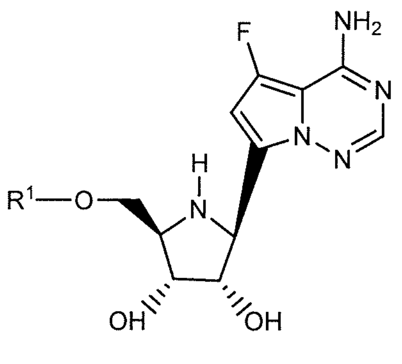
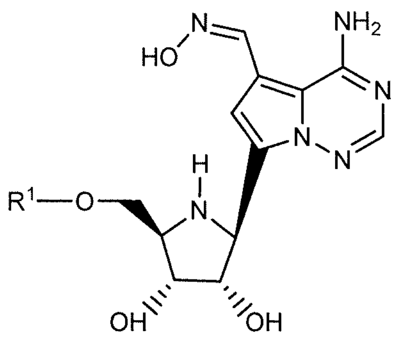
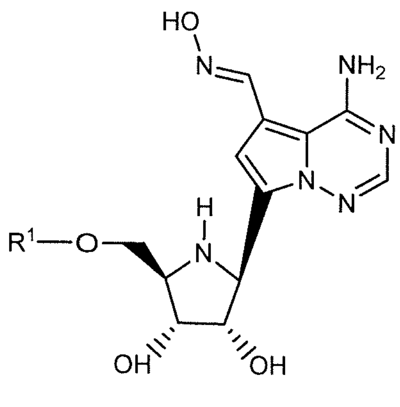

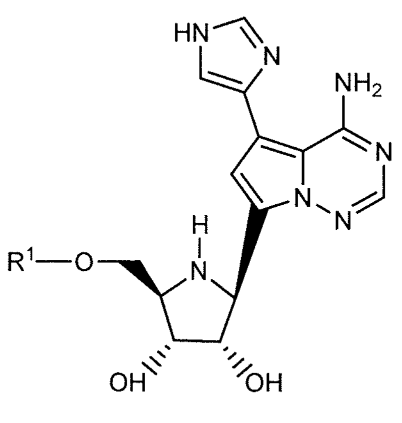
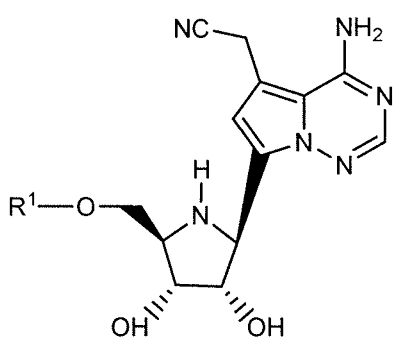
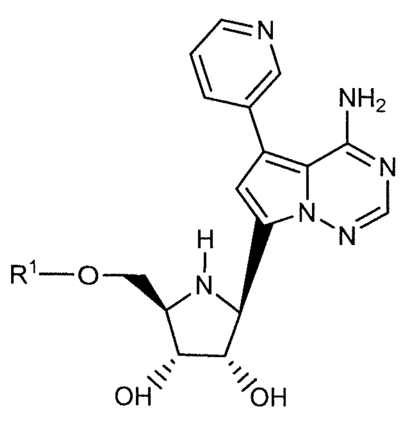

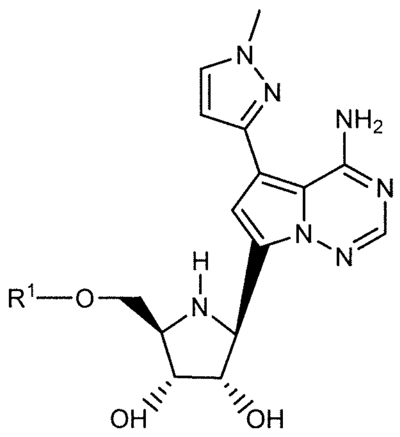

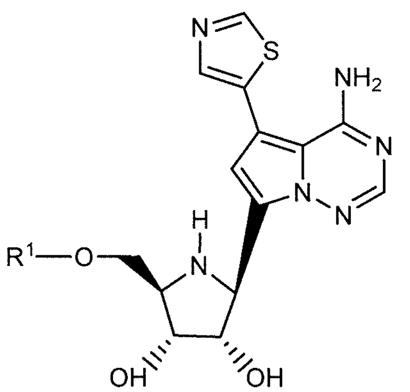

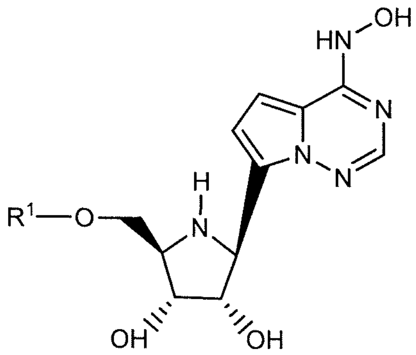

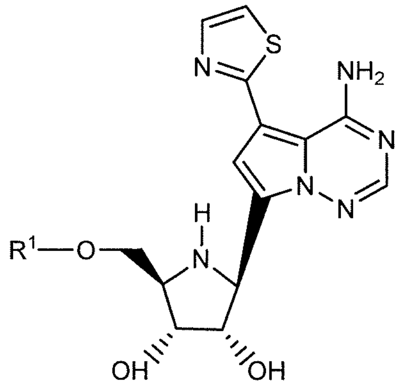

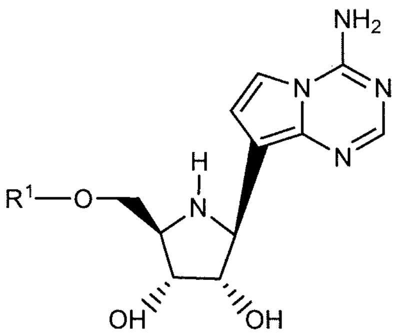

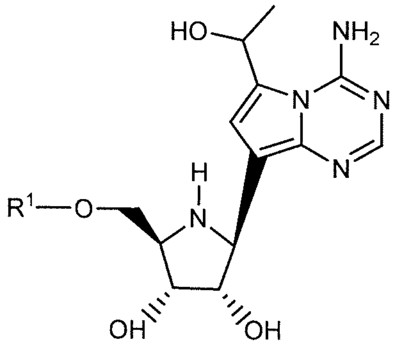

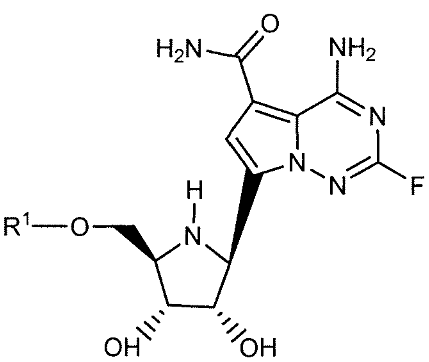

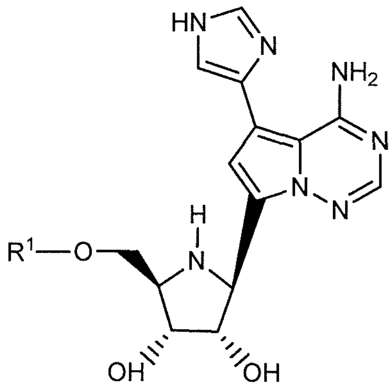

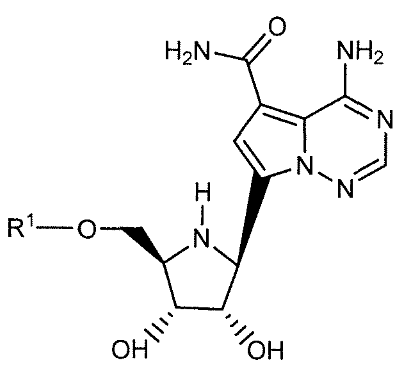

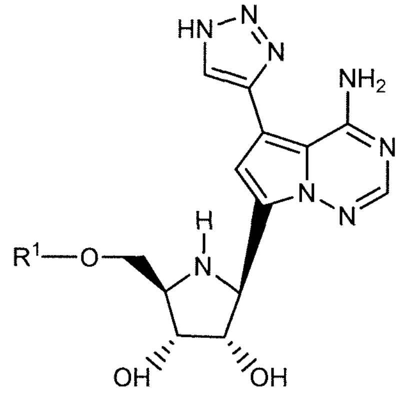

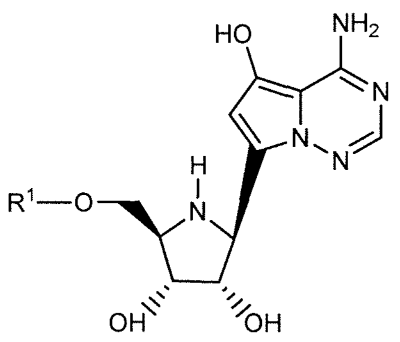

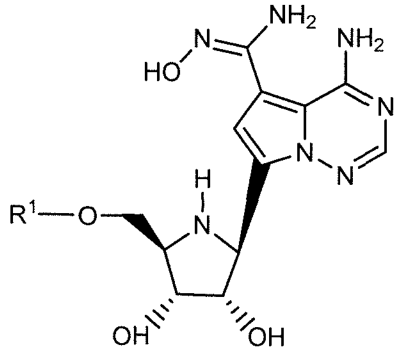

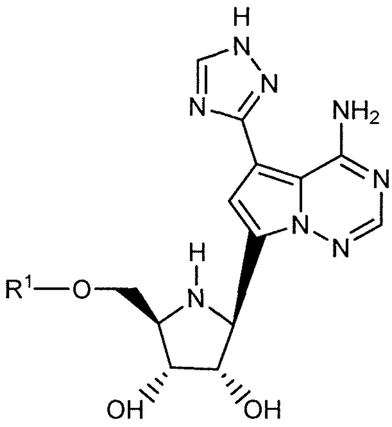
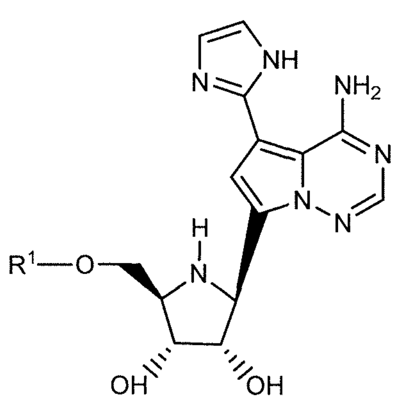
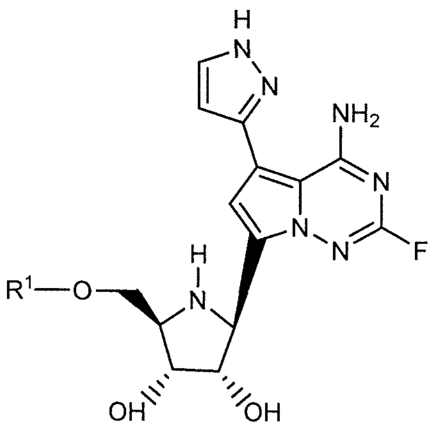

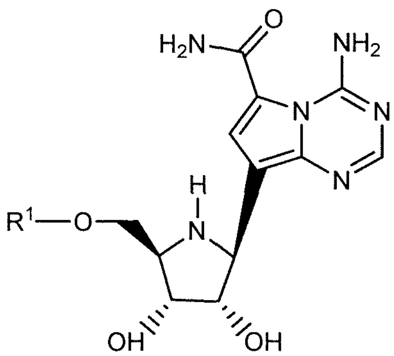
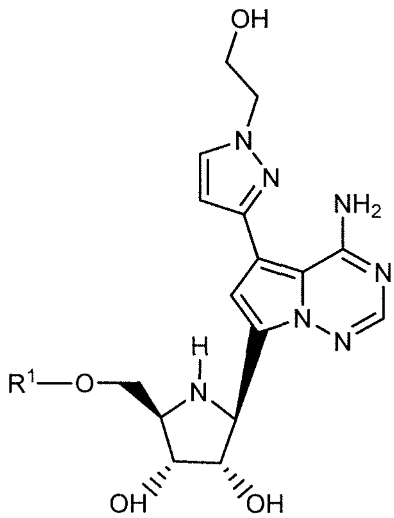
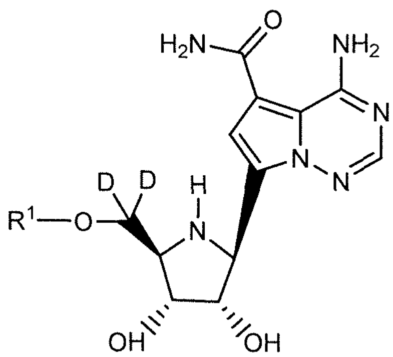

wherein $R^1$ is hydrogen, a group forming a prodrug, or a group selected from the group consisting of the following:

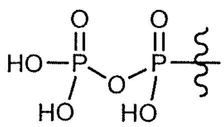 , 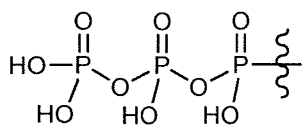

or a pharmaceutically acceptable salt thereof.

**9.** A compound represented by Formula (II):

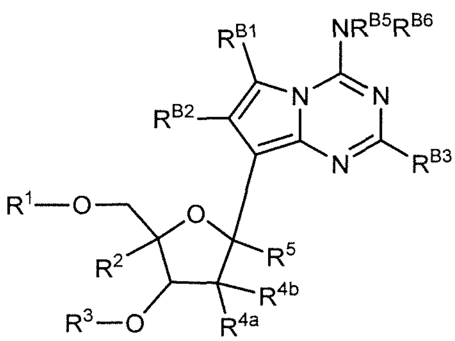 (II)

wherein

$R^{B1}$ is hydrogen, halogen, hydroxy, $-B(OH)_2$, amino optionally substituted with substituent group $\gamma$, an aromatic heterocyclyl optionally substituted with substituent group $\alpha$, alkyl optionally substituted with substituent group $\beta$, or a group represented by any one of the following formulae: $-C(=O)-N(R^{a1})(R^{a2})$, $-C(=NR^{a3})-NH_2$, and -C(=NOH)-H,
wherein $R^{a1}$ is hydrogen, $R^{a2}$ is hydrogen or alkyl optionally substituted with substituent group $\beta$, and $R^{a3}$ is hydrogen or hydroxy,
wherein

the substituent group $\alpha$: halogen and alkyl,
the substituent group 6: cyano and hydroxy, and
the substituent group $\gamma$: alkyl,

$R^{B2}$ is hydrogen,
$R^{B3}$ is hydrogen, halogen, amino, or alkyl,
$R^{B5}$ is hydrogen, alkyl, or a group forming a prodrug,
$R^{B6}$ is hydrogen, hydroxy, alkylcarbonyl, or a group forming a prodrug,
$R^1$ is hydrogen, a group forming prodrug, or a group selected from the group consisting of the following:

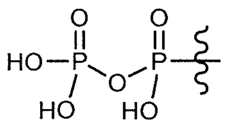 , 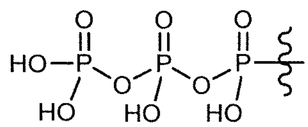

$R^2$ is hydrogen, halogen, C1-C3 alkyl, C2-C3 alkenyl, C2-C3 alkynyl, halo C1-C3 alkyl, halo C2-C3 alkenyl, or halo C2-C3 alkynyl,
$R^3$ is hydrogen or a group forming a prodrug,
$R^{4a}$ is hydrogen, hydroxy, or halogen,
$R^{4b}$ is hydrogen, halogen, C1-C3 alkyl, C1-C3 haloalkyl, C2-C3 alkenyl, or C2-C3 alkynyl,
$R^5$ is hydrogen, or cyano

or a pharmaceutically acceptable salt thereof.

**10.** A compound represented by Formula (II-a2-1):

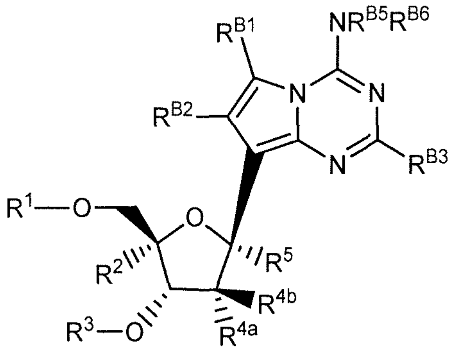

(II-a2-1)

wherein

$R^{B1}$ is hydrogen, halogen, hydroxy, $-B(OH)_2$, amino, an aromatic heterocyclyl optionally substituted with substituent group $\alpha$, alkyl optionally substituted with substituent group $\beta$, or a group represented by any one of the following formulae: $- C(=O)-N(R^{a1})(R^{a2})$, $-C(=NR^{a3})-NH_2$, and -C(=NOH)-H,
wherein $R^{a1}$ is hydrogen, $R^{a2}$ is hydrogen or alkyl optionally substituted with a substituent group $\beta$, and $R^{a3}$ is hydrogen or hydroxy,
wherein

the substituent group $\alpha$: halogen and alkyl, and
the substituent group 6: cyano and hydroxy,

$R^{B2}$ is hydrogen,
$R^{B3}$ is hydrogen, halogen, or alkyl,
$R^{B5}$ is hydrogen, or a group forming a prodrug,
$R^{B6}$ is hydrogen, hydroxy, alkylcarbonyl, or a group forming a prodrug,
$R^1$ is hydrogen, a group forming prodrug, or a group selected from the group consisting of the following:

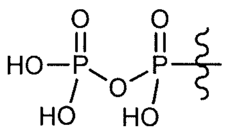
,
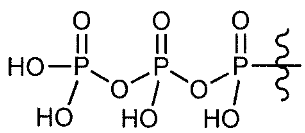

$R^2$ is hydrogen, or halogen,
$R^3$ is hydrogen or a group forming a prodrug,
$R^{4a}$ is hydroxy, or halogen,
$R^{4b}$ is hydrogen, or halogen,
$R^5$ is hydrogen, or cyano

or a pharmaceutically acceptable salt thereof.

**11.** A compound selected from the group consisting of those represented by the following formulae:

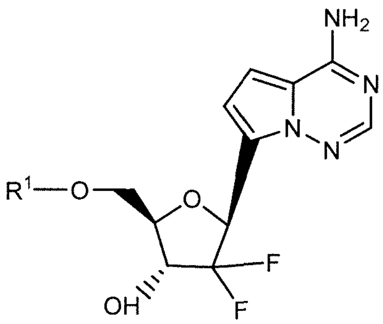
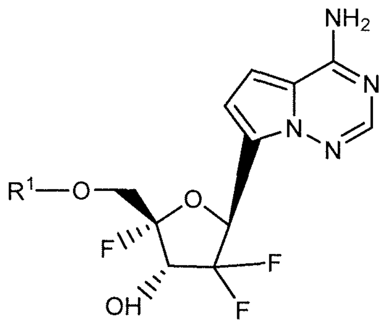
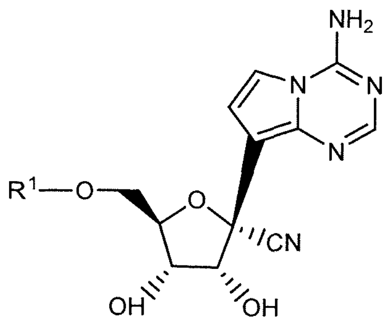
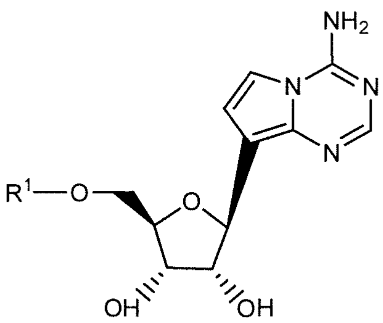
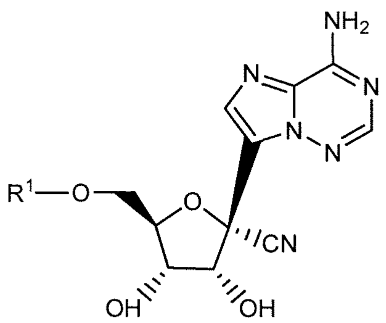
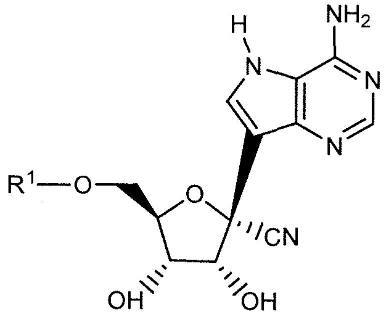
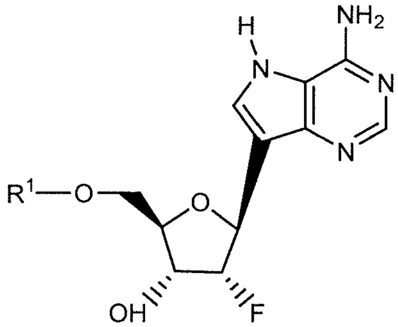

wherein $R^1$ is hydrogen, a group forming a prodrug, or a group selected from the group consisting of the following:

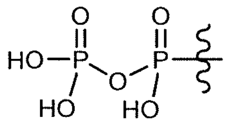
,
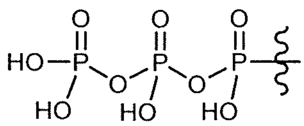

or a pharmaceutically acceptable salt thereof.

**12.** The compound according to any one of claims 1 to 11, wherein

the groups forming the prodrugs of $R^1$ and $R^3$ are groups selected from those represented by the following formulae:

a) $-C(=O)-P^{R0}$;
b) $-C(=O)-L-P^{R1}$;
c) $-C(=O)-L-O-P^{R0}$;
d) $-C(=O)-L-S-P^{R0}$;
e) $-C(=O)-L-N(-P^{R2})_2$;
f) $-C(=O)-C(P^{R3})_2-NH_2$;
g) $-C(=O)-O-P^{R0}$;
h) $-C(P^{R4})_2-O-C(=O)-P^{R5}$;
i) $-C(P^{R4})_2-O-C(=O)-L-P^{R6}$;
j) $-C(P^{R4})_2-O-C(=O)-O-P^{R5}$;
k) $-C(P^{R4})_2-O-C(=O)-O-L-P^{R6}$;
l) $-P(=O)(-OP^{R7})_2$;
m) $-P(=O)(-OP^{R7})-N(-P^{R8})_2$; and
n) $-P(=O)(-N(-P^{R8})_2)_2$,

the groups forming the prodrugs of $R^{A5}$, $R^{B5}$, $R^{C5}$, $R^{A6}$, $R^{B6}$, $R^{C6}$, and $R^6$ are groups selected from those represented by the following formulae:

a) $-C(=O)-P^{R0}$;
g) $-C(=O)-O-P^{R0}$;
o) $-C(=O)-O-L-P^{R1}$;
p) $-C(=O)-L-O-C(=O)-P^{R0}$; and
q) $-C(P^{R4})_2-P^{R1}$; or,

the groups forming prodrugs of $R^1$ and $R^3$ may be taken together to form a group represented by the following formula: $-P(=O)(-OP^{R7})$, and
the groups forming prodrugs of $R^1$ and $R^6$ may be taken together to form a group represented by the following formula: $-P(=O)(-OP^{R7})$, or $-P(=O)(-N(-P^{R8})_2)$,
wherein
L's are each independently a linear or branched alkylene,
$P^{R0}$s are each independently alkyl optionally substituted with substituent group A, a carbocyclyl optionally substituted with substituent group B, or a heterocyclyl optionally substituted with the substituent group B,
$P^{R1}$s are each independently a carbocyclyl optionally substituted with substituent group B, or a heterocyclyl optionally substituted with substituent group B,
$P^{R2}$s are each independently alkyl optionally substituted with substituent group A, a carbocyclyl optionally substituted with substituent group B, or a heterocyclyl optionally substituted with substituent group B, or
two $P^{R2}$s may be taken together with an adjacent atom to form a heterocycle optionally substituted with substituent group E,
$P^{R3}$s are each independently hydrogen or alkyl optionally substituted with substituent group C,
$P^{R4}$s are each independently hydrogen or alkyl,
$P^{R5}$s are each independently alkyl optionally substituted with substituent group A, a carbocyclyl optionally substituted with substituent group B, or a heterocyclyl optionally substituted with substituent group B,
$P^{R6}$s are each independently a carbocyclyl optionally substituted with substituent group B, or a heterocyclyl

optionally substituted with substituent group B,
$P^{R7}$s are each independently hydrogen, alkyl optionally substituted with substituent group D, phenyl optionally substituted with substituent group E, or naphthyl optionally substituted with substituent group E, or
two $P^{R7}$s may be taken together with adjacent atoms to form a heterocycle optionally substituted with substituent group E, and
$P^{R8}$s are each independently hydrogen or alkyl optionally substituted with a substituent group F,
wherein
the substituent group A: halogen;
the substituent group B: hydroxy, alkyl, and oxo;
the substituent group C: a carbocyclyl optionally substituted with substituent group G, a heterocyclyl optionally substituted with substituent group G, and alkylthio;
the substituent group D: a carbocyclyl optionally substituted with substituent group G, a heterocyclyl optionally substituted with substituent group G, a carbocyclylalkyloxy optionally substituted with substituent group G, alkyloxy, alkyloxycarbonyl, alkylcarbonyloxy, alkylthio, and alkylcarbonylthio;
the substituent group E: halogen, alkyl, and alkyloxy;
the substituent group F: a carbocyclyl, a heterocyclyl, halogen, and alkyloxycarbonyl; and
the substituent group G: halogen and cyano,

or a pharmaceutically acceptable salt thereof.

**13.** The compound according to any one of claims 1 to 11,
wherein

$R^3$, $R^6$, $R^{A5}$, $R^{B5}$, $R^{C5}$, $R^{A6}$, $R^{B6}$, and $R^{C6}$ are groups other than the group forming a prodrug, and
$R^1$ is hydrogen or a group selected from those represented by the following formulae:

a) $-C(=O)-P^{R0}$;
b) $-C(=O)-L-P^{R1}$;
f) $-C(=O)-C(P^{R3})_2-NH_2$;
g) $-C(=O)-O-P^{R0}$;
i) $-C(P^{R4})_2-O-C(=O)-L-P^{R6}$;
l) $-P(=O)(-OP^{R7})_2$; and
m) $-P(=O)(-OP^{R7})-N(-P^{R8})_2$, and

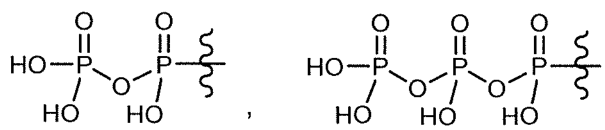

wherein

L's are each independently a linear or branched alkylene,
$P^{R0}$s are each independently alkyl, a carbocyclyl optionally substituted with substituent group B, or a heterocyclyl optionally substituted with substituent group B,
$P^{R1}$s are each independently a carbocyclyl optionally substituted with substituent group B, or a heterocyclyl optionally substituted with substituent group B,
$P^{R4}$s are each independently hydrogen or alkyl,
$P^{R6}$s are each independently a carbocyclyl optionally substituted with substituent group B, or a heterocyclyl optionally substituted with substituent group B,
$P^{R7}$s are each independently hydrogen, alkyl optionally substituted with substituent group D, or phenyl optionally substituted with substituent group E,
$P^{R8}$s are each independently hydrogen or alkyl optionally substituted with substituent group F,
wherein
the substituent group B: hydroxy, alkyl, and oxo;
the substituent group D: a carbocyclylalkyloxy optionally substituted with substituent group G, and alkyloxy;
the substituent group E: halogen, alkyl, and alkyloxy;
the substituent group F: alkyloxycarbonyl; and

the substituent group G: halogen and cyano,

or a pharmaceutically acceptable salt thereof.

**14.** The compound according to claim 13, wherein

$R^3$, $R^6$, $R^{A5,}$ $R^{B5}$, $R^{C5}$, $R^{A6}$, $R^{B6}$, and $R^{C6}$ are groups other than the group forming a prodrug, and $R^1$ is hydrogen or a group selected from those represented by the following formulae:

a) -C(=O)-$P^{R0}$;
b) -C(=O)-L-$P^{R1}$; and

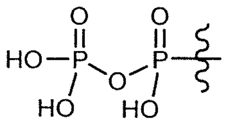 , 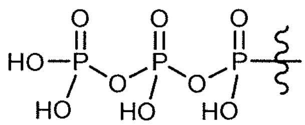

wherein

L's are each independently a linear or branched alkylene,
$P^{R0}$s are each independently alkyl, or a carbocyclyl optionally substituted with a substituent group B, and
$P^{R1}$s are each independently a carbocyclyl optionally substituted with substituent group B,
wherein
the substituent group B: hydroxy and alkyl,

or a pharmaceutically acceptable salt thereof.

**15.** The compound according to any one of claims 1 to 7, and 9 to 10, wherein

$R^1$, $R^3$, $R^6$, $R^{A6}$, $R^{B6}$, and $R^{C6}$ are groups other than the group forming a prodrug, and
$R^{A5,}$ $R^{B5}$, and $R^{C5}$ are each independently hydrogen or a group selected from those represented by the following formula:

a) -C(=O)-$P^{R0}$;

wherein $P^{R0}$s are each independently alkyl,

or a pharmaceutically acceptable salt thereof.

**16.** The compound according to any one of claims 1, 2, 4 to 7, and 9 to 10,

wherein $R^1$, $R^3$, $R^{A5,}$ $R^{B5}$, $R^{C5}$, $R^{A6}$, $R^{B6}$, and $R^{C6}$ are groups other than the group forming a prodrug, and $R^6$s are each independently hydrogen or a group selected from those represented by the following formula:

a)-C(=O)-$P^{R0}$,

wherein $P^{R0}$ is alkyl,

or a pharmaceutically acceptable salt thereof.

**17.** A pharmaceutical composition comprising the compound according to any one of claims 1 to 16 or a pharmaceutically acceptable salt thereof.

**18.** The pharmaceutical composition according to claim 17, wherein the pharmaceutical composition is an antiviral agent.

**19.** An antiviral agent comprising the compound according to any one of claims 1 to 16 or a pharmaceutically acceptable

salt thereof.

**20.** A method for treating and/or preventing a viral infectious disease, comprising administering the compound according to any of claims 1 to 16 or a pharmaceutically acceptable salt thereof.

**21.** The compound according to any one of claims 1 to 16, or a pharmaceutically acceptable salt thereof, for use in treating and/or preventing a viral infectious disease.

## INTERNATIONAL SEARCH REPORT

International application No.
**PCT/JP2022/031308**

**A. CLASSIFICATION OF SUBJECT MATTER**

***C07D 487/04***(2006.01)i; ***A61K 31/53***(2006.01)i; ***A61P 31/16***(2006.01)i
FI: C07D487/04 140; A61K31/53; A61P31/16

According to International Patent Classification (IPC) or to both national classification and IPC

**B. FIELDS SEARCHED**

Minimum documentation searched (classification system followed by classification symbols)

C07D487/04; A61K31/53; A61P31/16

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | JP 2016-504284 A (BIOCRYST PHARMACEUTICALS, INC.) 12 February 2016 (2016-02-12) claims, paragraphs [0002]-[0004], [0117]-[0128], [0147]-[0162], [0183], example 5 | 1-8, 12-21 |
| A | JP 2019-069986 A (GILEAD SCIENCES, INC.) 09 May 2019 (2019-05-09) paragraph [0014] | 1-8, 12-21 |
| A | JP 2020-534361 A (JANSSSEN BIOPHARMA, INC) 26 November 2020 (2020-11-26) claims | 1-8, 12-21 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **09 November 2022** | **22 November 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2022/031308**

**Box No. III Observations where unity of invention is lacking (Continuation of item 3 of first sheet)**

This International Searching Authority found multiple inventions in this international application, as follows:

The inventions in claims 1-21 share the common technical feature of a chemical structure in which a base (in particular, a fused triazine base) is C-substituted at ribose/iminoribose, but such a chemical structure is known at the time of filing this application as disclosed in JP 2020-534361 A, JP 2015-509983 A, JP 2013-535453 A, etc., the common technical feature is not a special technical feature.

Also, there are no other same or corresponding special technical features between the inventions in claims 1-21.

In addition, the inventions in claims 1-21 are classified into inventions 1-3 having different special technical features.

Invention 1: the invention in claims 1-8, and the invention of parts referring to claims 1-8 in claims 12-21 relate to a compound characterized by a structure in which A set forth in claim 1 is bonded to iminoribose.

Invention 2: the invention in claims 9-10, the invention of part relating to two formulae having a 1,3,5-triazine-containing fused ring system in the invention in claim 11, and the invention of parts referring to claims 9-10 or said part of claim 11 in claims 12-21 relate to a compound characterized by a ribose structure in which a base formed by 1,3,5-triazine being fused to pyrrole is C-substituted.

Invention 3: the invention of part relating to five formulae other than the two formulae having a 1,3,5-triazine-containing fused ring system of Invention 2 in the invention in claim 11, and the invention of part referring to said part of claim 11 in the invention in claims 12-14 and 17-21 relate to a compound having a combination of ribose/iminoribose structure and a base different from invention 1 or invention 2.

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **claims 1-8 (all) and 12-21 (parts referring to claims 1-8)**

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

**INTERNATIONAL SEARCH REPORT**
**Information on patent family members**

International application No.
**PCT/JP2022/031308**

| Patent document cited in search report | Publication date (day/month/year) | Patent family member(s) | Publication date (day/month/year) |
|---|---|---|---|
| JP 2016-504284 A | 12 February 2016 | US 2015/0291596 A1<br>claims, paragraphs [0002]-[0003], [0248]-[0260], [0275]-[0290], [0305], example 5<br>WO 2014/078778 A2<br>EP 2919785 A2 | |
| JP 2019-069986 A | 09 May 2019 | US 2013/0243725 A1<br>claims<br>WO 2013/138236 A1<br>EP 2834258 A1<br>CN 104185638 A | |
| JP 2020-534361 A | 26 November 2020 | US 2020/0277321 A1<br>claims<br>WO 2019/053696 A1<br>EP 3684782 A1<br>KR 10-2020-0098483 A<br>CN 111542531 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 1999019338 A
- US 6066722 A
- WO 2002018371 A
- WO 2003080620 A
- WO 2006002231 A
- WO 2007069924 A
- WO 2012074912 A
- WO 2013158746 A
- WO 2014078778 A
- WO 2014186465 A
- WO 2018199048 A
- WO 2018230479 A
- WO 2019140365 A
- WO 2004096286 A
- WO 2016069825 A
- WO 2016069826 A
- WO 2016069827 A
- CN 112010916 A
- CN 112062800 A
- WO 2008141079 A
- WO 2008089105 A
- WO 2021040356 A
- WO 2014035140 A
- WO 2010002877 A
- WO 2012037038 A
- WO 2016069975 A
- CN 112778310 A
- WO 2015069939 A
- WO 2015148746 A
- WO 2014093924 A
- WO 2003093290 A
- WO 2005123087 A
- WO 2006050161 A
- EP 71227 A


### Non-patent literature cited in the description


- *American Society for Microbiology Antimicrobial Agents and Chemotherapy,* April 2002, vol. 46 (4), 977-981
- *Antiviral Chemistry & Chemotherapy,* vol. 14, 235-241
- *American Society for Microbiology Antimicrobial Agents and Chemotherapy,* August 2018, vol. 62 (8
- **TOOTS et al.** *Sci. Transl. Med.,* 23 October 2019, vol. 11, eaax5866
- *n engl j med,* 05 November 2020, vol. 383 (19), 1813
- *n engl j med,* 04 March 2021, vol. 384 (9), 795
- **Q. CAI et al.** *Engineering,* 2020, vol. 6, 1192-1198
- *NATURE COMMUNICATIONS,* 2021, vol. 12 (1735), 1-13
- **SHEAHAN et al.** *Sci. Transl. Med.,* 2020, vol. 12, 1-15
- *Nature Microbiology,* January 2021, vol. 6, 11-18
- *NATURE COMMUNICATIONS,* 2021, vol. 12 (2295), 1-8
- *Antimicrobial Agents and Chemotherapy,* April 2021, vol. 65 (4), e02479-20
- *Prog. Med.,* 1985, vol. 5, 2157-2161
- Isotopes in the Physical and Biomedical Sciences. Labeled Compounds (Part A). 1987, vol. 1